# EUROPEAN PATENT SPECIFICATION

(11) **EP 2 804 850 B1**
(45) Date of publication and mention of the grant of the patent: **29.08.2018**
(21) Application number: 13738187.7
(22) Date of filing: 21.01.2013
(51) Int. Cl.: C07C 237/22, C07C 271/22, C07C 271/44, A61K 31/16, A61K 31/357, A61K 31/381, A61K 31/40, A61K 31/415, A61K 31/4164, A61K 31/4192, A61K 31/426, A61K 31/435, A61K 31/495, A61P 35/00, C07K 5/062

(54) **THERAPEUTICALLY ACTIVE COMPOUNDS AND THEIR METHODS OF USE**
THERAPEUTISCHE WIRKSTOFFVERBINDUNGEN UND VERFAHREN ZU IHRER VERWENDUNG
COMPOSÉS THÉRAPEUTIQUEMENT ACTIFS ET LEURS MÉTHODES D'UTILISATION

(30) Priority: 19.01.2012 WO PCT/CN2012/070607
(43) Date of publication of application: 26.11.2014
(73) Proprietor: Agios Pharmaceuticals, Inc., Cambridge, MA 02139 (US)
(72) Inventor: POPOVICI-MULLER, Janeta, Windham, New Hampshire 03087 (US); SAUNDERS, Jeffrey, O., Lincoln, MA 01773 (US); SALITURO, Francesco, G., Marlborough, MA 01752 (US); CAI, Zhenwei, Skillman, NJ 08558 (US); YAN, Shunqi, Irvine, CA 92603 (US); ZHOU, Ding, Xuhui District, Shanghai 200231 (CN)
(74) Representative: Bösl, Raphael Konrad
(86) International application number: PCT/CN2013/070755
(87) International publication number: WO 2013/107405

(56) References cited:
- WO-A1-2012/009678
- WO-A1-2012/009678
- WO-A1-2013/107291
- KR-A- 20050 036 293
- MAISON W ET AL: "Multicomponent synthesis of novel amino acid-nucleobase chimeras: a versatile approach to PNA-monomers", BIOORGANIC & MEDICINAL CHEMISTRY, PERGAMON, GB, vol. 8, no. 6, 1 June 2000 (2000-06-01), pages 1343-1360, XP027411646, ISSN: 0968-0896 [retrieved on 2000-06-01]
- DATABASE REGISTRY [Online] CHEMICAL ABSTRACTS SERVICE, COLUMBUS, OHIO, US; 3 July 2008 (2008-07-03), XP3031091, Database accession no. 1032450-21-7
- DATABASE REGISTRY CAS 05 August 2008 STN, XP003031090 Database accession no. 1038821-72-5
- DATABASE REGISTRY CAS 03 July 2008 STN, XP003031091 Database accession no. 1032450-21-7
- POPOVICI-MULLER, JANETA ET AL.: 'Discovery of the First Potent Inhibitors of Mutant IDH1 That Lower Tumor 2-HG in Vivo' ACS MEDICINAL CHEMISTRY LETTERS vol. 3, no. 10, 17 September 2012, pages 850 - 855, XP055159509

## Description

### CLAIM OF PRIORITY

This application claims priority from International Application No. PCT/CN2012/070607, filed January 19, 2012.

### BACKGROUND OF INVENTION

Isocitrate dehydrogenases (IDHs) catalyze the oxidative decarboxylation of isocitrate to 2-oxoglutarate (*i.e.,* α-ketoglutarate). These enzymes belong to two distinct subclasses, one of which utilizes NAD(+) as the electron acceptor and the other NADP(+). Five isocitrate dehydrogenases have been reported: three NAD(+)-dependent isocitrate dehydrogenases, which localize to the mitochondrial matrix, and two NADP(+)-dependent isocitrate dehydrogenases, one of which is mitochondrial and the other predominantly cytosolic. Each NADP(+)-dependent isozyme is a homodimer.

IDH1 (isocitrate dehydrogenase 1 (NADP+), cytosolic) is also known as IDH; IDP; IDCD; IDPC or PICD. The protein encoded by this gene is the NADP(+)-dependent isocitrate dehydrogenase found in the cytoplasm and peroxisomes. It contains the PTS-1 peroxisomal targeting signal sequence. The presence of this enzyme in peroxisomes suggests roles in the regeneration of NADPH for intraperoxisomal reductions, such as the conversion of 2, 4-dienoyl-CoAs to 3-enoyl-CoAs, as well as in peroxisomal reactions that consume 2-oxoglutarate, namely the alpha-hydroxylation of phytanic acid. The cytoplasmic enzyme serves a significant role in cytoplasmic NADPH production.

The human IDH1 gene encodes a protein of 414 amino acids. The nucleotide and amino acid sequences for human IDH1 can be found as GenBank entries NM_005896.2 and NP_005887.2 respectively. The nucleotide and amino acid sequences for IDH1 are also described in, *e.g.,* Nekrutenko et al., Mol. Biol. Evol. 15:1674-1684(1998); Geisbrecht et al., J. Biol. Chem. 274:30527-30533(1999); Wiemann et al., Genome Res. 11:422-435(2001); The MGC Project Team, Genome Res. 14:2121-2127(2004); Lubec *et al.,* Submitted (DEC-2008) to UniProtKB; Kullmann *et al.,* Submitted (JUN-1996) to the EMBL/GenBank/DDBJ databases; and Sjoeblom et al., Science 314:268-274(2006).

Non-mutant, *e.g*., wild type, IDH1 catalyzes the oxidative decarboxylation of isocitrate to α-ketoglutarate thereby reducing NAD⁺ (NADP⁺) to NADP (NADPH), *e.g.,* in the forward reaction:

Isocitrate + NAD⁺ (NADP⁺) → α-KG + CO₂ + NADH (NADPH) + H⁺.

It has been discovered that mutations of IDH1 present in certain cancer cells result in a new ability of the enzyme to catalyze the NADPH-dependent reduction of α-ketoglutarate to *R*(-)-2-hydroxyglutarate (2HG). The production of 2HG is believed to contribute to the formation and progression of cancer (Dang, L et al, Nature 2009, 462:739-44).

The inhibition of mutant IDH1 and its neoactivity is therefore a potential therapeutic treatment for cancer. Accordingly, there is an ongoing need for inhibitors of IDH1 mutants having alpha hydroxyl neoactivity.

### SUMMARY OF INVENTION

The present invention is directed to a compound of formula I or a pharmaceutically acceptable salt or hydrate thereof, wherein: 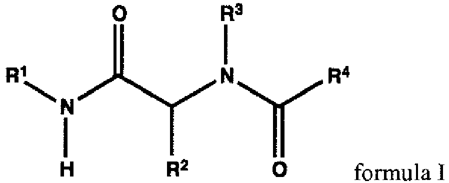 R¹ is: 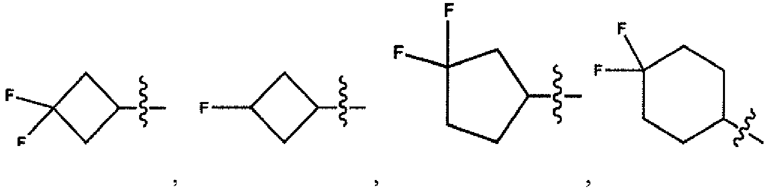 or 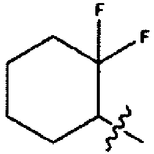 ;each R² and R³ is independently selected from optionally substituted aryl or optionally substituted heteroaryl;
R⁴ is a fully saturated heterocyclyl, -CH(R⁵)N(R⁵)-aryl, -CH(R⁵)N(R⁵)-heterocyclyl, - CH(R⁵)N(R⁵)-carbocyclyl, -CH₂-heterocyclyl, 1H-indol-2-yl, indolin-2-yl, 1,2,3,4-tetrahydroquinolin-2-yl, imidazo[1,2-*a*]pyridine-5-yl, 5,6,7,8-tetrahydroimidazo[1,2-*a*]pyridine-5-yl, -(CR⁵R⁶)₁₋₄N(R⁵)C(O)O(C₁-C₆ alkyl), or -(CR⁵R⁶)₁₋₄N(R⁵)SO₂(C₁-C₆ alkyl), wherein each saturated heterocyclyl, heteroaryl, aryl, heterocyclyl, or carbocyclyl is independently optionally substituted;
each R⁵ is independently selected from hydrogen and methyl; and
each R⁶ is independently selected from hydrogen, methyl, CH₂OH, CH(CH₃)OH, CH₂NH₂, or CH(CH₃)NH₂; wherein:
"heterocyclyl" refers to a monocyclic, bicyclic or tricyclic, ring structure that is not fully aromatic and includes one to four heteroatoms independently selected from N, O, or S in one or more of the rings; and
"carbocyclyl" refers to a monocyclic, bicyclic or tricyclic, hydrocarbon ring system that is not fully aromatic, wherein any ring atom capable of substitution can be substituted by one or more substituents.

Furthermore, the present invention is directed to a compound of formula I or a pharmaceutically acceptable salt or hydrate thereof, wherein: 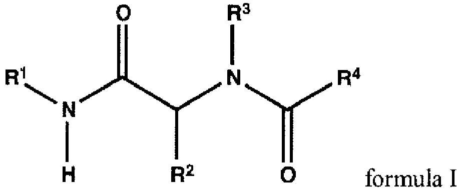 R¹ is: 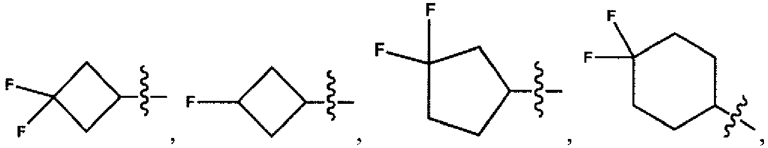 or 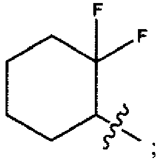 each R² and R³ is independently selected from optionally substituted aryl or optionally substituted heteroaryl; R⁴ is saturated heterocyclyl, -CH(R⁵)N(R⁵)-aryl, -CH(R⁵)N(R⁵)-heterocyclyl, -CH(R⁵)N(R⁵)-carbocyclyl, -CH₂-heterocyclyl, 1H-indol-2-yl, indolin-2-yl, 1,2,3,4-tetrahydroquinolin-2-yl, imidazo[1,2-*a*]pyridine-5-yl, 5,6,7,8-tetrahydroimidazo[1,2-*a*]pyridine-5-yl, -(CR⁵R⁶)₁₋₄N(R⁵)C(O)O(C₁-C₆ alkyl), or -(CR⁵R⁶)₁₋₄N(R⁵)SO₂(C₁-C₆ alkyl), wherein each saturated heterocyclyl, heteroaryl, aryl, heterocyclyl, or carbocyclyl is independently optionally substituted; or
R⁴ is -CH₂-heteroaryl wherein heteroaryl is imidazolyl substituted with one or two R⁷ groups selected from the group consisting of -CN, -CH₂OH, Cl, and F; or heteroaryl is triazolyl, tetrazolyl or pyridinyl optionally substituted with one or two R⁷ groups; or
R⁴ is -CH(R⁵)N(R⁵)-heteroaryl wherein heteroaryl is pyrimidinyl optionally substituted with one or two R⁷ groups selected from the group consisting of Cl and F;
each R⁵ is independently selected from hydrogen and methyl; and
each R⁶ is independently selected from hydrogen, methyl, CH₂OH, CH(CH₃)OH, CH₂NH₂, or CH(CH₃)NH₂.

The present invention is also directed to a pharmaceutical composition comprising a compound of the present invention and a pharmaceutically acceptable carrier. The present invention is also directed to a compound or pharmaceutical composition according to the present invention for use in a method of treating a cancer characterized by the presence of an IDH1 mutation, wherein the IDH1 mutation results in a new ability of the enzyme to catalyze the NADPH-dependend reduction of α-ketoglutarate to R(-)-2-hydroxyglutarate in a patient.

Described herein are compounds for use in methods of treating a cancer characterized by the presence of a mutant allele of IDH1 or IDH2. The methods comprise the step of administering to a subject in need thereof a compound of formula I, or a pharmaceutically acceptable salt or hydrate thereof, wherein: 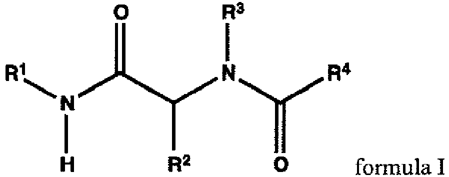
R¹ is optionally substituted C₄-C₆ carbocyclyl;
each R² and R³ is independently selected from optionally substituted aryl or optionally substituted heteroaryl;
R⁴ is saturated heterocyclyl, -CH(R⁵)N(R⁵)-heteroaryl, -CH(R⁵)N(R⁵)-aryl, -CH(R⁵)N(R⁵)-heterocyclyl, -CH(R⁵)N(R⁵)-carbocyclyl, hctcroaralkyl, -CH₂-heterocyclyl, 1H-indol-2-yl, indolin-2-yl, 1,2,3,4-tetrahydroquinolin-2-yl, imidazo[1,2-*a*]pyridine-5-yl, 5,6,7,8-tetrahydroimidazo[1,2-*α*]pyridine-5-yl, -(CR⁵R⁶)₁₋₄N(R⁵)C(O)O(C₁-C₆ alkyl), or -(CR⁵R⁶)₁₋₄N(R⁵)SO₂(C₁-C₆ alkyl), wherein each saturated heterocyclyl, heteroaryl, aryl, heterocyclyl, or carbocyclyl is independently optionally substituted;
each R⁵ is independently selected from hydrogen and methyl; and
each R⁶ is independently selected from hydrogen, methyl, CH₂OH, CH(CH₃)OH, CH₂NH₂, or CH(CH₃)NH₂; and provided that:
   (i) R⁴ is other than thien-2-ylmethyl, 1H-benizimidazol-1-ylmethyl, 1H-indol-3-ylmethyl, or 1H-benzotriazol-1-ylmethyl; and (ii) the compound is not N-[2-[[2-(cyclohexylamino)-1-(3-hydroxyphenyl)-2-oxoethyl]phenylamino]-2-oxoethyl]-carbamic acid 1,1-dimethylethyl ester or N-[2-[(2-benzoylphenyl)[2-(cyclohexylamino)-1-(3-hydroxyphenyl)-2-oxoethyl]amino]-2-oxoethyl]-carbamic acid 1,1-dimethylethyl ester.

The compound of formula I inhibits mutant IDH1/2, particularly mutant IDH1 having alpha hydroxyl neoactivity. Also described herein are pharmaceutical compositions comprising a compound of formula I.

### DETAILED DESCRIPTION OF THE INVENTION

### Definitions:

The term "halo" or "halogen" refers to any radical of fluorine, chlorine, bromine or iodine.

The term "alkyl" refers to a hydrocarbon chain that may be a straight chain or branched chain, containing the indicated number of carbon atoms. For example, C₁-C₁₂ alkyl indicates that the group may have from 1 to 12 (inclusive) carbon atoms in it. The term "haloalkyl" refers to an alkyl in which one or more hydrogen atoms are replaced by halo, and includes alkyl moieties in which all hydrogens have been replaced by halo (e.g., perfluoroalkyl). The terms "arylalkyl" or "aralkyl" refer to an alkyl moiety in which an alkyl hydrogen atom is replaced by an aryl group. Arylalkyl or aralkyl includes groups in which more than one hydrogen atom has been replaced by an aryl group. Examples of "arylalkyl" or "aralkyl" include benzyl, 2-phenylethyl, 3-phenylpropyl, 9-fluorenyl, benzhydryl, and trityl groups. The terms "heteroarylalkyl" or "heteroaralkyl" refer to an alkyl moiety in which an alkyl hydrogen atom is replaced by a heteroaryl group. Heteroarylalkyl or heteroaralkyl includes groups in which more than one hydrogen atom has been replaced by a heteroaryl group.

The term "alkylene" refers to a divalent alkyl, e.g., -CH₂-, -CH₂CH₂-, and -CH₂CH₂CH₂-.

The term "alkenyl" refers to a straight or branched hydrocarbon chain containing 2-12 carbon atoms and having one or more double bonds. Examples of alkenyl groups include, but are not limited to, allyl, propenyl, 2-butenyl, 3-hexenyl and 3-octenyl groups. One of the double bond carbons may optionally be the point of attachment of the alkenyl substituent. The term "alkynyl" refers to a straight or branched hydrocarbon chain containing 2-12 carbon atoms and characterized in having one or more triple bonds. Examples of alkynyl groups include, but are not limited to, ethynyl, propargyl, and 3-hexynyl. One of the triple bond carbons may optionally be the point of attachment of the alkynyl substituent.

The term "alkoxy" refers to an -O-alkyl radical. The term "haloalkoxy" refers to an alkoxy in which one or more hydrogen atoms are replaced by halo, and includes alkoxy moieties in which all hydrogens have been replaced by halo (e.g., perfluoroalkoxy).

The term "carbocyclyl" refers to a monocyclic, bicyclic or tricyclic, hydrocarbon ring system that is not fully aromatic, wherein any ring atom capable of substitution can be substituted by one or more substituents. A carbocyclyl can be fully or partially saturated. A bicyclic or tricylic carbocyclyl may contain one (in the case of a bicycle) or up to two (in the case of a tricycle) aromatic rings, as long as at least one ring in the carbocyclyl is non-aromatic. Unless otherwise specified, any ring atom capable of substitution in a carbocyclyl can be substituted by one or more substituents.

The term "aryl" refers to a fully aromatic monocyclic, bicyclic, or tricyclic hydrocarbon ring system. Examples of aryl moieties are phenyl, naphthyl, and anthracenyl. Unless otherwise specified, any ring atom in an aryl can be substituted by one or more substituents.

The term "cycloalkyl" as employed herein refers to a saturated cyclic, bicyclic, tricyclic, or polycyclic hydrocarbon group. Unless otherwise specified, any ring atom can be substituted by one or more substituents. The cycloalkyl groups can contain fused rings. Fused rings are rings that share a common carbon atom. Examples of cycloalkyl moieties include, but are not limited to, cyclopropyl, cyclohexyl, methylcyclohexyl, adamantyl, and norbornyl. Unless otherwise specified, any ring atom can be substituted by one or more substituents.

The term "heterocyclyl" refers to a monocyclic, bicyclic or tricyclic, ring structure that is not fully aromatic and includes one to four heteroatoms independently selected from N, O, or S in one or more of the rings. A heterocyclyl can be fully or partially saturated. A bicyclic or tricylic heterocyclyl may contain one (in the case of a bicycle) or up to two (in the case of a tricycle) aromatic rings, as long as at least one ring in the heterocyclyl is non-aromatic. Unless otherwise specified, any ring atom capable of substitution in a heterocyclyl can be substituted by one or more substituents. Heterocyclyl groups include, for example, thiophene, thianthrene, furan, pyran, isobenzofuran, chromene, xanthene, phenoxathiin, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like.

The term "heteroaryl" refers to a monocyclic, bicyclic, or tricyclic ring system having 1-3 heteroatoms if monocyclic, 1-6 heteroatoms if bicyclic, or 1-9 heteroatoms if tricyclic, said heteroatoms independently selected from O, N, or S, wherein each ring in a heteroaryl is fully aromatic. Unless otherwise specified, any ring atom capable of substitution in a heteroaryl can be substituted by one or more substituents. The terms "hetaralkyl" and "heteroaralkyl", as used herein, refers to an alkyl group substituted with a heteroaryl group. The ring heteroatoms of the compounds provided herein include N-O, S(O), and S(O)₂.

The term "substituted" refers to the replacement of a hydrogen atom with another moiety. Typical substituents include alkyl (e.g., C1, C2, C3, C4, C5, C6, C7, C8, C9, C10, C11, C12 straight or branched chain alkyl), cycloalkyl, haloalkyl (e.g., perfluoroalkyl such as CF₃), aryl, heteroaryl, aralkyl, heteroaralkyl, heterocyclyl, alkenyl, alkynyl, cycloalkenyl, heterocycloalkenyl, alkoxy, haloalkoxy (e.g., perfluoroalkoxy such as OCF₃), halo, hydroxy, carboxy, carboxylate, cyano, nitro, amino, alkyl amino, SO₃H, sulfate, phosphate, methylenedioxy (-O-CH₂-O- wherein oxygens are attached to vicinal atoms), ethylenedioxy, oxo (not a substituent on heteroaryl), thioxo (e.g., C=S) (not a substituent on heteroaryl), imino (alkyl, aryl, aralkyl), S(O)ₙalkyl (where n is 0-2), S(O)ₙ aryl (where n is 0-2), S(O)ₙ heteroaryl (where n is 0-2), S(O)ₙ heterocyclyl (where n is 0-2), amine (mono-, di-, alkyl, cycloalkyl, aralkyl, heteroaralkyl, aryl, heteroaryl, and combinations thereof), ester (alkyl, aralkyl, heteroaralkyl, aryl, heteroaryl), amide (mono-, di-, alkyl, aralkyl, heteroaralkyl, aryl, heteroaryl, and combinations thereof), sulfonamide (mono-, di-, alkyl, aralkyl, heteroaralkyl, and combinations thereof). In one aspect, the substituents on a group are independently any one single, or any subset of the aforementioned substituents. In another aspect, a substituent may itself be substituted with any one of the above substituents.

As used herein, the term "elevated levels of 2HG" means 10%, 20% 30%, 50%, 75%, 100%, 200%, 500% or more 2HG then is present in a subject that does not carry a mutant IDH1 or IDH2 allele. The term "elevated levels of 2HG" may refer to the amount of 2HG within a cell, within a tumor, within an organ comprising a tumor, or within a bodily fluid.

The term "bodily fluid" includes one or more of amniotic fluid surrounding a fetus, aqueous humour, blood (*e.g.,* blood plasma), serum, Cerebrospinal fluid, cerumen, chyme, Cowper's fluid, female ejaculate, interstitial fluid, lymph, breast milk, mucus (*e.g.,* nasal drainage or phlegm), pleural fluid, pus, saliva, sebum, semen, serum, sweat, tears, urine, vaginal secretion, or vomit.

As used herein, the terms "inhibit" or "prevent" include both complete and partial inhibition and prevention. An inhibitor may completely or partially inhibit.

The term "treat" means decrease, suppress, attenuate, diminish, arrest, or stabilize the development or progression of a cancer (e.g., a cancer delineated herein), lessen the severity of the cancer or improve the symptoms associated with the cancer.

As used herein, an amount of a compound effective to treat a disorder, or a "therapeutically effective amount" refers to an amount of the compound which is effective, upon single or multiple dose administration to a subject, in treating a cell, or in curing, alleviating, relieving or improving a subject with a disorder beyond that expected in the absence of such treatment.

As used herein, the term "subject" is intended to include human and non-human animals. Exemplary human subjects include a human patient having a disorder, e.g., a disorder described herein or a normal subject. The term "non-human animals" of the invention includes all vertebrates, e.g., non-mammals (such as chickens, amphibians, reptiles) and mammals, such as non-human primates, domesticated and/or agriculturally useful animals, e.g., sheep, dog, cat, cow, pig, etc.

### Compounds

Provided is a compound having formula I or a pharmaceutically acceptable salt or hydrate thereof, wherein: 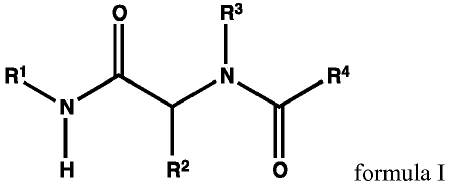
R¹ is optionally substituted C₄-C₆ carbocyclyl;
each R² and R³ is independently selected from optionally substituted aryl or optionally substituted heteroaryl;
R⁴ is saturated heterocyclyl, -CH(R⁵)N(R⁵)-heteroaryl, -CH(R⁵)N(R⁵)-aryl, -CH(R⁵)N(R⁵)-heterocyclyl, -CH(R⁵)N(R⁵)-carbocyclyl, heteroaralkyl, -CH₂-heterocyclyl, 1H-indol-2-yl, indolin-2-yl, 1,2,3,4-tetrahydroquinolin-2-yl, imidazo[1,2-*a*]pyridine-5-yl, 5,6,7,8-tetrahydroimidazo[1,2-*a*]pyridine-5-yl, -(CR⁵R⁶)₁₋₄N(R⁵)C(O)O(C₁-C₆ alkyl), or -(CR⁵R⁶)₁₋₄N(R⁵)SO₂(C₁-C₆ alkyl), wherein each saturated heterocyclyl, heteroaryl, aryl, heterocyclyl, or carbocyclyl is independently optionally substituted;
each R⁵ is independently selected from hydrogen and methyl; and
each R⁶ is independently selected from hydrogen, methyl, CH₂OH, CH(CH₃)OH, CH₂NH₂, or CH(CH₃)NH₂; and provided that:
   (i) R⁴ is other than thien-2-ylmethyl, 1H-benizimidazol-1-ylmethyl, 1H-indol-3-ylmethyl, or 1H-benzotriazol-1-ylmethyl; and (ii) the compound is not N-[2-[[2-(cyclohexylamino)-1-(3-hydroxyphenyl)-2-oxoethyl]phenylamino]-2-oxoethyl]-carbamic acid 1,1-dimethylethyl ester or N-[2-[(2-benzoylphenyl)[2-(cyclohexylamino)-1-(3-hydroxyphenyl)-2-oxoethyl]amino]-2-oxoethyl]-carbamic acid 1,1-dimethylethyl ester.

Provided is also a compound having formula I or a pharmaceutically acceptable salt or hydrate thereof, wherein: 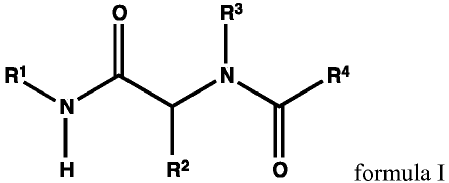
R¹ is C₄-C₆ carbocyclyl optionally substituted with one to three R⁷ groups;
each R² and R³ is independently selected from aryl or heteroaryl, wherein said aryl or heteroaryl is independently optionally substituted with one to three R⁷ groups or acrylamido;
R⁴ is saturated heterocyclyl, -CH(R⁵)N(R⁵)-heteroaryl, -CH(R⁵)N(R⁵)-aryl, -CH(R⁵)N(R⁵)-heterocyclyl, -CH(R⁵)N(R⁵)-carbocyclyl, heteroaralkyl, -CH₂-heterocyclyl, 1H-indol-2-yl, indolin-2-yl, 1,2,3,4-tetrahydroquinolin-2-yl, imidazo[1,2-*a*]pyridine-5-yl, 5,6,7,8-tetrahydroimidazo[1,2-*a*]pyridine-5-yl, -(CR⁵R⁶)₁₋₄N(R⁵)C(O)O(C₁-C₆ alkyl), or -(CR⁵R⁶)₁₋₄N(R⁵)SO₂(C₁-C₆ alkyl), wherein each saturated heterocyclyl, heteroaryl, aryl, heterocyclyl, or carbocyclyl is independently optionally substituted with one to three R⁷ groups;
each R⁵ is independently selected from hydrogen and methyl;
each R⁶ is independently selected from hydrogen, methyl, CH₂OH, CH(CH₃)OH, CH₂NH₂, or CH(CH₃)NH₂;
each R⁷ is independently halo, -CF₃, -CN, -OR⁸, -N(R⁸)₂, -C(O)CH₃, -C(O)OCH₃,-SO₂(C₁-C₃ alkyl), -C(O)N(R⁸)₂, -O(CH₂)₂-OR⁸, SO₂N(R⁸)₂, heteroaryl, -C₁-C₃ haloalkyl, C₁-C₃ alkyl optionally substituted with -OR⁸ or -N(R⁸)₂; and
each R⁸ is independently H or C₁-C₃ alkyl; and provided that:
   (i) R⁴ is other than thien-2-ylmethyl, 1H-benizimidazol-1-ylmethyl, 1H-indol-3-ylmethyl, or 1H-benzotriazol-1-ylmethyl; and (ii) the compound is not N-[2-[[2-(cyclohexylamino)-1-(3-hydroxyphenyl)-2-oxoethyl]phenylamino]-2-oxoethyl]-carbamic acid 1,1-dimethylethyl ester or N-[2-[(2-benzoylphenyl)[2-(cyclohexylamino)-1-(3-hydroxyphenyl)-2-oxoethyl]amino]-2-oxoethyl]-carbamic acid 1,1-dimethylethyl ester.
R¹ may be optionally substituted C₄-C₆ cycloalkyl. R¹ may be C₄-C₆ cycloalkyl optionally substituted with one to three R⁷ groups. R¹ may be C₄-C₆ cycloalkyl optionally substituted with one to two R⁷ groups and R⁷ is a halo. R¹ may be 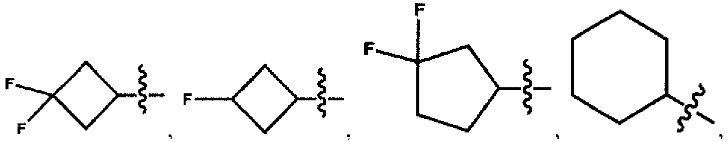 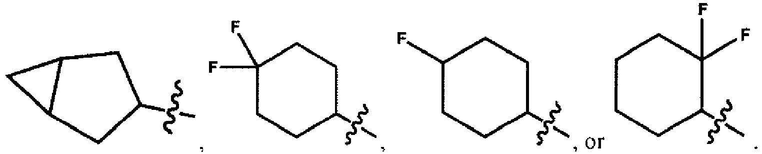
R¹ is:

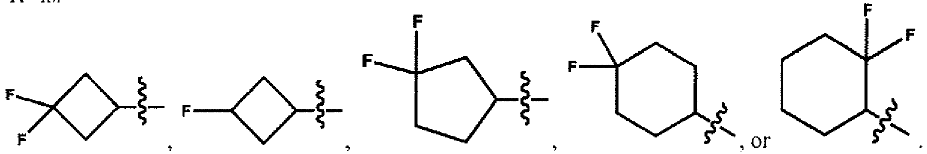

In another embodiment, R² is optionally substituted aryl. In one aspect of this embodiment, R² is aryl optionally substituted with one to three R⁷ groups. In another aspect of this embodiment, R² is phenyl optionally substituted with one to two R⁷ groups and each R⁷ is independently F, Cl or methyl.

In another embodiment, R³ is optionally substituted aryl. In one aspect of this embodiment, R³ is aryl optionally substituted with one to three R⁷ groups. In another aspect of this embodiment, R³ is indazolyl. In another aspect of this embodiment, R³ is phenyl optionally substituted with one to two R⁷ groups wherein each R⁷ is independently F, CN, SO₂N(R⁸)₂, or heteroaryl. In yet another aspect of this embodiment, R³ is phenyl optionally substituted with one to two R⁷ groups wherein each R⁷ is independently F, CN, -SO₂NH₂, -SO₂NH(CH₃), acrylamido or oxadiazolyl.

In another embodiment, R⁴ is 4-6 membered saturated heterocyclyl, -CH₂-heteroaryl, -CH₂-heterocyclyl, -CH(R⁵)N(R⁵)-heteroaryl, 1H-indol-2-yl, indolin-2-yl, 1,2,3,4-tetrahydroquinolin-2-yl, imidazo[1,2-*a*]pyridine-5-yl, 5,6,7,8-tetrahydroimidazo[1,2-*a*]pyridine-5-yl, -(CR⁵R⁶)₁₋₄N(R⁵)C(O)O(C₁-C₆ alkyl), or -(CR⁵R⁶)₁₋₄N(R⁵)SO₂(C₁-C₆ alkyl), wherein each said saturated heterocyclyl, heteroaryl, or heterocyclyl is independently optionally substituted with one to three R⁷ groups. In an aspect of this embodiment, each R⁷ is independently halo, -CF₃, -CN, -OR⁸, -N(R⁸)₂, -C(O)CH₃, -C(O)OCH₃, -SO₂(C₁-C₃ alkyl), -C(O)N(R⁸)₂, - O(CH₂)₂-OR⁸, pyrimidinyl, pyridyl, -C₁-C₃ haloalkyl, or C₁-C₃ alkyl optionally substituted with -OR⁸ or -N(R⁸)₂. In another aspect of this embodiment, R⁴ is: 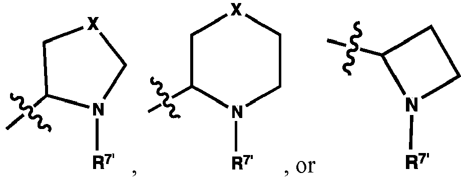 wherein X is CH(R⁷"), O, NH, or NC(O)CH₃; R^{7'} is H, -C(O)CH₃, -C(O)OCH₃, -SO₂(C₁-C₃ alkyl), -C(O)N(R⁸)₂, pyrimidinyl, pyridyl; and R^{7"} is H, -O(CH₂)₂-OCH₃, -O(CH₂)₂-OH, OH, OCH₃, NH₂, or F. In another aspect of this embodiment, R⁴ is -CH₂-NH(heteroaryl) or -CH(CH₂OH)-NH(heteroaryl), wherein heteroaryl is pyridinyl or pyrimidinyl each optionally substituted with one or two R⁷ groups (e.g., each R⁷ is independently halo such as Cl or F). In another aspect of this embodiment, R⁴ is -CH₂-heteroaryl wherein heteroaryl is imidazolyl, triazolyl, pyridinyl or tetrazolyl, each of imidazolyl, triazolyl, pyridinyl or tetrazolyl optionally substituted with one to two R⁷ groups (e.g., each R⁷ is independently halo (e.g., F or Cl), -CF₃, -CN, -CH₂OH, or -CH₃). In another aspect of this embodiment, R⁴ is -(CR⁵R⁶)N(R⁵)C(O)O(C₁-C₄ alkyl) wherein each R⁵ is independently H or methyl and R⁶ is methyl or CH₂OH. In another aspect of this embodiment, R⁴ is 1,2,3,4-tetrahydroquinolin-2-yl, or 5,6,7,8-tetrahydroimidazo[1,2-*a*]pyridine-5-yl.

In another embodiment, exemplary compounds of formula I are depicted below in Table 1.

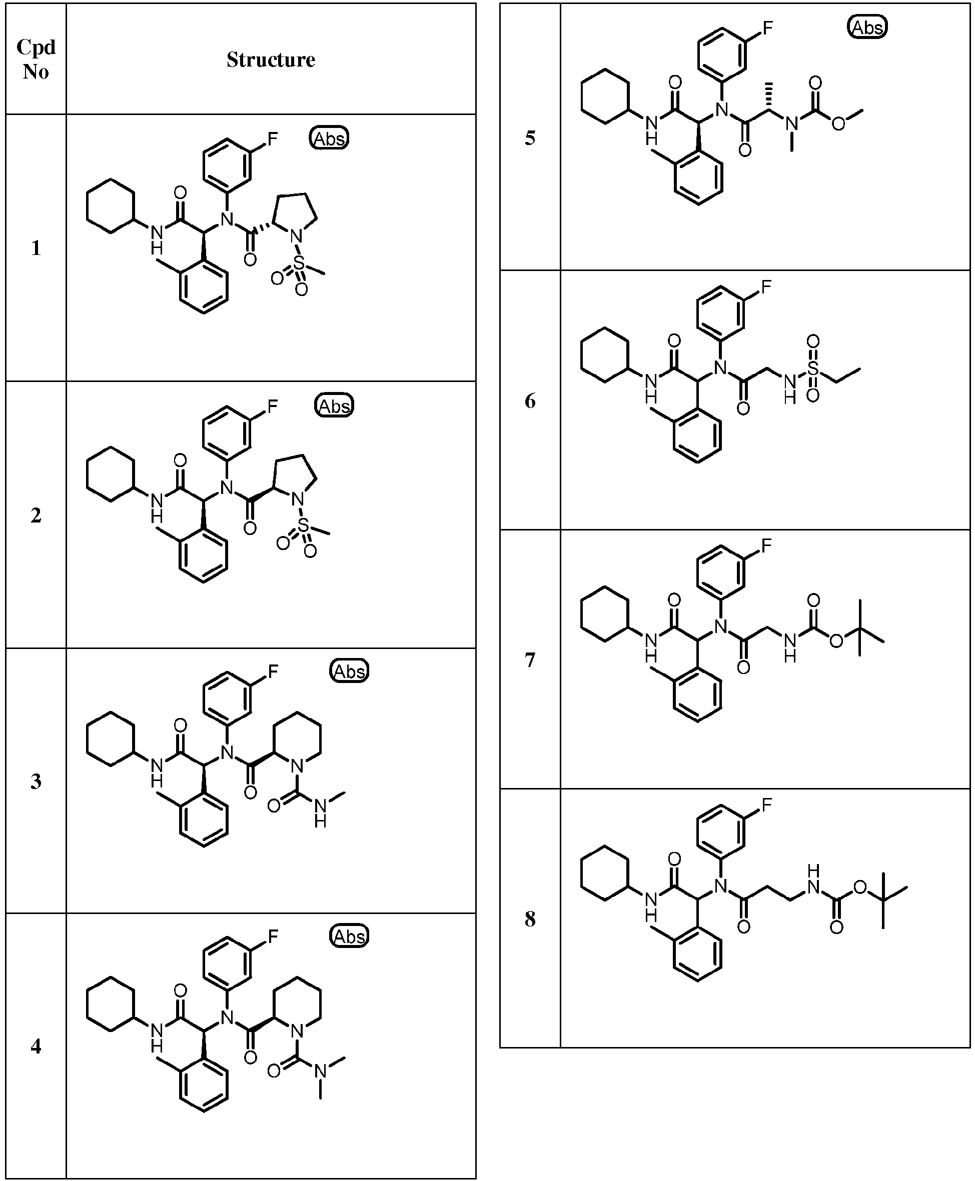

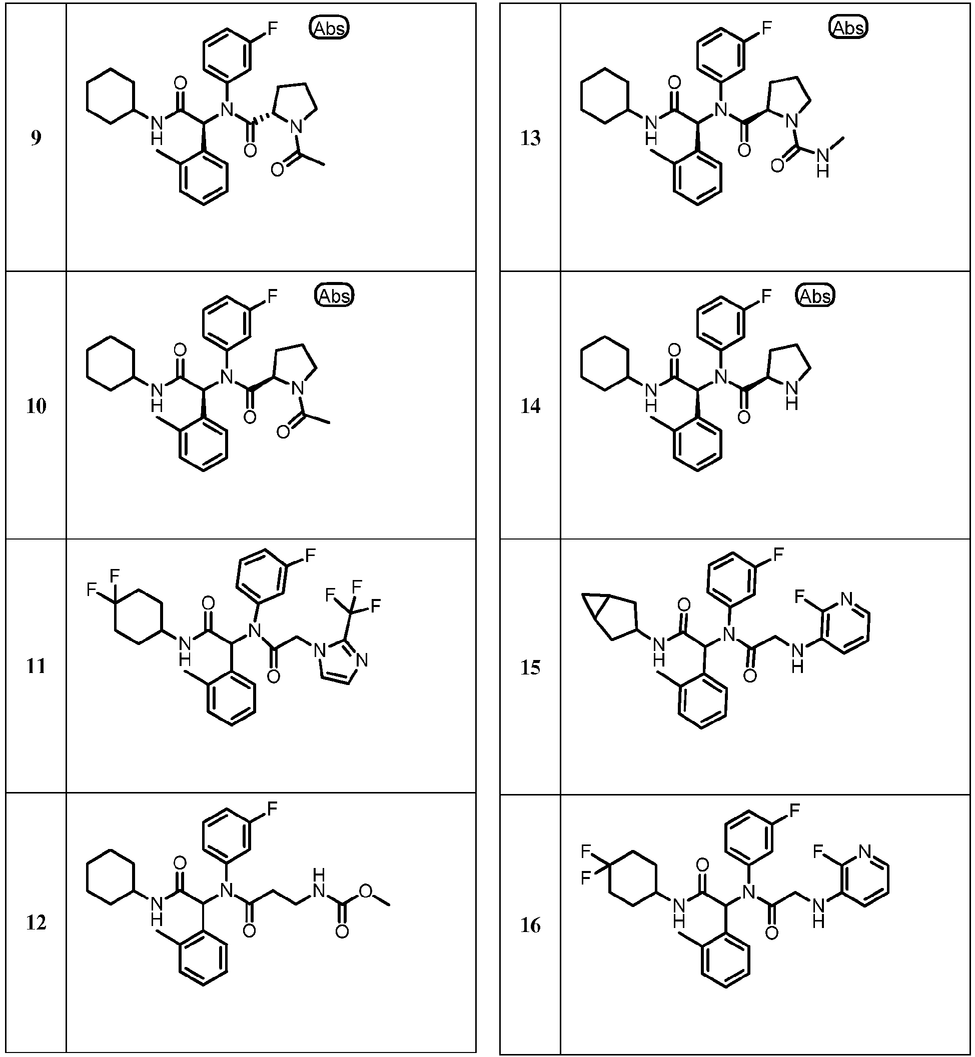

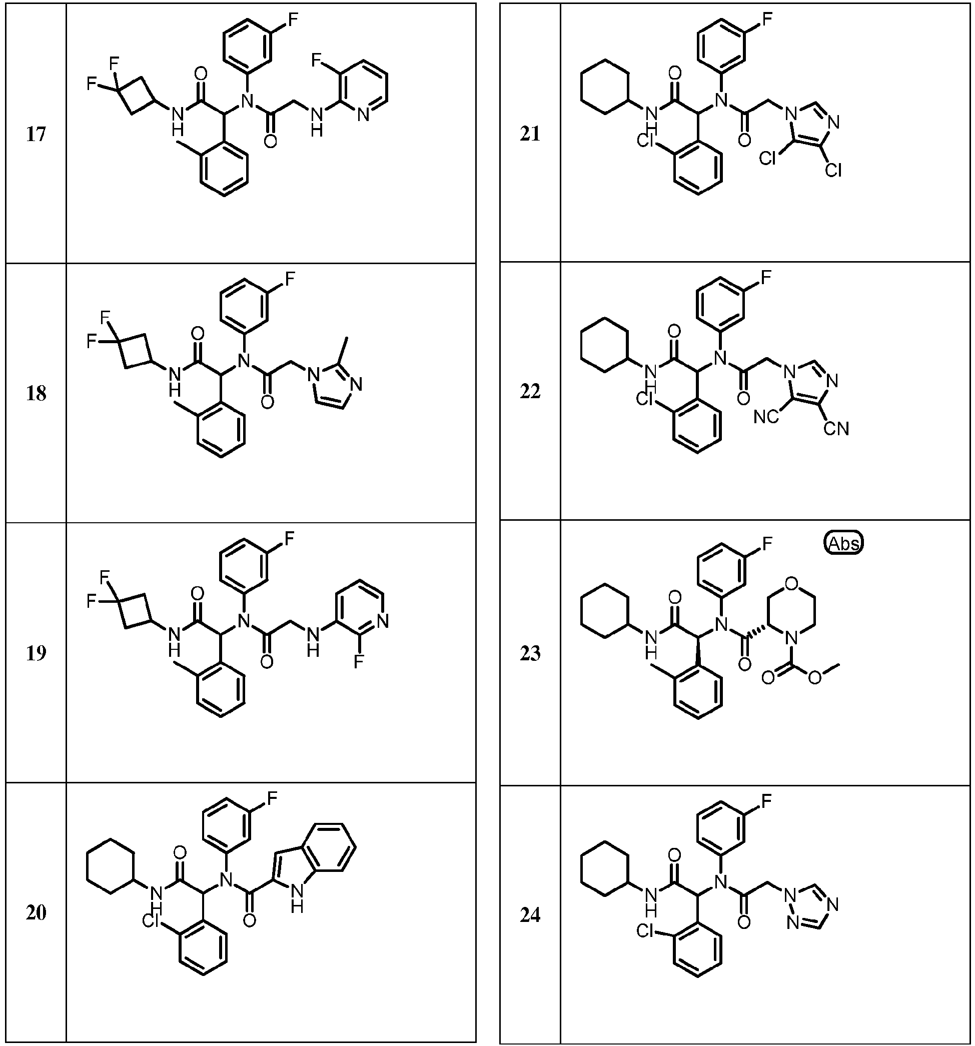

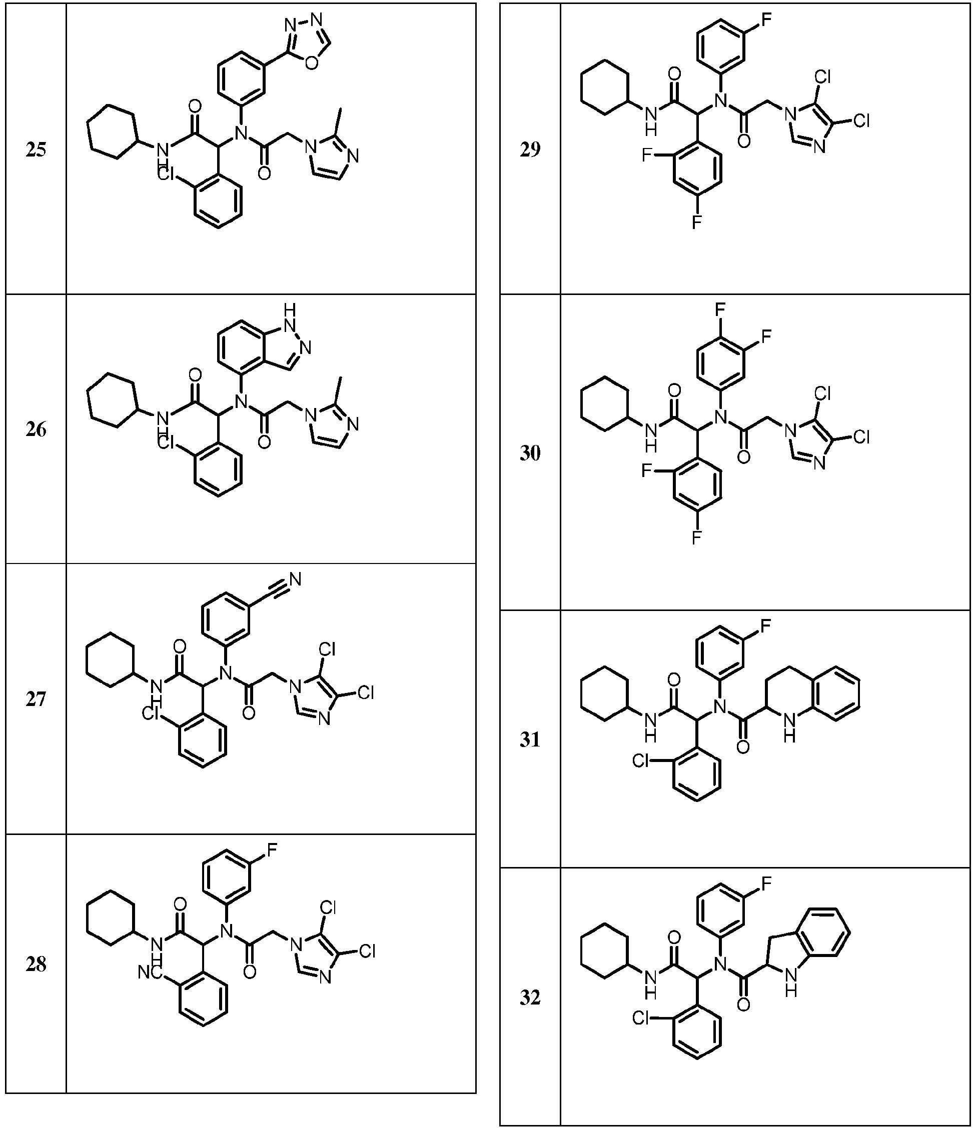

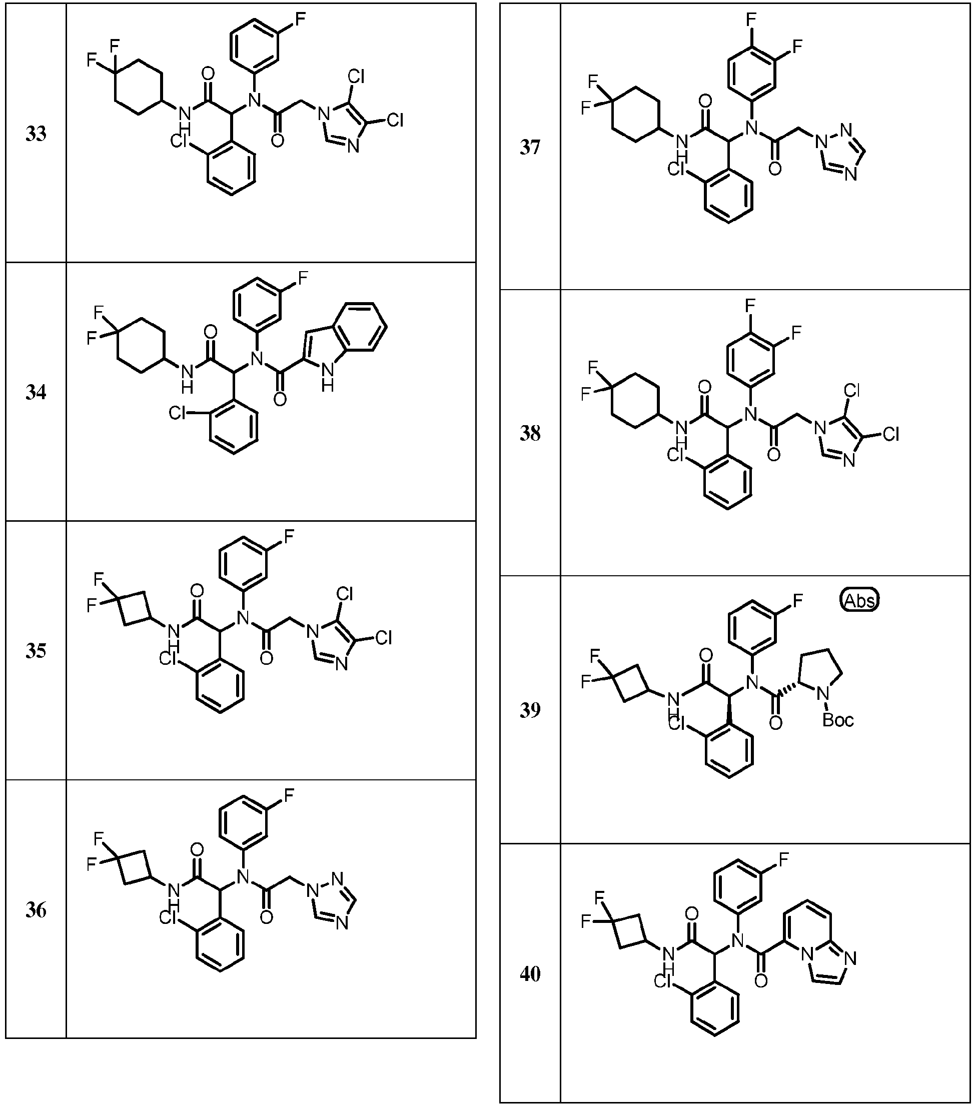

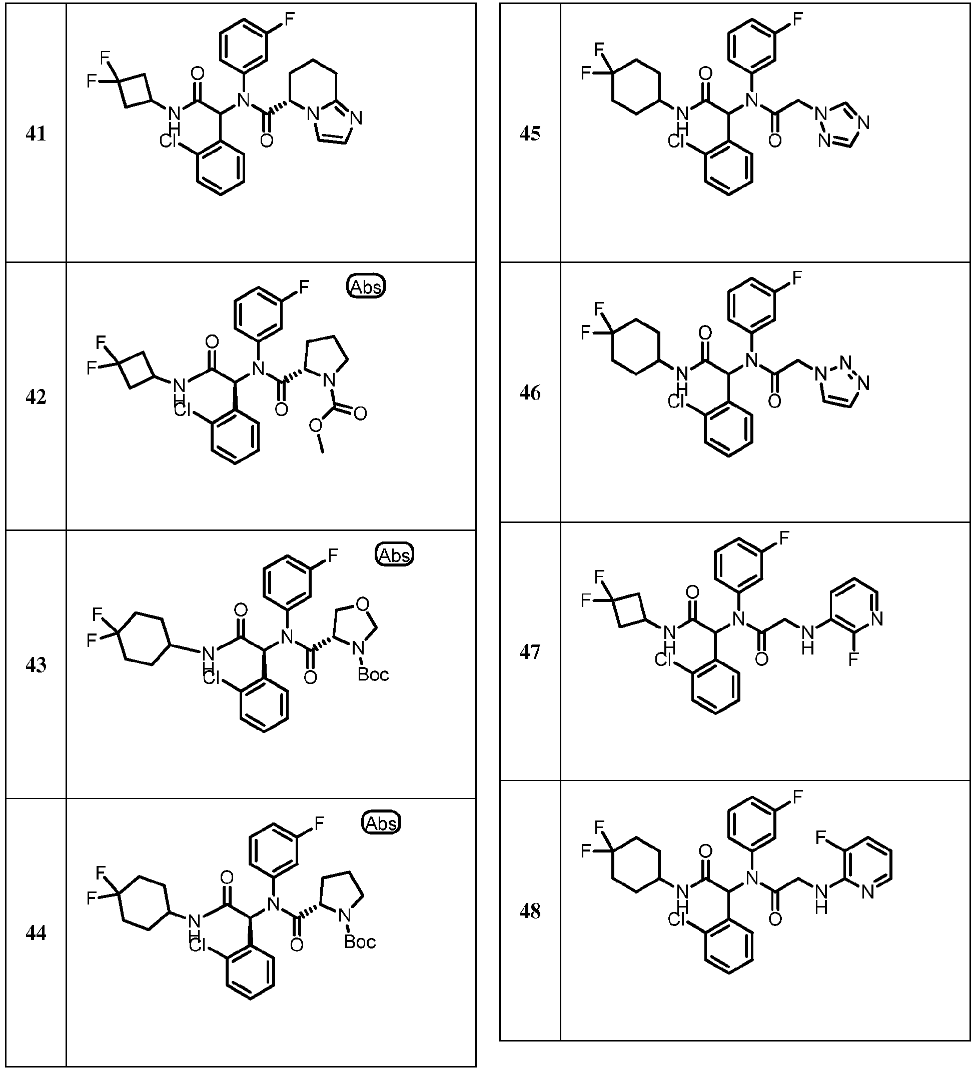

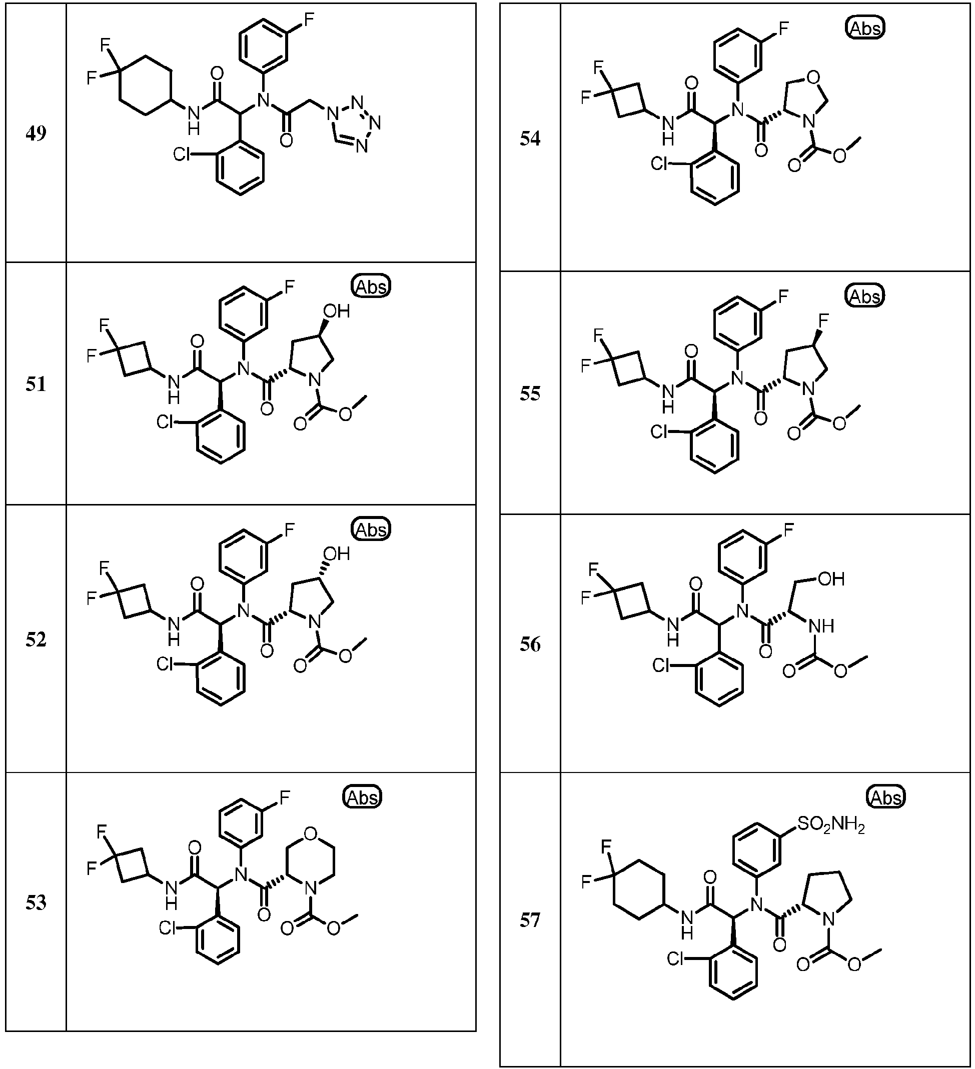

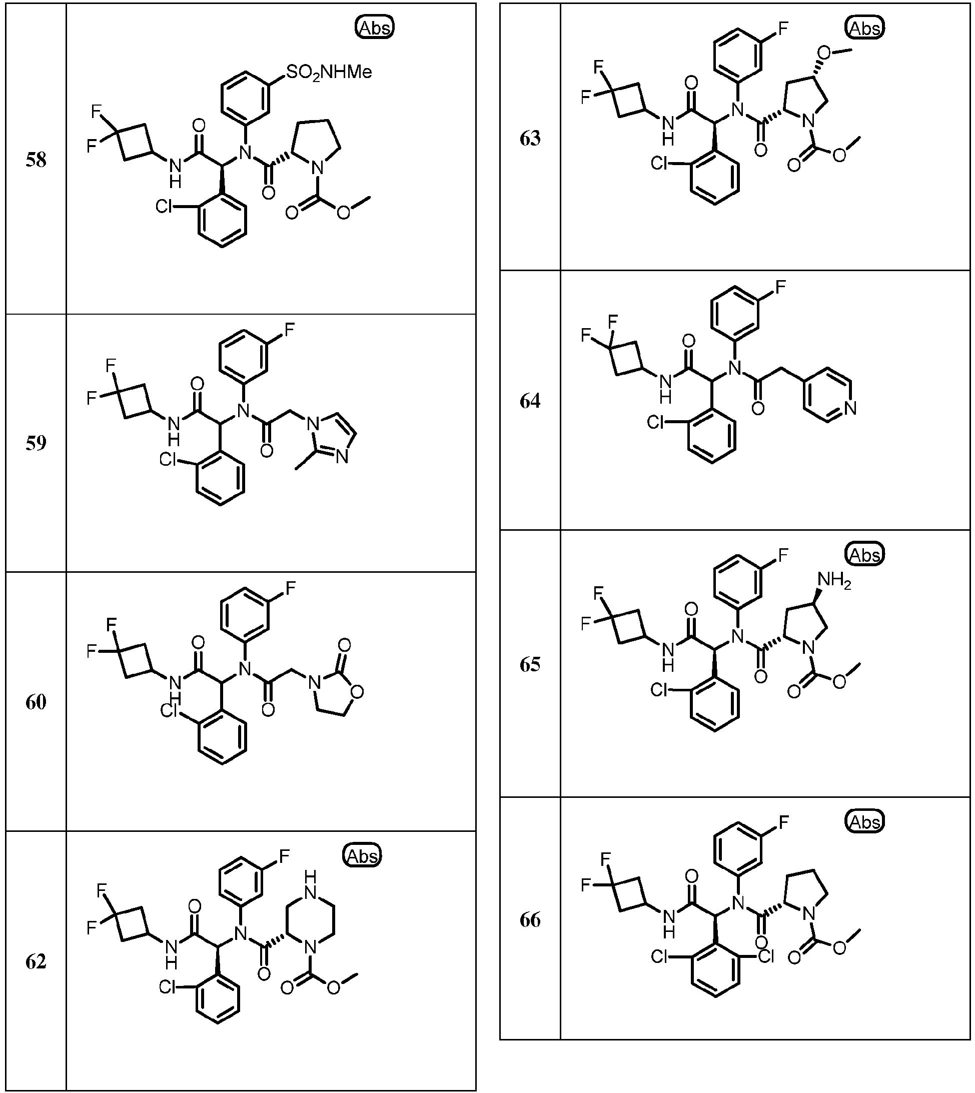

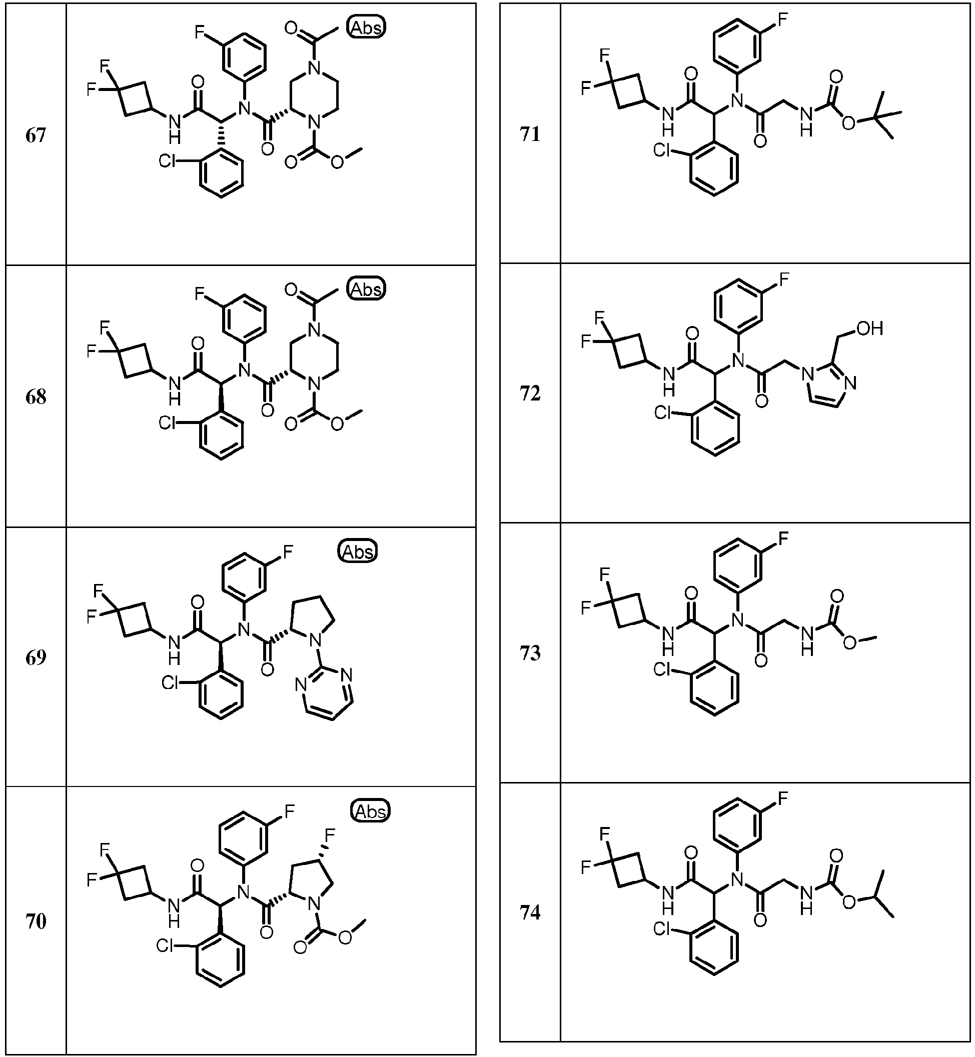

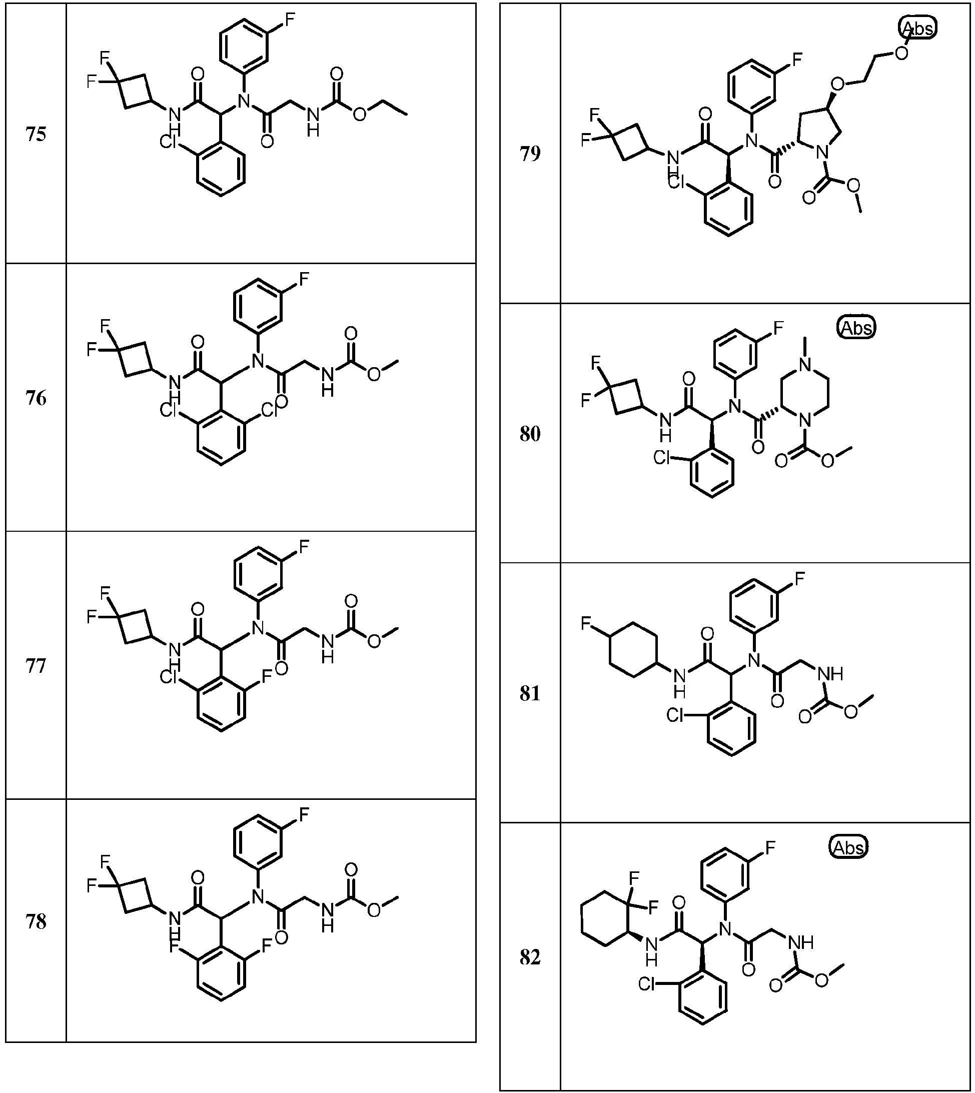

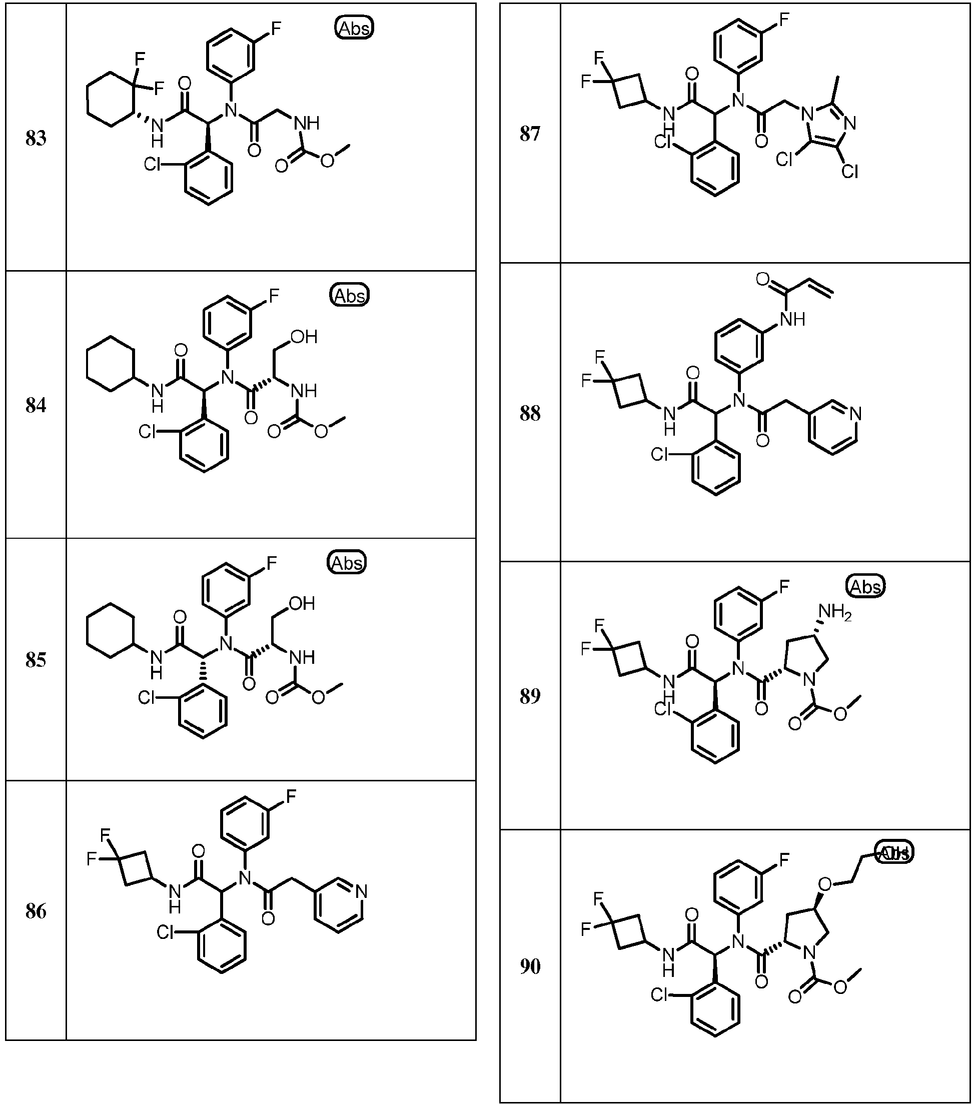

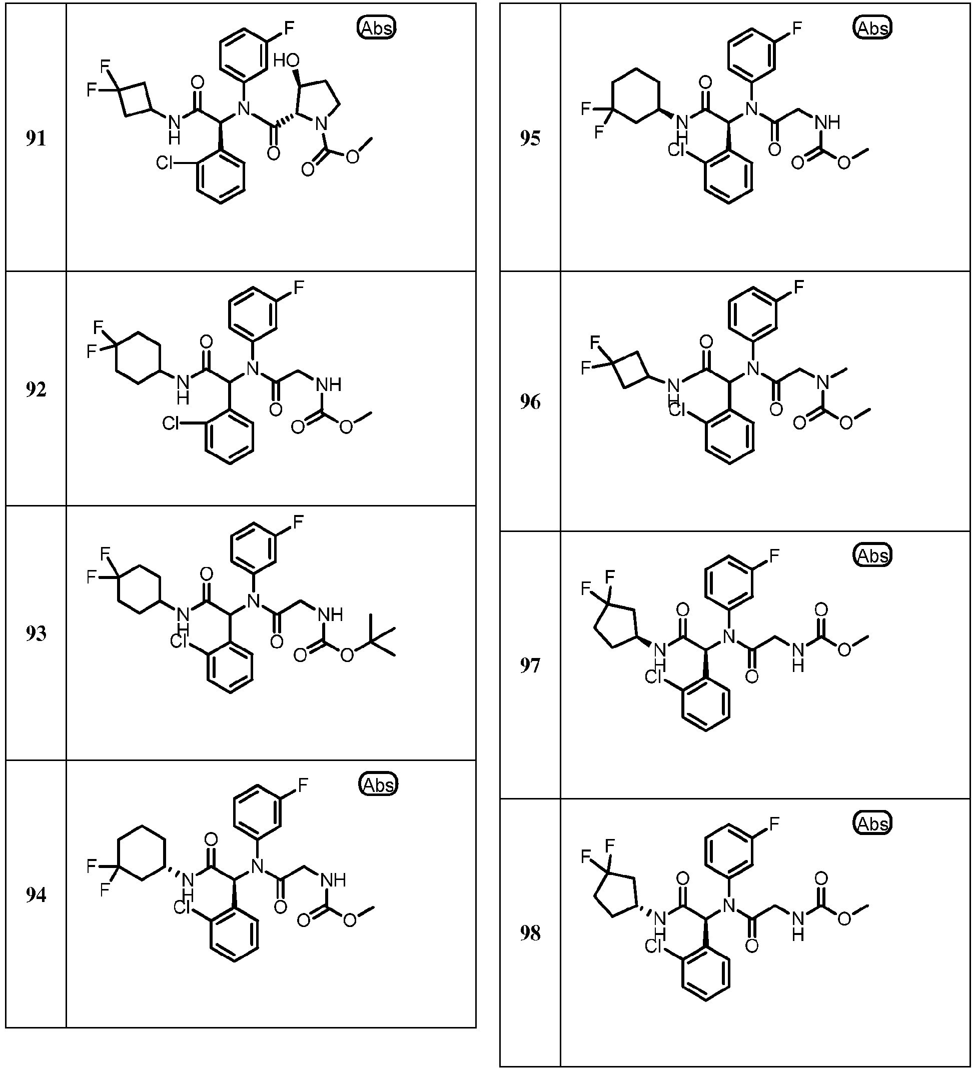

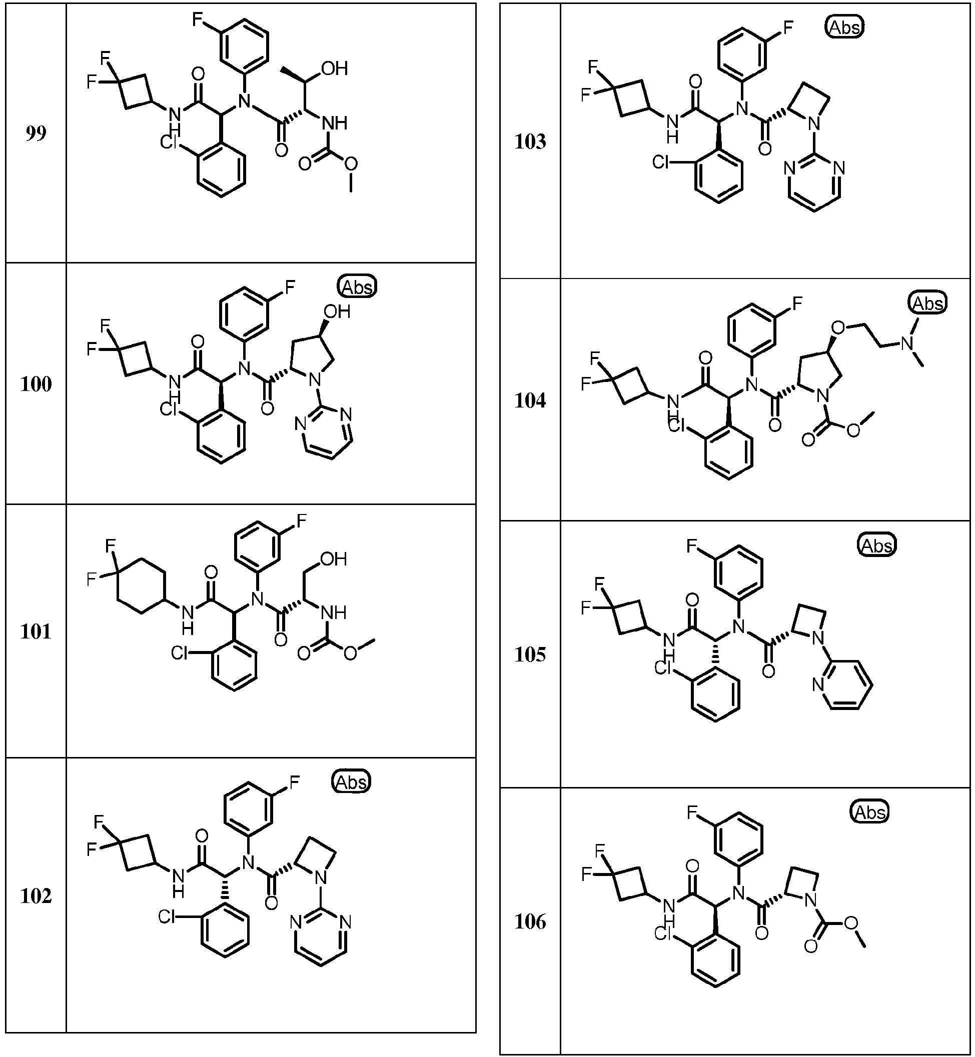

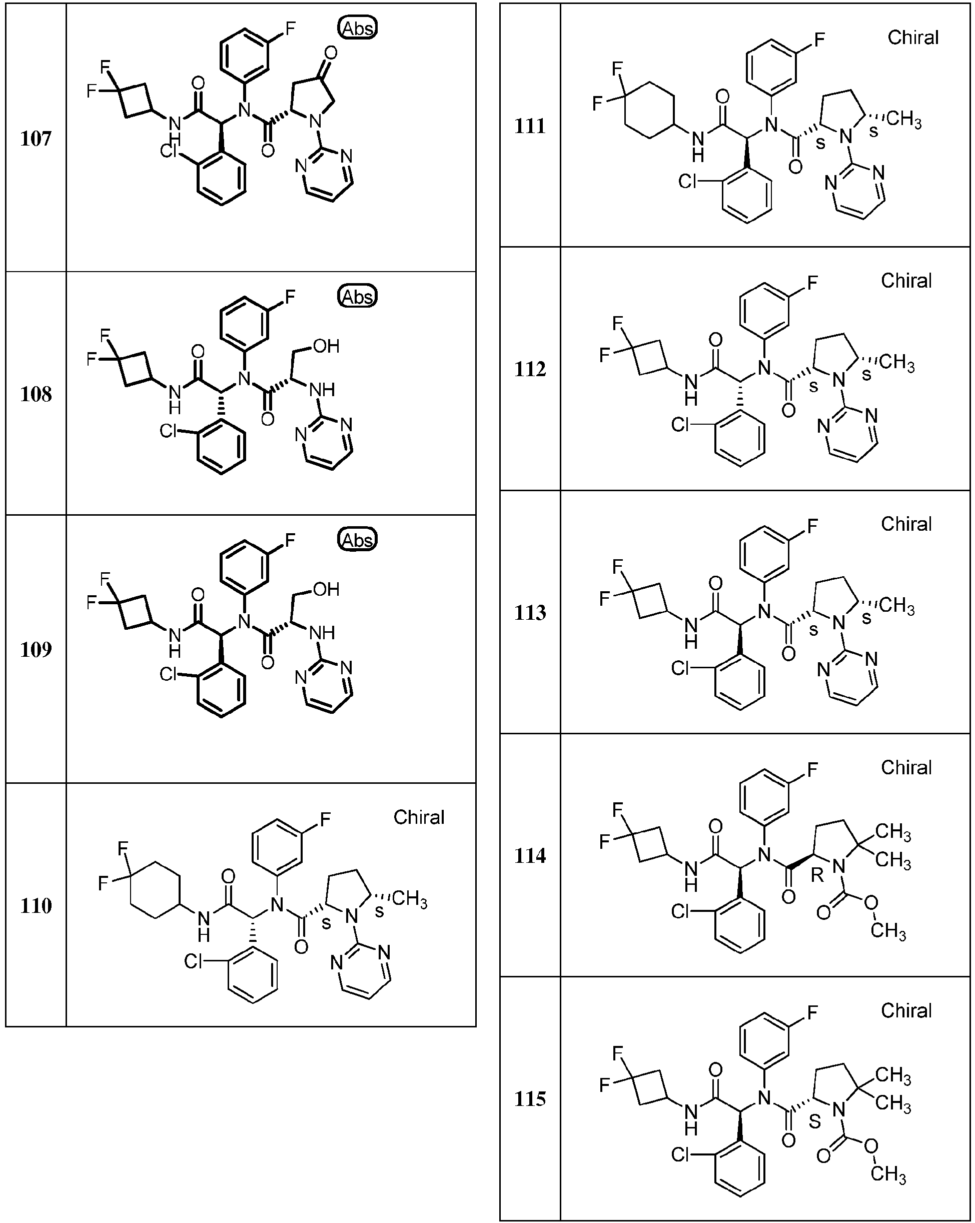

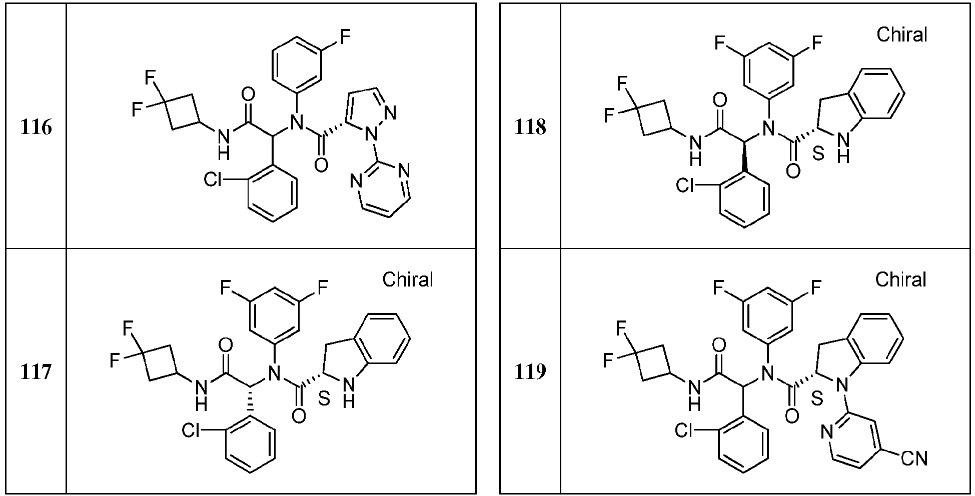

The compounds of this invention may contain one or more asymmetric centers and thus occur as racemates, racemic mixtures, scalemic mixtures, and diastereomeric mixtures, as well as single enantiomers or individual stereoisomers that are substantially free from another possible enantiomer or stereoisomer. The term "substantially free of other stereoisomers" as used herein means a preparation enriched in a compound having a selected stereochemistry at one or more selected stereocenters by at least about 60%, 65%, 70%, 75%, 80%, 85%, 90%, 95%, 96%, 97%, 98%, or 99%. The term "enriched" means that at least the designated percentage of a preparation is the compound having a selected stereochemistry at one or more selected stereocenters. Methods of obtaining or synthesizing an individual enantiomer or stereoisomer for a given compound are known in the art and may be applied as practicable to final compounds or to starting material or intermediates.

In one embodiment, the compound is enriched in a specific stereoisomer by at least about 60%, 65%, 70%, 75%, 80%, 85%, 90%, 95%, 96%, 97%, 98%, or 99%.

The compounds of formula I may also comprise one or more isotopic substitutions. For example, H may be in any isotopic form, including ¹H, ²H (D or deuterium), and ³H (T or tritium); C may be in any isotopic form, including ¹²C, ¹³C, and ¹⁴C; O may be in any isotopic form, including ¹⁶O and ¹⁸O; and the like.

Unless otherwise indicated when a disclosed compound is named or depicted by a structure without specifying the stereochemistry and has one or more chiral centers, it is understood to represent all possible stereoisomers of the compound.

The compounds of this invention may also be represented in multiple tautomeric forms, in such instances, the invention expressly includes all tautomeric forms of the compounds described herein, even though only a single tautomeric form may be represented (e.g., alkylation of a ring system may result in alkylation at multiple sites, the invention expressly includes all such reaction products). All such isomeric forms of such compounds are expressly included in the present invention.

Compounds described herein may be prepared following procedures detailed in the examples and other analogous methods known to one skilled in the art. The suitability of a chemical group in a compound structure for use in the synthesis of another compound is within the knowledge of one of ordinary skill in the art. Synthetic chemistry transformations and protecting group methodologies (protection and deprotection) useful in synthesizing the applicable compounds are known in the art and include, for example, those described in Larock R, Comprehensive Organic Transformations, VCH Publishers (1989); Greene, TW et al., Protective Groups in Organic Synthesis, 3rd Ed., John Wiley and Sons (1999); Fieser, L et al., Fieser and Fieser's Reagents for Organic Synthesis, John Wiley and Sons (1994); and Paquette, L, ed., Encyclopedia of Reagents for Organic Synthesis, John Wiley and Sons (1995) and subsequent editions thereof.

Combinations of substituents and variables envisioned by this invention are only those that result in the formation of stable compounds.

It may be convenient or desirable to prepare, purify, and/or handle a corresponding salt of the active compound, for example, a pharmaceutically acceptable salt. Examples of pharmaceutically acceptable salts are discussed in Berge et al., 1977, "Pharmaceutically Acceptable Salts." J. Pharm. Sci. Vol. 66, pp. 1-19.

For example, if the compound is anionic, or has a functional group which may be anionic *(e.g.,* -COOH may be -COO⁻), then a salt may be formed with a suitable cation. Examples of suitable inorganic cations include, but are not limited to, alkali metal ions such as Na⁺ and K⁺, alkaline earth cations such as Ca²⁺ and Mg²⁺, and other cations such as Al³⁺. Examples of suitable organic cations include, but are not limited to, ammonium ion (*i.e.,* NH₄⁺) and substituted ammonium ions (*e.g.,* NH₃R⁺, NH₂R²⁺, NHR³⁺, NR⁴⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

If the compound is cationic, or has a functional group that may be cationic (*e.g.,* -NH₂ may be -NH₃⁺), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucoheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. Examples of suitable polymeric organic anions include, but are not limited to, those derived from the following polymeric acids: tannic acid, carboxymethyl cellulose.

Unless otherwise specified, a reference to a particular compound also includes salt forms thereof.

### Compositions and routes of administration

The compounds utilized in the methods described herein may be formulated together with a pharmaceutically acceptable carrier or adjuvant into pharmaceutically acceptable compositions prior to be administered to a subject. In another embodiment, such pharmaceutically acceptable compositions further comprise additional therapeutic agents in amounts effective for achieving a modulation of disease or disease symptoms, including those described herein.

The term "pharmaceutically acceptable carrier or adjuvant" refers to a carrier or adjuvant that may be administered to a subject, together with a compound of this invention, and which does not destroy the pharmacological activity thereof and is nontoxic when administered in doses sufficient to deliver a therapeutic amount of the compound.

Pharmaceutically acceptable carriers, adjuvants and vehicles that may be used in the pharmaceutical compositions of this invention include, but are not limited to, ion exchangers, alumina, aluminum stearate, lecithin, self-emulsifying drug delivery systems (SEDDS) such as d-α-tocopherol polyethyleneglycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Cyclodextrins such as α-, β-, and γ-cyclodextrin, or chemically modified derivatives such as hydroxyalkylcyclodextrins, including 2- and 3-hydroxypropyl-β-cyclodextrins, or other solubilized derivatives may also be advantageously used to enhance delivery of compounds of the formulae described herein.

The pharmaceutical compositions of this invention may be administered orally, parenterally, by inhalation spray, topically, rectally, nasally, buccally, vaginally or via an implanted reservoir, preferably by oral administration or administration by injection. The pharmaceutical compositions of this invention may contain any conventional non-toxic pharmaceutically-acceptable carriers, adjuvants or vehicles. In some cases, the pH of the formulation may be adjusted with pharmaceutically acceptable acids, bases or buffers to enhance the stability of the formulated compound or its delivery form. The term parenteral as used herein includes subcutaneous, intracutaneous, intravenous, intramuscular, intraarticular, intraarterial, intrasynovial, intrasternal, intrathecal, intralesional and intracranial injection or infusion techniques.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, or carboxymethyl cellulose or similar dispersing agents which are commonly used in the formulation of pharmaceutically acceptable dosage forms such as emulsions and or suspensions. Other commonly used surfactants such as Tweens or Spans and/or other similar emulsifying agents or bioavailability enhancers which are commonly used in the manufacture of pharmaceutically acceptable solid, liquid, or other dosage forms may also be used for the purposes of formulation.

The pharmaceutical compositions of this invention may be orally administered in any orally acceptable dosage form including, but not limited to, capsules, tablets, emulsions and aqueous suspensions, dispersions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions and/or emulsions are administered orally, the active ingredient may be suspended or dissolved in an oily phase is combined with emulsifying and/or suspending agents. If desired, certain sweetening and/or flavoring and/or coloring agents may be added.

The pharmaceutical compositions of this invention may also be administered in the form of suppositories for rectal administration. These compositions can be prepared by mixing a compound of this invention with a suitable non-irritating excipient which is solid at room temperature but liquid at the rectal temperature and therefore will melt in the rectum to release the active components. Such materials include, but are not limited to, cocoa butter, beeswax and polyethylene glycols.

Topical administration of the pharmaceutical compositions of this invention is useful when the desired treatment involves areas or organs readily accessible by topical application. For application topically to the skin, the pharmaceutical composition should be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene polyoxypropylene compound, emulsifying wax and water. Alternatively, the pharmaceutical composition can be formulated with a suitable lotion or cream containing the active compound suspended or dissolved in a carrier with suitable emulsifying agents. Suitable carriers include, but are not limited to, mineral oil, sorbitan monostearate, polysorbate 60, cetyl esters wax, cetearyl alcohol, 2-octyldodecanol, benzyl alcohol and water. The pharmaceutical compositions of this invention may also be topically applied to the lower intestinal tract by rectal suppository formulation or in a suitable enema formulation. Topically-transdermal patches are also included in this invention.

The pharmaceutical compositions of this invention may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. When the compositions of this invention comprise a combination of a compound of the formulae described herein and one or more additional therapeutic or prophylactic agents, both the compound and the additional agent should be present at dosage levels of between about 1 to 100%, and more preferably between about 5 to 95% of the dosage normally administered in a monotherapy regimen. The additional agents may be administered separately, as part of a multiple dose regimen, from the compounds of this invention. Alternatively, those agents may be part of a single dosage form, mixed together with the compounds of this invention in a single composition.

The compounds described herein can, for example, be administered by injection, intravenously, intraarterially, subdermally, intraperitoneally, intramuscularly, or subcutaneously; or orally, buccally, nasally, transmucosally, topically, in an ophthalmic preparation, or by inhalation, with a dosage ranging from about 0.5 to about 100 mg/kg of body weight, alternatively dosages between 1 mg and 1000 mg/dose, every 4 to 120 hours, or according to the requirements of the particular drug. The methods herein contemplate administration of an effective amount of compound or compound composition to achieve the desired or stated effect. Typically, the pharmaceutical compositions of this invention will be administered from about 1 to about 6 times per day or alternatively, as a continuous infusion. Such administration can be used as a chronic or acute therapy. The amount of active ingredient that may be combined with the carrier materials to produce a single dosage form will vary depending upon the host treated and the particular mode of administration. A typical preparation will contain from about 5% to about 95% active compound (w/w). Alternatively, such preparations contain from about 20% to about 80% active compound.

Lower or higher doses than those recited above may be required. Specific dosage and treatment regimens for any particular subject will depend upon a variety of factors, including the activity of the specific compound employed, the age, body weight, general health status, sex, diet, time of administration, rate of excretion, drug combination, the severity and course of the disease, condition or symptoms, the subject's disposition to the disease, condition or symptoms, and the judgment of the treating physician.

Upon improvement of a subject's condition, a maintenance dose of a compound, composition or combination of this invention may be administered, if necessary. Subsequently, the dosage or frequency of administration, or both, may be reduced, as a function of the symptoms, to a level at which the improved condition is retained when the symptoms have been alleviated to the desired level. Subjects may, however, require intermittent treatment on a long-term basis upon any recurrence of disease symptoms.

The pharmaceutical compositions described above comprising a compound of formula I or a compound described in any one of the embodiments herein, may further comprise another therapeutic agent useful for treating cancer.

### Methods of Use

Provided is a method for inhibiting a mutant IDH1 or IDH2 activity comprising contacting a subject in need thereof a compound of structural formula I, a compound described in any one of the embodiments herein, or a pharmaceutically acceptable salt thereof. In one embodiment, the cancer to be treated is characterized by a mutant allele of IDH1 or IDH2 wherein the IDH1 or IDH2 mutation result in a new ability of the enzyme to catalyze the NADPH-dependent reduction of α-ketoglutarate to *R*(-)-2-hydroxyglutarate in a subject. In one aspect of this embodiment, the mutant IDH1 has an R132X mutation. In one aspect of this embodiment, the R132X mutation is selected from R132H, R132C, R132L, R132V, R132S and R132G. In another aspect, the R132X mutation is R132H or R132C. In yet another aspect, the R132X mutation is R132H.

Also provided are methods of treating a cancer characterized by the presence of a mutant allele of IDH1 comprising the step of administering to subject in need thereof (a) a compound of formula I, a compound described in any one of the embodiments herein, or a pharmaceutically acceptable salt thereof, or (b) a pharmaceutical composition comprising (a) and a pharmaceutically acceptable carrier.

In one embodiment, the cancer to be treated is characterized by a mutant allele of IDH1 wherein the IDH1 mutation result in a new ability of the enzyme to catalyze the NADPH-dependent reduction of α-ketoglutarate to *R*(-)-2-hydroxyglutarate in a patient. In one aspect of this embodiment, the IDH1 mutation is an R132X mutation. In another aspect of this embodiment, the R132X mutation is selected from R132H, R132C, R132L, R132V, R132S and R132G. In another aspect, the R132X mutation is R132H or R132C. A cancer can be analyzed by sequencing cell samples to determine the presence and specific nature of (e.g., the changed amino acid present at) a mutation at amino acid 132 of IDH1.

Without being bound by theory, applicants believe that mutant alleles of IDH1 wherein the IDH1 mutation result in a new ability of the enzyme to catalyze the NADPH-dependent reduction of α-ketoglutarate to *R*(-)-2-hydroxyglutarate, and in particular R132H mutations of IDH1, characterize a subset of all types of cancers, without regard to their cellular nature or location in the body. Thus, the compounds and methods of this invention are useful to treat any type of cancer that is characterized by the presence of a mutant allele of IDH1 imparting such acitivity and in particular an IDH1 R132H or R132C mutation.

In one aspect of this embodiment, the efficacy of cancer treatment is monitored by measuring the levels of 2HG in the subject. Typically levels of 2HG are measured prior to treatment, wherein an elevated level is indicative of the use of the compound of formula I to treat the cancer. Once the elevated levels are established, the level of 2HG is determined during the course of and/or following termination of treatment to establish efficacy. In certain embodiments, the level of 2HG is only determined during the course of and/or following termination of treatment. A reduction of 2HG levels during the course of treatment and following treatment is indicative of efficacy. Similarly, a determination that 2HG levels are not elevated during the course of or following treatment is also indicative of efficacy. Typically, the these 2HG measurements will be utilized together with other well-known determinations of efficacy of cancer treatment, such as reduction in number and size of tumors and/or other cancer-associated lesions, improvement in the general health of the subject, and alterations in other biomarkers that are associated with cancer treatment efficacy.

2HG can be detected in a sample by LC/MS. The sample is mixed 80:20 with methanol, and centrifuged at 3,000 rpm for 20 minutes at 4 degrees Celsius. The resulting supernatant can be collected and stored at -80 degrees Celsius prior to LC-MS/MS to assess 2-hydroxyglutarate levels. A variety of different liquid chromatography (LC) separation methods can be used. Each method can be coupled by negative electrospray ionization (ESI, -3.0 kV) to triple-quadrupole mass spectrometers operating in multiple reaction monitoring (MRM) mode, with MS parameters optimized on infused metabolite standard solutions. Metabolites can be separated by reversed phase chromatography using 10 mM tributyl-amine as an ion pairing agent in the aqueous mobile phase, according to a variant of a previously reported method (Luo et al. J Chromatogr A 1147, 153-64, 2007). One method allows resolution of TCA metabolites: t = 0, 50% B; t = 5, 95% B; t= 7, 95% B; t= 8, 0% B, where B refers to an organic mobile phase of 100% methanol. Another method is specific for 2-hydroxyglutarate, running a fast linear gradient from 50% -95% B (buffers as defined above) over 5 minutes. A Synergi Hydro-RP, 100mm × 2 mm, 2.1 µm particle size (Phenomonex) can be used as the column, as described above. Metabolites can be quantified by comparison of peak areas with pure metabolite standards at known concentration. Metabolite flux studies from ¹³C-glutamine can be performed as described, *e.g.,* in Munger et al. Nat Biotechnol 26, 1179-86, 2008.

In one embodiment 2HG is directly evaluated.

In another embodiment a derivative of 2HG formed in process of performing the analytic method is evaluated. By way of example such a derivative can be a derivative formed in MS analysis. Derivatives can include a salt adduct, *e.g.,* a Na adduct, a hydration variant, or a hydration variant which is also a salt adduct, e.g., a Na adduct, e.g., as formed in MS analysis.

In another embodiment a metabolic derivative of 2HG is evaluated. Examples include species that build up or are elevated, or reduced, as a result of the presence of 2HG, such as glutarate or glutamate that will be correlated to 2HG, *e.g.,* R-2HG.

Exemplary 2HG derivatives include dehydrated derivatives such as the compounds provided below or a salt adduct thereof:

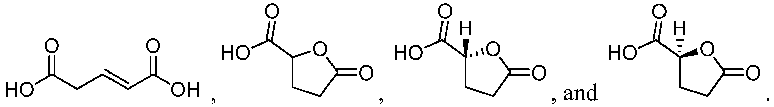

In one embodiment the cancer is a tumor wherein at least 30, 40, 50, 60, 70, 80 or 90% of the tumor cells carry an IDH1 mutation, and in particular an IDH1 R132H or R132C mutation, at the time of diagnosis or treatment.

IDH1 R132X mutations are known to occur in certain types of cancers as indicated in Table 2, below.

**Table 2. IDH mutations associated with certain cancers**

| **Cancer Type** | **IDH1 R132X Mutation** | **Tumor Type** |
|---|---|---|
| brain tumors | R132H | primary tumor |
| | R132C | primary tumor |
| | R132S | primary tumor |
| | R132G | primary tumor |
| | R132L | primary tumor |
| | R132V | primary tumor |
| fibrosarcoma | R132C | HT1080 fibrosarcoma cell line |
| Acute Myeloid Leukemia (AML) | R132H | primary tumor |
| | R132G | primary tumor |
| | R132C | primary tumor |
| Prostate cancer | R132H | primary tumor |
| | R132C | primary tumor |
| Acute lymphoblastic leukemia (ALL) | R132C | primary tumor |
| paragangliomas | R132C | primary tumor |

IDH1 R132H mutations have been identified in glioblastoma, acute myelogenous leukemia, sarcoma, melanoma, non-small cell lung cancer, cholangiocarcinomas, chondrosarcoma, myelodysplastic syndromes (MDS), myeloproliferative neoplasm (MPN), colon cancer, and angio-immunoblastic non-Hodgkin's lymphoma (NHL). Accordingly, in one embodiment, the methods described herein are used to treat glioma (glioblastoma), acute myelogenous leukemia, sarcoma, melanoma, non-small cell lung cancer (NSCLC) or cholangiocarcinomas, chondrosarcoma, myelodysplastic syndromes (MDS), myeloproliferative neoplasm (MPN), colon cancer, or angio-immunoblastic non-Hodgkin's lymphoma (NHL) in a patient.

Accordingly in one embodiment, the cancer is a cancer selected from any one of the cancer types listed in Table 2, and the IDH R132X mutation is one or more of the IDH1 R132X mutations listed in Table 2 for that particular cancer type.

Treatment methods described herein can additionally comprise various evaluation steps prior to and/or following treatment with a compound of formula I or a compound described in any one of the embodiments described herein.

In one embodiment, prior to and/or after treatment with a compound of Structural formula I or a compound described in any one of the embodiments described herein, the method further comprises the step of evaluating the growth, size, weight, invasiveness, stage and/or other phenotype of the cancer.

In one embodiment, prior to and/or after treatment with a compound of formula I or a compound described in any one of the embodiments described herein, the method further comprises the step of evaluating the IDH1 genotype of the cancer. This may be achieved by ordinary methods in the art, such as DNA sequencing, immuno analysis, and/or evaluation of the presence, distribution or level of 2HG.

In one embodiment, prior to and/or after treatment with a compound of formula I or a compound described in any one of the embodiments described herein, the method further comprises the step of determining the 2HG level in the subject. This may be achieved by spectroscopic analysis, *e.g.,* magnetic resonance-based analysis, *e.g.,* MRI and/or MRS measurement, sample analysis of bodily fluid, such as serum or spinal cord fluid analysis, or by analysis of surgical material, *e.g.,* by mass-spectroscopy.

### Combination therapies

In some embodiments, the methods described herein comprise the additional step of co-administering to a subject in need thereof a second therapy *e.g.,* an additional cancer therapeutic agent or an additional cancer treatment. Exemplary additional cancer therapeutic agents include for example, chemotherapy, targeted therapy, antibody therapies, immunotherapy, and hormonal therapy. Additional cancer treatments include, for example: surgery, and radiation therapy. Examples of each of these treatments are provided below.

The term "co-administering" as used herein with respect to an additional cancer therapeutic agents means that the additional cancer therapeutic agent may be administered together with a compound of this invention as part of a single dosage form (such as a composition of this invention comprising a compound of the invention and an second therapeutic agent as described above) or as separate, multiple dosage forms. Alternatively, the additional cancer therapeutic agent may be administered prior to, consecutively with, or following the administration of a compound of this invention. In such combination therapy treatment, both the compounds of this invention and the second therapeutic agent(s) are administered by conventional methods. The administration of a composition of this invention, comprising both a compound of the invention and a second therapeutic agent, to a subject does not preclude the separate administration of that same therapeutic agent, any other second therapeutic agent or any compound of this invention to said subject at another time during a course of treatment. The term "co-administering" as used herein with respect to an additional cancer treatment means that the additional cancer treatment may occur prior to, consecutively with, concurrently with or following the administration of a compound of this invention.

In some embodiments, the additional cancer therapeutic agent is a chemotherapy agent. Examples of chemotherapeutic agents used in cancer therapy include, for example, antimetabolites (*e.g.,* folic acid, purine, and pyrimidine derivatives), alkylating agents (*e.g.,* nitrogen mustards, nitrosoureas, platinum, alkyl sulfonates, hydrazines, triazenes, aziridines, spindle poison, cytotoxic agents, topoisomerase inhibitors and others) and hypomethylating agents (e.g., decitabine (5-aza-deoxycytidine), zebularine, isothiocyanates, azacitidine (5-azacytidine), 5-flouro-2'-deoxycytidine, 5,6-dihydro-5-azacytidine and others). Exemplary agents include Aclarubicin, Actinomycin, Alitretinoin, Altretamine, Aminopterin, Aminolevulinic acid, Amrubicin, Amsacrine, Anagrelide, Arsenic trioxide, Asparaginase, Atrasentan, Belotecan, Bexarotene, bendamustine, Bleomycin, Bortezomib, Busulfan, Camptothecin, Capecitabine, Carboplatin, Carboquone, Carmofur, Carmustine, Celecoxib, Chlorambucil, Chlormethine, Cisplatin, Cladribine, Clofarabine, Crisantaspase, Cyclophosphamide, Cytarabine, Dacarbazine, Dactinomycin, Daunorubicin, Decitabine, Demecolcine, Docetaxel, Doxorubicin, Efaproxiral, Elesclomol, Elsamitrucin, Enocitabine, Epirubicin, Estramustine, Etoglucid, Etoposide, Floxuridine, Fludarabine, Fluorouracil (5FU), Fotemustine, Gemcitabine, Gliadel implants, Hydroxycarbamide, Hydroxyurea, Idarubicin, Ifosfamide, Irinotecan, Irofulven, Ixabepilone, Larotaxel, Leucovorin, Liposomal doxorubicin, Liposomal daunorubicin, Lonidamine, Lomustine, Lucanthone, Mannosulfan, Masoprocol, Melphalan, Mercaptopurine, Mesna, Methotrexate, Methyl aminolevulinate, Mitobronitol, Mitoguazone, Mitotane, Mitomycin, Mitoxantrone, Nedaplatin, Nimustine, Oblimersen, Omacetaxine, Ortataxel, Oxaliplatin, Paclitaxel, Pegaspargase, Pemetrexed, Pentostatin, Pirarubicin, Pixantrone, Plicamycin, Porfimer sodium, Prednimustine, Procarbazine, Raltitrexed, Ranimustine, Rubitecan, Sapacitabine, Semustine, Sitimagene ceradenovec, Strataplatin, Streptozocin, Talaporfin, Tegafur-uracil, Temoporfin, Temozolomide, Teniposide, Tesetaxel, Testolactone, Tetranitrate, Thiotepa, Tiazofurine, Tioguanine, Tipifarnib, Topotecan, Trabectedin, Triaziquone, Triethylenemelamine, Triplatin, Tretinoin, Treosulfan, Trofosfamide, Uramustine, Valrubicin, Verteporfin, Vinblastine, Vincristine, Vindesine, Vinflunine, Vinorelbine, Vorinostat, Zorubicin, and other cytostatic or cytotoxic agents described herein.

Because some drugs work better together than alone, two or more drugs are often given at the same time. Often, two or more chemotherapy agents are used as combination chemotherapy.

In some embodiments, the additional cancer therapeutic agent is a differentiation agent. Such differentiation agent includes retinoids (such as all-trans-retinoic acid (ATRA), 9-cis retinoic acid, 13-cis-retinoic acid (13-cRA) and 4-hydroxy-phenretinamide (4-HPR)); arsenic trioxide; histone deacetylase inhibitors HDACs (such as azacytidine (Vidaza) and butyrates (e.g., sodium phenylbutyrate)); hybrid polar compounds (such as hexamethylene bisacetamide ((HMBA)); vitamin D; and cytokines (such as colony-stimulating factors including G-CSF and GM-CSF, and interferons).

In some embodiments the additional cancer therapeutic agent is a targeted therapy agent. Targeted therapy constitutes the use of agents specific for the deregulated proteins of cancer cells. Small molecule targeted therapy drugs are generally inhibitors of enzymatic domains on mutated, overexpressed, or otherwise critical proteins within the cancer cell. Prominent examples are the tyrosine kinase inhibitors such as Axitinib, Bosutinib, Cediranib, dasatinib, erlotinib, imatinib, gefitinib, lapatinib, Lestaurtinib, Nilotinib, Semaxanib, Sorafenib, Sunitinib, and Vandetanib, and also cyclin-dependent kinase inhibitors such as Alvocidib and Seliciclib. Monoclonal antibody therapy is another strategy in which the therapeutic agent is an antibody which specifically binds to a protein on the surface of the cancer cells. Examples include the anti-HER2/neu antibody trastuzumab (HERCEPTIN®) typically used in breast cancer, and the anti-CD20 antibody rituximab and Tositumomab typically used in a variety of B-cell malignancies. Other exemplary antibodies include Cetuximab, Panitumumab, Trastuzumab, Alemtuzumab, Bevacizumab, Edrecolomab, and Gemtuzumab. Exemplary fusion proteins include Aflibercept and Denileukin diftitox. In some embodiments, the targeted therapy can be used in combination with a compound described herein, *e.g.,* a biguanide such as metformin or phenformin, preferably phenformin.

Targeted therapy can also involve small peptides as "homing devices" which can bind to cell surface receptors or affected extracellular matrix surrounding the tumor. Radionuclides which are attached to these peptides (*e.g.,* RGDs) eventually kill the cancer cell if the nuclide decays in the vicinity of the cell. An example of such therapy includes BEXXAR®.

In some embodiments, the additional cancer therapeutic agent is an immunotherapy agent. Cancer immunotherapy refers to a diverse set of therapeutic strategies designed to induce the subject's own immune system to fight the tumor. Contemporary methods for generating an immune response against tumors include intravesicular BCG immunotherapy for superficial bladder cancer, and use of interferons and other cytokines to induce an immune response in renal cell carcinoma and melanoma subjects.

Allogeneic hematopoietic stem cell transplantation can be considered a form of immunotherapy, since the donor's immune cells will often attack the tumor in a graft-versus-tumor effect. In some embodiments, the immunotherapy agents can be used in combination with a compound or composition described herein.

In some embodiments, the additional cancer therapeutic agent is a hormonal therapy agent. The growth of some cancers can be inhibited by providing or blocking certain hormones. Common examples of hormone-sensitive tumors include certain types of breast and prostate cancers. Removing or blocking estrogen or testosterone is often an important additional treatment. In certain cancers, administration of hormone agonists, such as progestogens may be therapeutically beneficial. In some embodiments, the hormonal therapy agents can be used in combination with a compound or a composition described herein.

Other possible additional therapeutic modalities include imatinib, gene therapy, peptide and dendritic cell vaccines, synthetic chlorotoxins, and radiolabeled drugs and antibodies.

### EXAMPLES

### General procedures for the preparation of 1,1-difluoro-3-isocyanocyclobutane

### Method A:

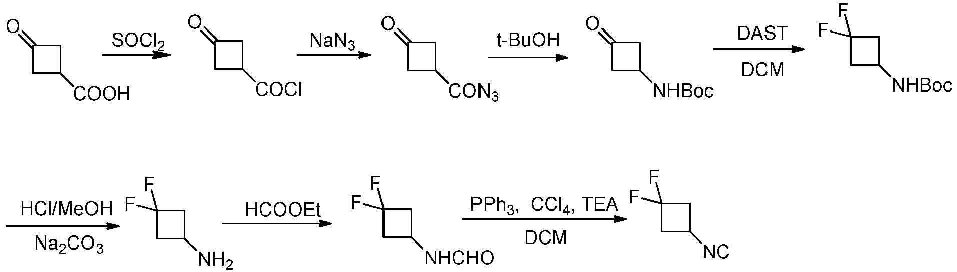

***Step A: tert-Butyl 3-oxocyclobutylcarbamate.*** To a solution of 3-oxocyclobutanecarboxylic acid (10 g, 88 mmol) in dry DCM (60 mL) at 0 °C, SOCl₂ (20 mL) was added dropwise. The mixture was heated to reflux for 1.5 h and then evaporated in vacuo. The resulting mixture was co-evaporated twice with toluene (2 x 8 mL) and the residue was dissolved in acetone (30 mL), followed by adding dropwise to a solution of NaN₃ (12 g, 185.0 mmol) in H₂O (35 mL) at 0°C. After addition, the mixture was stirred for another 1 h and then treated with ice (110 g). The resulting mixture was extracted with Et₂O (2 x100 mL). Combined organic layers were washed with brine, dried over anhydrous Mg₂SO₄ and concentrated to about 15 mL solution. Toluene (2 x 30 mL) was added into the residue and the mixture was co-evaporated twice to remove Et₂O (about 30 mL solution left each time to avoid explosion). The resulting toluene solution was heated to 90°C until the evolution of N₂ had ceased. 40 mL of t-BuOH was added into the reaction mixture and the resulting mixture was stirred overnight at 90°C. The mixture was cooled and concentrated. The residue was purified by flash column using petroleum ether / EtOAc (V:V, 7:1 to 5:1) as eluent to afford the desired product as a white solid (7.0 g, yield: 43%). MS: 186.1 (M+1)⁺.

***Step B: Tert-butyl 3,3-difluorocyclobutylcarbamate.*** To a solution of *tert*-butyl-3-oxocyclo-butylcarbamate (2.56 g, 111.07 mmol) in dry DCM (190 mL), DAST (diethylaminosulfur trifluoride) (41.0 mL, 222.14 mmol) was added dropwise at 0°C under the atmosphere of N₂. The mixture was then allowed to warm up to r.t and stirred overnight. The resulting mixture was slowly added into a pre-cooled saturated aq. NaHCO₃ solution and extracted with DCM (3 x 200 mL). Combined organic layers were washed with brine, dried over anhydrous MgSO₄, and concentrated in vacuo. The residue was purified by column chromatography using petroleum ether / EtOAc (V:V, 15:1) as eluent to afford the desired product (12.1 g, 52.6% yield). ¹H NMR (400 MHz, DMSO-d₆) : δ 4.79 (s, 1H), 4.07 (s, 1H), 2.98 (s, 2H), 2.58 - 2.29 (m, 2H), 1.46 (s, 9H). MS: 208.1 (M+1)⁺.

***Step C: N-(3,3-difluorocyclobutyl)formamide.*** To a solution of MeOH (170 mL) and CH₃COCl (65 mL), *tert*-butyl 3,3-difluoro-cyclobutylcarbamate (12.1 g, 58.42 mmol) was added in one portion dropwise at 0 °C. The reaction mixture was stirred at this temperature for 20 min, and then allowed to warm up to r.t. and stirred for another 1.5 hr. The reaction mixture was concentrated and dissolved in H₂O (200 mL). The resulting mixture was extracted by Et₂O (150 mL) and the aqueous layer was adjusted to pH=11 with solid Na₂CO₃ and extracted by DCM (2 x 150 mL). The combined organic layers were dried over anhydrous MgSO₄, filtered and concentrated in vacuo with cold-water bath (<20 °C). The residue was dissolved in HCOOEt (90 mL), and transferred into a sealed pressure tube. The reaction mixture was heated to 80°C and stirred overnight. The solvent was removed, and the residue was purified by column chromatography using petroleum ether / EtOAc (V:V, 1:1 to 1:3) as eluent to afford the desired product (4.08 g, 51.7% yield). MS: 136.1 (M+1)⁺.

***Step D: 1,1-Difluoro-3-isocyanocyclobutane.*** To a solution of *N*-(3,3-difluorocyclobutyl)-formamide (2.0 g, 14.81 mmol) and PPh₃ (4.27 g, 16.29 mmol) in DCM (35 mL) were added CCl₄ (1.43 mL, 14.81 mmol) and TEA (2.06 mL, 14.81 mmol). The reaction mixture was stirred at 45°C overnight under the atmosphere of N₂. The resulting mixture was evaporated in vacuo at 0°C. The residue was suspended in Et₂O (25 mL) at 0°C for 30 min and then filtered. The filtrate was evaporated to about 5 mL at 0 °C under reduced pressure. The residue was purified by column chromatography using Et₂O as eluent to afford the desired product (1.67 g, 76% yield) which was used directly in the next step.

### Method B:

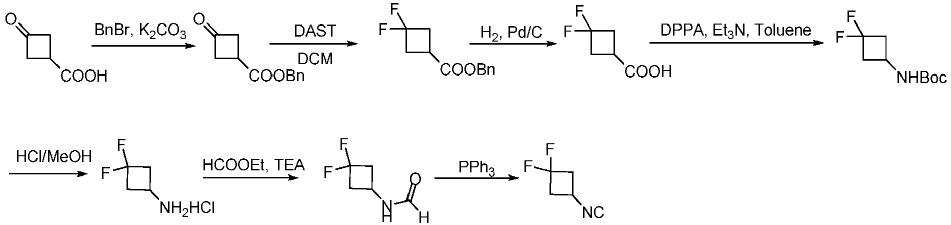

***Step A: Benzyl 3-oxocyclobutanecarboxylate.*** A mixture of 3-oxocyclobutanecarboxylic acid (5 g, 44 mmol), potassium carbonate (12 g, 88 mmol) and benzyl bromide (11.2 g, 66 mmol) in acetone (50 mL) was refluxed for 16 hr. Solvent was then removed under reduced pressure and the residue was partitioned between ethyl acetate and water. Combined organic layers were dried over anhydrous MgSO₄, filtered and concentrated. The residue was purified with silica gel chromatography eluting with a gradient of 100% hexane to 96% hexane / EtOAc to give the desired compound (8.1 g, 90% yield). ¹H NMR (400 MHz, CDCl₃): δ 7.45 - 7.27 (m, 5H), 5.19 (s, 2H), 3.55 - 3.36 (m, 2H), 3.33 - 3.11 (m, 3H).

***Step B: Benzyl 3,3-difluorocyclobutanecarboxylate.*** To a solution of benzyl 3-oxocyclobutanecarboxylate (1.23g, 6.03 mmol) in DCM (35 mL) was added DAST (0.8 mL, 6.03 mmol) dropwise under nitrogen. The mixture was stirred at r.t. for 16 h and then diluted with DCM. After washed successively in sequence with saturated sodium bicarbonate, IN aq. hydrochloric acid, and brine, the organic layer was dried over anhydrous sodium sulfate, filtered and concentrated. The crude product was purified by silica gel chromatography with 93% hexane / EtOAc as eluent to give the desired compound as an oil (1 g, 71% yield). ¹H NMR (400 MHz, CDCl₃): δ 7.47 - 7.27 (m, 5H), 5.16 (s, 2H), 3.09 - 2.95 (m, 1H), 2.90 - 2.60 (m, 4H).

***Step C: 3,3-Difluorocyclobutanecarboxylic acid.*** Benzyl 3,3-difluorocyclobutanecarboxylate (0.84 g, 3.72 mmol) was dissolved in ethanol (40 mL), and approximately 0.02 g palladium on activated carbon was added. The mixture was stirred at room temperature for 12 h under the atmosphere of H₂ and then filtered through a pad of Celite. The filtrates were concentrated and dried in vacuo to give the desired compound (0.46 g, 90% yield). ¹H NMR (400 MHz, CDCl₃): δ 3.16 - 2.55 (m, 5H).

***Step D: Tert-butyl 3,3-difluorocyclobutylcarbamate.*** Benzyl 3,3-difluorocyclobutanecarboxylic acid (3.7 g, 27.3 mmol), DPPA (7.87 g, 27 mmol) and TEA (2.87 g, 28.4 mmol) were dissolved in *t*-BuOH (25 mL). The mixture was refluxed for 5 h and then diluted with ethyl acetate (about 200 mL). The organic phase was washed twice with 5% citric acid and saturated sodium hydrogen carbonate respectively, dried over anhydrous Mg₂SO₄ and evaporated under reduced pressure. The residue was purified by silica gel chromatography with 50% hexane / EtOAc to give the desired product (3.96 g, 70% yield). MS: 208.1 (M+1)⁺.

***Step E: 3,3-Difluorocyclobutanamine hydrochloride.*** To a cold solution of MeOH (170 mL) and CH₃COCl (65 mL) was added *tert*-butyl 3,3-difluorocyclobutylcarbamate (12.1 g, 58.4 mmol) dropwise at 0 °C. After the completion of the addition, the mixture was stirred at this temperature for 20 min and then allowed to warm up to room temperature. The reaction mixture was stirred for another 1.5 h and then concentrated to give the crude product which was precipitated in ether to give the desired product as a white solid. MS: 108.1 (M+1)⁺. ***Step F: N-(3,3-difluorocyclobutyl)formamide.*** The mixture of 3,3-difluorocyclobutanamine hydrochloride (6.5 g, 60.7 mmol) and TEA (3 eq) in HCOOEt (90 mL) was stirred at 80 °C overnight in a sealed pressure tube. The solvent was removed under vacuo and the residue was purified by column chromatography with 50% petroleum ether / EtOAc to 25% petroleum ether / EtOAc to give the desired product (6.3 g, 70% yield). ¹H NMR (400 MHz, DMSO-d₆): δ 8.54 (s, 1H), 8.01 - 7.89 (m, 1H), 4.16 - 3.84 (m, 1H), 3.06 - 2.73 (m, 2H), 2.72 - 2.33 (m, 2H). MS: 136.1 (M+1)⁺.

***Step G: 1,1-Difluoro-3-isocyanocyclobutane.*** The compound was synthesized via the general procedure as the step D in method A set forth above.

### General procedures for the preparation of 1-fluoro-3-isocyanocyclobutane

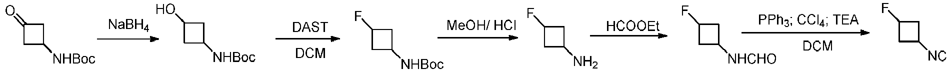

***Step A: Tert-butyl 3-hydroxycyclobutylcarbamate.*** To a solution of *tert*-butyl 3-oxocyclobutylcarbamate (2 g, 10.8 mmol, 2 eq) in EtOH (20 mL) was added NaBH₄ (204 mg, 1 eq) at 0 °C. The mixture was then allowed to warm to r.t. and stirred for 30 min. The mixture was concentrated in vacuo and the residue was purified by flash chromatography using petroleum ether / EtOAc (V:V, 2:1 to pure EtOAc) as eluent to afford the desired product as a white solid (1.9 g, 94% yield). MS: 188.1 (M+1)⁺.

***Step B: Tert-butyl 3-fluorocyclobutylcarbamate.*** To a solution *tert*-butyl 3-hydroxycyclobutyl - carbamate (1 g, 5.35 mmol) in dry DCM (20 mL) at -70 °C was added dropwise DAST (1g, 0.85 mL, 1.17 eq) under the atmosphere of N₂. The mixture was then slowly warmed to r.t. and stirred overnight. The resulting mixture was washed with diluted aq. NaHCO₃. The organic layer was dried over anhydrous Mg₂SO₄ and concentrated. The residue was purified by flash chromatography using petroleum ether / EtOAc (V:V, 20:1 to 2:1) as eluent to afford a white solid as the desired product (310 mg, 30.7% yield). MS : 190.1 (M+1)⁺.

***Step C: 3-Fluorocyclobutanamine.*** The compound was synthesized via the general procedure as the step E in method A set forth above.

***Step D: N-(3-fluorocyclobutyl)formamide.*** The compound was synthesized via the general procedure as the step F in method A set forth above. ¹H NMR (400 MHz, CDCl₃): δ 8.10 (s, 1H), 5.94-5.89 (brs, 1H), 5.32-5.25 (m, 0.5H), 5.18-5.11 (m, 0.5H), 4.63-4.42 (m, 1H), 2.76-2.62 (m, 2H), 2.44-2.31 (m, 2H).

***Step E: 1-Fluoro-3-isocyanocyclobutane.*** The compound was synthesized via the general procedure as the step G in method A set forth above.

### General procedures for the preparation of 1,1-difluoro-3-isocyanocyclopentane

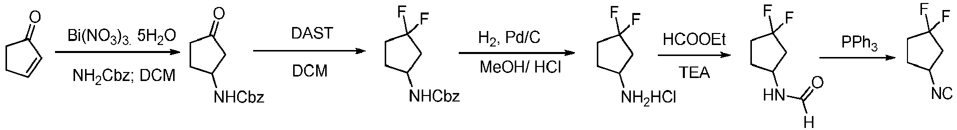

***Step A: Benzyl 3-oxocyclopentylcarbamate.*** To a solution of cyclopent-2-enone (400 mg, 4.88 mmol) and CbzNH₂ (740 mg, 4.88 mmol) in DCM (1 mL) was added Bi(NO₃)₃.5H₂O (240 mg, 0.49 mmol). The resulting syrup was vigorously stirred at r.t. overnight. DCM (8 mL) was then added to dilute the reaction mixture. The mixture was filtered and the filtrate was washed with saturated aq. NaHCO₃ (4 mL), dried over anhydrous MgSO₄ and concentrated. The residue was purified by column chromatography using PE/EA (V:V, 5:1 to 2:1) as eluent to afford the desired product as an oil product (470 mg, 41.3% yield). ¹H NMR (400 MHz, CDCl₃) : δ 7.46-7.26 (m, 5H), 5.15 (s, 2H), 5.05-4.95 (m, 1H), 4.30-4.25 (m, 1H), 2.70-2.60 (m, 1H), 2.45-2.05 (m, 5H). MS : 234.1 (M+1)⁺.

***Step B: Benzyl 3,3-difluorocyclopentylcarbamate.*** Benzyl 3-oxocyclopentylcarbamate (170 mg, 0.73 mmol) was dissolved in DCM (3 mL) and cooled to 0°C. DAST (488 mg, 3.03 mmol) was added dropwise at the temperature. The mixture was then allowed to warm up to r.t. and stirred overnight. The resulting mixture was added slowly to a pre-cooled saturated aq. NaHCO₃. The mixture was extracted with DCM (3 x 20 mL). Combined organic layers were washed with brine, dried over MgSO₄, filtered and concentrated in vacuo. The residue was purified by column chromatography using petroleum ether / EtOAc (V:V, 15:1 to 10:1) as eluent to afford the desired product as a white solid (110 mg, 59.1% yield). MS: 256.1 (M+1)⁺. ***Step C: 3,3-Difluorocyclopentanamine hydrochloride.*** A mixture of benzyl 3,3-difluorocyclopentylcarbamate (205 mg, 0.78 mmol) and 10% Pd/C (50 mg) in MeOH (5 mL) was stirred overnight under the atmosphere of H₂ (1 atm) at r.t. The reaction mixture was filtered and washed with MeOH. The filtrate was combined and treated with 30 mL of HCl/MeOH (2 N). The mixture was stirred for 20 min and evaporated in vacuo to afford the desired product as a white solid (125 mg, 99% yield). ¹H NMR (400 MHz, CDCl₃): δ 8.41 (brs, 3H), 3.70-3.61 (m, 1H), 2.40-1.90 (m, 5H). MS: 122.1 (M+1)⁺.

***Step D: N-(3,3-difluorocyclopentyl)formamide.*** The compound was synthesized via the general procedure for the synthesis of 1,1-Difluoro-3-isocyanocyclobutane as step F in method B set forth above. ¹H NMR (400 MHz, CDCl₃): δ 8.10 (d, *J* = 7.2 Hz, 1H), 6.93 (s, 1H), 4.59 - 4.30 (m, 1H), 2.58 - 1.59 (m, 6H).

***Step E: 1,1-Difluoro-3-isocyanocyclopentane.*** The compound was synthesized via the general procedure for the synthesis of 1,1-Difluoro-3-isocyanocyclobutane as step G in method B set forth above.

### General procedures for the preparation of (1R,2S)-1,2-difluoro-4-isocyanocyclopentane

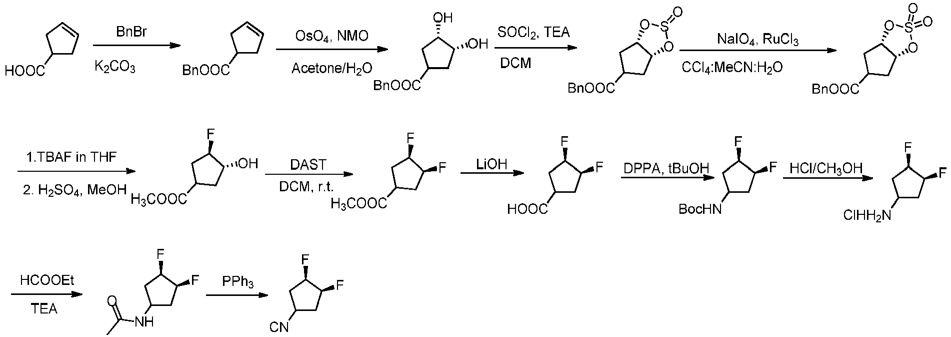

***Step A: Benzyl cyclopent-3-enecarboxylate.*** To a mixture of cyclopent-3-enecarboxylic acid (10 g, 89.3 mmol) and K₂CO₃ (24.6 g, 178.6 mmol) in acetone (150 mL) was added BnBr (16.8 g, 98.2 mmol). The mixture was stirred at 60 °C for 2 hr. The resulting mixture was filtered. The filtrate was concentrated and the residue was purified by flash chromatography using petroleum ether / EtOAc (V:V, 200:1) as eluent to give the desired product as a clear liquid (17 g, 95% yield).

***Step B: (3R,4S)-benzyl 3,4-dihydroxycyclopentanecarboxylate.*** To a mixture of benzyl cyclopent-3-enecarboxylate (15 g, 74.3 mmol) in acetone / water (V:V, 4:1, 150 mL) was added NMO (26 g, 223 mmol) and a catalytic amount of OsO₄ (472 mg, 1.9 mmol) at r.t.. The mixture was stirred at r.t. for 3 hr. The reaction was treated by addition of a few drops of saturated aq. NaHSO₃. The resulting mixture was concentrated and the residue was extracted by EtOAc (2 x 100 mL). The combined organic layers were washed with brine, dried over anhydrous Mg₂SO₄ and concentrated. The residue was purified by flash chromatography using petroleum ether / EtOAc (V:V, 5:1) as eluent to give the desired product as a white solid (13 g, 74% yield). ¹H NMR (400 MHz, CDCl₃): δ 7.36-7.26 (m, 5H), 5.09 (s, 2H), 4.16 (s, 2H), 3.20-3.18 (m, 1H), 2.94 (s, 2H), 2.12-1.97 (m, 4H).

***Step C: Benzyl (3a*α*,6a*α*)-(tetrahydro-4H-cyclopenta-1,3,2-dioxathiol-5-yl)carboxylate S-dioxide.*** To a mixture of (3*R*,4*S*)-benzyl 3,4-dihydroxycyclopentanecarboxylate (12 g, 50.8 mmol) and TEA (15.4 g, 152.4 mmol) in dry DCM (150 mL) at 0 °C was added SOCl₂ (9.1 g, 76.2 mmol). The mixture was stirred at this temperature for 10 min. The resulting mixture was washed with water and brine, dried over anhydrous Mg₂SO₄ and concentrated. The residue was purified by flash chromatography using petroleum ether / EtOAc (V:V, 6:1) as eluent to give the desired product as a white foam (11 g, 78% yield). ¹H NMR (400 MHz, CDCl₃): δ 7.39-7.26 (m, 5H), 5.43 (dd, *J* = 4.0, 1.2 Hz, 1H), 5.21 (dd, *J* = 4.0, 1.2 Hz, 1H), 5.13 (s, 2H), 3.66-3.60 (m, 0.5H), 3.05-3.00 (m, 0.5H), 2.53-2.48 (m, 1H), 2.37-2.32 (m, 1H), 2.19-2.01 (m, 2H).

***Step D: Benzyl (3a*α*,6a*α*)-(tetrahydro-4H-cyclopenta-1,3,2-dioxathiol-5-yl)carboxylate S,S-dioxide.*** To a mixture of benzyl (3a**α**,6a**α**)-(tetrahydro-4H-cyclopenta-1,3,2-dioxathiol-5-yl)-carboxylate *S*-dioxide (10 g, 35.5 mmol) in CCl₄/MeCN/H₂O (V:V:V, 1:1:1.5, 150 mL) was added NaIO₄ (15 g, 71 mmol) and RuCl₃ (184 mg, 8.9 mmol). The mixture was stirred at r.t. for 2.5 hr. The resulting mixture was partitioned between DCM and water. The organic layer was separated, washed with brine, dried over anhydrous Mg₂SO₄ and concentrated. The residue was purified by flash chromatography using petroleum ether / EtOAc (V:V, 5:1) as eluent to give the desired product as a white solid (8 g, 76% yield). ¹H NMR (400 MHz, CDCl₃): δ 7.40-7.26 (m, 5H), 5.35 (dd, J= 4.4, 1.6 Hz, 2H), 5.15 (s, 2H), 3.40-3.35 (m, 1H), 2.51-2.18 (m, 2H), 2.18-2.04 (m, 2H).

***Step E: (3R,4R)-methyl 3-fluoro-4-hydroxycyclopentanecarboxylate.*** To a mixture of (3aα,6aα)-(tetrahydro-4H-cyclopenta-1,3,2-dioxathiol-5-yl)carboxylate *S,S*-dioxide (8g, 26.8 mmol) in MeCN (100 mL) was added TBAF (1M sol. in THF, 40 mL) at r.t. The mixture was stirred at 90 °C for 1 h under an atmosphere of N₂. The mixture was concentrated. To the residue were added methanol (50 mL) and conc. H₂SO₄ (3 mL). The resulting mixture was stirred at 90 °C for another 1 hr. The mixture was concentrated and adjusted to pH 7 with saturated aq. NaHCO₃ and extracted with DCM. Combined organic layers were dried over anhydrous Mg₂SO₄ and concentrated. The residue was purified by flash chromatography using petroleum ether / EtOAc (V:V, 5:1) as eluent to give the desired product as a yellow oil (3.8 g, 76% yield).

***Step F: (3R,4S)-methyl 3,4-difluorocyclopentanecarboxylate.*** To a mixture of (3*R*,4*R*)-methyl 3-fluoro-4-hydroxycyclopentanecarboxylate (3.8 g, 23.4 mmol) in dry DCM (50 mL) was added DAST (1.2 eq) at 0 °C under the atmosphere of N₂. The reaction mixture was stirred at r.t. for 1.5 hr. The resulting mixture was washed with water. The organic layer was dried over anhydrous MgSO₄ and concentrated. The residue was purified by flash chromatography using petroleum ether / EtOAc (V:V, 10:1) as eluent to give the desired product as a yellow oil (1 g, 31% yield).

***Step G: (3R,4S)-3,4-difluorocyclopentanecarboxylic** acid.* To a solution of (3*R*,4*S*)-methyl 3,4-difluorocyclopentanecarboxylate (1 g, 6.1 mmol) in MeOH (5 mL) at 0 °C was added aq. LiOH (4 mL). The mixture was stirred at r.t. for 1 h and then adjusted to pH=2 with 2 N HCl. The resulting mixture was extracted with EtOAc (2 x 20 mL). The combined organic layers were dried over anhydrous MgSO₄ and concentrated. The residue was purified by flash chromatography using EtOAc as eluent to give the desired product as a yellow solid (400 mg, 44% yield).

***Step H: Tert-butyl (3R,4S)-3,4-difluorocyclopentylcarbamate.*** The compound was synthesized via the general procedure for the synthesis of 1,1-Difluoro-3-isocyanocyclobutane as step D in method B set forth above. ¹H NMR (400 MHz, CDCl₃): δ 4.97-4.93 (m, 1H), 4.86-4.81 (m, 2H), 4.16-4.14 (m, 1H), 2.44-2.34 (m, 2H), 1.93-1.84 (m, 2H), 1.34 (s, 9H).

***Step I: (3R,4S)-3,4-difluorocyclopentanamine hydrochloride.*** The compound was synthesized via the general procedure for the synthesis of 1,1-Difluoro-3-isocyanocyclobutane as step E in method B set forth above.

***Step J: N-((3R,4S)-3,4-difluorocyclopentyl)acetamide.*** The compound was synthesized via the general procedure for the synthesis of 1,1-Difluoro-3-isocyanocyclobutane as step F in method B set forth above. ¹H NMR (400 MHz, CDCl₃): δ 8.10 (s, 1H), 5.94 (brs, 1H), 5.04-4.99 (m, 1H), 4.91-4.86 (m, 1H), 4.60-4.55 (m, 1H), 2.49-2.36 (m, 2H), 1.99-1.88 (m, 2H). ***Step K: (1R,2S)-1,2-difluoro-4-isocyanocyclopentane.*** The compound was synthesized via the general procedure for the synthesis of 1,1-Difluoro-3-isocyanocyclobutane as step G in method B set forth above.

### General procedures for the preparation of 1,1-difluoro-4-isocyanocyclohexane

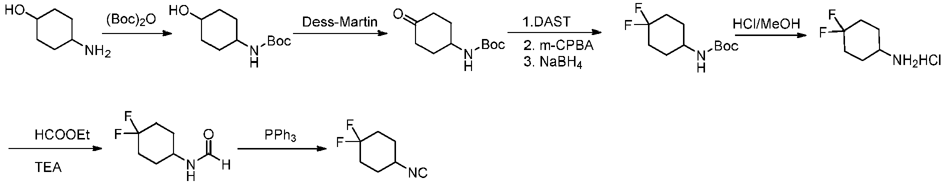

***Step A: Tert-butyl 4-hydroxycyclohexylcarbamate.*** To a solution of 4-aminocyclohexanol (23 g, 0.2 mol) and Et₃N (60 g, 0.6 mol) in THF (230 mL) was added (Boc)₂O (87 g, 0.4 mol). The resulting solution was stirred at r.t. overnight. The solvent was removed under reduced pressure and the residue was extracted with EtOAc (3 x 200 mL). The combined organic layers were washed with water (2 x 200 mL) and brine (200 mL), dried over anhydrous Na₂SO₄ and concentrated. The residue was purified by column chromatography on silica gel using DCM/ MeOH (V:V, 20:1) to afford the desired product as a white solid (34 g, 79% yield). MS: 216.2 (M+1)⁺.

***Step B: Tert-butyl 4-oxocyclohexylcarbamate.*** To a solution of *tert*-butyl 4-hydroxycyclohexylcarbamate (10.0 g, 46.5 mmol) in DCM (100 mL) was added Dess-Martin periodinane (39.4 g, 92.9 mmol) portionwise. The resulting solution was stirred at r.t. overnight and treated with aq. Na₂S₂O₃ solution and extracted with DCM (3 x 100 mL). The combined organic layers were washed with water (2 x 100 mL) and brine (100 mL), dried over anhydrous Na₂SO₄, and concentrated. The residue was purified by column chromatography on silica gel using petroleum ether / EtOAc (V:V, 10:1) to afford desired product as a white solid (7.0 g, 70% crude yield).

***Step C: Tert-butyl 4,4-difluorocyclohexylcarbamate.*** To a solution of *tert*-butyl 4-oxocyclohexylcarbamate (2.13 g, 10 mmol) in dry DCM (25 mL) was added DAST (2.58 g, 16 mmol) dropwise at -5 °C under nitrogen protection. After addition, the reaction mixture was stirred at r.t overnight. The reaction mixture was poured into ice water slowly and extracted by DCM (3 x 100 mL). The combined organic layers were washed with 2 N aq. NaHCO₃ and brine, dried over anhydrous Na₂SO₄, filtered and concentrated in vacuum. The residue was purified by column chromatography using petroleum ether / EtOAc (V:V, 5:1) as eluent to afford a mixture of the title compound (∼70%) and the byproduct *tert-butyl 4-fluorocyclohex-3-enylcarbamate* (∼30%) as a light-yellow solid.

To the above mixtures (2.52 g, 10.7 mmol) in DCM (25 mL) was added m-CPBA (2.20 g, 12.9 mmol) by portions at 0 °C while keeping the internal temperature below 5 °C. After addition, the reaction mixture was stirred at r.t. overnight. The saturated aq. Na₂S₂O₃ (8.0 mL) was added to the reaction mixture at 0 °C. The resulting mixture was stirred at this temperature for 40 min, and then extracted by DCM (3 x 5.0 mL). The combined organic layers were washed with brine, dried over anhydrous Na₂SO₄, and evaporated in vacuum. The residue was used directly in the next step without further purification

To the above residue in MeOH (15 mL) was added NaBH₄ (0.202 g, 5.35 mmol) at 0 °C. The reaction mixture was stirred at r.t overnight. Water (0.38 g) was added dropwise to quench the reaction at 0 °C. The resulting mixture was stirred at this temperature for 30 min, and concentrated in vacuo. The residue was purified by column chromatography using DCM as eluent to afford the pure compound as a white solid (1.4 g, 56% yield two steps). ¹H NMR (400 MHz, CDCl₃): δ 4.46 (s, 1H), 3.59 (s, 1H), 2.25 - 1.69 (m, 6H), 1.61 - 1.20 (m, 11H). MS: 236.2 (M+ 1)⁺.

***Step D: 4,4-Difluorocyclohexanamine hydrochloride.*** A mixture of *tert-butyl* 4,4-difluorocyclohexylcarbamate (6.0 g, 25.5 mmol) and 6 N HCl/MeOH (60 mL) was stirred at r.t. for 2 hr. The solvent was concentrated to give the crude product which was directly used in next step without further purification. ¹H NMR (400 MHz, CD₃OD): δ 4.89 (s, 2H), 3.32-3.26 (m, 1H), 2.14-2.01 (m, 4H), 2.02-1.85 (m, 2H), 1.74-1.65 (m, 2H). MS: 136.1 (M+1)⁺.

***Step E: N-(4,4-Difluorocyclohexyl)formamide.*** A mixture of 4,4-difluorocyclohexanamine (crude 3.4 g, 25.2 mmol), TEA (3 eq) and ethyl formate (35 mL) was stirred at 110 °C overnight in a sealed tube. The solvent was removed and the residue was purified by column chromatography using DCM / MeOH (V:V, 10:1) as eluent to afford the desired product (2.5 g, 61% yield in two steps). ¹H NMR (400 MHz, CDCl₃): δ 8.14 (s, 1H), 5.98 (s, 1H), 3.93 (m, 1H), 2.54 - 2.19 (m, 1H), 2.15 - 1.39 (m, 7H). MS: 164.1 (M+1)⁺.

***Step F: 1,1-Difluoro-4-isocyanocyclohexane.*** A mixture of *N*-(4,4-difluorocyclohexyl)-formamide (2.5 g, 15.3 mmol), PPh₃ (4.4 g, 16.8 mmol), CCl₄ (2.3 g, 15.1 mmol), Et₃N (1.5 g, 14.9 mmol) and DCM (50 mL) was heated to 45 °C and stirred overnight. The resulting mixture was evaporated in vacuo and the residue was suspended in Et₂O (125 mL) at 0 °C. The filtrate was concentrated and the residue was purified by column chromatography on silica gel eluted with Et₂O to afford the desired product as a yellow oil (1.9 g, 86% yield) which was used directly in the next step.

### General procedures for the preparation of 1-fluoro-4-isocyanocyclohexane

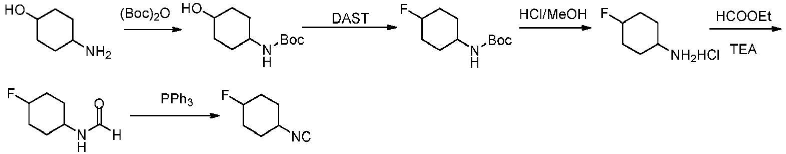

1-Fluoro-4-isocyanocyclohexane were synthesized via the general procedure for the preparation of 1,1-difluoro-4-isocyanocyclohexane set forth above.

### General procedures for the UGI reaction:

A mixture of aldehyde (3.5 mmol) and aniline (3.5 mmol) in MeOH (8 mL) was stirred at r.t. for 30 min. Then acid (3.5 mmol) was added and the reaction mixture was stirred for another 30 min. The isocyanide (3.5 mmol) was then added and the reaction mixture was stirred at r.t. overnight. The mixture was treated with H₂O, and the resulting mixture was partitioned between EtOAc and H₂O. The organic layer was separated, washed with brine, dried over anhydrous Na₂SO₄, and then concentrated. The residue was purified by silica gel column chromatography using DCM/MeOH as eluent to afford the desired product.

**EXAMPLE 1** The following analogs were synthesized via the general procedure for the UGI reaction set forth above, using the appropriate aldehyde, amine, carboxylic acid, and isocyanide.

### Compound 20

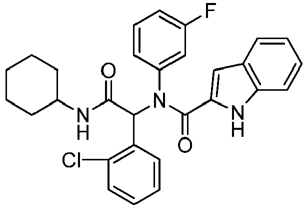

¹H NMR (400 MHz, CDCl₃): δ 9.45 (s, 1H), 7.40 - 7.36 (m, 3H), 7.27 - 7.12 (m, 5H), 7.09 - 6.96 (m, 3H), 6.68 (s, 1H), 5.70 (d, *J* = 8.0 Hz, 1H), 5.21 (d, J= 1.4 Hz, 1H), 3.95 - 3.85 (m, 1H), 2.07 - 1.96 (m, 4H), 1.92 - 1.90 (m, 1H), 1.69 - 1.54 (m, 2H), 1.39 - 1.33 (m, 2H), 1.25 - 1.01 (m, 3H). MS: 504.2 (M+1)⁺.

### Compound 32

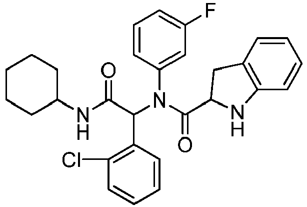

¹H NMR (400 MHz, CDCl₃): δ 7.73 (m, 1H), 7.36 (m, 1H), 7.24 - 6.83 (m, 7H), 6.74 - 6.34 (m, 3H), 5.78 - 5.59 (m, 1H), 4.42 - 4.32 (m, 1H), 3.92 - 3.72 (m, 1H), 3.32 - 3.22 (m, 1H), 2.99 - 2.80 (m, 1H), 2.04 - 1.82 (m, 2H), 1.78 - 1.53 (m, 4H), 1.41 - 1.29 (m, 2H), 1.16 - 1.00 (m, 2H). MS: 506.2 (M+1)⁺.

### Compound 24

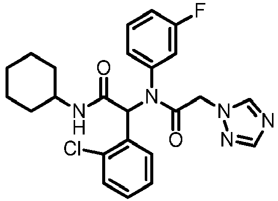

¹H NMR (400 MHz, CDCl₃): δ 8.20 (s, 1H), 7.90 (s, 1H), 7.76 (m, 1H), 7.36 (d, *J* = 8.0 Hz, 1H), 7.20 - 7.16 (m, 1H), 7.05 - 6.94 (m, 3H), 6.73 (m, 1H), 6.44 (s, 1H), 5.72 (d, *J* = 7.9 Hz, 1H), 4.77 (m, 2H), 3.88 - 3.80 (m, 1H), 1.99 - 1.88 (m, 2H), 1.76 - 1.56 (m, 3H), 1.36 - 1.33 (m, 2H), 1.21 - 0.95 (m, 3H). MS: 470.2 (M+1)⁺.

### Compound 34

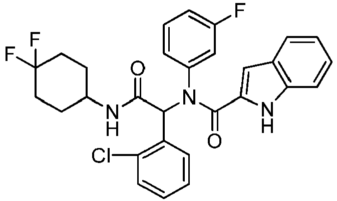

¹H NMR (400 MHz, DMSO-d₆): δ 11.66 (s, 1H), 8.47 (m, 1H), 7.50 - 6.74 (m, 11H), 6.72 - 6.44 (m,1H), 5.10 (m, 1H), 3.95 (m, 1H), 2.41 - 1.63 (m, 6H), 1.63 - 0.99 (m, 2H). MS: 539.1 (M+1)⁺.

### Compound 45

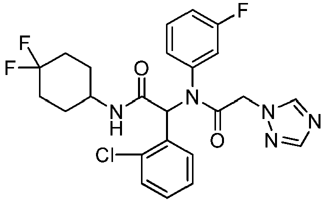

¹H NMR (400 MHz, DMSO-d₆): δ 8.37 (m, 2H), 7.95 (s, 1H), 7.89 - 7.56 (m, 1H), 7.45 - 6.72 (m, 7H), 6.31 (s, 1H), 4.88 (m, 2H), 3.88 (m, 1H), 2.25 (m, 1H), 1.94 (m, 5H), 1.48 (m, 1H), 1.26 (m, 1H). MS: 506.1 (M+1)⁺.

### Compound 37

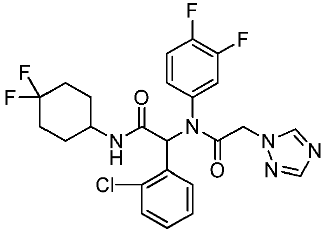

¹H NMR (400 MHz, CDCl₃): δ 8.19 (s, 1H), 7.92 (s, 1H), 7.47 - 7.06 (m, 4H), 7.06 - 6.88 (m, 2H), 6.38 (s, 1H), 5.58 (s, 1H), 4.75 (m, 2H), 4.13 - 3.77 (m, 1H), 2.03 (m, 4H), 1.65 - 1.14 (m, 6H). MS: 524.1 (M+1)⁺.

### Compound 46

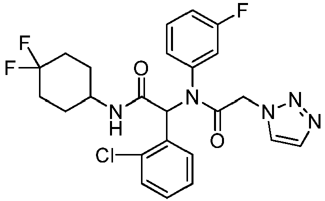

¹H NMR (400 MHz, DMSO-d₆): δ 8.42 (m, 1H), 8.12 - 7.63 (m, 3H), 7.19 (m, 7H), 6.31 (s, 1H), 5.06 (m, 2H), 3.86 (m, 1H), 2.24 (m, 1H), 1.86 (m, 5H), 1.59 - 0.97 (m, 2H). MS: 506.1 (M+1)⁺.

### Compound 49

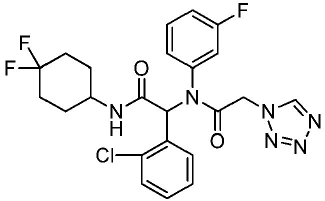

¹H NMR (400 MHz, DMSO-d₆): δ 9.34 (s, 1H), 8.45 (m, 1H), 7.84 (brs, 1H), 7.51 - 6.68 (m, 7H), 6.52 - 6.07 (m, 1H), 5.41 (m, 1H), 5.13 (m, 1H), 3.88 (m 1H), 2.24 (s, 1H), 2.08 - 1.06 (m, 7H). MS: 507.1(M+1)⁺.

### Compound 93

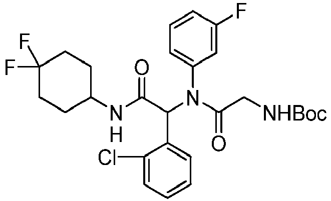

¹H NMR (400 MHz, CDCl₃): δ 7.36 (d, *J* = 7.9 Hz, 2H), 7.26 - 7.08 (m, 2H), 7.08 - 6.84 (m, 3H), 6.42 (s, 1H), 5.70 (d, *J* = 7.7 Hz, 1H), 5.39 (s, 1H), 4.66 - 3.90 (m, 2H), 3.66 (m, 2H), 2.58 - 1.70 (m, 7H), 1.49 (s, 1H), 1.43 (s, 9H). MS: 554.2 (M+1)⁺.

### Compound 44

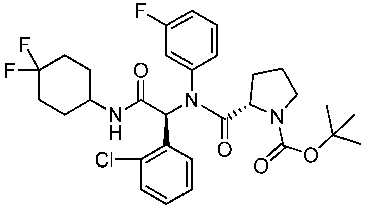

¹H NMR (400 MHz, CDCl₃): δ 7.61 (m, 1H), 7.33 (m, 1H), 7.24 - 6.83 (m, 5H), 6.54 (m, 1H), 5.77 (m, 1H), 5.20 - 4.73 (m, 2H), 4.48 - 3.82 (m, 4H), 2.53 - 2.19 (m, 2H), 2.04-1.81 (m, 9H), 1.71-1.43 (m, 9 H). MS: 594.2 (M+1)⁺.

### Compound 48

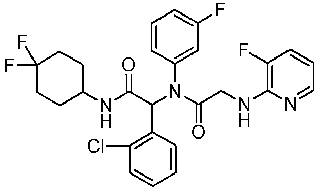

¹H NMR (400 MHz, DMSO-d₆): δ 8.36 (d, *J* = 7.1 Hz, 1H), 7.78 (d, *J* = 4.9 Hz, 1H), 7.50 - 6.79 (m, 7H), 6.61 - 6.41 (m, 2H), 6.32 (m, 1H), 4.11 - 3.73 (m, 2H), 3.63 (m, 1H), 2.35 - 1.05 (m, 8H). MS: 548.2 (M+1)⁺.

### Compound 40

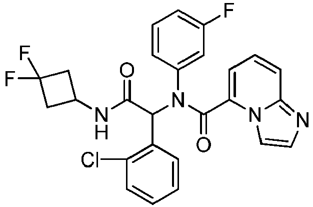

¹H NMR (400 MHz, CDCl₃) : δ 9.63 (d, *J* = 7.1 Hz, 1H), 7.62 (d, *J* = 8.9 Hz, 1H), 7.42 - 7.37 (m, 2H), 7.24 - 7.00 (m, 7H), 6.65 (s, 1H), 6.49 (s, 1H), 6.11 (d, *J* = 6.2 Hz, 1H), 4.37 (m, 1H), 3.16 - 2.96 (m, 2H), 2.64 - 2.45 (m, 2H). MS: 513.2 (M+1)⁺.

### Compound 41

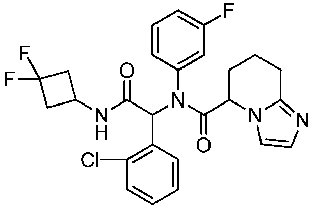

¹H NMR (400 MHz, CDCl₃): δ 7.72 (m, 1H), 7.37 (m, 1H), 7.21 (m, 1H), 7.10 - 6.42 (m, 8H), 4.61 (m, 1H), 4.30 (m, 1H), 2.98 (m, 2H), 2.80 (m 2H), 2.58 - 2.27 (m, 2H), 2.02 (m, 2H), 1.65 - 1.51 (m, 2H). MS: 517.2 (M+1)⁺.

### Compound 47

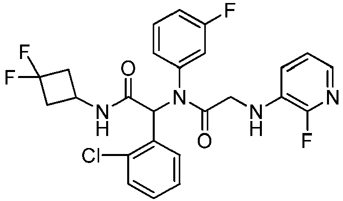

¹H NMR (400 MHz, DMSO-d₆): δ 8.87 (d, *J* = 5.9 Hz, 1H), 7.96 - 7.53 (m, 1H), 7.12 (m, 9H), 6.33 (s, 1H), 5.86 (s, 1H), 4.11 (m, 1H), 3.82 (m, 1H), 3.50 (m 1H), 2.94 (m, 2H), 2.34 (m, 2H). MS: 521.1(M+1)⁺.

### Compound 59

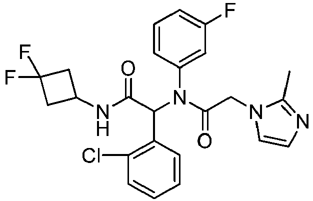

¹H NMR (400 MHz, DMSO-d₆): δ 8.91 (d, *J* = 6.3 Hz, 1H), 7.87 - 7.51 (m, 1H), 7.51 - 6.73 (m, 9H), 6.32 (s, 1H), 4.83 (d, *J* = 17.0 Hz, 1H), 4.54 (d, *J* = 17.6 Hz, 1H),4.12 (m, 1H), 2.94 (m, , 2H), 2.40-2.31 (m, 2H), 2.20 (s, 3H). MS: 491.1(M+1)⁺.

### Compound 36

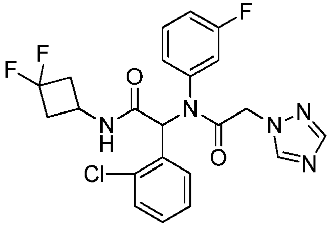

¹H NMR (400 MHz, DMSO-d₆): δ 8.86 (d, *J* = 6.1 Hz, 1H), 8.39 (s, 1H), 7.93 (s, 1H), 7.42 (d, *J* = 8.0 Hz, 1H), 7.18 (m, 3H), 7.07 (m, 2H), 6.87 (m, 2H), 6.28 (s, 1H), 4.98 (m, 1H), 4.73 (m, 1H), 4.20 - 3.97 (m, 1H), 2.88 (m, 2H), 2.52 - 2.26 (m, 2H).MS: 477.1 (M+1)⁺.

### Compound 86

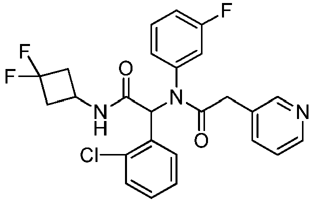

¹H NMR (400 MHz, DMSO-d₆): δ 8.86 (d, *J* = 6.1 Hz, 1H), 8.40 (d, *J* = 4.7 Hz, 1H), 8.21 (s, 1H), 7.58 - 6.81 (m, 10H), 6.35 (s, 1H), 4.17 - 4.08 (m, 1H), 3.53 (m, 2H), 3.01 - 2.86 (m, 2H), 2.58 (m, 1H), 2.42 (m, 1H). MS: 488.1 (M+1)⁺.

### Compound 64

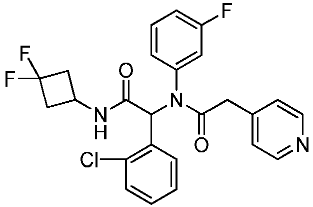

¹H NMR (400 MHz, CDCl₃): δ 8.46 (d, *J* = 6.0 Hz, 2H), 7.50 - 7.30 (m, 2H), 7.23 - 6.86 (m, 6H), 6.47 (d, *J* = 8.7 Hz, 2H), 4.30 (s, 1H), 3.49 (s, 2H), 3.11 - 2.87 (m, 2H), 2.53 (s, 1H), 2.41 - 2.25 (m, 1H). MS : 488 (M+1)⁺.

### Compound 39

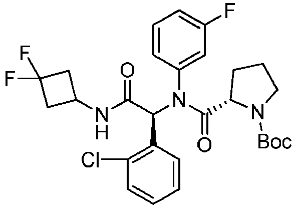

¹H NMR (400 MHz, CDCl₃) : δ 7.59 (s, 0.42H), 7.30 (t, *J* = 8.1 Hz, 1H), 7.14 (m, 2H), 7.06 - 6.95 (m, 2H), 6.88 (dd, *J* = 9.5, 7.2 Hz, 1.43H), 6.78 - 6.60 (m, 0.43H), 6.51 (s, 0.58H), 6.34 - 6.10 (m, 1H), 4.31 (s, 1H), 4.24 - 4.13 (m, 1H), 3.46 (m, 2H), 3.00 (m, 2H), 2.73 - 2.34 (m, 2H), 2.10 - 1.74 (m, 4H), 1.47 (m, 9H). MS: 566.2 (M+1)⁺.

### Example 2. Preparation of N-cyclohexyl-2-(2-(4,5-dichloro-1H-imidazol-1-yl)-N-(3,4-difluorophenyl)acetamido)-2-(2,4-difluorophenyl)acetamide.

Compound **30** was prepared according to the following scheme, using the following protocol.

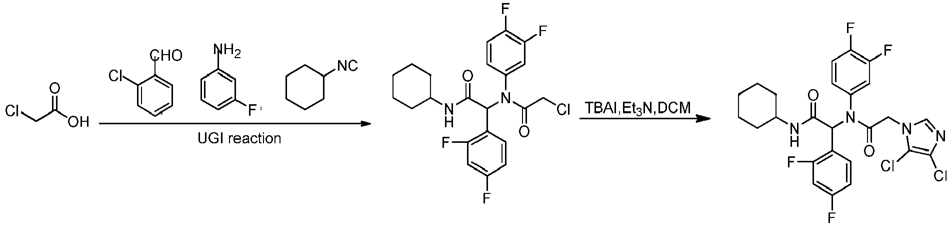

***Step A: 2-chloro-N-(2-(cyclohexylamino)-1-(2,4-difluorophenyl)-2-oxoethyl)-N-(3,4-difluorophenyl)acetamide.*** A mixture of 2,4-difluorobenzaldehyde (500 mg, 3.5 mmol) and 3,4-difluoroaniline (455 mg, 3.5 mmol) in MeOH (8 mL) was stirred at r.t. for 30 min. 2-chloroacetic acid (294 mg, 3.5 mmol) was added and the reaction mixture stirred for 10 min. Cyclohexyl isocyanide (382 mg, 3.5 mmol) was then added and the reaction mixture was stirred at r.t. overnight. The resulting mixture was partitioned between EA and H₂O. The organic layer was separated, washed with brine, dried over Na₂SO₄, filtered and concentrated. The residue was purified by column chromatography to afford the desired product (1.1 g, 67% yield) as a white solid. MS: 457.1 (M+1)⁺.

***Step B: Compound 30.*** To a solution of 2-chloro-N-(2-(cyclohexylamino)-1-(2,4-difluorophenyl)-2-oxoethyl)-N-(3,4-difluorophenyl)acetamide (200 mg, 0.44 mmol), 4,5-dichloro-1H-imidazole (121 mg, 0.88 mmol) and Et₃N (178 mg, 1.76 mmol) in DCM (6 mL) was added TBAI (162 mg, 0.44 mmol). The mixture was stirred at r.t. overnight. The resulting mixture was partitioned between DCM and H₂O. The organic layer was separated, washed with brine, dried over anhydrous Na₂SO₄, filtered and concentrated. The residue was purified by gel column to afford the desired product as a yellow solid (160 mg, 64% yield). ¹H NMR (400 MHz, DMSO-d₆): δ 8.22 (s, 1H), 7.90 (m, 1H), 7.69 (s, 1H), 7.58 - 7.17 (m, 2H), 7.11 - 6.65 (m, 3H), 6.22 (s, 1H), 4.68 (m, 2H), 3.60 (m, 1H), 1.63 (m, 5H), 1.35 - 0.80 (m, 6H). MS: 571.1 (M+1)⁺.

The following analogs were synthesized via the procedure set forth above, using the appropriate aldehyde, amine, carboxylic acid, isocyanide and halo-substituted aromatic ring or heteroaromatic ring using the reagents and solvents set forth above; and purified via various methods including TLC, Chromatography, HPLC or chiral HPLC.

### Compound 21

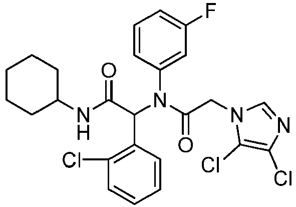

¹H NMR (400 MHz, CDCl₃) : δ 7.82 - 7.80 (m, 1H), 7.38 (d, *J* = 4.3 Hz, 2H), 7.23 - 7.15 (m, 1H), 7.06 - 6.94 (m, 3H), 6.44 (s, 1H), 5.59 (d, *J* = 7.8 Hz, 1H), 4.49 - 4.35 (m, 2H), 3.88 - 3.79 (m, 1H), 1.94 (m, 2H), 1.70 (m, 2H), 1.43 - 1.22 (m, 3H), 1.21 - 0.95 (m, 3H). MS: 537.1 (M+1)⁺.

### Compound 22

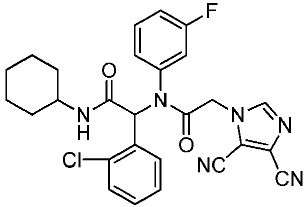

¹H NMR (400 MHz, CDCl₃) : δ 7.75 (s, 1H), 7.40 (d, *J* = 7.9 Hz, 1H), 7.25 - 7.17 (m, 1H), 7.06 - 6.96 (m, 3H), 6.89 - 6.53 (m, 1H), 6.45 (s, 1H), 5.54 (d, *J* = 7.9 Hz, 1H), 4.66 (s, 2H), 3.84 (m, 1H), 1.94 (m, 2H), 1.79 - 1.71 (m, 1H), 1.67 - 1.56 (m, 2H), 1.42 - 1.29 (m, 2H), 1.22 - 0.96 (m, 3H). MS: 519.1 (M+1)⁺.

### Compound 27

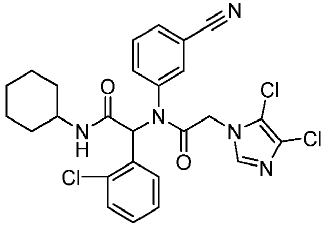

¹H NMR (400 MHz, CDCl₃) : δ 8.35 (m, 2H), 7.92 - 6.67 (m, 7H), 6.33 (s, 1H), 4.67 (m, 2H), 3.63 (m, 1H), 1.63 (m, 5H), 1.30 - 1.00 (m, 5H). MS: 544.1 (M+1)⁺.

### Compound 28

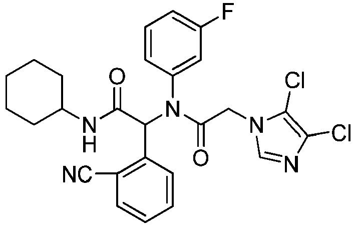

¹H NMR (400 MHz, DMSO-d₆): δ 8.32 (d, *J* = 7.4 Hz, 1H), 7.92 - 7.64 (m, 2H), 7.50 - 7.18 (m, 3H), 7.09 (m, 2H), 6.78 (m, 1H), 6.32 (s, 7H), 4.67 (m, 2H), 3.73 - 3.50 (m, 1H), 1.82 - 1.39 (m, 5H), 1.37 - 0.73 (m, 5H). MS: 528.1 (M+1)⁺.

### Compound 29

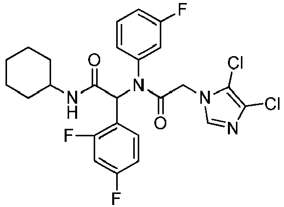

¹H NMR (400 MHz, DMSO-d₆): δ 8.17 (d, *J* = 7.4 Hz, 1H), 7.68 (m, 2H), 7.29 (m, 1H), 7.14 (m, 2H), 6.86 (m, 3H), 6.21 (s, 1H), 4.61 (m, 2H), 3.71 - 3.42 (m, 1H), 1.61 (m, 5H), 1.33 - 0.66 (m, 5H). MS: 538.1 (M+1)⁺.

### Compound 35

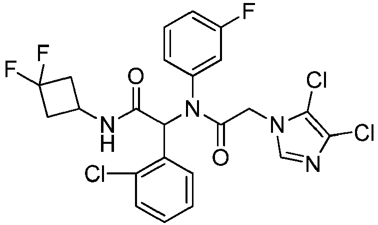

¹H NMR (400 MHz, DMSO-d₆) : δ 8.87 (d, *J* = 5.9 Hz, 1H), 7.70 (m, 2H), 7.44 (d, *J* = 7.9 Hz, 1H), 7.22 (m, 2H), 7.09 (m, 2H), 6.86 (m, 2H), 6.31 (s, 1H), 4.65 (m, 2H), 4.09 (m, 1H), 3.05 - 2.81 (m, 2H), 2.59 - 2.19 (m, 2H). MS: 544.1 (M+1)⁺.

### Compound 33

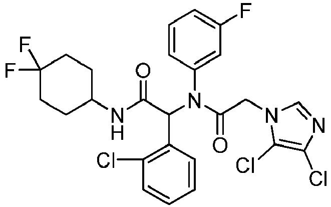

¹H NMR (400 MHz, CDCl₃) : δ 7.90 - 7.59 (m, 1H), 7.39 - 7.31 (m, 2H), 7.23 - 7.09 (m, 2H), 7.03 - 6.91 (m, 3H), 6.42 (d, *J* = 5.7 Hz, 1H), 5.89 - 5.75 (m, 1H), 4.40 (m, 2H), 4.19 - 3.87 (m, 1H), 2.52 - 1.90 (m, 5H), 1.89 - 1.72 (m, 2H), 1.48 - 1.29 (m, 1H). MS: 573.2 (M+1)⁺.

### Compound 38

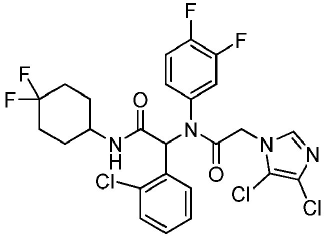

¹H NMR (400 MHz, CDCl₃) : δ 7.38 (m, 3H), 7.24 (m, 1H), 7.01 (m, 3H), 6.39 (s, 1H), 5.64 (m, 1H), 4.39 (m, 3H), 3.94 (m, 1H), 1.90 (m, 4H), 1.64 (m, 2H), 1.73 - 1.01 (m, 2H). MS: 591.1 (M+1)⁺.

### Compound 87

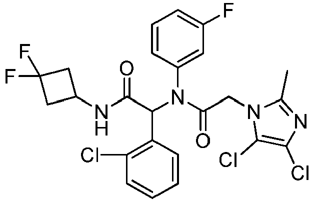

¹H NMR (400 MHz, DMSO-d₆): δ 8.88 (m, 1H), 8.06 - 7.54 (m, 1H), 7.51 - 6.59 (m, 7H), 6.31 (s, 1H), 4.66-4.57 (m, 1H), 4.44-4.38 (m, 1 H), 4.16-4.09 (m, 1H), 2.99-2.90 (m, 2H), 2.48 - 2.34 (m, 2H), 2.22 (s, 3H). MS: 559.1(M+1)⁺.

### Compound 72

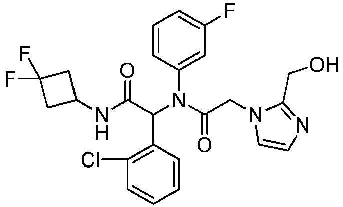

¹H NMR (400 MHz, CD₃OD) : δ 7.86 - 7.60 (m, 1H), 7.41 (d, *J* = 7.9 Hz, 1H), 7.22 (t, *J* = 7.7 Hz, 2H), 7.13 (s, 1H), 7.10 - 6.93 (m, 5H), 6.51 (s, 1H), 4.95 (s, 1H), 4.75 - 4.70 (m, 1H), 4.63 (s, 2H), 4.25 - 4.23 (m, 1H), 3.02 - 2.84 (m, 2H), 2.63 - 2.46 (m, 2H). MS: 507.2 (M+1)⁺.

### Compound 11

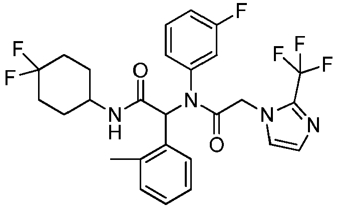

¹H NMR (400 MHz, DMSO-d6): δ 8.17-8.13 (m, 1H), 7.89-7.86 (m, 1H), 7.41-7.07 (m, 6H), 6.87 (t, 1H, J=7.6 Hz), 6.70-6.69 (m, 1H), 6.51-6.50 (m, 1H), 6.20 (s,1H), 4.82-4.76 (m,2H), 3.84 (s, 1H), 2.38 (s,3H), 2.01-1.76 (m, 6H), 1.51-1.43 (m, 1H), 1.31-1.23 (m, 1H); MS: 552.6 (M+ 1)⁺.

### Compound 18

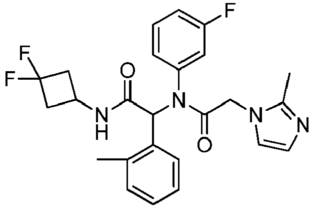

¹H NMR (400 MHz, CDCl3): δ 7.54 (br, 1H), 7.07 (m, 2H), 6.89 (m, 3H), 6.65 (s, 1H), 6.40 (s, 1H), 6.30 (m, 3H), 4.31 (s, 2H), 4.17 (m, 1), 2.85 (m, 2H), 2.30-2.17 (m, 7H), 1.92(m, 2H); MS: 470.9 (M+1)⁺.

### Example 3. Preparation of N-(1-(2-chlorophenyl)-2-(cyclohexylamino)-2-oxoethyl)-N-(3-fluorophenyl)-1,2,3,4-tetrahydroquinoline-2-carboxamide.

Compound **31** was prepared according to the following scheme, using the following protocol.

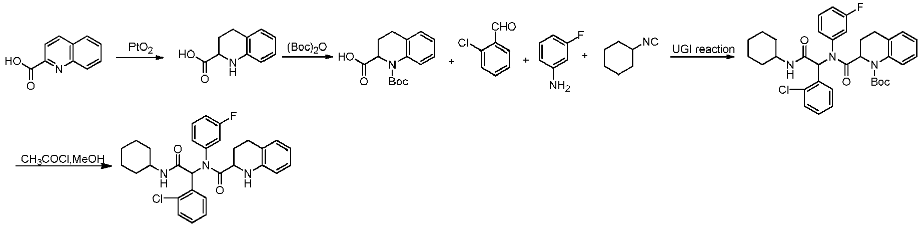

***Step A: 1,2,3,4-tetrahydroquinoline-2-carboxylic acid.*** A solution of quinoline-2-carboxylic acid (500 mg, 2.9 mmol) and platinum oxide (32 mg, 0.14 mmol) in MeOH (6 mL) was stirred under hydrogen atmosphere for 2.5 hr. The mixture was filtered and the filtrate was concentrated to afford the crude product as an oil (500 mg, 97% yield).

***Step B: 1-(tert-butoxycarbonyl)-1,2,3,4-tetrahydroquinoline-2-carboxylic acid.*** To a solution of triethylamine (1.1 g, 11.2 mmol) in DCM (5 mL) was added to 1,2,3,4-tetrahydro -quinoline-2-carboxylic acid (500 mg, 2.82 mmol) in DCM (1 mL), followed by addition of di-*tert*-butyl dicarbonate (924 mg, 4.24 mmol). The mixture was stirred at r.t. overnight and treated with water. The resulting mixture was extracted with DCM (2 x 20 mL). The combined organic layer was dried over anhydrous Na₂SO₄ and concentrated to afford the desired product (230 mg, 29% yield).

***Step C: tert-Butyl 2-((1-(2-chlorophenyl)-2-(cyclohexylamino)-2-oxoethyl)(3-fluorophenyl)-carbamoyl)-3,4-dihydroquinoline-1(2H)-carboxylate.*** The compound was synthesized via the general procedure for UGI reaction set forth above.

***Step D: Compound 31.*** To a solution of *tert*-butyl 2-((1-(2-chlorophenyl)-2-(cyclohexyl-amino)-2-oxoethyl)(3-fluorophenyl)carbamoyl)-3,4-dihydroquinoline-1(2*H*)-carboxylate (120 mg, 0.19 mmol) in MeOH (1 mL) was added to acetyl chloride (1 mL) in MeOH (2 mL) at 0 °C and stirred at r.t. for 1 hr. The mixture was evaporated and the residue was dissolved in H₂O and then aq. NaOH (1N) was added to the solution to adjust pH to 9. The solution was extracted with EtOAc. The combined organic layers were dried over anhydrous Na₂SO₄ and concentrated. The residue was purified by prep-TLC to afford the desired product as a white solid (46 mg, 47% yield). ¹H NMR (400 MHz, CDCl₃): δ 7.73 (s, 1H), 7.42 - 7.25 (m, 2H), 7.21 - 7.15 (m, 1H), 7.15 - 7.07 (m, 1H), 6.99 (m, 3H), 6.92 - 6.82 (m, 2H), 6.65 - 6.53 (m, 2H), 6.39 (s, 1H), 5.70 (m, 1H), 3.94 (m, 1H), 3.82 (m, 1H), 2.75 - 2.62 (m, 1H), 2.54 - 2.38 (m, 1H), 1.96 (m, 2H), 1.86 (m, 1H), 1.71 (m, 2H), 1.39 - 1.21 (m, 4H), 1.17 - 1.03 (m, 2H). MS: 520.2 (M+1)⁺.

### Example 4. Preparation of N-(3-(1,3,4-oxadiazol-2-yl)phenyl)-N-(1-(2-chlorophenyl)-2-(cyclohexylamino)-2-oxoethyl)-2-(2-methyl-1H-imidazol-1-yl)acetamide.

Compound **25** was prepared according to the following scheme, using the following protocol.

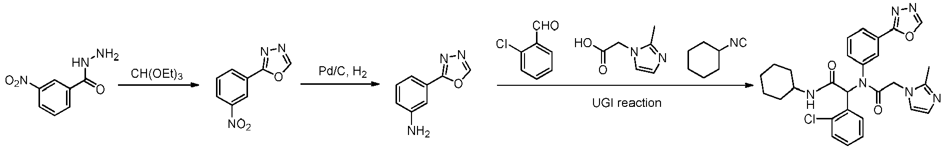

***Step A: 2-(3-nitrophenyl)-1,3,4-oxadiazole.*** A mixture of 3-nitrobenzohydrazide (1.8 g, 10 mmol), triethoxymethane (5 mL) and 0.1 mL CH(OEt)₃ was stirred at 100 °C for 3 hr. The mixture was concentrated to small volume and cooled down to give a colorless crystal. The solid was collected by filtration and dried under vacuum to afford the desired compound (1.5 g, 78.9% yield). MS: 192.2 (M+1)⁺.

***Step B: 3-(1,3,4-oxadiazol-2-yl)aniline.*** A mixture of 2-(3-nitrophenyl)-1,3,4-oxadiazole (1 g, 5.2 mmol) and 10% palladium on carbon (0.1 g) in methanol (20 mL) was stirred at r.t. for 16 h under the atmosphere of hydrogen. The mixture was filtered through Celite and the filtrate was concentrated to give the desired compound (840 mg, quant.). MS: 162.2 (M+1)⁺.

***Step C: Compound 25.*** 2-Chlorobenzaldehyde (112 mg, 0.805 mmol), 3-(1,3,4-oxadiazol-2-yl)aniline (130 mg, 0.805 mmol), 2-(2-methyl-1H-imidazol-1-yl)acetic acid (120 mg, 0.805 mmol) and isocyanocyclohexane (87.9 mg, 0.1 mL, 0.805 mmol) were used for the UGI reaction and after purification gave the desired compound (20 mg, 5% yield). ¹H NMR (400 MHz, CDCl₃): δ 8.66-8.40 (m, 2H), 8.21-7.92 (m, 2H), 7.55-7.29 (m, 2H), 7.23-6.90 (m, 4H), 6.80 (d, *J* = 0.8 Hz, 1H), 6.46 (s, 1H), 5.95-5.28 (brs, 1H), 4.37 (dd, *J* = 1.6, 2.8 Hz, 2H), 3.81-3.78 (m, 1H), 2.29 (s, 3H), 1.95-0.99 (m, 10H). MS: 533.2 (M+1)⁺.

### Example 5. Preparation of 2-(2-chlorophenyl)-N-(3,3-difluorocyclobutyl)-2-(N-(3-fluorophenyl)-2-(2-oxooxazolidin-3-yl)acetamido)acetamide

Compound **60** was prepared according to the following scheme, using the following protocol.

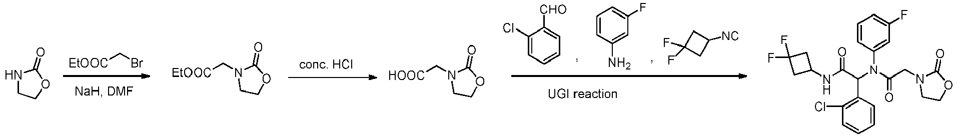

***Step A: Ethyl 2-(2-oxooxazolidin-3-yl)acetate.*** To a solution of oxazolidin-2-one (1.0 g, 11.49 mmol) in dry DMF (10 mL) was added ethyl 2-bromoacetate (1.91 g, 11.49 mmol) and sodium hydride (0.46 g, 11.49 mmol) at r.t. The mixture was stirred at r.t. for 1 hr. The resulting mixture was partitioned between ethyl acetate and brine. The aqueous layer was extracted by ethyl acetate three times (3 x 30 mL). The combined organic layers were dried over anhydrous sodium sulfate, filtered, and concentrated. The crude product was purified by flash chromatography using DCM/MeOH (V:V, 100:1) as eluent to give desired compound (750 mg, 37.7% yield). MS: 174.1 (M+1)⁺.

***Step B: 2-(2-oxooxazolidin-3-yl)acetic acid.*** A mixture of ethyl 2-(2-oxooxazolidin-3-yl)acetate (200 mg, 1.16 mmol) in conc. HCl (2 mL) was stirred at room temperature (r.t.) overnight. The mixture was concentrated and the residue was dissolved in acetone and dried over anhydrous Na₂SO₄. The acetone was evaporated to give the desired compound (150 mg, 89.5% yield). MS: 146.0 (M+1)⁺.

***Step C: Compound 60.*** A mixture of 2-chlorobenzaldehyde (0.11 mL, 1 mmol) and 3-fluoroaniline (0.1 mL, 1 mmol), 2-(2-oxooxazolidin-3-yl)acetic acid (150 mg, 1 mmol) and 3,3-difluoroisocyanobutane (80% purity, 176 mg, 1.2 mmol) were used for UGI reaction to give the desired compound (216.7 mg, 29% yield). ¹H NMR (400 MHz, DMSO-d₆): δ 8.88 (d, *J* = 6.2 Hz, 1H), 7.83 - 7.37 (m, 2H), 7.34 - 7.16 (m, 2H), 7.08 (m, 2H), 6.92 - 6.56 (m, 2H), 6.32 (s, 1H), 4.32 - 4.21 (m, 2H), 4.13 (m, 1H), 3.83 (m, 1H), 3.69 - 3.51 (m, 3H), 3.04 - 2.86 (m, 2H), 2.69 - 2.52 (m, 1H), 2.46 - 2.33 (m, 1H). MS: 496.1 (M+1)⁺.

### Example 6. Preparation of methyl 2-((1-(2-chlorophenyl)-2-(3,3-difluorocyclobutylamino)-2-oxoethyl)(3-fluorophenyl)amino)-2-oxoethylcarbamate

Compound **73** was prepared according to the following scheme, using the following protocol.

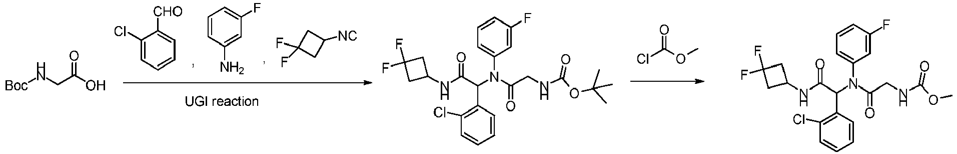

***Step A: tert-Butyl 2-((1-(2-chlorophenyl)-2-(3,3-difluorocyclobutylamino)-2-oxoethyl)(3-fluorophenyl)amino)-2-oxoethylcarbamate. Compound 71.*** A mixture of 2-chlorobenzaldehyde (0.31 mL, 2.74 mmol) and 3-fluoroaniline (0.26 mL, 2.74 mmol), 2-(*tert*-butoxycarbonyl-amino)acetic acid (479 mg, 2.74 mmol) and 3,3-difluoroisocyanobutane (66% purity, 500 mg, 4.27 mmol) were used for the UGI reaction to give the desired compound (717 mg, 41.9 % yield). ¹H NMR (400 MHz, DMSO-d₆): δ 8.88 (m, 1H), 7.61 (m, 1H), 7.42 (d, *J* = 7.9 Hz, 1H), 7.32 - 6.42 (m, 7H), 6.33 (s, 1H), 4.12 (m, 1H), 3.72 - 3.48 (m, 1H), 3.28-3.23 (m, 1H), 3.05 - 2.75 (m, 2H), 2.55-2.50 (m, 1H), 2.50-2.40 (m, 1H), 1.37 (s, 9H). MS: 526.2 (M+1)⁺.

***Step B: 2-Amino-N-(1-(2-chlorophenyl)-2-(3,3-difluorocyclobutylamino)-2-oxoethyl)-N-(3-fluorophenyl)acetamide hydrochloride.*** A mixture of *tert*-butyl 2-((1-(2-chlorophenyl)-2-(3,3-difluorocyclobutylamino)-2-oxoethyl)(3-fluorophenyl)amino)-2-oxoethylcarbamate (500 mg) in 4N HCl in EA (20 mL) was stirred at r.t. overnight. The mixture was filtered to give the desired product (quant.). MS: 426.2 (M+1)⁺.

***Step C: Compound 73.*** To a solution of 2-amino-N-(1-(2-chlorophenyl)-2-(3,3-difluoro cyclobutylamino)-2-oxoethyl)-N-(3-fluorophenyl) acetamide hydrochloride (100 mg) in saturated aq. NaHCO₃ (10 mL) and THF (10 mL) was added methyl chloroformate (1 mL). The mixture was stirred at r.t. overnight and treated with saturated aq. NaHCO₃ to pH=9. The resulting mixture was extracted with EtOAc. The combined organic layers were dried, filtered and concentrated. The residue was purified by flash chromatography using DCM/MeOH (V:V, 20:1) as eluent to give the desired product (66.5 mg, 60% yield). ¹H NMR (400 MHz, DMSO-d₆): δ 8.83 (m, 1H), 7.63 (m, 1H), 7.39 (d, *J* = 7.8 Hz, 1H), 7.27 - 7.13 (m, 3H), 7.05 (m, 2H), 6.84 (d, *J* = 7.6 Hz, 1H), 6.72 (m, 1H), 6.28 (s, 1H), 4.09 (dt, J= 8.0, 6.1 Hz, 1H), 3.58 (m, 1H), 3.49 (s, 3H), 3.31 - 3.24 (m, 1H), 2.97 - 2.84 (m, 2H), 2.57 (m, 1H), 2.40 (m, 1H). MS: 484.1 (M+1)⁺.

The following analogs were synthesized via the procedure set forth above.

### Compound 75

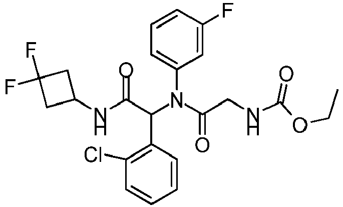

¹H NMR (400 MHz, DMSO-d₆): δ 8.88 (d, *J* = 4.4 Hz, 1H), 7.67 (brs, 1H), 7.42 (d, *J* = 7.5 Hz, 1H), 7.22 (dt, *J* = 13.6, 6.8 Hz, 3H), 7.07 (t, *J* = 6.8 Hz, 2H), 6.87 (d, *J* = 7.6 Hz, 1H), 6.66 (brs, 1H), 6.31 (s, 1H), 4.17 - 4.05 (m, 1H), 3.97 (m, 2H), 3.60 (m, 2H), 3.03 - 2.85 (m, 2H), 2.59 (m, 1H), 2.44 (m, 1H), 1.14 (t, *J* = 7.1 Hz, 3H). MS: 498.1 (M+1)⁺.

### Compound 74

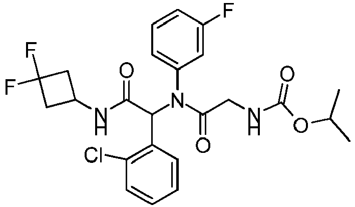

¹H NMR (400 MHz, DMSO-d₆): δ 8.84 (m, 1H), 7.48 (m, 1H), 7.39 (m, 1H), 7.22 - 7.15 (m, 2H), 7.07 - 6.99 (m, 3H), 6.84 (d, *J* = 7.2 Hz, 2H), 6.28 (s, 1H), 4.72 - 4.65 (m, 1H), 4.10 - 4.05 (m, 1H), 3.60 - 3.48 (m, 2H), 2.89 (m, 2H), 2.54 (m, 1H), 2.42 (m, 1H), 1.12 (d, *J* = 6.2 Hz, 6H). MS: 512.1 (M+1)⁺.

### Compound 1

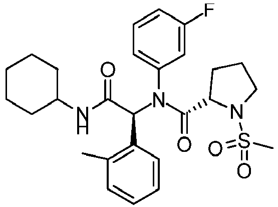

1H NMR (400 MHz, DMSO-d6): δ 8.04-7.95 (m, 1H), 7.78-7.75 (d, 1H, J=10), 7.14-6.23 (m, 8H), 4.06-4.02 (m, 1H), 3.62 (s, 1H), 3.39-3.36 (m, 1H), 3.28-3.26 (m, 1H), 2.89-2.86 (m, 3H), 2.36-2.34 (d, 3H, J=6), 1.93-1.52 (m, 9H), 1.30-0.85 (m, 6H); MS: 516.0 (M+1)+.

### Compound 2

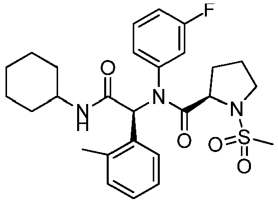

¹H NMR (400 MHz, DMSO-d6): δ 7.78-7.55 (m, 2H), 7.13-6.08 (m, 7H), 5.94 (s, 1H), 3.79 (s, 1H), 3.40-3.39 (m, 1H), 3.17-3.11 (m, 2H), 2.63 (s, 3H), 2.14 (s, 3H), 1.78-1.29 (m, 9H), 1.08-0.61 (m, 5H); MS: 516.2 (M+1)⁺.

### Compound 5

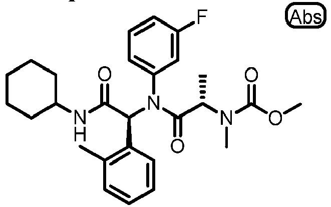

¹H NMR (400 MHz, CDCl₃): δ7.71 (br, 1H), 7.10 (br, 2H), 6.87-6.68 (m, 4H), 6.36-6.32 (br, 2H), 4.68-4.66 (m, 0.5H), 4.64-4.59 (br, 0.5H), 3.85-3.84 (br, 1H), 3.60 (s, 2H), 3.40-3.34 (br, 1H), 2.90-2.88 (br, 3H), 2.38 (s, 3H), 1.96-1.93 (br, 2H), 1.68-1.65 (br, 2H), 1.36-1.26 (br, 6H), 1.11-1.07 (br, 3H); MS: 484.1 (M+1)⁺.

### Compound 6

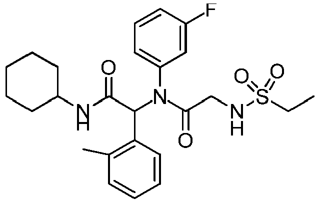

¹H NMR (400 MHz, MeOD-d4): δ 8.03 (m, 0.77H), 7.77 (m, 0.65H), 7.31 (br, 1H), 7.10-7.01 (m, 3H), 6.86 (m, 1H), 6.72 (m, 1H), 6.22 (s, 1H), 3.66-3.62 (m, 2H), 2.99-2.93(q, 2H), 2.36 (s, 3H), 1.79-1.52 (m, 4H), 1.29-0.98 (9H); MS: 490.2 (M+1)⁺.

### Compound 7

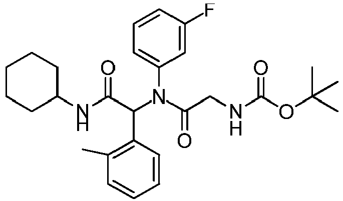

. ¹H NMR (400 MHz, MeOD-d4): δ 7.73 (br, 1H), 7.15 (d, *J* = 7.6, 1H), 7.09-7.09 (m, 1), 6.99-6.94 (m, 1H). 6.80-6.78 (m, 1H), 6.57 (br, 0.7H), 6.38 (s, 1H), 3.78-3.68 (m, 2H), 3.50-3.39 (d, 1H), 2.33 (s, 3H), 1.9-1.93 (m, 1H), 1.80-1.71 (m, 4H), 1.46-1.04 (m, 12H); MS: 498.1 (M+1)⁺.

### Compound 8

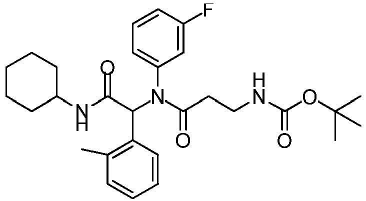

¹H NMR (400 MHz, DMSO-d6): δ7.80-7.72 (br, 1.7H), 7.10-7.08 (d, 2H), 7.02-6.94 (m, 2H), 6.84 (t, J = 8, 1H), 6.69 (d, J = 7.6, 1H), 6.61 (s, 1H), 6.22 (s, 1H), 3.62 (m, 1H), 3.13-2.50 (m, 2H), 2.34 (s, 3H), 2.27-2.23 (m, 1.5H), 2.04-2.00 (br, 1.3H), 1.78-1.52 (m, 5.5H), 1.52-1.11 (m, 12H); MS: 512.1 (M+1)⁺.

### Compound 9

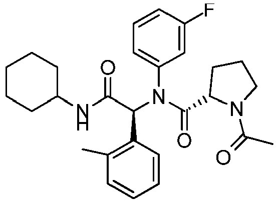

¹H NMR (400 MHz, DMSO-d6): δ 7.99-7.75 (m, 2H), 7.29-6.59 (m, 7H), 6.22 (s, 1H), 4.12-4.04 (m, 1H), 3.63-3.62 (m, 1H), 3.51-3.42 (m, 2H), 2.35 (s, 3H), 1.99 (s, 3H), 1.73-1.52 (m, 8H), 1.29-0.85 (m, 7H); MS: 480.1 (M+1)⁺.

### Compound 10

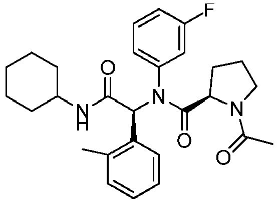

¹H NMR (400 MHz, DMSO-d6): δ 7.81-7.79 (d, J = 10.4 1H), 7.10-6.61 (m, 8H), 6.22 (s, 1H), 4.04-3.99 (d, J = 22.4, 1H), 3.64-3.33 (m, 3H), 2.33 (s, 3H), 1.99-1.53 (m, 12H), 1.32-0.63 (m, 5H); MS: 480.1 (M+1)⁺.

### Compound 12

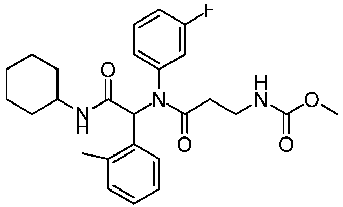

¹H NMR (400 MHz, DMSO-d6): δ7.80 (br, 1H), 7.72 (br, 0.8H), 7.09-7.06 (d, 2H), 7.02-6.94 (m, 3H), 6.84 (t, 1H), 6.70 (d, 1H), 6.22 (s, 1H), 3.63 (m, 1H), 3.46 (s, 3H), 3.20-3.08 (m, 2H), 2.34 (s,3H), 2.30-2.24 (m, 1H), 2.06-2.01 (m, 1H), 1.77-1.52 (m, 6H), 1.29-1.23 (br, 1H), 1.19-0.94 (m, 3H); MS: 470.1 (M+1)⁺.

### Compound 14

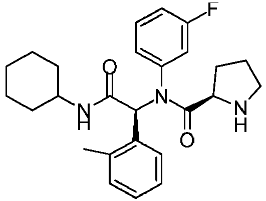

¹H NMR (400 MHz, DMSO-d6): δ 8.06-8.05 (d, J = 0.8, 1H), 7.85 (s, 1H), 7.16-6.82 (m, 8H), 6.66-6.12 (m, 2H), 3.62-3.58 (m, 2H), 3.33-3.29 (m, 1H), 3.10-2.81 (m, 1H), 2.45 (s, 3H), 1.77-1.52 (m, 8H), 1.29-0.47 (m, 6H); MS: 437.8 (M+1)⁺.

### Compound 16

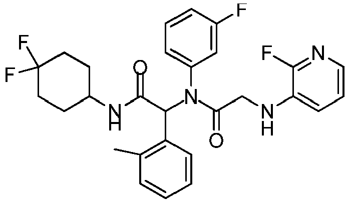

¹H NMR (400 MHz, DMSO-d6): 8.16 (br, 1H), 7.84 (br, 1H), 7.36 (d, 1H, *J* = 4.8), 7.14-7.02 (m, 5H), 6.90-6.84 (m, 2H), 6.75 (d, 1H, *J* = 8.4), 6.22 (s, 1H), 5.84 (t, 1H, *J* = 5.2), 3.84-3.79 (m, 2H), 3.49 (d, 1H, *J* = 12.4), 2.39 (s, 3H), 1.92-1.80 (m, 6H), 151-1.49 (m, 1H), 1.36-1.31 (m, 1H); MS: 528.7 (M+1)⁺.

### Compound 17

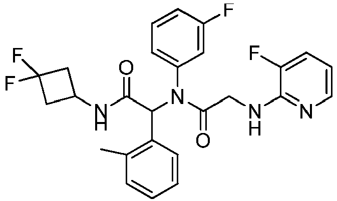

¹H NMR (400 MHz, DMSO-d₆): 8.60 (m, 1H), 7.80 (d, 1H, *J* = 4.8), 7.39-7.34 (m, 1H), 7.19-7.05 (s, 4H), 6.90 (t, 1H, *J* = 4.0), 6.67-6.56 (m, 4H), 6.24 (s, 1H), 4.11(br, 1H), 3.96 (dd, 1H, *J* = 15.2, 3.2), 3.62 (dd, 1H, *J* = 15.2, 3.2), 2.95 (br, 1H), 2.40(s, 3H), 1.31-1.18 (m, 4H); MS: 500.7 (M+1)⁺.

### Compound 19

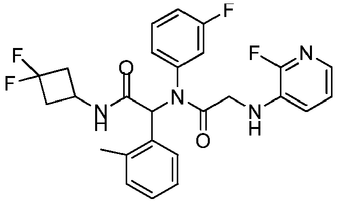

¹H NMR (400 MHz, DMSO-d6): δ 8.65 (s, 1H), 7.77-7.35 (m, 2H), 7.15-7.03 (m, 5H), 6.90-6.67 (m, 4H), 6.21(s, 1H), 5.81 (m, 1H), 4.08(m, 1H), 3.82-3.76 (m, 1H), 3.46 (m, 1H), 2.92 (m, 2H), 2.38 (m, 5H); MS: 500.9 (M+1)⁺.

### Compound 23

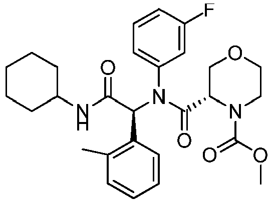

¹H NMR (400 MHz, DMSO-d6): δ 8.06-7.89 (m, 2H), 7.31-6.34 (m, 8H), 4.31-4.23 (m, 1H), 3.84-3.48 (m, 8H), 3.29-3.26 (m, 2H), 2.30 (s, 3H), 1.77-1.53 (m, 5H), 1.30-0.94 (m, 5H); MS: 512.0 (M+1)⁺.

### Example 7. Preparation of N-(3-(N-(1-(2-chlorophenyl)-2-(3,3-difluorocyclobutylamino)-2-oxoethyl)-2-(pyridin-3-yl)acetamido)phenyl)acrylamide

Compound **88** was prepared according to the following scheme, using the following protocol.

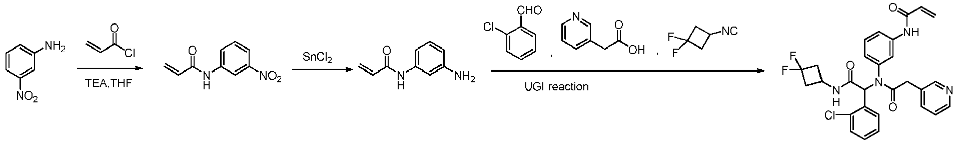

***Step A: N-(3-nitrophenyl)acrylamide.*** To a solution of 3-nitroaniline (500 mg, 3.62 mmol) in dry THF (5 mL) was added TEA (0.75 mL, 5.43 mmol). The mixture was stirred at r.t. for 10 min followed by dropwise addition of acryloyl chloride (0.59 mL, 7.24 mmol) at 0 °C. The mixture was then stirred at r.t. for 2 hr. The resulting mixture was concentrated in vacuo and the crude product was purified by column chromatography using DCM as eluent to give the desired product (300 mg, 41.5 %). MS: 193.1 (M+1)⁺.

***Step B: N-(3-aminophenyl)acrylamide.*** To a solution of *N*-(3-nitrophenyl)acrylamide (200 mg, 1.042 mmol) in a mixture of MeOH (4 mL) and THF (4 mL) was added SnCl₂ (99 mg, 5.21 mmol). The mixture was stirred at r.t. overnight and then concentrated. The residue was treated with saturated aq. Na₂CO₃ to pH=12. The mixture was extracted with EtOAc. The combined organic layers were dried, filtered and concentrated. The residue was purified by column chromatography using DCM/MeOH (V:V, 10:1) as eluent to give the desired compound (168 mg, quant.). MS: 163.1 (M+1)⁺.

***Step C: Compound 88.*** A mixture of 2-chlorobenzaldehyde (0.07 mL, 0.617 mmol) and N-(3-aminophenyl)acrylamide (100 mg, 0.617 mmol), 2-(pyridin-3-yl)acetic acid (84.6 mg, 0.617 mmol) and 3,3-difluoroisocyanobutane (80% purity, 101 mg, 0.864 mmol) were used for UGI reaction to give the desired compound (172 mg, 51.8% yield). ¹H NMR (400 MHz, DMSO-d₆): δ 10.17 (m, 1H), 8.77 (d, *J* = 6.3 Hz, 1H), 8.41 (d, *J* = 3.5 Hz, 1H), 8.25 (s, 1H), 7.69 (m, 3H), 7.40 (d, *J* = 7.8 Hz, 1H), 7.30 (dd, *J* = 7.8, 4.8 Hz, 1H), 7.12 (m, *J* = 14.9, 7.2 Hz, 3H), 6.89 (d, *J* = 7.3 Hz, 1H), 6.45 (brs, 1H), 6.35 (s, 1H), 6.25 (m, 1H), 5.76 (d, *J* = 10.0 Hz, 1H), 4.18 - 4.07 (m, 1H), 3.47 (m, 2H), 3.02 - 2.84 (m, 2H), 2.62 (m, 1H), 2.47 - 2.30 (m, 1H). MS: 539.2 (M+1)⁺.

### Example 8. Preparation of N-cyclohexyl-2-[(2-imidazol-1-yl-acetyl)-thiophen-2-ylmethyl-amino]-2-o-tolyl-acetamide.

Compound **26** was prepared according to the following scheme, using the following protocol.

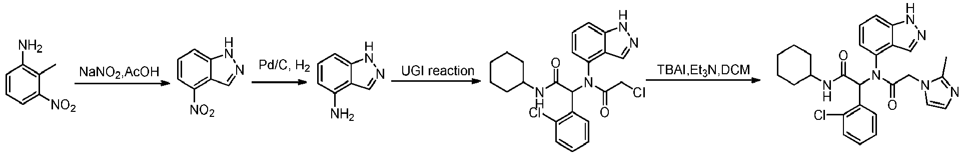

***Step A: 4-Nitro-1H-indazole.*** To a solution of 2-methyl-3-nitroaniline (500 mg, 3.29 mmol) in AcOH (10 mL) was added a solution of sodium nitrite (250 mg, 3.62 mmol) in H₂O (1 mL). the reaction was stirred at r.t. overnight. The mixture was poured into ice-water and the precipitate was collected by filtration. The filtrate was treated with aq. NaOH (1 N) and adjusted to pH=9 and filtrated again. The precipitate was dried under vacuum to afford the desired product (400 mg, 74% yield). MS: 164.0 (M+1)⁺.

***Step B: 1H-Indazol-4-amine.*** A solution of 4-nitro-1H-indazole (100 mg, 0.61 mmol) and Pd/C in EtOH (3 mL) was stirred at r.t. for 2 h under atmosphere of hydrogen. The mixture was filtered and the filtrate was concentrated to afford the crude product (80 mg, 98% yield) without further purification. MS: 134.1 (M+1)⁺.

***Step C: 2-Chloro-N-(1-(2-chlorophenyl)-2-(cyclohexylamino)-2-oxoethyl)-N-(1H-indazol-4-yl)acetamide.*** A mixture of 2-chlorobenzaldehyde (85 mg, 0.60 mmol), 1H-indazol-4-amine (80 mg, 0.6 mmol), 2-chloroacetic acid (57 mg, 0.60 mmol) and cyclohexyl isocyanide (65 mg, 0.60 mmol) were used for UGI reaction to afford the desired product as yellow solid (130 mg, 46% yield). MS: 459.1 (M+1)⁺.

***Step D: Compound 26.*** To a solution of 2-chloro-N-(1-(2-chlorophenyl)-2-(cyclohexylamino)-2-oxoethyl)-N-(1H-indazol-4-yl) acetamide (60 mg, 0.13 mmol), 2-methyl-1H-imidazole (21 mg, 0.26 mmol) and Et₃N (53 mg, 0.52 mmol) in DCM (3 mL) was added TBAI (48 mg, 0.13 mmol). The mixture was stirred at r.t. overnight. The resulting mixture was partitioned between DCM and H₂O. The organic layer was separated, washed with brine, dried over anhydrous Na₂SO₄, filtered and concentrated. The residue was purified by prep-TLC to afford the desired product (20 mg, 30% yield) as yellow solid. ¹H NMR (400 MHz, CDCl₃) : δ 8.62 (m, 1H), 7.91 (s, 1H), 7.72 (d, *J* = 7.3 Hz, 1H), 7.56 (m, 1H), 7.38 - 7.30 (m, 1H), 7.21 (d, *J* = 8.1 Hz, 1H), 7.11 - 6.96 (m, 2H), 6.93 - 6.64 (m, 4H), 5.65 - 5.49 (m, 1H), 3.84 (m, 1H), 3.40 (s, 2H), 2.21 (s, 3H), 1.91 - 1.77 (m, 2H), 1.59 (m, 2H), 1.39 - 1.30 (m, 3H), 1.23 - 1.07 (m, 3H). MS: 519.2 (M+1)⁺.

### Example 9. Preparation of (S)-methyl 2-(((S)-1-(2-chlorophenyl)-2-((3,3-difluorocyclobutyl) amino)-2-oxoethyl)(3-fluorophenyl)carbamoyl)pyrrolidine-1-carboxylate.

Compound **42** was prepared according to the following scheme, using the following protocol.

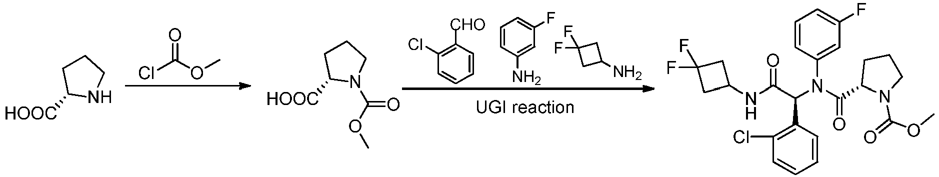

***Step A: (S)-1-(Methoxycarbonyl)pyrrolidine-2-carboxylic acid.*** To a solution of **(*S*)-pyrrolidine-**2-carboxylic acid (1 g, 8.7 mmol) in a solution of THF (10 mL) and saturated NaHCO₃ (10 mL) at 0 °C was added methyl chloroformate (1.65 g, 17.4 mmol). The mixture was stirred at r.t. overnight and extracted with EtOAc (2 x 30 mL). The combined organic layers were concentrated to afford the desired product (1.2 g, 80% yield) as colorless oil.

***Step B: Compound 42.*** The product was synthesized via the procedure for UGI reaction set forth above. ¹H NMR (400 MHz, CDCl₃): δ 7.63 (s, 0.4H), 7.34 (m, 3H), 7.27 - 7.07 (m, 7H), 7.03 - 6.86 (m, 4H), 6.73 - 6.39 (m, 2H), 6.13 (s, 1H), 4.39 (m, 1H), 4.23 - 4.08 (m, 1H), 3.72 (m, 2H), 3.58 - 3.37 (m, 2H), 3.12 - 2.92 (m, 2H), 2.79 - 2.31 (m, 2H), 2.09 - 1.86 (m, 3H), 1.77 - 1.66 (m, 1H). MS: 524.1 (M+1)⁺.

The following analogs were synthesized via the UGI reaction procedure set forth above, using the appropriate aldehyde, amine, carboxylic acid, and isocyanide; and purified via various methods including TLC, Chromatography, HPLC or chiral HPLC.

### Compound 84

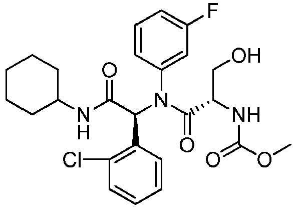

¹H NMR (400 MHz, CDCl₃): δ 7.41 (s, 1H), 7.00 (m, 7H), 6.60 (s, 1H), 5.73 (m, 2H), 4.48 (s, 1H), 3.82 (m, 2H), 3.62 (m, 4H), 3.27 (s, 1H), 1.98 (s, 2H), 1.48 - 1.01 (m, 8H). MS: 506.2 (M+ 1)⁺.

### Compound 85

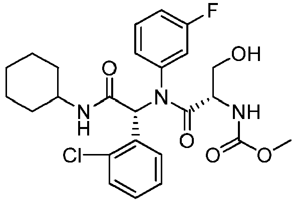

¹H NMR (400 MHz, CDCl₃): δ 7.36 (d, *J* = 8.0 Hz, 1H), 7.21 (m, 2H), 7.04 (m, 4H), 6.19 (s, 1H), 5.78 (m, 2H), 4.33 (m, 1H), 3.92 - 3.78 (m, 2H), 3.66 (m, 5H), 3.04 (s, 1H), 1.94 (m, 2H), 1.72 (m, 1H), 1.44 - 1.04 (m, 7H). MS: 506.2 (M+1)⁺.

### Compound 56

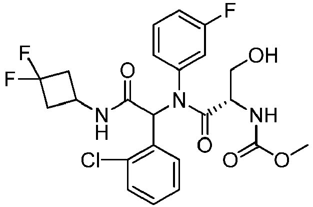

¹H NMR (400 MHz, CDCl₃): δ 7.35 (d, *J* = 8.0 Hz, 1H), 7.27 (d, *J* = 5.2 Hz, 1H), 7.22 - 7.10 (m, 2H), 7.06 - 6.73 (m, 4H), 6.61 (m, 1H), 5.78 (d, *J* = 5.5 Hz, 1H), 4.52 - 4.08 (m, 2H), 3.86 - 3.55 (m, 5H), 2.99 (m, 3H), 2.67 - 2.35 (m, 2H). MS: 514.1 (M+1)⁺.

### Compound 96

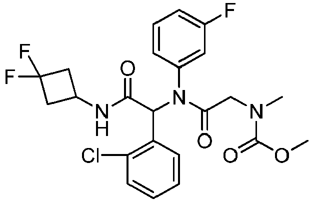

¹H NMR (400 MHz, CDCl₃): δ 7.33 (m, 2H), 7.23 - 7.07 (m, 2H), 7.05 - 6.88 (m, 3H), 6.62 (s, 1H), 6.52 - 6.24 (m, 1H), 4.49 - 4.22 (m, 1H), 3.84 - 3.52 (m, 5H), 3.12 - 2.94 (m, 4H), 2.92 - 2.30 (m, 3H). MS: 498.1 (M+1)⁺.

### Compound 99

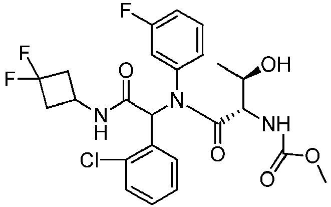

¹H NMR (400 MHz, CDCl₃): δ 7.39 (m, 1H), 7.26 - 7.10 (m, 1H), 7.10 - 6.78 (m, 4H), 6.72 - 6.36 (m, 2H), 5.72 (s, 1H), 4.32 (m, 1H), 4.09 (m, 1H), 3.86-3.70 (m, 3H), 2.98 (m, 7H), 2.61 (m, 1H), 1.06 (m, 3H). MS: 528.1 (M+1)⁺.

### Compound 66

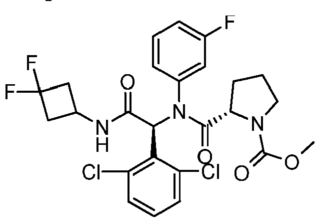

¹H NMR (400 MHz, CDCl₃): δ 8.27 (m, 1H), 7.69 - 7.53 (m, 1H), 7.38 (m, 1H), 7.16 - 6.93 (m, 4H), 6.32 - 6.16 (m, 1H), 4.41 (s, 1H), 4.00 (t, *J* = 7.3 Hz, 1H), 3.76 (d, *J* = 2.2 Hz, 3H), 3.53 (m, 2H), 3.13 - 2.76 (m, 4H), 2.17 - 1.96 (m, 2H), 1.94 - 1.76 (m, 2H). MS: 558.1(M+1)⁺.

### Compound 58

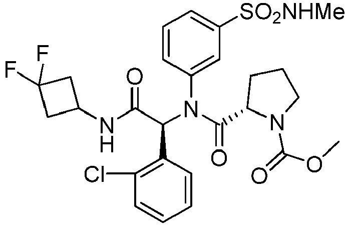

¹H NMR (400 MHz, CDCl₃): δ 8.30-6.73 (m, 9H), 6.56 (m, 2H), 6.26 (m, 3H), 5.02 (m, 1H), 4.49 - 3.92 (m, 2H), 3.88 - 3.57 (m, 3H), 3.52 - 3.26 (m, 2H), 3.01 (m, 2H), 2.37 (m, 5H), 2.17 - 1.79 (m, 5H). MS: 599.1 (M+1)⁺.

### Compound 52

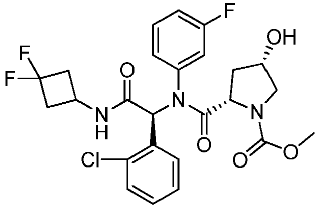

¹H NMR (400 MHz, CDCl₃): δ 7.40 - 7.30 (m, 1H), 7.18 (m, 3H), 7.00 (m, 3H), 6.86 - 6.58 (m, 1H), 6.30 (m, 2H), 4.37 - 4.25 (m, 2H), 3.73 (m, 3H), 3.43 (m, 1H), 3.11 - 2.90 (m, 2H), 2.68 - 2.47 (m, 2H), 2.41 (m, 1H), 2.04 (m, 3H). MS: 540.1 (M+1)⁺.

### Compound 51

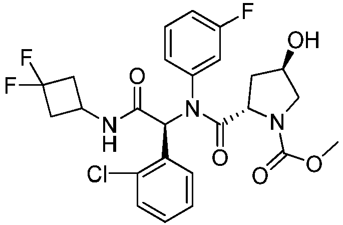

¹H NMR (400 MHz, CD₃OD): δ 7.62 (m, 1H), 7.44 - 6.23 (m, 7H), 4.34 (m, 2H), 4.21 (m, 1H), 3.72 (m, 3H), 3.62 - 3.34 (m, 2H), 2.91 (m, 2H), 2.52 (m, 2H), 1.95 (m, 2H). MS: 539.1 (M+1)⁺.

### Compound 91

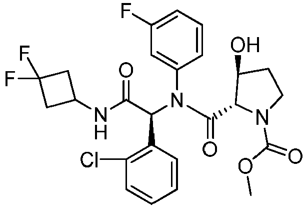

¹H NMR (400 MHz, CDCl₃): δ 7.39 (m, 2H), 7.26 - 7.12 (m, 3H), 7.12 - 6.85 (m, 2H), 6.68 (m, 1H), 6.40 - 6.27 (m, 1H), 4.48 (m, 1H), 4.34 (m, 1H), 4.16 (m, 1H), 3.79 - 3.46 (m, 5H), 3.01 (m, 2H), 2.67 - 2.14 (m, 3H), 1.90 - 1.78 (m, 1H). MS: 540.1 (M+1)⁺.

### Compound 53

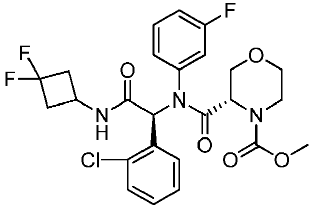

¹H NMR (400 MHz, CDCl₃): δ 7.69 (s, 1H), 7.29 (d, *J =* 7.0 Hz, 1H), 7.20 - 7.08 (m, 2H), 7.05 - 6.93 (m, 3H), 6.92 - 6.65 (m, 2H), 4.40 (m, 2H), 4.02 (m, 1H), 3.84 (s, 1H), 3.75 (s, 3H), 3.66 - 3.40 (m, 2H), 3.40 - 3.29 (m, 2H), 2.98 (m, 2H), 2.71 - 2.56 (m, 1H), 2.44 - 2.26 (m, 1H), 2.17 (s, 2H). MS: 540.1 (M+1)⁺.

### Compound 97

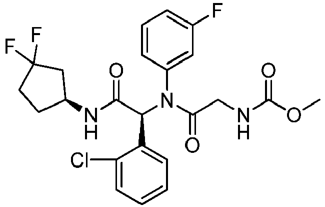

¹H NMR (400 MHz, CDCl₃): δ 7.38 (d, *J* = 8.5 Hz, 1H), 7.19 (s, 2H), 6.99 (m, 3H), 6.43 (s, 1H), 5.90 (s, 1H), 5.53 (s, 1H), 4.54 (s, 1H), 3.72 (m, 4H), 2.61 (s, 1H), 2.06 (s, 4H), 1.66 (s, 1H), 1.28 (s, 4H). MS: 498 (M+1)⁺.

### Compound 98

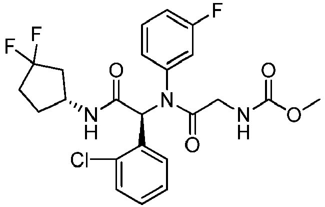

¹H NMR (400 MHz, CDCl₃): δ 7.36 (d, J= 8.0 Hz, 1H), 7.17 (dd, *J* = 10.6, 4.3 Hz, 2H), 7.05 - 6.90 (m, 3H), 6.44 (s, 1H), 6.06 (d, *J* = 6.7 Hz, 1H), 5.55 (s, 1H), 4.52 (m, 1H), 3.78 (m, 1H), 3.67 (s, 4H), 2.53 (m, 1H), 2.40 - 2.02 (m, 3H), 2.01 - 1.74 (m, 2H). MS: 498 (M+1)⁺.

### Compound 92

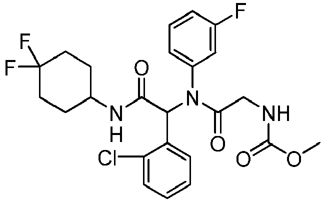

¹H NMR (400 MHz, CDCl₃): δ 7.36 (d, *J* = 8.0 Hz, 1H), 7.28 - 7.07 (m, 2H), 6.97 (m, 3H), 6.42 (d, *J* = 8.3 Hz, 1H), 5.78 (s, 1H), 5.55 (s, 1H), 5.24 (m, 1H), 4.11 (m, 1H), 3.87 - 3.73 (m, 1H), 3.67 (s, 4H), 2.62 - 1.65 (m, 7H), 1.47 (m, 1H); MS: 512.1 (M+1)⁺ .

### Compound 76

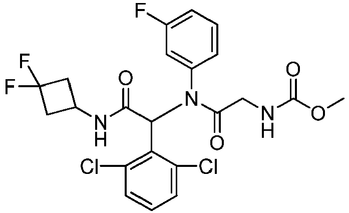

¹H NMR (400 MHz, DMSO-d₆): δ 8.54 - 8.37 (m, 1H), 7.77 (m, 1H), 7.64 - 7.24 (m, 4H), 7.23 - 7.08 (m, 2H), 6.67 (s, 1H), 6.34 - 6.10 (m, 1H), 4.14 (s, 1H), 3.52 (s, 3H), 3.50 - 3.44 (m, 2H), 2.90 (s, 2H), 2.58 (m, 2H). MS: 518.3 (M+1)⁺.

### Compound 77

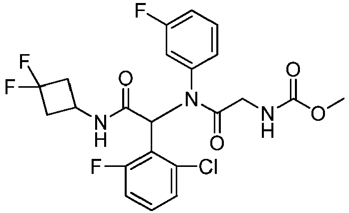

¹H NMR (400 MHz, DMSO-d₆): δ 8.54 (s, 1H), 7.71 (s, 1H), 7.51 - 7.24 (m, 3H), 7.16 (m, 1H), 6.94 (m, 1H), 6.48 (m, 2H), 4.17 - 4.03 (m, 1H), 3.59 (m, 1H), 3.52 (s, 3H), 2.92 (m, 2H), 2.58 (m, 1H), 2.50 (m, 1H).MS:501.1 (M+1)⁺.

### Compound 78

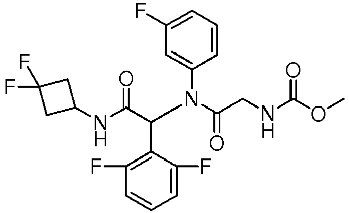

¹H NMR (400 MHz, DMSO-d₆): δ 8.54 (s, 1H), 7.70 (s, 1H), 7.51 - 7.11 (m, 4H), 6.96 (t, *J* = 8.8 Hz, 2H), 6.46 (m, 2H), 4.21 - 3.99 (m, 1H), 3.56 (m, 1H), 3.52 (s, 3H), 2.93 (d, *J* = 7.8 Hz, 2H), 2.66 - 2.53 (m, 1H), 2.51 (s, 1H), 2.49 - 2.35 (m, 1H). MS: 485.1 (M+1)⁺.

### Compound 106

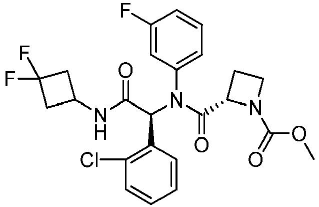

¹H NMR (400 MHz, CDCl₃): δ 7.33 (m, 1H), 7.28 - 6.98 (m, 5H), 6.92 (t, *J* = 7.5 Hz, 1H), 6.59 (s, 2H), 4.62 - 4.48 (m, 1H), 4.37 (s, 1H), 4.10 - 3.97 (m, 1H), 3.79 (m, 1H), 3.62 (s, 3H), 3.13 - 2.91 (m, 2H), 2.62 (s, 2H), 2.29 (m, 1H), 2.09 (m, 1H).MS: 510.1 (M+1)⁺.

### Compound 101

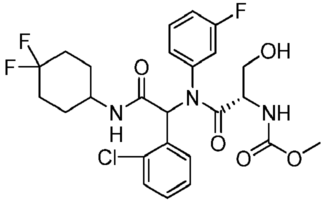

¹H NMR (400 MHz, CDCl₃): δ 7.38 (dd, *J* = 8.0, 3.3 Hz, 1H), 7.26 - 6.75 (m, 6H), 6.61 (t, *J* = 4.5 Hz, 1H), 6.22 (m, 1H), 5.84 - 5.65 (m, 1H), 5.22 (m, 1H), 4.61 - 3.91 (m, 2H), 3.89 - 3.54 (m, 5H), 3.10 (d, *J* = 66.3 Hz, 1H), 2.65 - 1.77 (m, 7H), 1.67 - 1.39 (m, 1H); MS: 542.1 (M+1)⁺.

### Compound 57

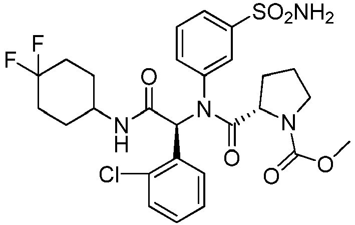

¹H NMR (400 MHz, CDCl₃): δ 8.53 - 6.68 (m, 7H), 6.65 - 5.66 (m, 2H), 5.18 (m, 3H), 4.38 - 3.85 (m, 2H), 3.85 - 3.12 (m, 5H), 2.62 - 1.34 (m, 12H). MS: 613.2 (M+1)⁺.

### Compound 81

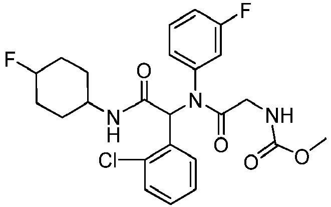

¹H NMR (400 MHz, CDCl₃): δ 7.34 (d, *J* = 7.9 Hz, 1H), 7.26 (s, 2H), 7.15 (m, 2H), 7.04 - 6.78 (m, 3H), 6.41 (s, 1H), 5.57 (m, 2H), 4.77 (m, 1H), 4.00 - 3.51 (m, 4H), 1.97 (m, 1H), 1.89 - 1.67 (m, 1H), 1.69 - 1.30 (m, 7H). MS: 494.2 (M+1)⁺.

### Example 10. Preparation of (2S,4R)-methyl 2-(((S)-1-(2-chlorophenyl)-2-((3,3-difluorocyclo -butyl)amino)-2-oxoethyl)(3-fluorophenyl)carbamoyl)-4-fluoropyrrolidine-1-carboxylate

Compound **55** was prepared according to the following scheme, using the following protocol. 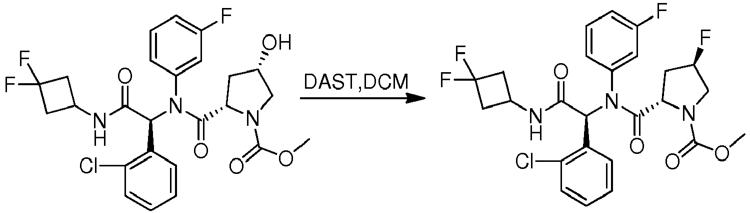 DAST (48 mg, 0.29 mmol) was added to a solution of compound **52** (40 mg, 0.074 mmol) in anhydrous DCM (2 mL) at 0 °C and stirred at r.t. for 2 hr. The reaction was concentrated and the residue was purified by prep-TLC to afford the desired product as white solid (30 mg, 75% yield). ¹H NMR (400 MHz, CDCl₃): δ 7.64 - 7.27 (m, 2H), 7.26 - 6.83 (m, 6H), 6.70 - 6.49 (m, 1H), 6.32 - 6.02 (m, 1H), 5.15 (m, 1H), 4.34 (m, 2H), 3.99 - 3.51 (m, 5H), 3.00 (m, 2H), 2.72 - 2.31 (m, 2H), 2.31 - 2.02 (m, 2H); MS: 542.1 (M+1)⁺.

Compound **70** was prepared according to the above scheme using the corresponding starting material.

### Compound 70

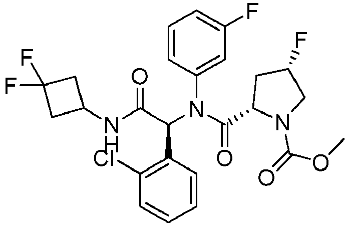

¹H NMR (400 MHz, CDCl₃): δ 7.80 - 6.49 (m, 10H), 6.32 (m, 1H), 5.13 (m, 1H), 4.30 (m, 2H), 3.98 - 3.34 (m, 5H), 3.01 (m, 2H), 2.74 - 2.42 (m, 2H), 2.19 (m, 2H). MS: 542.1 (M+1)⁺.

**Example 11. Preparation of (2S,4R)-methyl 2-(((S)-1-(2-chlorophenyl)-2-((3,3-difluorocyclo -butyl)amino)-2-oxoethyl)(3-fluorophenyl)carbamoyl)-4-fluoropyrrolidine-1-carboxylate** Compound **63** was prepared according to the following scheme, using the following protocol.

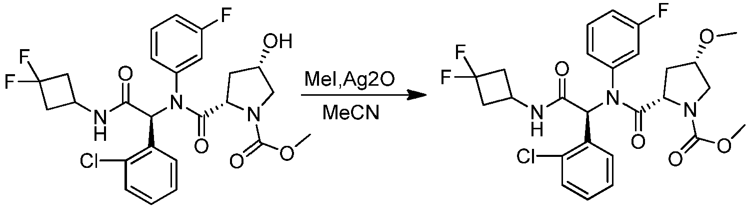

To a solution of compound **52** (60 mg, 0.11 mmol) in MeCN (3 mL) was added Ag₂O (39 mg, 0.17 mmol), followed by addition of MeI (187 mg, 1.32 mmol). The mixture was stirred at r.t. overnight and then filtered. The filtrate was purified by prep-TLC to afford the desired product as a white solid (30 mg, 49% yield). ¹H NMR (400 MHz, CDCl₃): δ 7.52 - 7.28 (m, 2H), 7.18 (m, 2H), 7.09 m, 1H), 7.02 - 6.85 (m, 2H), 6.59 (m, 1H), 6.16 (m, 1H), 4.36 (m, 1H), 4.14 (m, 1H), 3.92 - 3.74 (m, 2H), 3.71 (d, *J* = 7.9 Hz, 3H), 3.28 (d, *J* = 4.4 Hz, 3H), 2.99 (m, 2H), 2.75 - 2.26 (m, 2H), 2.17 (s, 2H), 2.06 - 1.86 (m, 1H). MS: 554.2. (M+1)⁺.

**Example 12. Preparation of (2S,4R)-methyl 2-(((S)-1-(2-chlorophenyl)-2-((3,3-difluorocyclo-butyl)amino)-2-oxoethyl)(3-fluorophenyl)carbamoyl)-4-fluoropyrrolidine-1-carboxylate** Compound **65** was prepared according to the following scheme, using the following protocol.

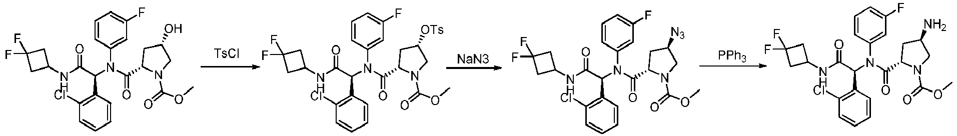

***Step A: (2S,4S)-Methyl 2-(((S)-1-(2-chlorophenyl)-2-((3,3-difluorocyclobutyl)amino)-2-oxo-ethyl)(3-fluorophenyl)carbamoyl)-4-(tosyloxy)pyrrolidine-1-carboxylate.*** TsCl (150 mg, 0.78 mmol) was added to a solution of compound 52 (210 mg, 0.39 mmol), 4-dimethylaminopyridine (24 mg, 0.20 mmol) and pyridine (0.1 mL, 1.17 mmol) in anhydrous DCM (5 mL). The mixture was stirred at r.t. overnight and then partitioned between EtOAc and H₂O. The organic layer was separated, washed with brine, dried over anhydrous Na₂SO₄, and concentrated. The residue was purified by column chromatography to afford the desired product (200 mg, 74% yield) as white solid.

***Step B: (2S,4R)-Methyl 4-azido-2-(((S)-1-(2-chlorophenyl)-2-(3,3-difluorocyclobutylamino)-2-oxoethyl)(3-fluorophenyl)carbamoyl)pyrrolidine-1-carboxylate.*** A mixture of (2*S*, 4*S*) - methyl 2-(((S)-1-(2-chlorophenyl)-2-((3,3-difluorocyclobutyl)amino)-2-oxo-ethyl)(3-fluoro - phenyl)carbamoyl)-4-(tosyloxy)pyrrolidine-1-carboxylate (100 mg, 0.14 mmol), and NaN₃ (49.5 mg, 0.6 mmol) in dry DMF (2 mL) was stirred at 60 °C for 1 hr. The mixture was treated with water and extracted with ethyl acetate (2 x 20 mL). The combined organic layers were washed with brine, dried over anhydrous Na₂SO₄ and concentrated to dryness to give the crude product which was used directly in the next step.

***Step C: Compound 65.*** A mixture of (2*S*,4*R*)-methyl 4-azido-2-(((S)-1-(2-chlorophenyl)-2-(3,3-difluoro-cyclobutylamino)-2-oxoethyl)(3-fluorophenyl)carbamoyl)pyrrolidine-1-carboxylate (50 mg, 0.072 mmol) and PPh₃ (71 mg, 0.27 mmol) in THF/water (V:V, 10:1, 2 mL) was stirred at 40 °C overnight. The mixture was treated with water and extracted with ethyl acetate (2 x 20 mL). The combined organic layers were dried over anhydrous Na₂SO₄ and concentrated to give the crude product which was purified by prep-TLC to afford the desired product (10 mg, 26% yield) as a yellow solid. ¹H NMR (400 MHz, CDCl₃): δ 7.66 - 7.28 (m, 2H), 7.22 - 6.87 (m, 5H), 6.59 (d, *J* = 48.6 Hz, 1H), 5.51 (m, 1H), 4.44-3.77 (m, 4H), 3.68 (d, *J* = 8.4 Hz, 3H), 3.55 (m, 1H), 3.36 (m, 1H), 3.02 - 2.76 (m, 3H), 2.63 - 2.25 (m, 3H), 2.14 (m, 1H), 2.04 - 1.90 (m, 1H). MS: 539.2 (M+1)⁺.

Compound **89** was prepared according to the above scheme using the corresponding starting material.

### Compound 89

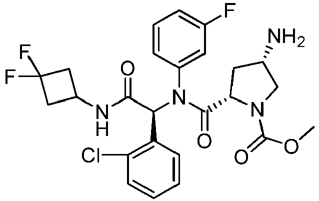

¹H NMR (400 MHz, DMSO-d₆): δ 7.77 (m, 2H), 7.49 - 5.90 (m, 6H), 4.57 - 3.91 (m, 6H), 3.81 - 3.48 (m, 4H), 3.34 - 2.84 (m, 2H), 2.79 - 2.03 (m, 4H). MS: 539.2 (M+1)⁺.

### Example 13. Preparation of (2S,4R)-methyl 2-(((S)-1-(2-chlorophenyl)-2-((3,3-difluoro-cyclobutyl)amino)-2-oxoethyl)(3-fluorophenyl)carbamoyl)-4-(2-methoxyethoxy)pyrrolidine-1-carboxylate

Compound 79 was prepared according to the following scheme, using the following protocol.

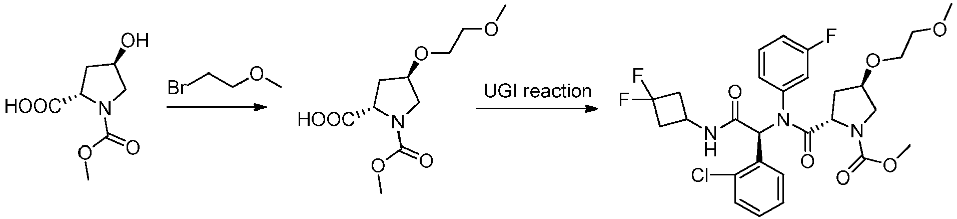

***Step A: (2S,4R)-1-(Methoxycarbonyl)-4-(2-methoxyethoxy)pyrrolidine-2-carboxylic acid.*** NaH (127 mg, 3.18 mmol) was added to a solution of (2*S*,4*R*)-4-hydroxy-1-(methoxycarbonyl)-pyrrolidine-2-carboxylic acid (200 mg, 1.06 mmol) in DMF (5 mL) at 0 °C under nitrogen atmosphere. After stirring at this temperature for 30 min, 1-bromo-2-methoxyethane (368 mg, 2.65 mmol) was added to the above mixture and the mixture was stirred at r.t. for 2 hr. The reaction mixture was treated with H₂O and extracted with EtOAc (2 x 20 mL). The aqueous layer was acidified with aq. HCl (2 N) to pH=4. The mixture was extracted with EtOAc and concentrated to afford the desired product as a yellow oil (195mg, 74% yield). MS: 248.1 (M+1)⁺.

***Step B: Compound 79.*** The compound was synthesized via the general procedure for UGI reaction set forth above. ¹H NMR (400 MHz, CDCl₃): δ 7.38 (m, 2H), 7.18 (m, 3H), 7.09 (t, *J* = 7.6 Hz, 1H), 7.03 - 6.84 (m, 2H), 6.75 - 6.40 (m, 1H), 6.28 - 5.96 (m, 1H), 4.31 (m, 2H), 4.09 (m, 1H), 3.71 (d, *J* = 8.7 Hz, 3H), 3.68 - 3.54 (m, 2H), 3.52-3.37 (m, 4H), 3.29 (s, 3H), 3.00 (m, 2H), 2.73 - 2.30 (m, 2H), 2.25 - 2.00 (m, 2H). MS: 598.2 (M+1)⁺.

### Example 14. Preparation of ((2S,4R)-methyl 2-(((S)-1-(2-chlorophenyl)-2-(3,3-difluorocyclo -butylamino)-2-oxoethyl)(3-fluorophenyl)carbamoyl)-4-(2-(dimethylamino)ethoxy)pyrrolidine-1-carboxylate

Compound **104** was prepared according to the following scheme, using the following protocol.

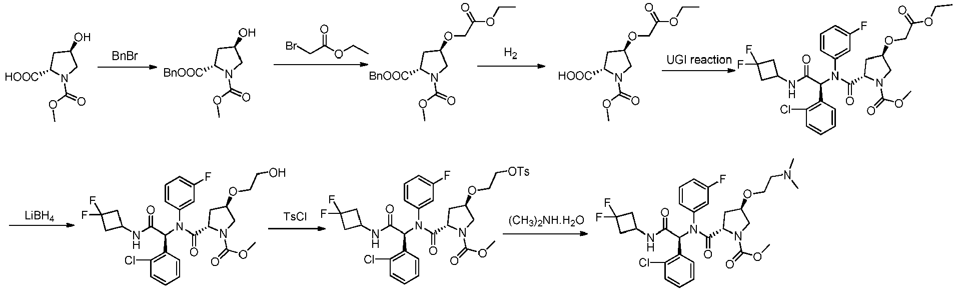

***Step A:* (2S,4R)-2-Benzyl 1-methyl 4-hydroxypyrrolidine-1,2-dicarboxylate.** To a solution of (2*S*,4*R*)-4-hydroxy-1-(methoxycarbonyl)pyrrolidine-2-carboxylic acid (1.7 g, 9.0 mmol) in MeOH (18 mL) at 0 °C was added a solution of Cs₂CO₃ (1.5 g, 4.5 mmol) in H₂O (12 mL). The solution was concentrated and the residue was dissolved in DMF (20 mL). After cooling to 0 °C, (bromomethyl)benzene (8.5 g, 9.0 mmol) was added to the mixture. The mixture was stirred at r.t. overnight, and then partitioned between water and EtOAc. The combined organic layers were washed with brine, dried over anhydrous Na₂SO₄ and concentrated. The residue was purified by column chromatography to afford the desired product as a colorless oil (2.0 g, 80% yield). MS: 280.1 (M+1)⁺.

***Step B: (2S,4R)-2-Benzyl 1-methyl 4-(2-ethoxy-2-oxoethoxy)pyrolidine-1,2-dicarboxylate.*** A solution of (2*S*,4*R*)-2-benzyl 1-methyl 4-hydroxypyrrolidine-1,2-dicarboxylate (2.0 g, 7.5 mmol) in anhydrous THF (10 mL) was added to a solution of ethyl 2-bromoacetate (1.5 g, 9.0 mmol), sodium hydride (360 mg, 9.0 mmol) and tetrabutylammonium iodide (276 mg, 0.75 mmol) in THF (15 mL) at r.t.. After stirring for 1.5 h, the mixture was treated with H₂O and extracted with EtOAc (2 x 30 mL). The combined organic layer were dried over anhydrous Na₂SO₄ and concentrated. The residue was purified by column chromatography to afford the desired product (1.4 g, 51% yield) as yellow oil. MS: 366.1 (M+1)⁺.

***Step C: (2S,4R)-4-(2-Ethoxy-2-oxoethoxy)-1-(methoxycarbonyl)pyrrolidine-2-carboxylic acid.*** Pd/C (70 mg, 10%) was added to a solution of (2*S*,4*R*)-2-benzyl 1-methyl-4-(2-ethoxy-2-oxoethoxy)pyrrolidine-1,2-dicarboxylate (700 mg, 1.9 mmol) in MeOH (8 mL). The mixture was stirred at r.t. under hydrogen atmosphere for 1 h, and then filtered. The filtrate was concentrated to afford the desired product as colorless oil (530 mg, 100% yield). MS: 276.1 (M+1)⁺.

***Step D: (2S,4R)-Methyl 2-(((S)-1-(2-chlorophenyl)-2-((3,3-difluorocyclobutyl)amino)-2-oxoethyl)(3-fluorophenyl)carbamoyl)-4-(2-ethoxy-2-oxoethoxy)pyrrolidine-1-carboxylate.*** The compound was synthesized via the general procedure for UGI reaction set forth above.

***Step F: Compound 90.*** LiBH₄ (20 mg, 0.91 mmol) was added to a solution of (2*S*,4*R*)-methyl 2-(((S)-1-(2-chlorophenyl)-2-((3,3-difluorocyclobutyl)amino)-2-oxoethyl)(3-fluorophenyl)carbamoyl)-4-(2-ethoxy-2-oxoethoxy)pyrrolidine-1-carboxylate (100 mg, 0.16 mmol) in anhydrous THF (3 mL) at r.t. The mixture was stirred for 1.5 h, and then treated with ice-water. The resulting mixture was extracted with EtOAc (2 x 10 mL). The combined organic layers were dried over anhydrous Na₂SO₄ and concentrated. The residue was purified by prep-TLC to afford the desired product (78 mg, 84% yield) as white solid. ¹H NMR (400 MHz, CDCl₃): δ 7.39 (m, 2H), 7.19 (m, 3H), 7.11 - 7.05 (m, 1H), 7.03 - 6.85 (m, 2H), 6.72 - 6.00 (m, 2H), 4.46 - 4.20 (m, 2H), 4.10 (s, 1H), 3.72 (d, *J* = 8.9 Hz, 3H), 3.67 - 3.50 (m, 4H), 3.47 - 3.36 (m, 2H), 3.11 - 2.89 (m, 2H), 2.73 - 2.31 (m, 2H), 2.24 - 1.99 (m, 2H). MS: 584.2 (M+1)⁺.

***Step G: (2S,4R)-Methyl 2-(((S)-1-(2-chlorophenyl)-2-((3,3-difluorocyclobutyl)amino)-2-oxo-ethyl)(3-fluorophenyl)carbamoyl)-4-(2-(tosyloxy)ethoxy)pyrrolidine-1-carboxylate.*** TsCl (150 mg, 0.78 mmol) was added to a solution of ***compound 90*** (0.39 mmol), 4-dimethylaminopyridine (24 mg, 0.20 mmol) and pyridine (0.1 mL, 1.17 mmol) in anhydrous DCM (5 mL). The mixture was stirred at r.t. overnight, and then partitioned between EtOAc and H₂O. The organic layer was washed with brine, dried over anhydrous Na₂SO₄, and concentrated. The residue was purified by column chromatography to afford the desired product (74% yield) as a white solid. MS: 738.2 (M+1)⁺.

***Step H: Compound 104.*** Dimethylamine aq. (1 mL) was added to a solution of (2*S*,4*R*)-methyl 2-(((*S*)-1-(2-chlorophenyl)-2-((3,3-difluorocyclobutyl)amino)-2-oxoethyl)(3-fluorophenyl)-carbamoyl)-4-(2-(tosyloxy)ethoxy)pyrrolidine-1-carboxylate (100 mg, 0.13 mmol) in THF (1 mL). The mixture was stirred at 90 °C in a sealed tube for 2 h, and then partitioned between EtOAc and H₂O. The organic layer was separated, dried over anhydrous Na₂SO₄ and concentrated. The residue was purified by prep-TLC to afford the desired product as a white solid (15 mg, 19% yield). ¹H NMR (400 MHz, CDCl₃): δ 7.39 (m, 2H), 7.23 - 7.13 (m, 2H), 7.12 - 7.04 (m, 1H), 7.01 - 6.82 (m, 2H), 6.70 - 6.43 (m, 1H), 6.36 - 5.98 (m, 1H), 4.30 (m, 3H), 3.72 (d, *J* = 6.4 Hz, 5H), 3.66 - 3.50 (m, 2H), 3.16 - 2.91 (m, 5H), 2.72 - 2.50 (m, 7H), 2.49 - 2.30 (m, 1H), 2.24 - 1.98 (m, 2H). MS: 611.2 (M+1)⁺.

### Example 15. Preparation of (S)-methyl 4-(((S)-1-(2-chlorophenyl)-2-((3,3-difluorocyclo-butyl)amino)-2-oxoethyl)(3-fluorophenyl)carbamoyl)oxazolidine-3-carboxylate

Compound **54** was prepared according to the following scheme, using the following protocol.

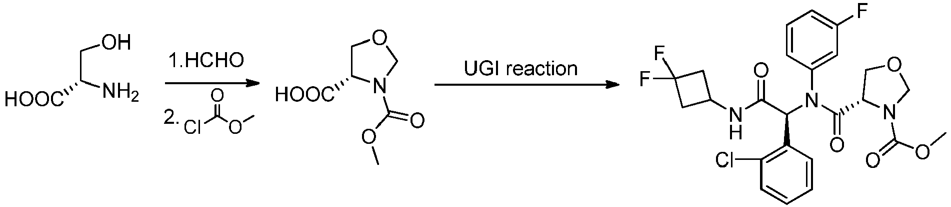

***Step A: (S)-3-(Methoxycarbonyl)oxazolidine-4-carboxylic acid.*** Formaldehyde (1.8 mL, 37% wt in water, 19.0 mmol) was added to a solution of (*S*)-2-amino-3-hydroxypropanoic acid (2 g, 19.0 mmol) in aq. NaOH (2 N, 10 mL) at 0 °C. After stirring at this temperature overnight, acetone (10 mL) and saturated aq. NaHCO₃ (1.6g, 19.0 mmol) were added to the solution, followed by dropwise addition of methyl chloroformate (3.6 g, 38.0 mmol). The mixture was stirred at r.t. for 4.5 h, and then extracted with EtOAc (2 x 40 mL). The organic layer was dried over anhydrous Na₂SO₄, filtered, and concentrated to afford the desired product as an oil (1.7 g, 51% yield).

***Step B: Compound 54.*** The compound was synthesized via the general procedure for the UGI reaction set forth above. ¹H NMR (400 MHz, CDCl₃): δ 7.41 (m, 2H), 7.05 (m, 5H), 6.60 (m, 2H), 5.14 - 4.68 (m, 2H), 4.29 (m, 2H), 4.15 - 3.85 (m, 2H), 3.72 (m, 3H), 2.99 (s, 2H), 2.49 (m, 2H). MS: 526.1 (M+1)⁺.

Compound **43** was prepared according to the above scheme using the corresponding starting material.

### Compound 43

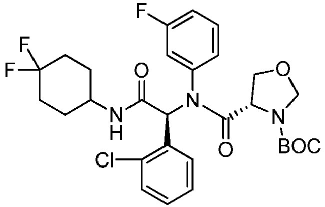

¹H NMR (400 MHz, CDCl₃): δ 7.61 (m, 1H), 7.33 (m, 1H), 7.24 - 6.83 (m, 5H), 6.54 (m, 1H), 5.77 (m, 1H), 5.20 - 4.73 (m, 2H), 4.48 - 3.82 (m, 4H), 2.53 - 2.19 (m, 1H), 2.04 (m, 3H), 1.81 (m, 2H), 1.71 (m, 2H), 1.55 - 1.43 (m, 9H). MS: 534.1 (M+1)⁺.

### Example 16. Preparation of (S)-methyl 4-acetyl-2-(((S)-1-(2-chlorophenyl)-2-(3,3-difluoro-cyclobutylamino)-2-oxoethyl)(3-fluorophenyl)carbamoyl)piperazine-1-carboxylate and (S)-methyl 2-(((S)-1-(2-chlorophenyl)-2-(3,3-difluorocyclobutylamino)-2-oxoethyl)(3-fluoro-phenyl)carbamoyl)-4-methylpiperazine-1-carboxylate

Compounds **62, 68** and **80** were prepared according to the following scheme, using the following protocol.

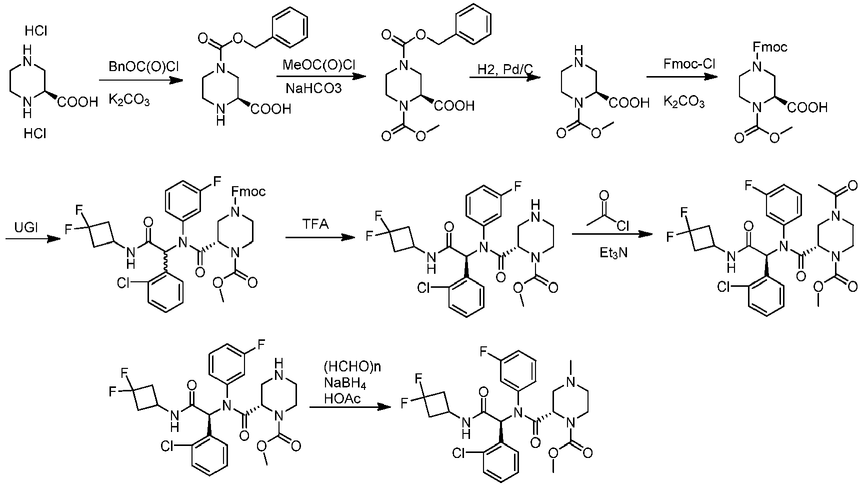

***Step A: (S)-4-(Benzyloxycarbonyl)piperazine-2-carboxylic acid.*** To a solution of (*S*)-piperazine-2-carboxylic acid dihydrochloride (2 g, 10 mmol) in H₂O (30 mL) was slowly added K₂CO₃ (1.4 g, 10 mmol), followed by addition of Cu₂(OH)₂CO₃ (1.0 g, 5 mmol). The mixture was heated to reflux for 2 hr. After cooling to 0 °C, a solution of benzyl chloroformate (2.8 g, 11 mmol) in toluene (15 mL) was added dropwise while keeping the pH of the resultant solution at 8-10. The resulting mixture was stirred at r.t. overnight and filtered. The filtrate was concentrated to give a blue solid to which water (30 mL) was added, followed by addition of conc. HCl until a clear solution was obtained. Na₂S (0.8 g, 10 mmol) was added into the mixture while a black precipitate was formed. The mixture was stirred for 1 h at r.t. and filtered. The filtrate was concentrated to give a white solid which was washed with methanol (60 mL) to give the desired product as a white solid (2.27 g, 86% yield). MS: 265 (M+1)⁺.

***Step B: (S)-4-(Benzyloxycarbonyl)-1-(methoxycarbonyl)piperazine-2-carboxylic acid.*** (*S*)-4-((Benzyloxy)carbonyl)piperazine-2-carboxylic acid (1 g, 3.8 mmol) was dissolved in a mixture of saturated aq. NaHCO₃ (5 mL) and THF (5 mL). The resulting mixture was cooled to 0 °C and methyl chloroformate (0.76 mL, 12 mmol) was added. The mixture was stirred at r.t. for 2 h, and then concentrated. The residue was extracted with EtOAc (3 x 50 mL). The combined organic layers were dried over anhydrous MgSO₄, and concentrated to give the desired product as a white solid (0.63 g, 52% yield). MS: 323 (M+1)⁺.

***Step C: (S)-1-(Methoxycarbonyl)piperazine-2-carboxylic acid.*** (*S*)-4-((Benzyloxy)carbonyl)-1-(methoxycarbonyl)piperazine-2-carboxylic acid (0.63 g, 2 mmol) was dissolved in methanol (20 mL) and then cooled to 0 °C. Pd/C (0.3 g) and conc. HCl (0.1 mL) was added into the mixture. The reaction was stirred under H₂ atmosphere at r.t. overnight and then filtered. The filtrate was concentrated to give the desired product as a white solid (0.4 g, 100% yield) which was used directly for next step.

***Step D: (S)-4-(((9H-Fluoren-9-yl)methoxy)carbonyl)-1-(methoxycarbonyl)piperazine-2-carboxylic acid.*** To a solution of (*S*)-1-(methoxycarbonyl)piperazine-2-carboxylic acid (0.4 g, 2 mmol) in 1,4-dioxane (2 mL) and H₂O (10 mL) was added K₂CO₃ (0.8 g, 5 mmol) at 0 °C, followed by addition of (9H-fluoren-9-yl)methyl chloroformate (0.76 g, 2.5 mmol). The reaction mixture was stirred at r.t. overnight and then concentrated. The residue was extracted with Et₂O (2 x 20 mL). The aqueous phase was acidified with conc. HCl to pH=2-3, and extracted with mixed solvent of DCM/CH₃OH (V:V, 10:1, 3 x 50 mL). The combined organic layers were dried over anhydrous Na₂SO₄ and concentrated. The residue was purified by column chromatography eluting with DCM/CH₃OH (V:V, 30:1 to 5:1) to give the desired product as a white solid (470 mg, 40% yield). MS: 411 (M+1)⁺.

***Step E: (S)-4-(9H-fluoren-9-yl)methyl 1-methyl 2-(((S)-1-(2-chlorophenyl)-2-(3,3-difluoro-cyclobutylamino)-2-oxoethyl)(3-fluorophenyl)carbamoyl)piperazine-1,4-dicarboxylate.*** The titled compound and its diastereomer were synthesized via the general procedure for UGI reaction set forth above. The two diastereomers were purified by prep-TLC (DCM/CH₃OH (V:V, 20:1) to give (*S*)-4-(9*H*-fluoren-9-yl)methyl 1-methyl 2-(((*R*)-1-(2-chlorophenyl)-2-(3,3-difluoro-cyclo-butylamino)-2-oxoethyl)(3-fluorophenyl)carbamoyl)piperazine-1,4-dicarboxylate (170 mg, 21% yield) and its isomer (*S*)-4-(9*H*-fluoren-9-yl)methyl 1-methyl 2-(((*S*)-1-(2-chlorophenyl)-2-(3,3-difluoro -cyclobutylamino)-2-oxoethyl)(3-fluorophenyl)carbamoyl)piperazine-1,4-dicarboxylate (200 mg, 21% yield). MS: 761 (M+1)⁺.

***Step F: (S)-Methyl 2-(((R)-1-(2-chlorophenyl)-2-(3,3-difluorocyclobutylamino)-2-oxoethyl)-(3-fluorophenyl)carbamoyl)piperazine-1-carboxylate.*** To a solution of (*S*)-4-(9*H*-fluoren-9-yl)-methyl 1-methyl 2-(*N*-((*R*)-(3,3-difluorocyclobutylcarbamoyl) (2-chlorophenyl)methyl)-*N*-(3-fluorophenyl)-carbamoyl) piperazine-1,4-dicarboxylate (170 mg, 0.23 mmol) in CH₃CN (3 mL) was added piperidine (0.6 mL). The resulting mixture was stirred at r.t. for 10 min, and then concentrated at 20 °C. The residue was purified by Prep-TLC using DCM/CH₃OH/NH₃.H₂O (V:V:V, 20:1:0.01) as eluent to give the desired compound (0. 1 g, 78% yield). MS: 539 (M+1)⁺. ***(S)-Methyl 2-(((S)-1-(2-chlorophenyl)-2-(3,3-difluorocyclobutylamino)-2-oxoethyl)-(3-fluorophenyl)carbamoyl)piperazine-1-carboxylate*** was prepared in the same procedure as that described above (Compound **62**) (80 mg, 57% yield). ¹H NMR (400 MHz, CDCl₃): δ 2.80-2.41 (m, 6H), 2.98-2.95 (m, 2H), 3.34-3.31(m, 1H), 3.73-3.53(m, 4H), 4.31 (bs, 1H), 4.66-4.51(m, 1H), 6.57 (s, 1H), 6.98-6.88 (m, 4H), 7.33-7.28 (m, 4H). MS: 539 (M+1)⁺.

***Step G: (S)-Methyl 4-acetyl-2-(((R)-1-(2-chlorophenyl)-2-(3,3-difluorocyclobutylamino)-2-oxoethyl)(3-fluorophenyl)carbamoyl)piperazine-1-carboxylate(Compound 67).*** To a solution of (*S*)-methyl 2-(*N*-((*R*)-(3,3-difluorocyclobutylcarbamoyl)(2-chlorophenyl)methyl)-*N-*(3-fluoro-phenyl)carbamoyl)piperazine-1-carboxylate (40 mg, 0.074mmol) in CH₃CN (5 mL) was added Et₃N (0.02 mL, 0.15 mmol) followed by acetyl chloride (0. 01 mL, 0.112 mmol) at 0 °C. The mixture was stirred at r.t. for 30 min and concentrated. The residue was purified by Prep-TLC using DCM/CH₃OH/aq. NH₃ (V:V:V, 20:1:0.01) as eluent to give the desired compound (20 mg, 46% yield). ¹H NMR (400 MHz, CDCl₃): δ 7.32-7.28 (m, 3H), 7.16-7.12 (m, 2H), 7.01-7.00 (m, 2H), 6.90-6.87 (m, 1H), 6.72-6.68 (m,1H), 4.68 (m, 1H), 4.35-4.34 (m, 1H), 3.76-3.66 (m, 4H), 3.02-2.88 (m, 3H), 2.74-2.39 (m, 4H), 2.25 (s, 3H), 1.94 (m, 2H). MS: 581(M+1)⁺. ***(S)-Methyl 4-acetyl-2-(((S)-1-(2-chlorophenyl)-2-(3,3-difluorocyclobutylamino)-2-oxoethyl) (3-fluorophenyl)carbamoyl)piperazine-1-carboxylate* (Compound 68)** was prepared via the same procedure (20 mg, 46% yield). ¹H NMR (400 MHz, CDCl₃): δ 801-7.31 (m, 4H), 7.15-6.85 (m, 4H), 6.67-6.65 (d, 1H), 4.61-4.51 (m, 2H), 3.98-3.77 (m, 5H), 3.33-3.23 (m, 2H), 3.06-2.62 (m, 4H), 2.19 (s, 3H). MS: 581(M+1)⁺.

***Step H. (S)-Methyl 2-(((S)-1-(2-chlorophenyl)-2-(3,3-difluorocyclobutylamino)-2-oxoethyl)-(3-fluorophenyl)carbamoyl)-4-methylpiperazine-1-carboxylate (Compound 80).*** To a solution of (*S*)-methyl 2-(*N*-((*S*)-(3,3-difluorocyclobutylcarbamoyl)(2-chlorophenyl)methyl)-*N*-(3-fluorophenyl)carbamoyl)piperazine-1-carboxylate (20 mg, 0.04 mmol) in CH₃OH (5 mL) was added paraformaldehyde (20 mg, 0. 2 mmol), followed by HOAc (0.1 mL). The reaction mixture was stirred at r.t. under the atmosphere of N₂ for 3 h and then NaBH₄ (10 mg, 0.08 mmol) was added. The resulting mixture was stirred overnight and concentrated. The residue was purified by prep-TLC using DCM/CH₃OH (V:V, 5:1) as eluent to give the desired compound (8 mg, 36% yield). ¹H NMR (400 MHz, CDCl₃): δ 7.32-7.28 (m, 3H), 7.16-7.12 (m, 2H), 7.01-7.00 (m, 2H), 6.90-6.87 (m, 1H), 6.72 (m, 1H), 4.69 (m, 1H), 4.35 (m, 1H), 3.76 (s,3H), 3.02-2.29 (m, 8H), 2.25 (s, 3H), 1.94 (m,2H). MS: 553 (M+1)⁺.

### Example 17. Preparation of (2S)-N-((S)-(3,3-difluorocyclobutylcarbamoyl)(2-chlorophenyl) methyl)-N-(3-fluorophenyl)-1-(pyrimidin-2-yl)pyrrolidine-2-carboxamide

Compound **69** was prepared according to the following scheme, using the following protocol.

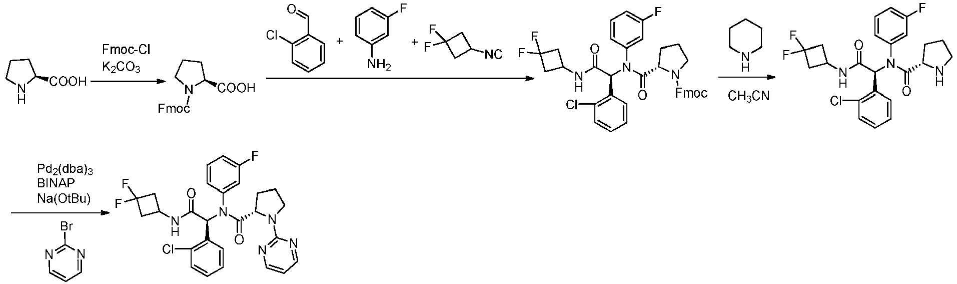

***Step A: (S)-1-(((9H-Fluoren-9-yl)methoxy)carbonyl)pyrrolidine-2-carboxylic acid.*** To a solution of (*S*)-pyrrolidine-2-carboxylic acid (1 g, 8.7 mmol) in 1,4-dioxane (4 mL) and H₂O (15 mL) was added K₂CO₃ (3.24 g, 23 mmol) followed by (9H-fluoren-9-yl)methyl chloroformate (2.3 g, 8.3 mmol) at 0 °C. The mixture was stirred at r.t. overnight and treated by H₂O (10 mL). The resulting mixture was extracted with Et₂O (2 x 20 mL). The aqueous phase was acidified with aq. HCl (1 M) to pH=2-3, and extracted with DCM (3 x 50 mL). The combined organic layers were dried over anhydrous Na₂SO₄ and concentrated to give the desired product as a white solid (2.8 g, 95% yield). MS: 338 (M+1)⁺.

***Step B: (S)-(9H-Fluoren-9-yl)methyl 2-(((S)-1-(2-chlorophenyl)-2-(3,3-difluorocyclobutyl-amino)-2-oxoethyl)(3-fluorophenyl)carbamoyl)pyrrolidine-1-carboxylate.*** 2-Chlorobenzaldehyde (230 mg, 1.5 mmol), 3-fluorobenzenamine (180 mg, 1.5 mmol), (*S*)-1-(((9*H*-fluoren-9-yl)-methoxy)-carbonyl)pyrrolidine-2-carboxylic acid (500 mg, 1.5 mmol) and 1,1-difluoro-3-isocyanocyclobutane (360 mg, 3.0 mmol) in MeOH (4 mL) were used for UGI reaction and the two isomers were purified by column chromatography eluting with DCM/acetone (V:V, 150:1 to 30:1), then with DCM/EtOAc (V:V, 1:1) to give the (*S*)-(9*H*-fluoren-9-yl)methyl-2-(((*R*)-1-(2-chloro-phenyl)-2-(3,3-difluoro -cyclobutylamino)-2-oxo-ethyl)(3-fluorophenyl)carbamoyl)pyrrolidine -1-carboxylate (350 mg, 33% yield) and (*S*)-(9*H*-fluoren-9-yl)methyl-2-(((*S*)-1-(2-chloro-phenyl)-2-(3,3-difluorocyclo-butylamino)-2-oxo-ethyl)(3-fluorophenyl)carbamoyl)pyrrolidine-1-carboxylate (370 mg, 33% yield). MS: 688(M+1)⁺.

***Step C: (S)-N-((S)-1-(2-chlorophenyl)-2-(3,3-difluorocyclobutylamino)-2-oxoethyl)-N-(3-fluorophenyl)pyrrolidine-2-carboxamide.*** To a solution of (*S*)-(9*H*-fluoren-9-yl)methyl 2-(*N-*((*S*)-(3,3-difluoro-cyclobutylcarbamoyl)(2-chlorophenyl)methyl)-*N*-(3-fluorophenyl) carbamoyl)pyrrolidine-1-carboxylate (370 mg, 0.53 mmol) in CH₃CN (5 mL) was added piperidine (1 mL) dropwise. The mixture was stirred at 40 °C overnight and treated with H₂O (10 mL). The resulting mixture was extracted with EtOAc (3 x 50 mL). The combined organic layers were dried over anhydrous MgSO₄ and concentrated to give the desired product (145 mg, 57% yield). MS: 466 (M+1)⁺.

***Step D: Compound 69.*** A mixture of (2*S*)-*N*-((*S*)-(3,3-difluorocyclobutylcarbamoyl)-(2-chlorophenyl)-methyl)-*N*-(3-fluorophenyl)pyrrolidine-2-carboxamide (40 mg, 0.08 mmol), 2-bromo-pyrimidine (100 mg, 0.5 mmol), Pd₂(dba)₃ (10 mg, 0.008 mmol), BINAP (10 mg, 0.012 mmol), NaO*t*Bu (24 mg, 0.2 mmol) in toluene (10 mL) was stirred at 100 °C under the atmosphere of N₂ overnight. The mixture was filtered, washed with EtOAc (3 x 20 mL). The combined organic layers were concentrated and the residue was purified by prep-TLC using Petroleum ether / EtOAc (V:V, 3:2) and then DCM/acetone (V:V, 30:1) as eluent to give the desired compound (10 mg, 23% yield). ¹H NMR (400 MHz, CDCl₃): δ 8.30-8.29 (m, 2H), 7.31-7.29 (m, 1H), 7.15-7.10 (m, 3H), 7.30-6.94 (m,2H), 6.88-6.83 (m, 1H), 6.50-6.48 (t, 1H), 6.43 (s, 1H), 6.20-6.18 (m, 1H), 4.41-4.38 (m, 1H), 4.30-4.30 (m, 1H), 3.77-3.64 (m, 2H), 3.01-2.93 (m, 2H), 2.50-2.35 (m, 2H), 2.16-2.07 (m, 2H), 1.97-1.83 (m, 2H). MS: 544 (M+1)⁺.

The following compounds were prepared according to the above scheme using the corresponding starting material.

### Compound 102

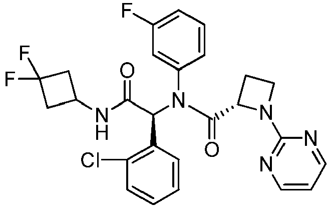

¹H NMR (400 MHz, CDCl₃): δ 8.23 (s, 2H), 7.36 (s, 2H), 7.11 (s, 5H), 6.47 (s, 3H), 4.77 (s, 1H), 4.23 (s, 2H), 3.95 (s, 1H), 3.01 (s, 2H), 2.30 (d, *J* = 110.8 Hz, 4H). MS: 530.2 (M+1)⁺.

### Compound 105

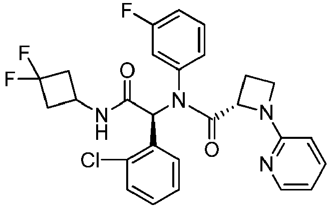

¹H NMR (400 MHz, CDCl₃): δ 7.98 (d, *J* = 4.3 Hz, 1H), 7.36 (d, *J* = 7.8 Hz, 1H), 7.16 (dd, *J* = 14.8, 7.2 Hz, 5H), 7.02 (t, *J* = 7.6 Hz, 1H), 6.89 (t, *J* = 7.5 Hz, 1H), 6.47 (m, 2H), 6.24 (d, *J* = 8.4 Hz, 1H), 4.64 (t, *J* = 7.6 Hz, 1H), 4.36 - 4.23 (m, 1H), 4.04 - 3.94 (m, 1H), 3.74 (q, *J* = 7.3 Hz, 1H), 3.04 - 2.78 (m, 2H), 2.73 - 2.28 (m, 3H), 1.95 (m, 2H). MS: 529.2 (M+1)⁺.

### Example 18. Preparation of (2S,4R)-N-((S)-1-(2-chlorophenyl)-2-(3,3-difluorocyclobutyl-amino)-2-oxoethyl)-N-(3-fluorophenyl)-4-hydroxy-1-(pyrimidin-2-yl)pyrrolidine-2-carbox-amide

Compound **100** was prepared according to the following scheme, using the following protocol.

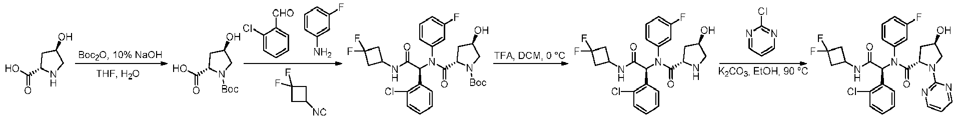

***Step A: (2S,4R)-1-(tert-Butoxycarbonyl)-4-hydroxypyrrolidine-2-carboxylic acid.*** To a mixture of (2*S*,4*R*)-4-hydroxypyrrolidine-2-carboxylic acid (2.5 g, 19 mmol) and 10% aqueous NaOH (8.0 mL) in 38 mL of a mixture of THF/H₂O (V:V, 2:1) was treated first with and then with Boc₂O (6.0 g, 28 mmol). The reaction mixture was stirred at r.t. overnight and then the THF was removed in vacuo. The residue was adjusted to pH=2 by the addition of 10% aqueous KHSO₄ and then extracted with EtOAc (3 x 30 mL). The combined organic layers were washed with water, brine and then dried over anhydrous Na₂SO₄. The solvent was removed in vacuo to give the crude product as a syrup which was used without further purification (4.2 g, 95% yield).

***Step B: (2S,4R)-tert-Butyl 2-(((S)-1-(2-chlorophenyl)-2-(3,3-difluorocyclobutylamino)-2-oxo-ethyl)(3-fluorophenyl)carbamoyl)-4-hydroxypyrrolidine-1-carboxylate.*** 2-chlorobenzaldehyde (598 mg, 4.27 mmol), 3-fluoroaniline (474 mg, 4.27 mmol), (2*S*,4*R*)-1-(*tert*-butoxycarbonyl)-4-hydroxypyrrolidine -2-carboxylic acid (987 mg, 4.27 mmol) and 1,1-difluoro-3-isocyanocyclobutane (500 mg, 4.27 mmol) were used for the UGI reaction and the diastereomers were purified by column chromatography using DCM/MeOH (V:V, 30:1) as eluent to afford the desired (2*S*,4*R*)-*tert*-butyl 2-(((*R*)-1-(2-chlorophenyl)-2-(3,3-difluorocyclobutylamino)-2-oxoethyl)(3-fluorophenyl)carbamoyl)-4-hydroxypyrrolidine-1-carboxylate as a white solid (1.0 g, 80% yield). MS: 582.2 (M+1)⁺.

***Step C: (2S,4R)-N-((S)-1-(2-Chlorophenyl)-2-(3,3-difluorocyclobutylamino)-2-oxoethyl)-N-(3-fluorophenyl)-4-hydroxypyrrolidine-2-carboxamide.*** To a solution of (2*S*,4*R*)-*tert*-butyl 2-(((*S*)-1-(2-chlorophenyl)-2-(3,3-difluorocyclobutylamino)-2-oxoethyl)(3-fluorophenyl)carbamoyl)-4-hydroxypyrrolidine-1-carboxylate (100 mg, 0.172 mmol) in DCM (3 mL) was added TFA (1 mL) dropwise at 0 °C. The resulting mixture was stirred at 0 °C for 1 h and treated with saturated NaHCO₃ until pH=7. The mixture was extracted with DCM (3 x 10 mL) and the combined organic layers were dried over anhydrous Na₂SO₄ and concentrated to give the crude product which was directly used in next step (80 mg, 96% yield). MS: 482.2 (M+1)⁺.

***Step D: Compound 100.*** To a solution of 2-chloropyrimidine (18 mg, 0.158 mmol) and (2*S*,4*R*)-*N*-((*S*)-1-(2-chlorophenyl)-2-(3,3-difluorocyclobutylamino)-2-oxoethyl)-*N*-(3-fluorophenyl)-4-hydroxypyrrolidine-2-carboxamide (70 mg, 0.146 mmol) in EtOH (2 mL) was added K₂CO₃ (42 mg, 0.304 mmol). The reaction vessel was sealed and irradiated by microwave at 90 °C for 10 min. After cooled to r.t., water (10 mL) was added and the resulting mixture was extracted with EtOAc (3 x 20 mL). The combined organic layers were washed with water (3 x 20 mL) and brine (20 mL), dried over anhydrous Na₂SO₄ and concentrated in vacuo. The residue was purified by column chromatography on silica gel using DCM/MeOH (V:V, 20:1) as eluent to afford the desired product as a white solid (15 mg, 19% yield). ¹H NMR (400 MHz, CD₃OD): δ 8.44 - 8.32 (m, 2H), 7.69 (m, 1H), 7.49 - 7.26 (m, 2H), 7.25 - 7.00 (m, 3H), 6.99 - 6.91 (m, 2H), 6.70 - 6.63 (m, 1H), 6.56 - 6.48 (m, 1H), 4.65 - 4.47 (m, 2H), 4.29 - 4.16 (m, 1H), 3.85 - 3.68 (m, 2H), 3.01 - 2.85 (m, 2H), 2.66 - 2.38 (m, 2H), 2.31 - 2.21 (m, 1H), 2.06 - 1.96 (m, 1H). MS: 560.1 (M+1)⁺.

### Example 19. Preparation of (2S,4R)-N-((S)-1-(2-chlorophenyl)-2-(3,3-difluorocyclobutyl-amino)-2-oxoethyl)-N-(3-fluorophenyl)-4-hydroxy-1-(pyrimidin-2-yl)pyrrolidine-2-carbox-amide

Compound **107** was prepared according to the following scheme, using the following protocol.

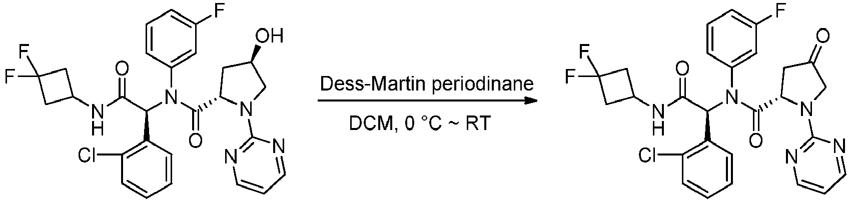

***Step A:*** To a stirred solution of (*2S*,*4R*)*-N*-((*S*)-1-(2-chlorophenyl)-2-(3,3-difluorocyclobutyl-amino)-2-oxoethyl)-*N*-(3-fluorophenyl)-4-hydroxy-1-(pyrimidin-2-yl)pyrrolidine-2-carboxamide (40 mg, 0.071 mmol) in DCM (10 mL) was added Dess-Martin reagent (46 mg, 0.108 mmol) at 0 °C. The resulting mixture was stirred at r.t. overnight and then treated with water (10 mL). The mixture was extracted with DCM (3 x 10 mL), washed with water (10 mL) and brine (10 mL). The organic phase was dried over anhydrous Na₂SO₄ and concentrated. The residue was purified by prep-HPLC to afford the desired product as a white solid (15 mg, 37% yield). ¹H NMR (400 MHz, CD₃OD): δ 8.47 (s, 2H), 7.72 - 7.61 (m, 1H), 7.45 - 7.31 (m, 2H), 7.26 - 7.17 (m, 2H), 7.12-6.90 (m, 4H), 6.81 (t, *J* = 4.6 Hz, 1H), 6.47 (m, 1H), 5.00 - 4.96 (m, 1H), 4.30 - 4.17 (m, 1H), 2.99 - 2.87 (m, 2H), 2.84 - 2.64 (m, 2H), 2.63 - 2.34 (m, 3H), 2.31 - 2.09 (m, 1H); MS: 558.1 (M+1)⁺.

### Example 20. Preparation of methyl 2-((1-(2-chlorophenyl)-2-(3,3-difluorocyclohexylamino)-2-oxoethyl) (3-fluoro-phenyl)amino)-2-oxoethylcarbamate

Compound **94** and **95** were prepared according to the following scheme, using the following protocol.

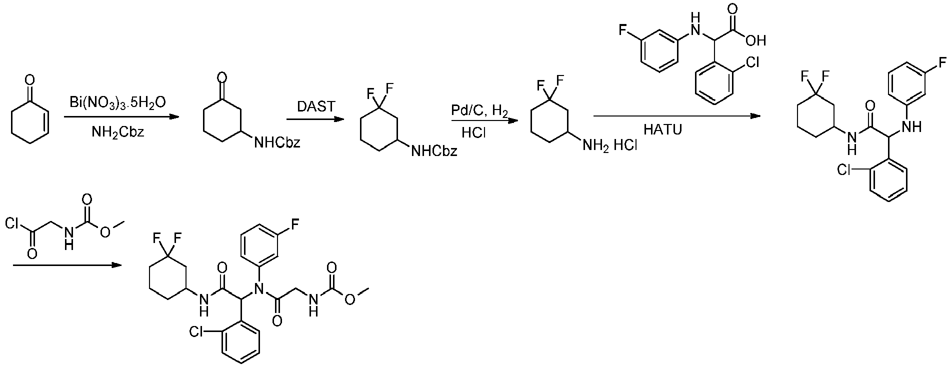

***Step A: (3-Oxo-cyclohexyl)-carbamic acid benzyl ester.*** Bismuth nitrate pentahydrate (1.52g, 3.13 mmol) was added to a mixture of benzyl carbamate (4.73 g, 31.3 mmol) and cyclohex-2-enone (3.0 mL, 31.3 mmol) in CH₂Cl₂ (3 mL), and the resulting syrup was vigorously stirred at room temperature overnight. The reaction mixture was filtered through a pad of Celite. The filtrate was washed with saturated aq. NaHCO₃. The organic layer was dried over anhydrous Na₂SO₄ and concentrated in vacuo. The residue was purified silica gel column chromatography using petroleum ether / EtOAc (V:V, 3:1) as eluent to provide the title compound as a pale yellow gum (5.35 g, 69% yield). ¹H NMR (400 MHz, CDCl₃) : δ 7.46 - 7.31 (m, 5H), 5.11 (s, 2H), 4.90 (s, 1H), 4.01 (s, 1H), 2.73 (m, 1H), 2.39 (m, 1H), 2.28 (m, 2H), 2.10 (m, 1H), 1.99 (m, 1H), 1.72 (m, 2H). MS: 248.1 (M+1)⁺.

***Step B: Benzyl 3,3-difluorocyclohexylcarbamate.*** To a solution of (3-oxo-cyclohexyl)-carbamic acid benzyl ester (0.25 g, 1 mmol) in CH₂Cl₂ (3 mL) at 0 °C under the atmosphere of nitrogen was added DAST (0.37 mL, 2 mmol) dropwise. After addition, the reaction mixture was allowed to warm to room temperature and stirred overnight and treated with saturated NaHCO₃ (5 mL). The organic layer was separated and the water phase was extracted with DCM (3 x 10 mL). The combined organic layers were washed with aq. NaHCO₃, brine, dried over anhydrous Na₂SO₄ and concentrated in vacuo. The residue was purified by column chromatography to give the desired product as white solid (110 mg, 40.7% yield). ¹H NMR (400 MHz, CDCl₃) : δ 7.52 - 7.31 (m, 5H), 5.12 (s, 2H), 4.95 (s, 1H), 3.95 (s, 1H), 2.34 (m, 1H), 2.05 - 1.40 (m, 6H). MS: 270.1 (M+1)⁺.

***Step C: 3,3-Difluorocyclohexanamine hydrochloride.*** A solution of benzyl 3,3-difluorocyclohexylcarbamate (1.42 g, 5.28 mmol) and Pd/C (10%, 0.3 g) in MeOH (40 mL) was stirred under the atmosphere of H₂ overnight. The reaction was filtered through the Celite pad and the filtrate was evaporated in vacuo to afford the desired product which was used directly without further purification (0.87 g, 95% yield). MS: 136.1 (M+1)⁺.

***Step D: 2-(2-Chlorophenyl)-N-(3,3-difluorocyclohexyl)-2-(3-fluorophenylamino)-acetamide.*** To a solution of (3-fluoro-phenylamino)-phenyl-acetic acid (0.87 g, 5.60 mmol) in DCM (20 mL) were added HATU (3.88 g, 10.2 mmol), DIPEA (2.53 mL, 15.3mmol) and 3,3-difluoro-cyclohexylamine hydrochloride (0.87 g, 5.09 mmol) at 0 °C. The reaction mixture was allowed to r.t. overnight and treated with 30 mL of water. The organic layer was separated and the water phase was extracted with DCM (3 x 20 mL). The combined organic layers were washed with saturated aq. NaHCO₃, brine, dried over anhydrous Na₂SO₄ and concentrated in vacuo. The residue was purified by flash column chromatography to give the desired product as an off-white solid (1.2 g, 97% yield, mixture of epimers). MS: 397.1 (M+1)⁺.

### Step E: Methyl 2-((1-(2-chlorophenyl)-2-(3,3-difluorocyclohexylamino)-2-oxoethyl) (3-fluorophenyl)amino)-2-oxoethylcarbamate.

To a suspension of 2-(methoxycarbonylamino)acetic acid (300 mg, 2.26 mmol) in DCM (4 mL) was added oxalyl chloride dropwise at 0 °C with one drop of DMF. The mixture was then allowed to warm up to r.t. and stirred for 2 hr. The solvent was removed in vacuo and the residue was used directly without further purification.

A mixture of 2-(2-chlorophenyl)-*N*-(3,3-difluorocyclohexyl)-2-(3-fluoro phenylamino)acetamide (300 mg, 0.756 mmol) and methyl 2-chloro-2-oxoethylcarbamate (230 mg, 1.51 mmol) in toluene (4 mL) was heated to 100 °C for 2 hr. The resulting mixture was cooled and diluted with DCM (30 mL) and saturated aq. NaHCO₃ (10 mL). The organic layer was separated and the aqueous phase was extracted with DCM (3 x 20 mL). The combined organic layers were washed with brine, dried over anhydrous Na₂SO₄ and concentrated in vacuo. The residue was purified by column chromatography to give the desired product as white solid (isomer A : 50 mg, 13% yield; isomer B: 40 mg, 10% yield). MS: 512.1 (M+1)⁺.
Isomer A: ¹H NMR (400 MHz, CDCl₃): δ 7.37 (d, *J* = 8.0 Hz, 1H), 7.29 (s, 1H), 7.18 (s, 2H), 6.98 (d, *J* = 29.8 Hz, 3H), 6.42 (s, 1H), 5.97 (s, 1H), 5.59 (s, 1H), 4.25 (s, 1H), 3.76 (m, 2H), 3.67 (s, 3H), 2.45-2.35 (m, 1H), 2.08-1.93 (m, 1H), 1.91-1.58 (m,6H).
Isomer B: ¹H NMR (400 MHz, CDCl₃): δ 7.36 (d, *J* = 7.9 Hz,1H), 7.19 (s, 1H), 7.02 (d, *J* = 4.1 Hz, 2H), 6.94 (s, 2H), 6.41 (s, 1H), 5.89 (s, 1H), 5.57 (s, 1H), 4.26 (m, 1H), 3.72 (m, 2H), 3.65 (s, 3H), 2.33- 2.22 (m, 1H), 2.08-1.96 (m, 1H), 1.88-1.53 (m, 6H).

### Example 21: (2S,5S)-N-((R)-1-(2-chlorophenyl)-2-(4,4-difluorocyclohexylamino)-2-oxoethyl)-N-(3-fluorophenyl)-5-methyl-1-(pyrimidin-2-yl)pyrrolidine-2-carboxamide (Compound 110) and (2S,5S)-N-((S)-1-(2-chlorophenyl)-2-(4,4-difluorocyclohexylamino)-2-oxo ethyl)-N-(3-fluorophenyl)-5-methyl-1-(pyrimidin-2-yl)pyrrolidine-2-carboxamide (Compound 111)

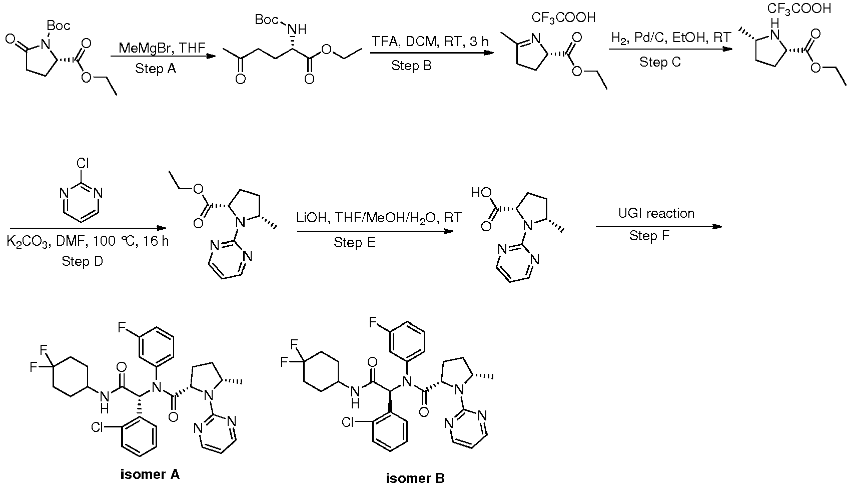

***Step A. (S)-ethyl 2-((tert-butoxycarbonyl)amino)-5-oxohexanoate.*** To a solution of (S)-1-tert-butyl 2-ethyl 5-oxopyrrolidine-1,2-dicarboxylate (4.0 g, 15.6 mmol) in THF (40 mL) was dropwise added a solution of methylmagnesium bromide (1 M, 16 mL) in THF at -40 °C. The resulting mixture was stirred at -40 °C to -20 °C for 3 hr and then at 0 °C overnight. The mixture was quenched with saturated aqueous NH₄Cl solution and then extracted with EtOAc (3 x 50 mL). The combined organic layers were washed with water (3 x 50 mL) and brine (50 mL), dried over anhydrous Na₂SO₄ and then concentrated in high vacuum. The residue was purified by column chromatography on silica gel (PE/EtOAc=20/1) to afford the desired product (2.7 g, 64% yield).

***Step B: (S)-ethyl 5-methyl-3,4-dihydro-2H-pyrrole-2-carboxylate 2,2,2-trifluoro acetate salt.*** To a solution of (S)-ethyl 2-(tert-butoxycarbonylamino)-5-oxohexanoate (2.7 g, 9.9 mmol) in DCM (30 mL) was added TFA (10 mL). The resulting mixture was stirred at r.t. for 3 hr. The solvent was removed under reduced pressure to afford the crude product (2.66 g, quant. yield) which was directly used in next step without further purification.

***Step C: (2S,5S)-ethyl 5-methylpyrrolidine-2-carboxylate 2,2,2-trifluoro acetate salt.*** To a solution of (S)-ethyl 5-methyl-3,4-dihydro-2H-pyrrole-2-carboxylate 2,2,2-trifluoroacetate (2.66 g, 9.89 mmol) in EtOH (30 mL) was added Pd/C (10%, 0.3 g). The mixture was purged with H₂ three times and then stirred at r.t. under H₂ atmosphere overnight. The resulting mixture was filtered and the filtrate was concentrated to afford desired product (1.6 g, 60% yield) which was directly used in the next step without further purification.

***Step D: (2S,5S)-ethyl 5-methyl-1-(pyrimidin-2-yl)pyrrolidine-2-carboxylate.*** To a solution of (2S,5S)-ethyl 5-methylpyrrolidine-2-carboxylate 2,2,2-trifluoroacetate (1.6 g, 5.9 mmol) in DMF (20 mL) was added K₂CO₃ (2.4 g, 17.4 mmol). The reaction was stirred at r.t. for 10 min followed by addition of 2-chloropyrimidine (1.0 g, 8.8 mmol). The resulting mixture was stirred at 100 °C overnight and then quenched with water (20 mL). The resulting mixture was extracted with EtOAc (3 x 30 mL). The combined organic layers were washed in sequence with water (3 x 30 mL) and brine (30 mL), dried over anhydrous Na₂SO₄ and then concentrated. The residue was purified by column chromatography on silica gel (DCM/MeOH = 30/1) to afford the desired product (800 mg, 57% yield).

***Step E: (2S,5S)-5-methyl-1-(pyrimidin-2-yl)pyrrolidine-2-carboxylic acid.*** To a solution of (2S,5S)-ethyl 5-methyl-1-(pyrimidin-2-yl)pyrrolidine-2-carboxylate (750 mg, 3.19 mmol) in THF/MeOH/H₂O (1/1/1, 10 mL) was added LiOH (153 mg, 6.38 mmol). The resulting mixture was stirred at r.t. for 2 hr and then acidified with 1 N HCl to adjust pH = 2. The mixture was extracted with DCM (5 x 15 mL). The combined organic layers were dried over anhydrous Na₂SO₄ and then concentrated to give the desired product (400 mg, 60% yield) which was directly used in the next step without further purification.

***Step F: (2S,5S)-N-((R)-1-(2-chlorophenyl)-2-(4,4-difluorocyclohexylamino)-2-oxoethyl)-N-(3-fluorophenyl)-5-methyl-1-(pyrimidin-2-yl)pyrrolidine-2-carboxamide and (2S,5S)-N-((S)-1-(2-chlorophenyl)-2-(4,4-difluorocyclohexylamino)-2-oxoethyl)-N-(3-fluorophenyl)-5-methyl-1-(pyrimidin-2-yl)pyrrolidine-2-carbox-amide.***

### General procedures for the UGI reaction:

A mixture of aldehyde (3.5 mmol) and aniline (3.5 mmol) in MeOH (8 mL) was stirred at room temperature for 30 min. Then acid (3.5 mmol) was added and the reaction mixture was stirred for another 30 min, followed by addition of isocyanide (3.5 mmol). The resulting mixture was then stirred at room temperature overnight and quenched with H₂O. The resulting mixture was partitioned between EtOAc and H₂O. The organic layer was washed with brine, dried over anhydrous Na₂SO₄, and then concentrated. The residue was purified by silica gel column chromatography using DCM/MeOH as eluent to afford the desired product.

The following analogs were synthesized via the procedure set forth herein, using the appropriate aldehyde, amine, carboxylic acid, isocyanide and using the reagents and solvents set forth herein, and purified via various standard methods.

### Compound 110: (2S,5S)-N-((R)-1-(2-chlorophenyl)-2-(4,4-difluorocyclohexylamino)-2-oxoethyl)-N-(3-fluorophenyl)-5-methyl-1-(pyrimidin-2-yl)pyrrolidine-2-carboxamide

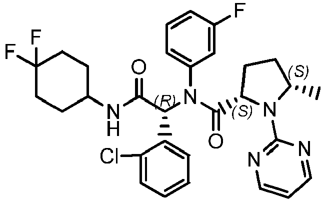

¹H NMR(400 MHz, CDCl₃) : δ 8.31 (d, *J* = 4.8 Hz, 2H), 7.67 (s, 1H), 7.32 (d, *J* = 8.1 Hz, 1H), 7.26 - 6.78 (m, 6H), 6.55 (d, *J* = 5.9 Hz, 1H), 6.52 - 6.47 (m, 1H), 6.01 - 5.88 (m, 1H), 4.41 - 4.25 (m, 2H), 4.05 - 3.90 (m, 1H), 2.26 - 1.68 (m, 11H), 1.64 - 1.42 (m, 2H), 1.39 (d, *J* = 8.4 Hz, 3H). MS: 586.3 (M+1)⁺.

### Compound 111: (2S,5S)-N-((S)-1-(2-chlorophenyl)-2-(4,4-difluorocyclohexylamino)-2-oxoethyl)-N-(3-fluorophenyl)-5-methyl-1-(pyrimidin-2-yl)pyrrolidine-2-carboxamide.

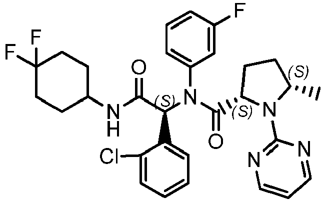

¹H NMR (400 MHz, CDCl₃) : δ 8.32 (d, *J* = 4.8 Hz, 2H), 7.65 (s, 1H), 7.36 (d, *J* = 8.2 Hz, 1H), 7.00 (m, 6H), 6.50 (m, 2H), 5.69 (s, 1H), 4.45 - 4.26 (m, 2H), 4.00 (m, 1H), 2.32 - 1.81 (m, 12H), 1.43 (m, 4H). MS: 586.3 (M+1)⁺.

### Compound 112: (2S,5S)-N-((R)-1-(2-chlorophenyl)-2-(3,3-difluorocyclobutylamino)-2-oxoethyl)-N-(3-fluorophenyl)-5-methyl-1-(pyrimidin-2-yl)pyrrolidine-2-carboxamide

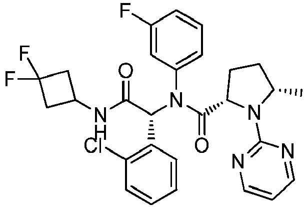

¹HNMR (400 MHz, CDCl₃) : δ 8.32 (d, *J* = 4.8 Hz, 2H), 7.64 (m, 1H), 7.55 - 7.34 (m, 1H), 7.18 -6.73 (m, 6H), 6.55 (s, 1H), 6.51 (t, *J* = 4.8 Hz, 1H), 6.47 - 6.38 (m, 1H), 4.48 - 4.19 (m, 3H), 3.06 - 2.97 (m, 2H), 2.63 - 2.28 (m, 2H), 2.26 - 1.83 (m, 4H), 1.35 (d, *J* = 6.4 Hz, 3H). MS: 558.2 (M+1)⁺.

### Compound 113: (2S,5S)-N-((S)-1-(2-chlorophenyl)-2-(3,3-difluorocyclobutylamino)-2-oxoethyl)-N-(3-fluorophenyl)-5-methyl-1-(pyrimidin-2-yl)pyrrolidine-2-carboxamide

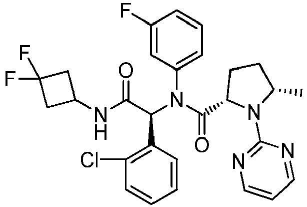

¹H NMR (400 MHz, CDCl₃) δ 8.33 (d, *J* = 4.8 Hz, 2H), 7.65 (m, 1H), 7.26 (m, 1H), 7.20 - 6.82 (m, 6H), 6.65 (m, 1H), 6.58 (s, 1H), 6.53 (t, *J* = 4.8 Hz, 1H), 4.31 (m, 3H), 3.10 - 2.97 (m, 2H), 2.61 - 2.29 (m, 2H), 2.23 - 1.85 (m, 4H), 1.27 (d, *J* = 3.3 Hz, 3H). MS: 558.2 (M+1)⁺.

### Example 22: (R)-methyl 5-(((S)-1-(2-chlorophenyl)-2-((3,3-difluorocyclobutyl)amino)-2-oxoethyl)(3-fluorophenyl)carbamoyl)-2,2-dimethylpyrrolidine-1-carboxylate (Compound 114) and (S)-methyl 5-(((S)-1-(2-chlorophenyl)-2-((3,3-difluorocyclobutyl)amino)-2-oxoethyl)(3-fluorophenyl)carbamoyl)-2,2-dimethylpyrrolidine-1-carboxylate (Compound 115)

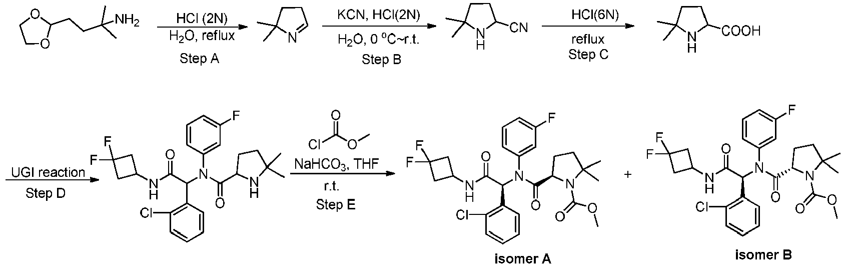

***Step A. Synthesis of 2,2-dimethyl-3,4-dihydro-2H-pyrrole.*** 3-[1,3]Dioxolan-2-yl-1,1-dimethyl-propylamine (4.8 g, 30 mmol) was dissolved in hot water (20 mL). The resulting solution was adjusted with 2 N HCl to pH=3 and then heated to reflux for 30 min. The mixture was neutralized with 6 N aq. KOH solution and extracted with chloroform (4 x 20 mL). The combined organic layers were dried over anhydrous Na₂SO₄ and then concentrated under high vacuum. The residue was purified by column chromatography to give the desired product (1.37 g, 47% yield) as clear pungent oil. ¹H NMR (400 MHz, CD₃OD): δ 7.38 (s, 1 H), 2.57 (t, *J* = 8.0 Hz, 2H), 1.60 (t, *J* = 8.0 Hz, 2H), 1.21 (s, 2H).
***Step B. Synthesis of 5,5-dimethylpyrrolidine-2-carbonitrile.*** To a solution of 2,2-Dimethyl-3,4-dihydro-2H-pyrrole (1.2 g, 12.2 mmole) in water (70 mL) at 0 °C was added KCN (1.6 g, 24.4 mmole). The solution was acidified with 2 N HCl solution to pH= 6. After stirred at 0 °C for 3 hr, the solution was neutralized with 2 N NaOH solution and extracted with chloroform (3 x 50 mL). The combined organic layers were dried over anhydrous Na₂SO₄, and concentrated. The residue was purified by column chromatography to give the desired product (0.67 g, 45% yield) as pale yellow oil. ¹H NMR (400 MHz, CD₃OD): δ 4.10 - 4.00 (m, 1 H), 2.30 - 2.17 (m, 2 H), 1.86-1.80 (m, 1 H), 1.71 - 1.60 (m, 2 H), 1.29 (s, 3H), 1.15 (s, 3H).
***Step C. 5,5-dimethylpyrrolidine-2-carboxylic acid.*** 5,5-Dimethyl-pyrrolidine-2- carbonitrile (0.67 g, 5.4 mmol) was dissolved in 6 N HCl. The mixture was refluxed overnight and then concentrated under high vacuum. The residue was precipitated by Et₂O to give the desired product (0.66 g, 86%). MS: 144 (M⁺+1).
***Step D. N-(1-(2-chlorophenyl)-2-(3,3-difluorocyclobutylamino)-2-oxoethyl)-N-(3-fluorophenyl)-5,5-dimethylpyrrolidine-2-carboxamide. The same as general procedure for UGI reaction set forth above.***
***Step E. (R)-methyl 5-(((S)-1-(2-chlorophenyl)-2-((3,3-difluorocyclobutyl)amino)-2-oxo-ethyl)(3-fluorophenyl)carbamoyl)-2,2-dimethylpyrrolidine-1-carboxylate and (S)-methyl 5-(((S)-1-(2-chlorophenyl)-2-((3,3-difluorocyclobutyl)amino)-2-oxoeth-yl)(3-fluorophenyl)carbamoyl)-2,2-dimethylpyrrolidine-1-carboxylate.*** To a solution of N-(1-(2-chloro phenyl)-2-(3,3-difluorocyclobutylamino)-2-oxoethyl)-N-(3-fluoro -phenyl)-5,5-dimethylpyrrolidine-2-carboxamide (20 mg, 0.04 mmol) in THF (10 mL) at 0 °C, was slowly added methyl carbonochloridate (0.5 mL) and satd. aq. NaHCO₃. The reaction mixture was stirred at r.t. overnight and then extracted with EtOAc (3 x 10 mL). The combined organic layers were dried over anhydrous Na₂SO₄, and then concentrated in high vacuum. The residue was purified by prep-TLC to give the two desired isomers.

### Compound 114: (R)-methyl 5-(((S)-1-(2-chlorophenyl)-2-((3,3-difluorocyclobutyl)amino)-2-oxo-eth-yl)(3-fluorophenyl)carbamoyl)-2,2-dimethylpyrrolidine-1-carboxylate (isomer A)

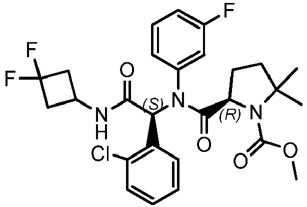

¹H NMR (400 MHz, CD₃OD): δ 8.35 (s, 1H), 7.35 - 7.25 (m, 2H), 7.15 - 7.00 (m, 1H), 6.90 - 6.84 (m, 3H), 6.80 - 6.75 (m, 1H), 6.35 - 6.22 (m, 1H), 4.49 - 4.42 (m, 1H), 4.10 - 4.00 (m, 1H), 3.74 (s, 3H), 3.20 - 2.85 (m, 3H), 2.80 - 2.70 (m, 1H), 2.20 - 1.90 (m, 2H), 1.85 - 1.75 (m, 1H), 1.70 - 1.50 (m, 2H), 1.49 (s, 3H), 1.30 (s, 3H). MS: 552 (M⁺+1).

### Compound 115: (S)-methyl 5-(((S)-1-(2-chlorophenyl)-2-((3,3-difluorocyclobutyl)amino)-2-oxoeth-yl)(3-fluorophenyl)carbamoyl)-2,2-dimethylpyrrolidine-1-carboxylate (isomer B)

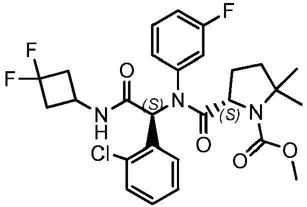

¹H NMR (400 MHz, CD₃OD): δ 7.69 (s, 1H), 7.40 - 7.10 (m, 3H), 7.00 - 6.80 (m, 3H), 6.75 - 6.55 (m, 1H), 6.40 - 6.20 (m, 1H), 4.40 - 4.20 (m, 2H), 3.70 (s, 3H), 3.10 - 2.90 (m, 2H), 2.63 - 2.25 (m, 2H), 2.20 - 1.95 (m, 1H), 1.90 - 1.60 (m, 4H), 1.70 - 1.50 (m, 2H), 1.45 (s, 3H), 1.28 (s, 3H). MS: 552 (M⁺+1).

### Example 23: (S)-N-((R)-1-(2-chlorophenyl)-2-((3,3-difluorocyclobutyl)amino)-2-oxo ethyl)-N-(3,5-difluorophenyl)indoline-2-carboxamide (Compound 117) and (S)-N-((S)-1-(2-chlorophenyl)-2-((3,3-difluorocyclobutyl)amino)-2-oxoethyl) -N-(3,5-difluorophenyl)indoline-2-carboxamide (Compound 118)

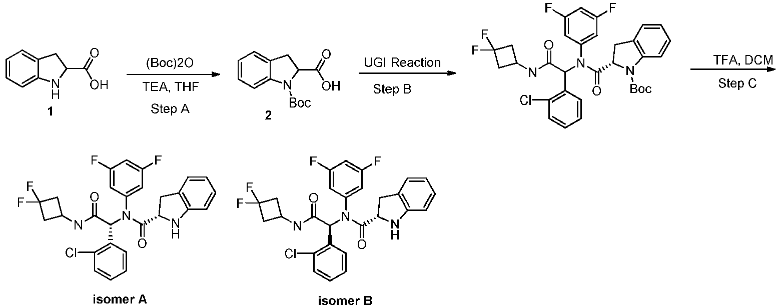

***Step A: 1-(tert-butoxycarbonyl)indoline-2-carboxylic** acid.* To a solution of indoline-2-carboxylic acid (1 g, 6.1 mmol) in THF (10 mL) was added TEA (2.6 mL, 18.3 mmol) and (Boc)₂O (2.8 mL, 12.3 mmol). The mixture was stirred at r.t. overnight and then concentrated under high vacuum. The residue was dissolved in 1 N NaOH (20 mL) and washed with ether (2 x 10 mL). The aqueous layer was acidified with 1 N HCl, and extracted with ether (3 x 10 mL). The organic layers were dried over anhydrous Na₂SO₄, and concentrated to give the desired product (1.3 g, 80.6% yield) as a white solid. MS: 264.1 (M+1)⁺.

***Step B: (2S)-tert-butyl 2-((1-(2-chlorophenyl)-2-((3,3-difluorocyclobutyl)amino)-2-oxoethyl)(3,5-difluorophenyl)carbamoyl)indoline-1-carboxylate. The same as general procedure for UGI reaction set forth above.***

***Step C: (S)-N-((R)-1-(2-chlorophenyl)-2-((3,3-difluorocyclobutyl)amino)-2-oxo-ethyl)-N-(3,5-difluorophenyl)indoline-2-carboxamide and (S)-N-((S)-1-(2-chloro phenyl)-2-((3,3-difluorocyclobutyl)amino)-2-oxoethyl)-N-(3,5-difluorophenyl)indoline-2-carboxamide.*** To a solution of (2S)-tert-butyl-2-((1-(2-chlorophenyl)-2-((3,3 -difluorocyclobutyl)amino)-2-oxoethyl)-(3,5-difluorophenyl)carbamoyl)indoline-1- carboxylate (500 mg, 0.8 mmol) in DCM (5 mL) was added TFA (3 mL) at 0 °C. The mixture was stirred at r.t. for 40 min and then concentrated under high vacuum. The residue was dissolved in DCM (5 mL) and TEA was added dropwise to adjust pH= 6. The solution was concentrated and the residue was purified by column chromatography on silica gel eluted with PE/EtOAc (10 : 1 to 6 : 1) to give the two desired isomers as white solid.

### Compound 117: (S)-N-((R)-1-(2-chlorophenyl)-2-((3,3-difluorocyclobutyl)amino)-2-oxo-ethyl)-N-(3,5-difluorophenyl)indoline-2-carboxamide (Isomer A):

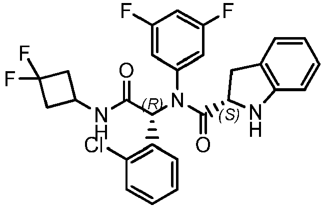

(80 mg, 19.0% yield). ¹H NMR (400 MHz, CDCl₃) : δ 7.38 (t, *J* = 6.9 Hz, 2H), 7.26 - 7.18 (m, 2H), 7.11 - 6.93 (m, 7H), 6.76 - 6.62 (m, 4H), 6.58 - 6.44 (m, 5H), 4.68 (s, 2H), 4.38 (dd, *J* = 10.6, 6.6 Hz, 2H), 4.19 (s, 4H), 3.28-3.22 (m, 2H), 2.97 - 2.82 (m, 5H), 2.47 - 2.15 (m, 4H). MS: 532.1 (M+1)⁺.

### Compound 118: (S)-N-((S)-1-(2-chlorophenyl)-2-((3,3-difluorocyclobutyl)amino)-2-oxo-ethyl)-N-(3,5-difluorophenyl)indoline-2-carboxamide (Isomer B):

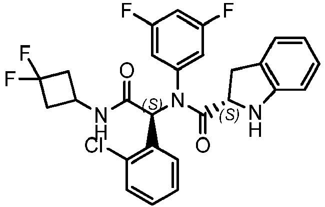

(85 mg, 20.2% yield). ¹H NMR (400 MHz, CDCl₃) : δ 7.38 (d, *J* = 7.8 Hz, 1H), 7.25 - 7.17 (m, 1H), 7.11 - 6.92 (m, 4H), 6.75 - 6.57 (m, 3H), 6.41 - 6.26 (m, 2H), 4.32 (dd, *J* = 10.3, 6.6 Hz, 2H), 3.23-3.18 (m, 1H), 3.05-2.92 (m, 3H), 2.60 - 2.30 (m, 2H). MS: 532.1 (M+1)⁺.

### Example 24: (2S)-N-(1-(2-chlorophenyl)-2-((3,3-difluorocyclobutyl)amino)-2-oxo ethyl)-1-(4-cyanopyridin-2-yl)-N-(3,5-difluorophenyl)indoline-2-carboxamide (Compound 119) General procedures for the Buchwald reaction:

A mixture of amine (0.30 mmol), aryl bromide (0.30 mmol), Cs₂CO₃ (129 mg, 0.39 mmol), Pd₂(dba)₃ (18 mg, 0.02 mmol) and Xant-Phos (9.4 mg, 0.02 mmol) in 1,4-dioxane (10 mL) was stirred under nitrogen atmosphere at 80 °C overnight. After filtration, the filtrate was concentrated in high vacuo and the residue was purified by prep-TLC to give the desired products.

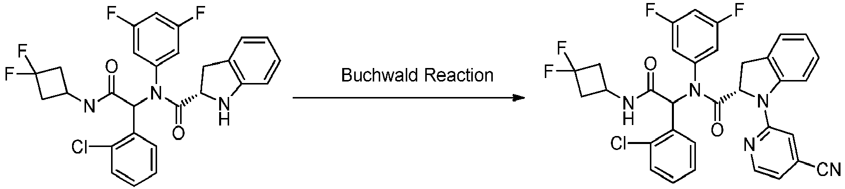

***The product was prepared followed the general procedure for Buchwald reaction set forth above.***
¹H NMR (400 MHz, CDCl₃) : δ 8.51-8.45 (m, 1H), 7.72 - 7.52 (m, 1H), 7.48 - 7.29 (m, 3H), 7.26 - 6.77 (m, 7H), 6.75 - 6.61 (m, 1H), 6.46 - 6.20 (m, 1H), 6.14 - 6.11 (m, 1H), 5.04 - 4.88 (m, 1H), 4.20 (s, 1H), 3.35 - 3.08 (m, 2H), 2.89 (s, 2H), 2.31 (s, 2H). MS: 634.2 (M+1)⁺.

### Example 25: N-(1-(2-chlorophenyl)-2-((3,3-difluorocyclobutyl)amino)-2-oxoethyl)-N-(3-fluorophenyl)-1-(pyrimidin-2-yl)-1H-pyrazole-5-carboxamide (Compound 116)

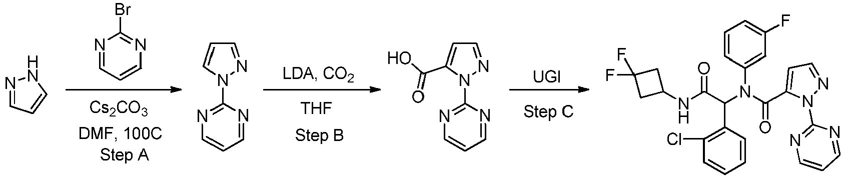

***Step A: 2-(1H-pyrazol-1-yl)pyrimidine.*** A mixture of 1H-pyrazole (1.7 g, 25.3 mmol), 2-bromopyrimidine (4.0 g, 25.3 mmol), Cs₂CO₃ (8.2 g, 25.3 mmol) in DMF (40 mL) was stirred at 100 °C overnight. The resulting reaction mixture was allowed to cool to r.t. and diluted with water. The mixture was extracted with DCM/ Isopropanol (3 : 1, 3 x 40 mL). The combined organic layers were dried over MgSO₄, and concentrated to give the desired product (3.5 g, 94.6% yield) as an orange solid.

***Step B: 1-(pyrimidin-2-yl)-1H-pyrazole-5-carboxylic acid.*** To a mixture of 2-(1H-pyrazol-1-yl)pyrimidine (400 mg, 2.7 mmol) and THF (10 mL) was added dropwise a solution of LDA (2.0 N, 1.7 mL) in hexane/THF/ethylbenzene at -78 °C over 10 min. Excess dry ice was then added into the above solution at that temperature. The resulting mixture was allowed to warm slowly to r.t. and then stirred for another 30 min. The mixture was then partitioned between water and ether. The aqueous layer was acidified by 2 N HCl solution to pH=3 and then extracted with methyl tert-butyl ether (3 x 10 mL). The combined organic layers were washed with brine, dried over anhydrous MgSO₄, and then concentrated to obtain the desired product (250 mg, 48.0% yield) as a white solid.

***Step C: N-(1-(2-chlorophenyl)-2-((3,3-difluorocyclobutyl)amino)-2-oxoethyl)-N-(3-fluorophenyl)-1-(pyrimidin-2-yl)-1H-pyrazole-5-carboxamide. The same as general procedure for UGI reaction set forth above.*** ¹H NMR (400 MHz, CDCl₃) : δ 8.79 (d, *J* = 4.8 Hz, 2H), 7.51 (d, *J* = 1.6 Hz, 1H), 7.36 -7.27 (m, 3H), 7.18 (t, *J* = 7.7 Hz, 1H), 7.11 - 6.96 (m, 3H), 6.90-6.84 (m, 1H), 6.68 (t, *J* = 8.1 Hz, 1H), 6.58 (s, 1H), 6.40 (d, *J* = 6.6 Hz, 1H), 6.29 (d, *J* = 1.6 Hz, 1H), 4.35 (s, 1H), 3.09 - 2.93 (m, 2H), 2.61 - 2.37 (m, 2H). MS: 541.1 (M+1)⁺.

### Example A: In Vitro Assays for IDH1m (R132H or R132C) Inhibitors

A test compound is prepared as 10 mM stock in DMSO and diluted to 50X final concentration in DMSO, for a 50 µl reaction mixture. IDH enzyme activity converting alpha-ketoglutarate to 2-hydroxyglutaric acid is measured using a NADPH depletion assay. In the assay the remaining cofactor is measured at the end of the reaction with the addition of a catalytic excess of diaphorase and resazurin, to generate a fluorescent signal in proportion to the amount of NADPH remaining. IDH1-R132 homodimer enzyme is diluted to 0.125 µg/ml in 40 µl of Assay Buffer(150 mM NaCl, 20 mM Tris-Cl pH 7.5, 10 mM MgCl2, 0.05% BSA, 2 mM b-mercaptoethanol); 1 µl of test compound dilution in DMSO is added and the mixture is incubated for 60 minutes at room temperature. The reaction is started with the addition of 10 µl of Substrate Mix (20 µl NADPH, 5 mM alpha-ketoglutarate, in Assay Buffer) and the mixture is incubated for 90 minutes at room temperature. The reaction is terminated with the addition of 25 µl of Detection Buffer (36 µg/ml diaphorase, 30 mM resazurin, in 1X Assay Buffer), and is incubated for 1 minute before reading on a SpectraMax platereader at Ex544/Em590.

Compounds are assayed for their activity against IDH1 R132C following the same assay as above with the following modifications: Assay Buffer is (50 mM potassium phosphate, pH 6.5; 40 mM sodium carbonate, 5 mM MgCl₂, 10% glycerol, 2 mM b-mercaptoethanol, and 0.03% BSA). The concentration of NADPH and alpha-ketoglutarate in the Substrate Buffer is 20 µM and 1 mM, respectively.

Representative compounds of formula I set forth in Table 1 were tested in this assay and the results are set forth below in Table 3. As used in Table 3, "A" refers to an inhibitory activity against IDH1 R132H or IDH1 R132C with an IC₅₀ ≤ 0.1 µM; "B" refers to an inhibitory activity against IDH1 R132H or IDH1 R132C with an IC₅₀ between 0.1 µM and 0.5 µM; "C" refers to an inhibitory activity against IDH1 R132H or IDH1 R132C with an IC₅₀ between 0.5 µM and 1 µM; "D" refers to an inhibitory activity against IDH1 R132H or IDH1 R132C with an IC₅₀ between 1 µM and 2 µM.

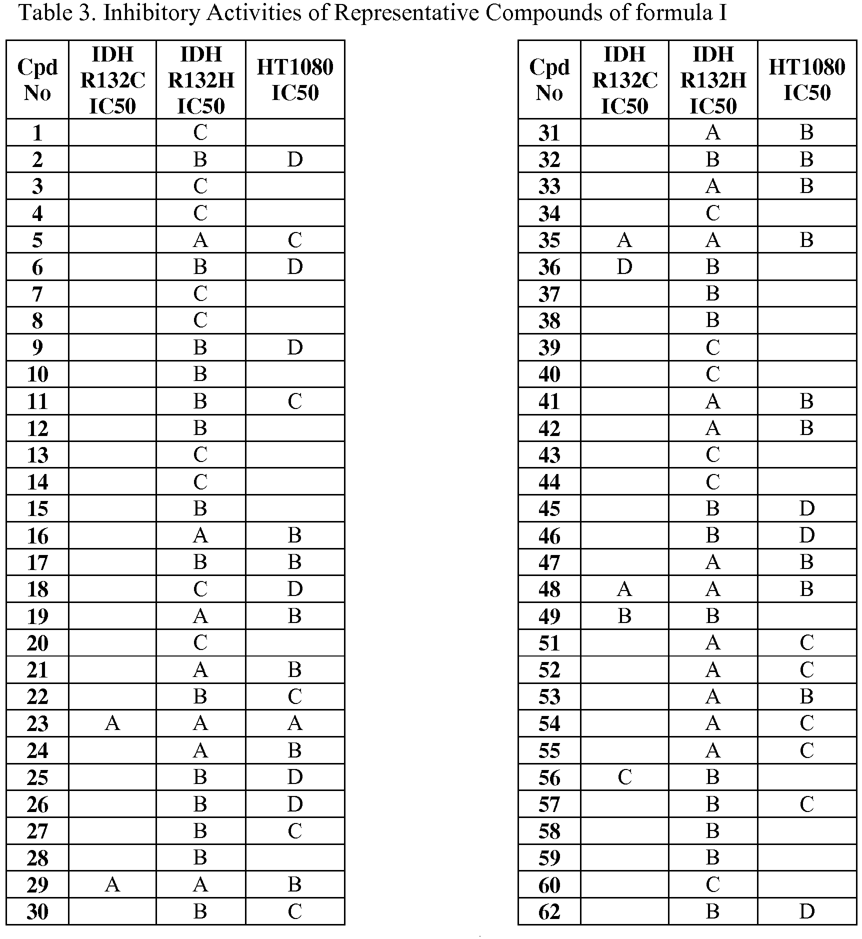

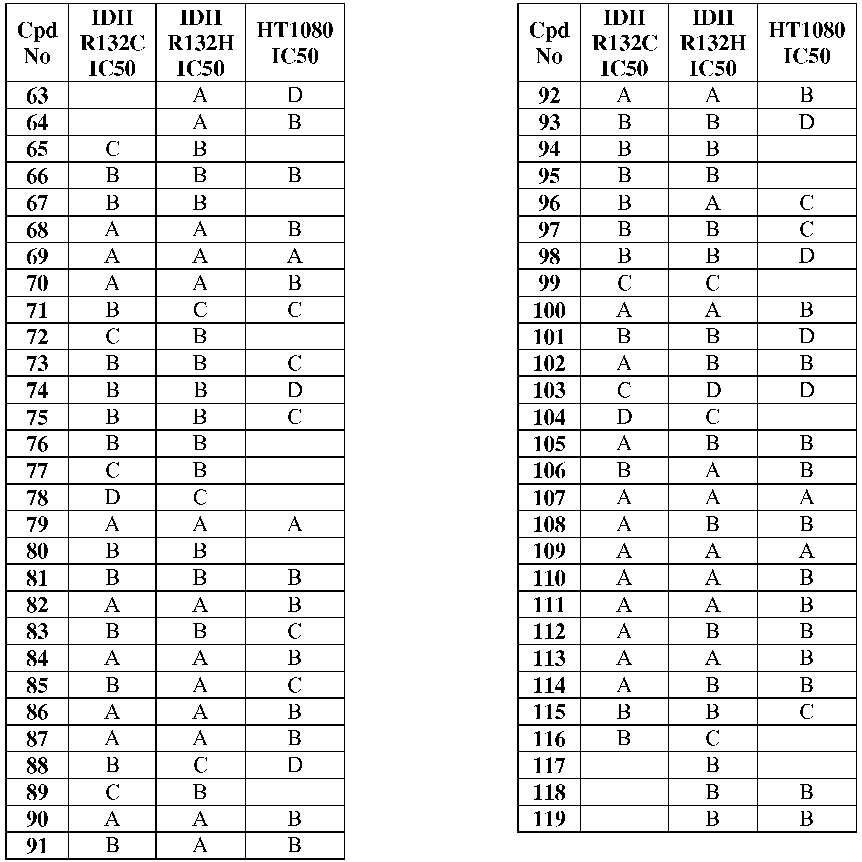

### Example B: Cellular Assays for IDH1m (R132H or R132C) Inhibitors.

Cells (HT1080 or U87MG) are grown in T125 flasks in DMEM containing 10% FBS, 1x penicillin/streptomycin and 500ug/mL G418 (present in U87MG cells only). They are harvested by trypsin and seeded into 96 well white bottom plates at a density of 5000 cell/well in 100 µl/well in DMEM with 10% FBS. No cells are placed in columns 1 and 12. Cells are incubated overnight at 37 °C in 5% CO₂. The next day test compounds are made up at 2x the final concentration and 100 µl are added to each cell well. The final concentration of DMSO is 0.2% and the DMSO control wells are plated in row G. The plates are then placed in the incubator for 48 hours. At 48 hours, 100 µl of media is removed from each well and analyzed by LC-MS for 2-HG concentrations. The cell plate is placed back in the incubator for another 24 hours. At 72 hours post compound addition, 10 mL/plate of Promega Cell Titer Glo reagent is thawed and mixed. The cell plate is removed from the incubator and allowed to equilibrate to room temperature. Then 100 µl of Promega Cell Titer Glo reagent is added to each well of media. The cell plate is then placed on an orbital shaker for 10 minutes and then allowed to sit at room temperature for 20 minutes. The plate is then read for luminescence with an integration time of 500ms.

The IC₅₀ for inhibition of 2-HG production (concentration of test compound to reduce 2HG production by 50% compared to control) in these two cell lines for various compounds of formula I is set forth in Table 2 above. As used in Table 2, "A" refers to an IC₅₀ for inhibition of 2-HG production ≤ 0.1 µM; "B" refers to an IC₅₀ for inhibition of 2-HG production between 0.1 µM and 0.5 µM; "C" refers to an IC₅₀ for inhibition of 2-HG production between 0.5µM and 1 µM; "D" refers to an IC₅₀ for inhibition of 2-HG production between 1µM and 2µM.

## Claims

1. A compound of formula I or a pharmaceutically acceptable salt or hydrate thereof, wherein: 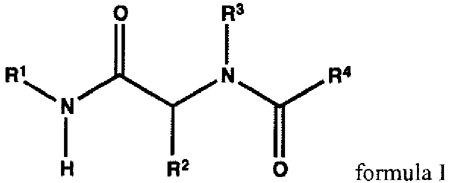 R¹ is: 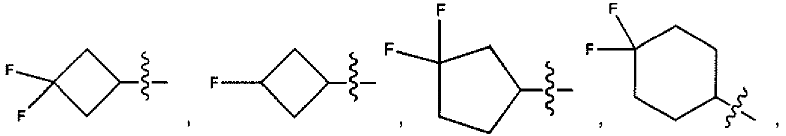 or 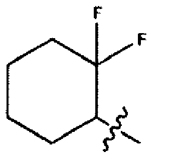
;each R² and R³ is independently selected from optionally substituted aryl or optionally substituted heteroaryl;
R⁴ is a fully saturated heterocyclyl, -CH(R⁵)N(R⁵)-aryl, -CH(R⁵)N(R⁵)-heterocyclyl, -CH(R⁵)N(R⁵)-carbocyclyl, -CH₂-heterocyclyl, 1H-indol-2-yl, indolin-2-yl, 1,2,3,4-tetrahydroquinolin-2-yl, imidazo[1,2-*a*]pyridine-5-yl, 5,6,7,8-tetrahydroimidazo[1,2-*a*]pyridine-5-yl, -(CR⁵R⁶)₁₋₄N(R⁵)C(O)O(C₁-C₆ alkyl), or -(CR⁵R⁶)₁₋₄N(R⁵)SO₂(C₁-C₆ alkyl), wherein each saturated heterocyclyl, heteroaryl, aryl, heterocyclyl, or carbocyclyl is independently optionally substituted;
each R⁵ is independently selected from hydrogen and methyl; and
each R⁶ is independently selected from hydrogen, methyl, CH₂OH, CH(CH₃)OH, CH₂NH₂, or CH(CH₃)NH₂; wherein:
"heterocyclyl" refers to a monocyclic, bicyclic or tricyclic, ring structure that is not fully aromatic and includes one to four heteroatoms independently selected from N, O, or S in one or more of the rings; and
"carbocyclyl" refers to a monocyclic, bicyclic or tricyclic, hydrocarbon ring system that is not fully aromatic, wherein any ring atom capable of substitution can be substituted by one or more substituents.

2. The compound of claim 1, wherein:
each R² and R³ is independently selected from aryl or heteroaryl, wherein said aryl or heteroaryl is independently optionally substituted with one to three R⁷ groups or acrylamido;
R⁴ is saturated heterocyclyl, -CH(R⁵)N(R⁵)-aryl, -CH(R⁵)N(R⁵)-heterocyclyl, -CH(R⁵)N(R⁵)-carbocyclyl, -CH₂-heterocyclyl, 1H-indol-2-yl, indolin-2-yl, 1,2,3,4-tetrahydroquinolin-2-yl, imidazo[1,2-*a*]pyridine-5-yl, 5,6,7,8-tetrahydroimidazo[1,2-*a*]pyridine-5-yl, -(CR⁵R⁶)₁₋₄N(R⁵)C(O)O(C₁-C₆ alkyl), or -(CR⁵R⁶)₁₋₄N(R⁵)SO₂(C₁-C₆ alkyl), wherein each saturated heterocyclyl, heteroaryl, aryl, heterocyclyl, or carbocyclyl is independently optionally substituted with one to three R⁷ groups;
each R⁵ is independently selected from hydrogen and methyl;
each R⁶ is independently selected from hydrogen, methyl, CH₂OH, CH(CH₃)OH, CH₂NH₂, or CH(CH₃)NH₂;
each R⁷ is independently halo, -CF₃, -CN, -OR⁸, -N(R⁸)₂, -C(O)CH₃,-C(O)OCH₃, -SO₂(C₁-C₃ alkyl), -C(O)N(R⁸)₂, -O(CH₂)₂-OR⁸, SO₂N(R⁸)₂, heteroaryl, -C₁-C₃ haloalkyl, C₁-C₃ alkyl optionally substituted with -OR⁸ or-N(R⁸)₂; and
each R⁸ is independently H or C₁-C₃ alkyl.

3. The compound of claim 1 or 2, wherein each R² and R³ is independently aryl optionally substituted with one to three R⁷ groups.

4. The compound of claim 3, wherein R² is phenyl optionally substituted with one to two R⁷ groups and each R⁷ is independently F, Cl or methyl.

5. The compound of claim 3, wherein R³ is phenyl optionally substituted with one to two R⁷ groups wherein each R⁷ is independently F, CN, -SO₂NH₂,-SO₂NH(CH₃), acrylamido or oxadiazolyl.

6. The compound of claim 3, wherein R⁴ is 4-6 membered saturated heterocyclyl, -CH₂-heterocyclyl, 1H-indol-2-yl, indolin-2-yl, 1,2,3,4-tetrahydroquinolin-2-yl, imidazo[1,2-*a*]pyridine-5-yl, 5,6,7,8-tetrahydroimidazo[1,2-*a*]pyridine-5-yl, -(CR⁵R⁶)₁₋₄N(R⁵)C(O)O(C₁-C₆ alkyl), or -(CR⁵R⁶)₁₋₄N(R⁵)SO₂(C₁-C₆ alkyl), wherein each said saturated heterocyclyl, heteroaryl, or heterocyclyl is independently optionally substituted with one to three R⁷ groups; or
R⁴ is: 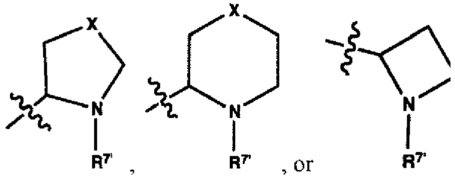
wherein X is CH(R⁷"), O, NH, or NC(O)CH₃; R⁷' is H, -C(O)CH₃,-C(O)OCH₃, -SO₂(C₁-C₃ alkyl), -C(O)N(R⁸)₂, pyrimidinyl, pyridyl; and R⁷" is H, -O(CH₂)₂-OCH₃, -O(CH₂)₂-OCH, OH, OCH₃, NH₂, or F; or
R⁴ is -(CR⁵R⁶)N(R⁵)C(O)O(C₁-C₄ alkyl) wherein each R⁵ is independently H or methyl and R⁶ is methyl or CH₂OH.

7. The compound of claim 5, wherein R⁴ is 1,2,3,4-tetrahydroquinolin-2-yl, or 5,6,7,8-tetrahydroimidazo[1,2-*a*]pyridine-5-yl.

8. A compound selected from any one of the following compounds:

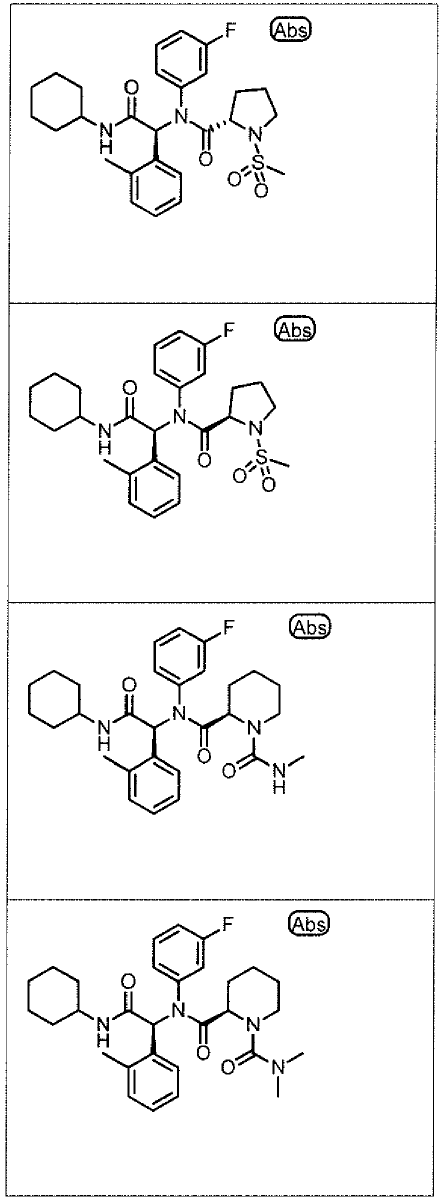

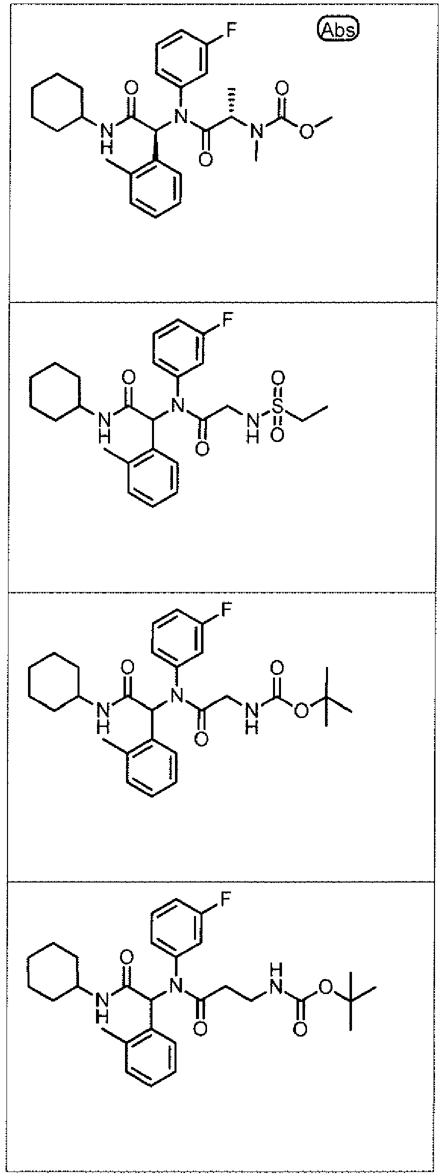

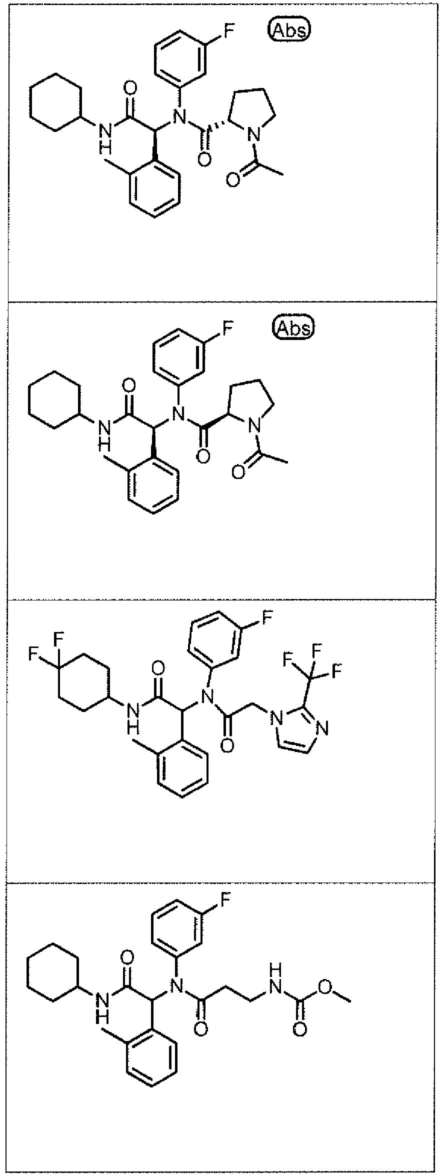

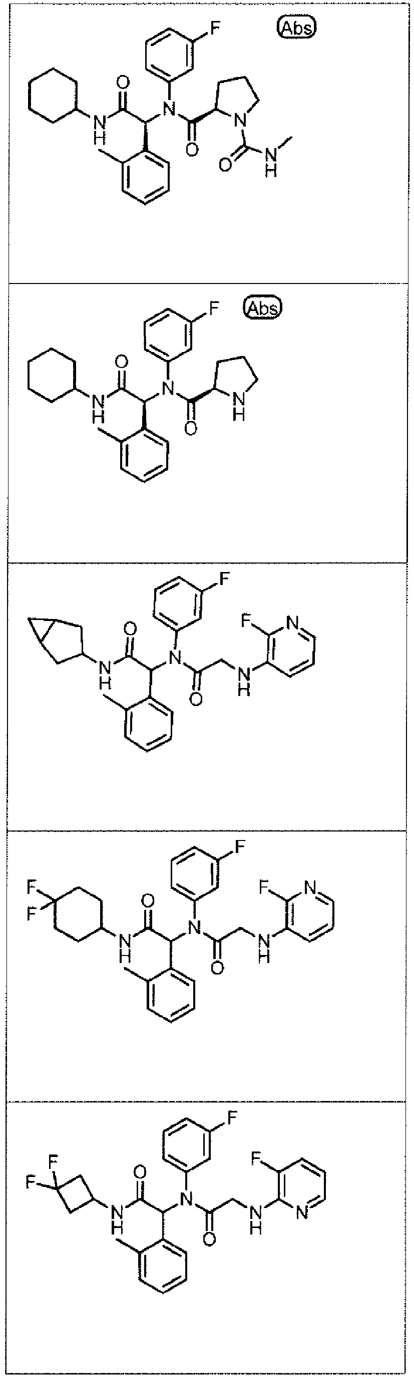

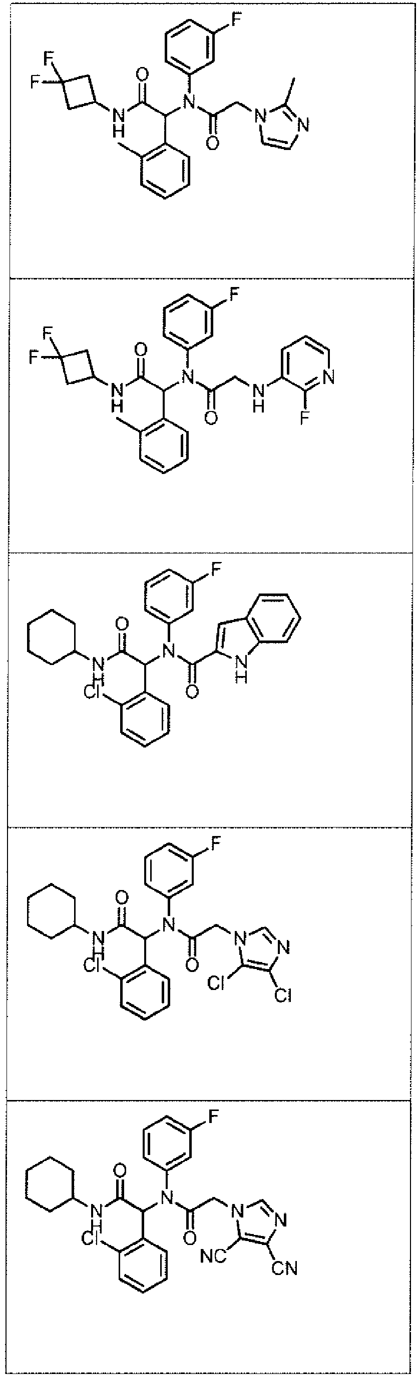

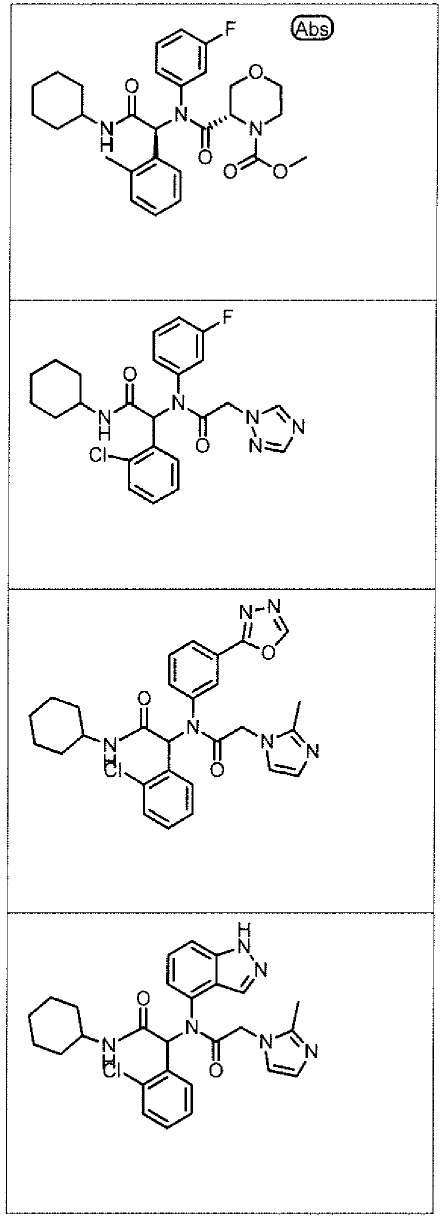

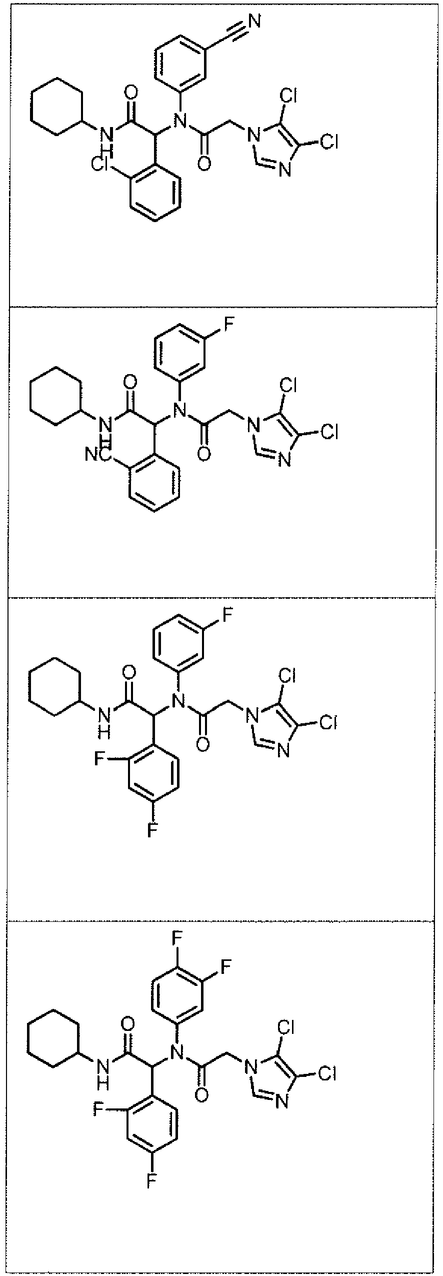

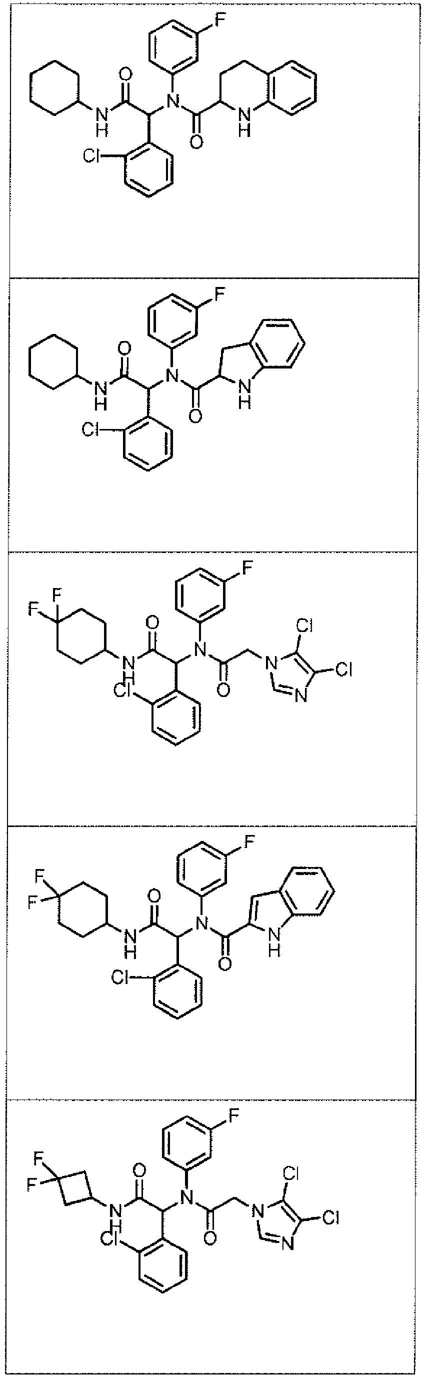

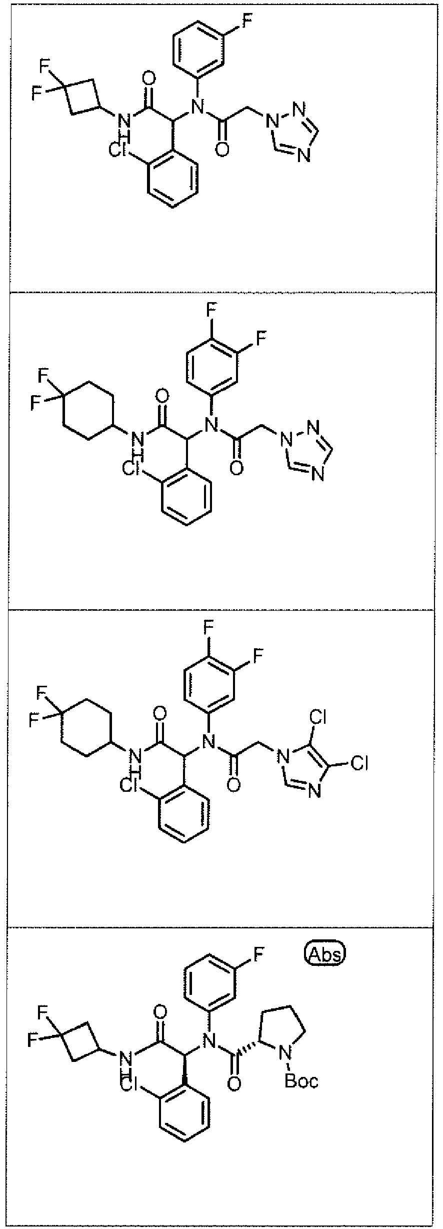

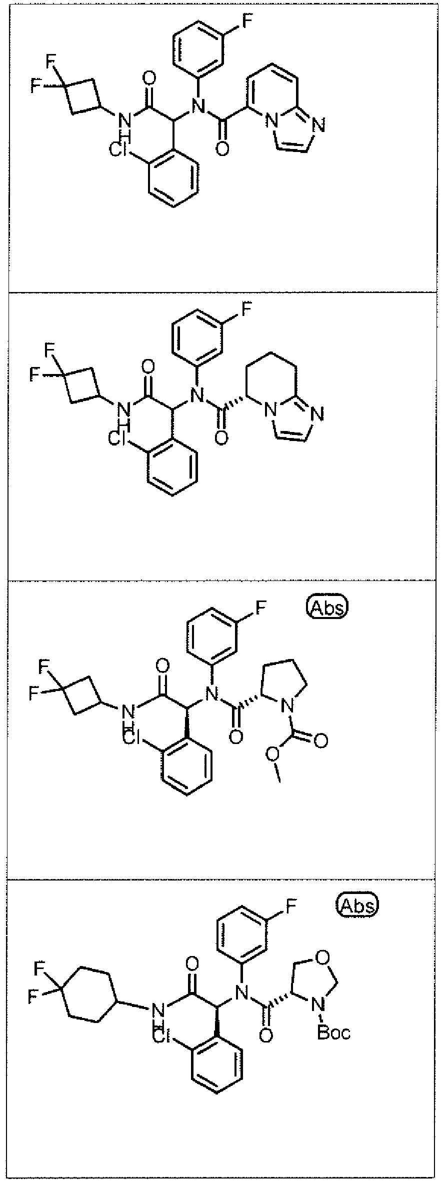

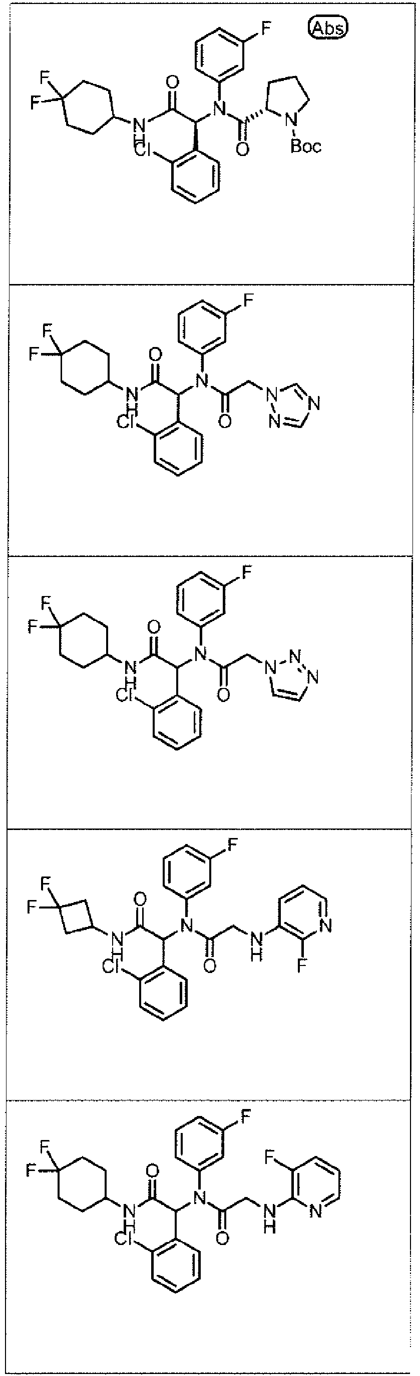

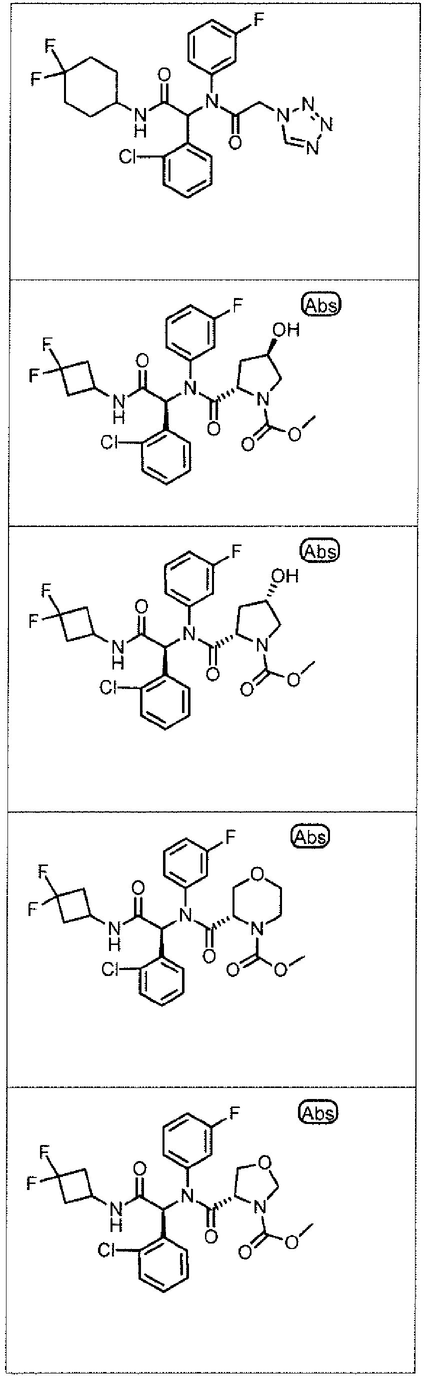

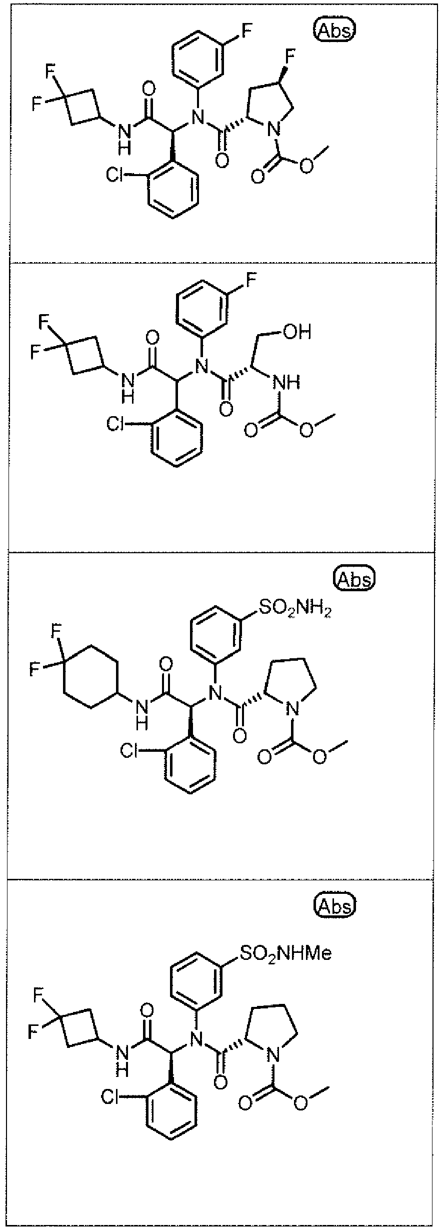

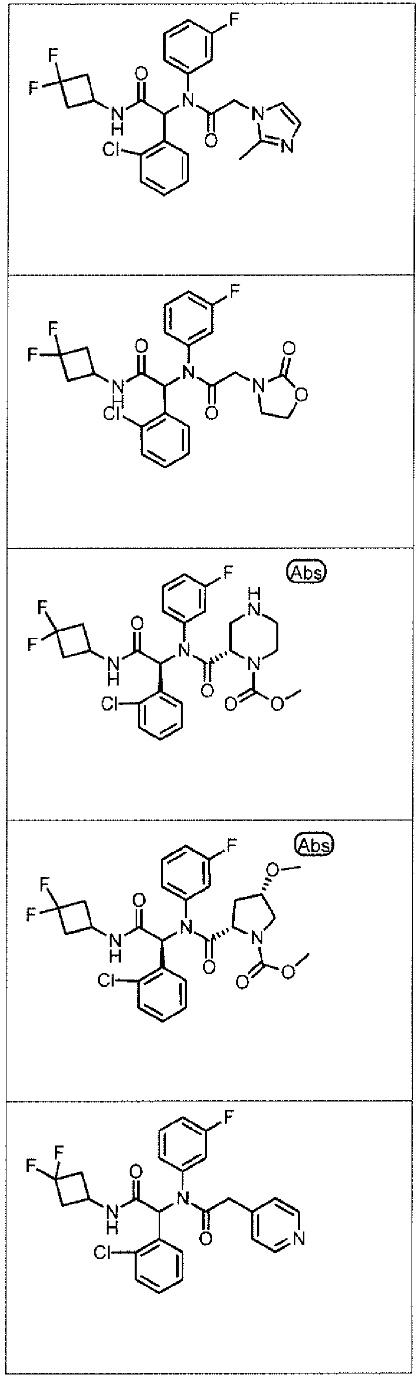

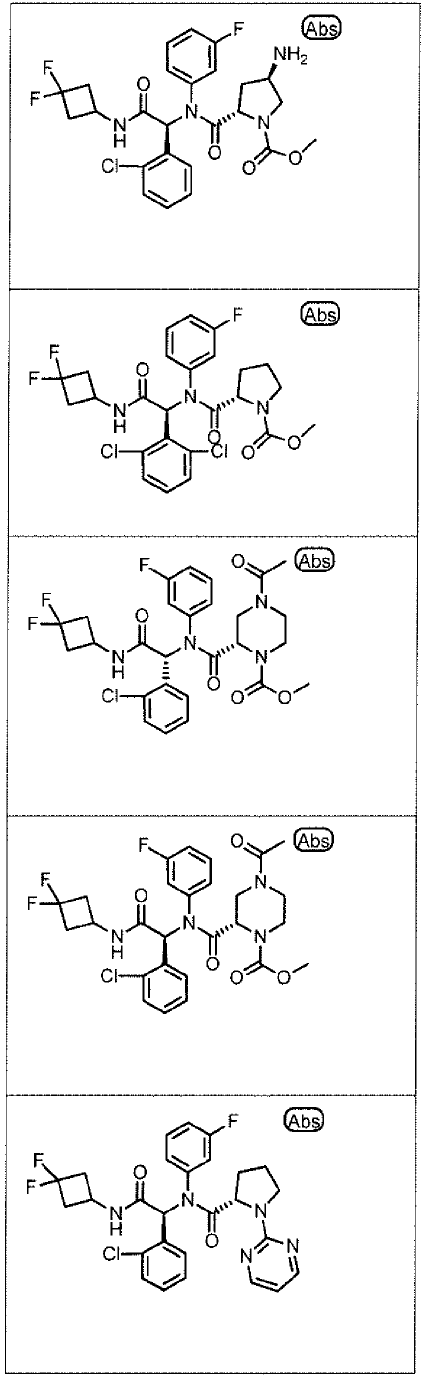

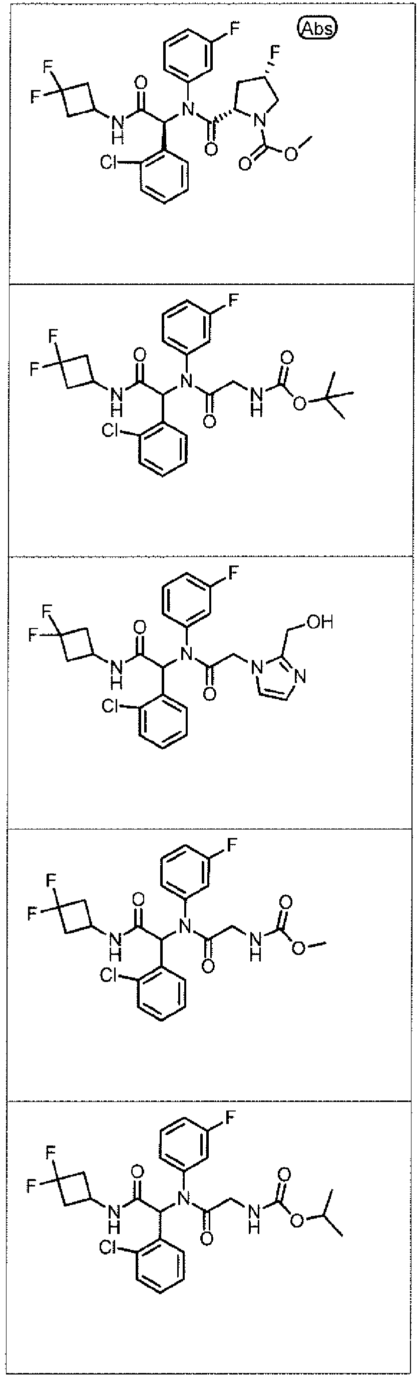

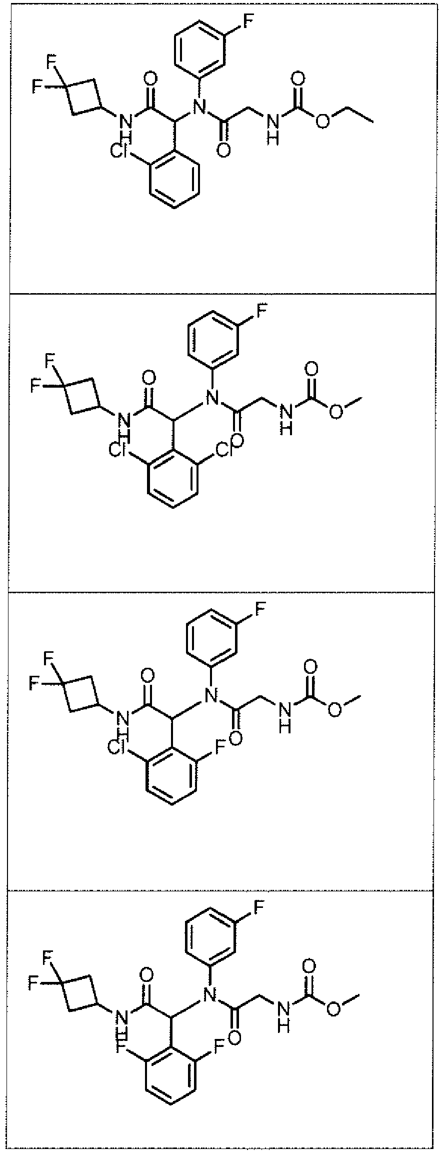

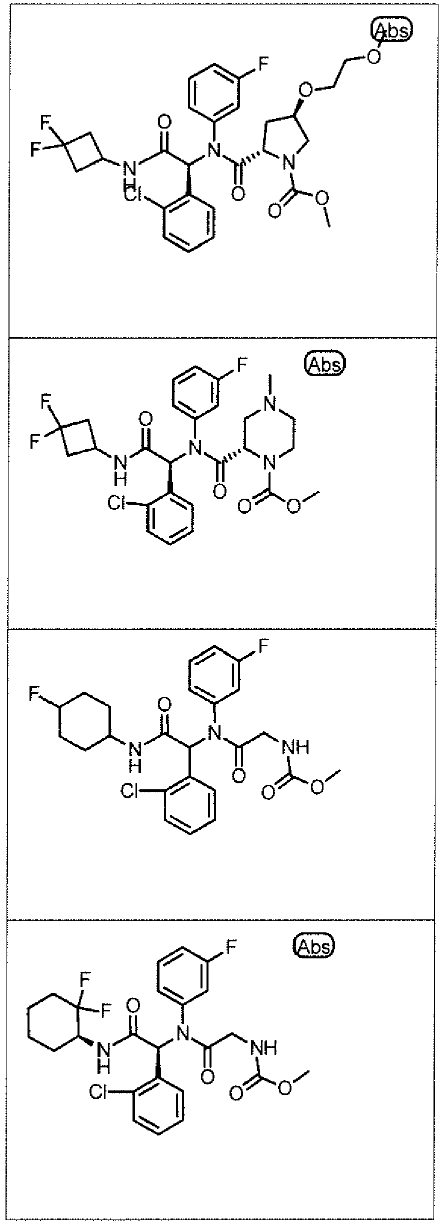

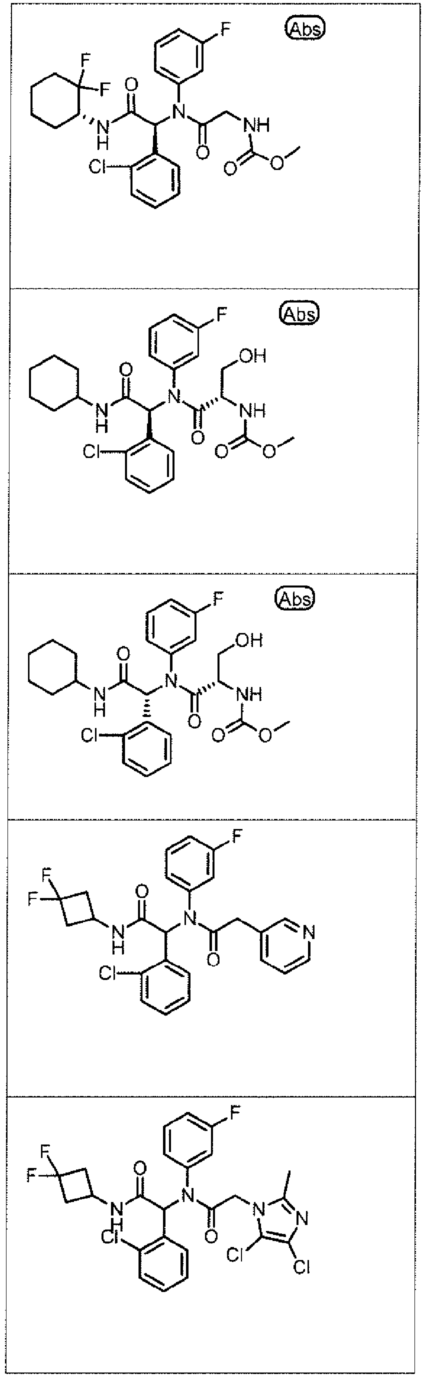

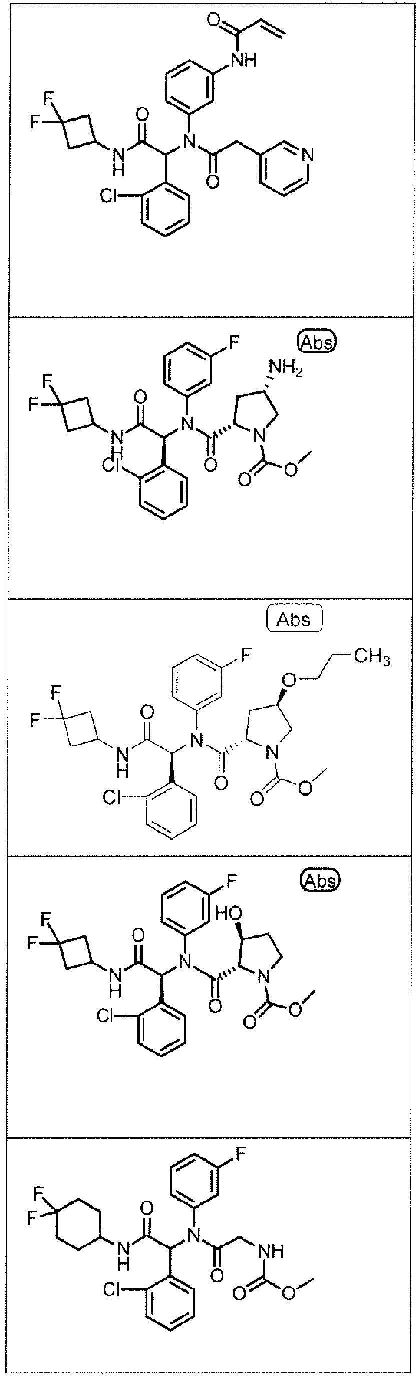

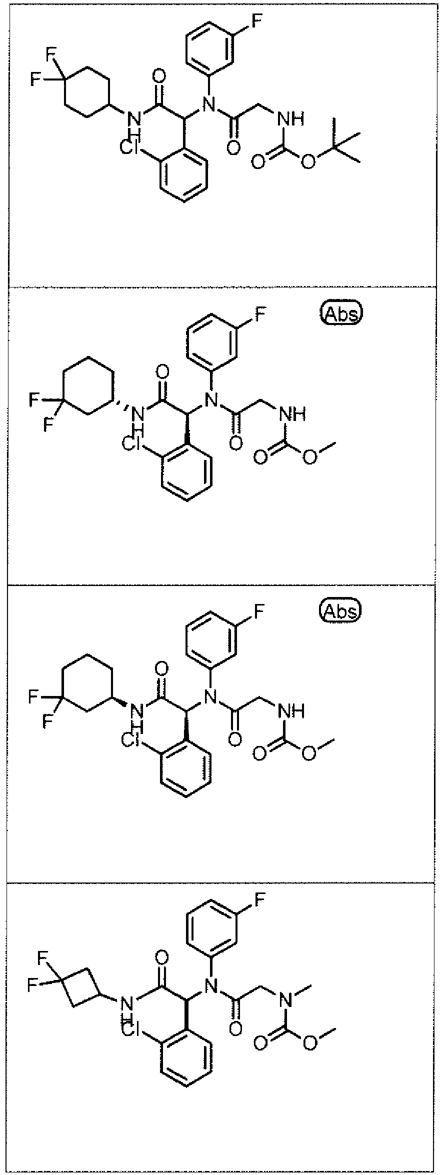

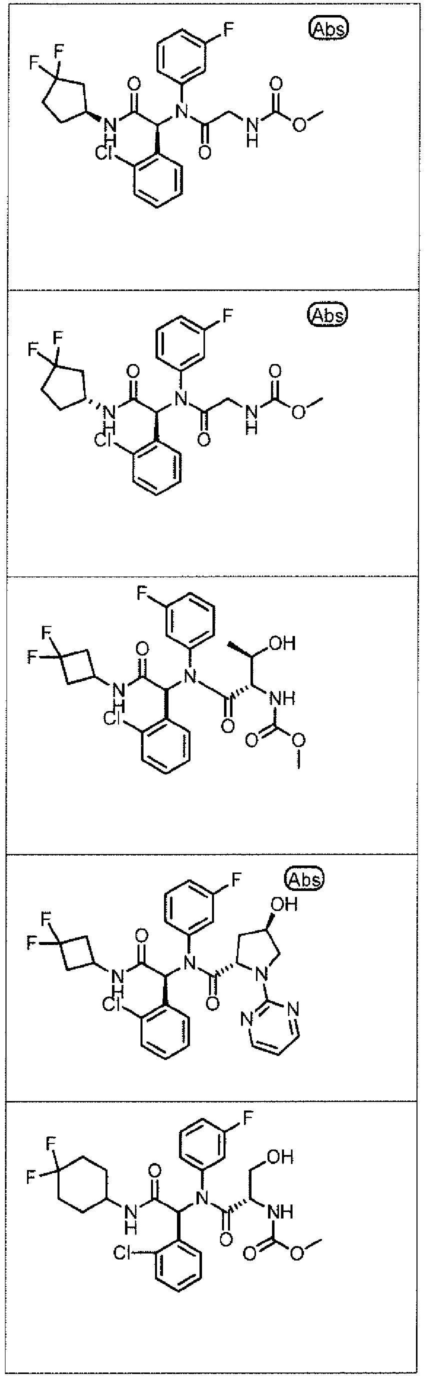

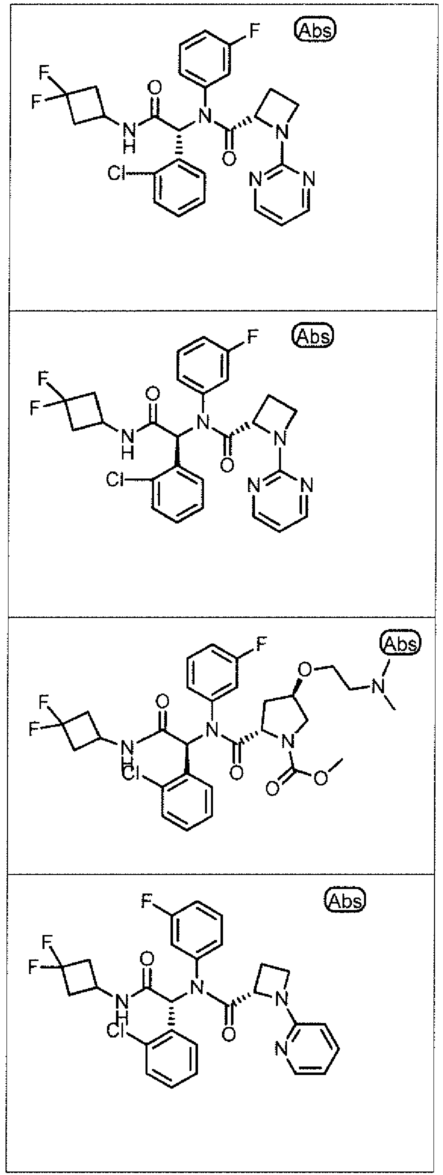

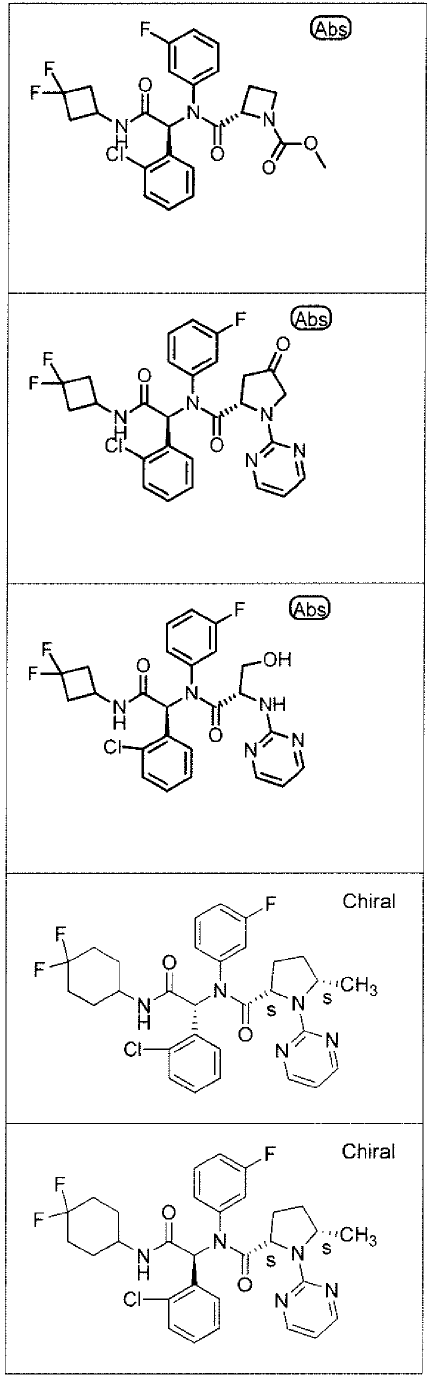

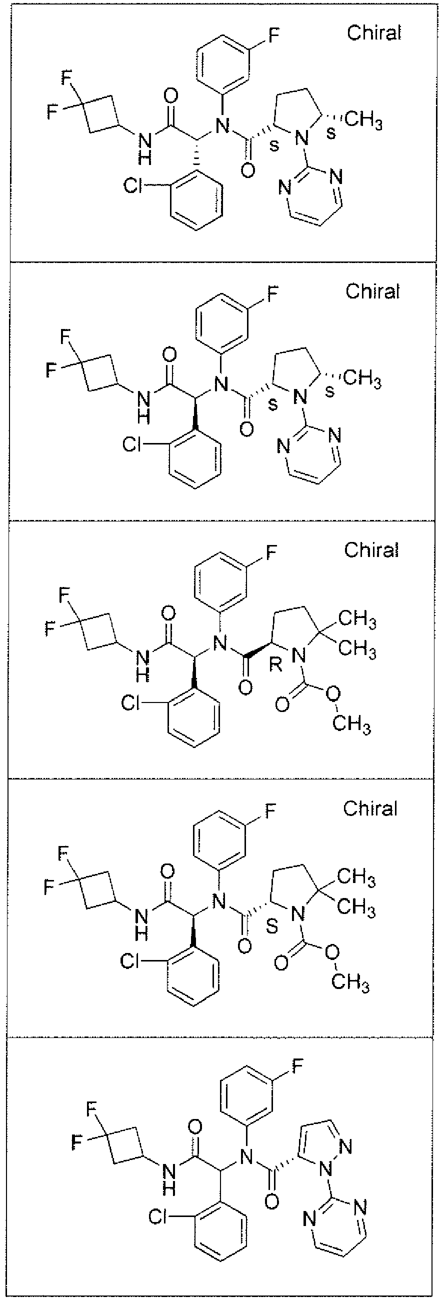

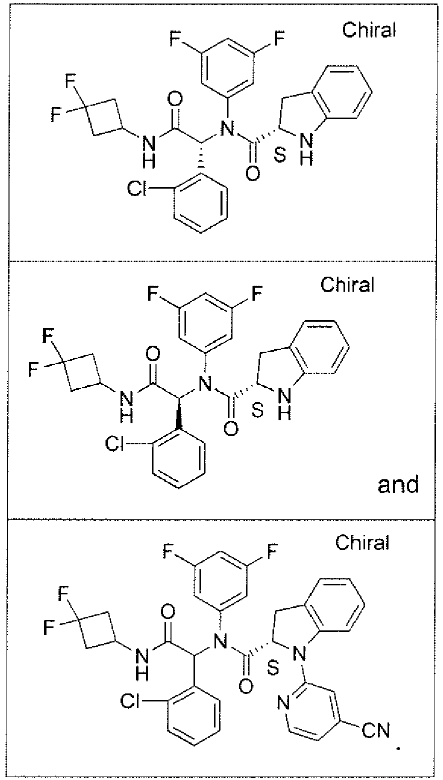

9. A compound of formula I or a pharmaceutically acceptable salt or hydrate thereof, wherein: 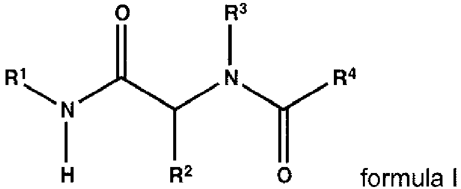 R¹ is: 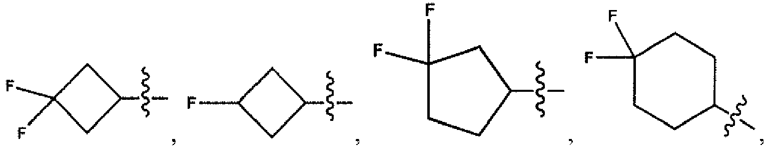 or 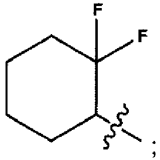
each R² and R³ is independently selected from optionally substituted aryl or optionally substituted heteroaryl;
R⁴ is saturated heterocyclyl, -CH(R⁵)N(R⁵)-aryl, -CH(R⁵)N(R⁵)-heterocyclyl, -CH(R⁵)N(R⁵)-carbocyclyl, -CH₂-heterocyclyl, 1H-indol-2-yl, indolin-2-yl, 1,2,3,4-tetrahydroquinolin-2-yl, imidazo[1,2-a]pyridine-5-yl, 5,6,7,8-tetrahydroimidazo[1,2-a]pyridine-5-yl, -(CR⁵R⁶)₁₋₄N(R⁵)C(O)O(C₁-C₆ alkyl), or -(CR⁵R⁶)₁₋₄N(R⁵)SO₂(C₁-C₆ alkyl), wherein each saturated heterocyclyl, heteroaryl, aryl, heterocyclyl, or carbocyclyl is independently optionally substituted; or
R⁴ is -CH₂-heteroaryl wherein heteroaryl is imidazolyl substituted with one or two R⁷ groups selected from the group consisting of-CN, -CH₂OH, Cl, and F; or heteroaryl is triazolyl, tetrazolyl or pyridinyl optionally substituted with one or two R⁷ groups; or
R⁴ is -CH(R⁵)N(R⁵)-heteroaryl wherein heteroaryl is pyrimidinyl optionally substituted with one or two R⁷ groups selected from the group consisting of Cl and F;
each R⁵ is independently selected from hydrogen and methyl; and
each R⁶ is independently selected from hydrogen, methyl, CH₂OH, CH(CH₃)OH, CH₂NH₂, or CH(CH₃)NH₂.

10. A compound selected from the group consisting of:
| | |
|---|---|
| **33** | 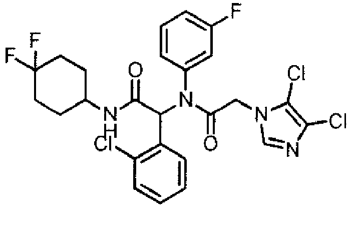 |
| **35** | 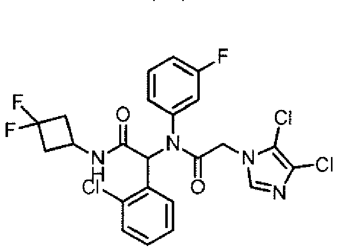 |
| **36** | 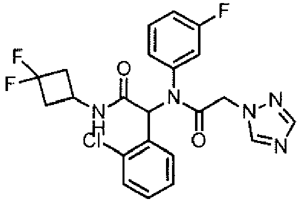 |
| **37** | 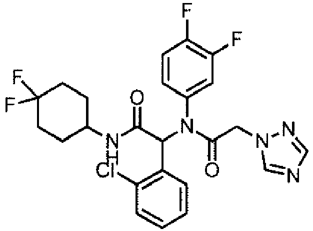 |
| **38** | 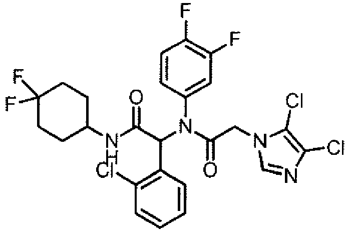 |
| **45** | 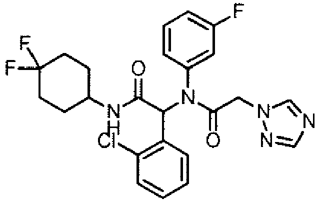 |
| **46** | 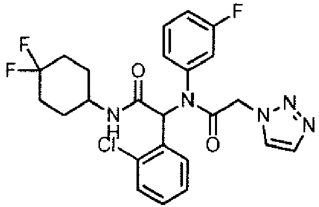 |
| **49** | 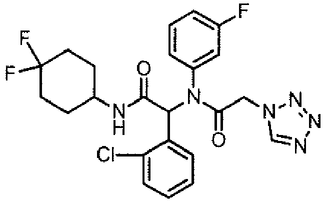 |
| **59** | 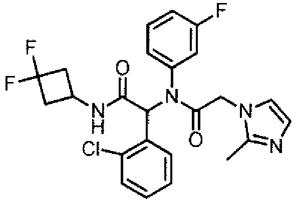 |
| **64** | 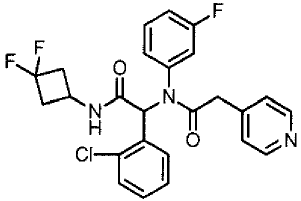 |
| **72** | 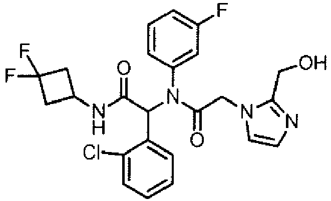 |
| **86** | 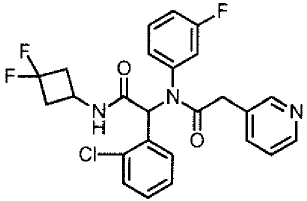 |
| **87** | 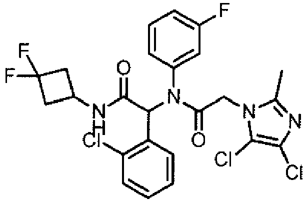 |
| **88** | 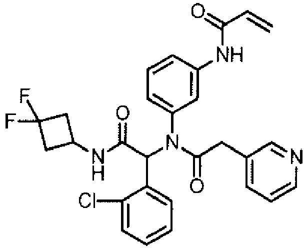 |
| **108** | 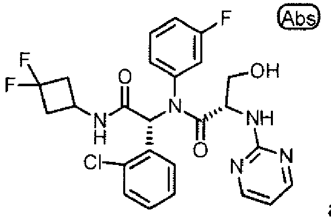 |
| | and |
| **109** | 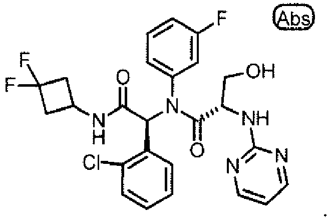 |

11. A pharmaceutical composition comprising a compound of any one of claims 1 to 10; and a pharmaceutically acceptable carrier.

12. The pharmaceutical composition of claim 11, further comprising a second therapeutic agent useful in the treatment of cancer.

13. A compound or pharmaceutical composition according to any one of claims 1 to 11 for use in a method of treating a cancer **characterized by** the presence of an IDH1 mutation, wherein the IDH1 mutation results in a new ability of the enzyme to catalyze the NADPH-dependent reduction of α-ketoglutarate to *R*(-)-2-hydroxyglutarate in a patient.

14. The compound or pharmaceutical composition for use of claim 13, wherein the IDH1 mutation is an IDH1 R132H or R132C mutation.

15. The compound or pharmaceutical composition for use of claim 13, wherein the cancer is selected from glioma (glioblastoma), acute myelogenous leukemia, melanoma, non-small cell lung cancer (NSCLC), cholangiocarcinomas, chondrosarcoma, myelodysplastic syndromes (MDS), myeloproliferative neoplasm (MPN), colon cancer in a patient.

16. The compound or pharmaceutical composition for use of any one of claims 13 to 15, further comprising administering to the patient in need thereof a second therapeutic agent useful in the treatment of cancer.

## Patentansprüche

1. Verbindung der Formel I oder ein pharmazeutisch verträgliches Salz oder Hydrat davon, wobei: 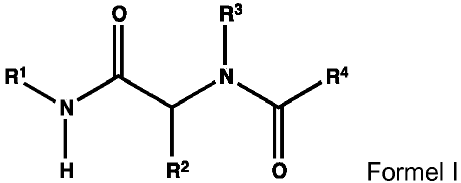 R¹: 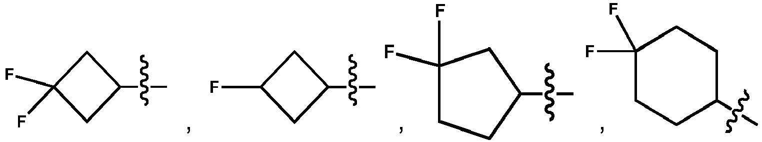 oder 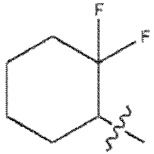 ist;
jedes R² und R³ unabhängig ausgewählt ist aus optional substituiertem Aryl oder optional substituiertem Heteroaryl;
R⁴ ein vollständig gesättigtes Heterocyclyl, -CH(R⁵)N(R⁵)-Aryl, -CH(R⁵)N(R⁵)-Heterocyclyl, -CH(R⁵)N(R⁵)-Carbocyclyl, -CH₂-Heterocyclyl, 1H-Indol-2-yl, Indolin-2-yl, 1,2,3,4-Tetrahydroquinolin-2-yl, Imidazo[1,2-a]pyridin-5-yl, 5,6,7,8-Tetrahydroimidazo[1,2-*a*]pyridin-5-yl, -(CR⁵R⁶)₁₋₄N(R⁵)C(O)O(C₁-C₆-Alkyl) oder -(CR⁵R⁶)₁₋₄N(R⁵)SO₂(C₁-C₆-Alkyl) ist, wobei jedes gesättigte Heterocyclyl, Heteroaryl, Aryl, Heterocyclyl oder Carbocyclyl unabhängig optional substituiert ist;
jedes R⁵ unabhängig ausgewählt aus Wasserstoff und Methyl ist; und jedes R⁶ unabhängig ausgewählt aus Wasserstoff, Methyl, CH₂OH, CH(CH₃)OH, CH₂NH₂, oder CH(CH₃)NH₂ ist; wobei:
"Heterocyclyl" sich auf eine monozyklische, bizyklische oder trizyklische Ringstruktur bezieht, die nicht vollständig aromatisch ist und ein bis vier Heteroatome beinhaltet, unabhängig ausgewählt aus N, O oder S in einem oder mehreren der Ringe; und
"Carbocyclyl" sich auf ein monozyklisches, bizyklisches oder trizyklisches Kohlenwasserstoffringsystem bezieht, das nicht vollständig aromatisch ist, wobei irgendein Ringatom, das zur Substitution geeignet ist, durch einen oder mehrere Substituenten substituiert sein kann.

2. Verbindung nach Anspruch 1, wobei:
jedes R² und R³ unabhängig ausgewählt ist aus Aryl oder Heteroaryl, wobei das Aryl oder Heteroaryl unabhängig optional substituiert mit ein bis drei R⁷-Gruppen oder Acrylamido ist;
R⁴ gesättigtes Heterocyclyl, -CH(R⁵)N(R⁵)-Aryl, -CH(R⁵)N(R⁵)-Heterocyclyl, -CH(R⁵)N(R⁵)-Carbocyclyl, -CH₂-Heterocyclyl, 1H-Indol-2-yl, Indolin-2-yl, 1,2,3,4-Tetrahydroquinolin-2-yl, Imidazo[1,2-*a*]pyridin-5-yl, 5,6,7,8-Tetrahydroimidazo[1,2-*a*]pyridin-5-yl, -(CR⁵R⁶)₁₋₄N(R⁵)C(O)O(C₁-C₆-Alkyl) oder -(CR⁵R⁶)₁₋₄N(R⁵)SO₂(C₁-C₆-Alkyl) ist, wobei jedes gesättigte Heterocyclyl, Heteroaryl, Aryl, Heterocyclyl oder Carbocyclyl unabhängig optional substituiert mit ein bis drei R⁷-Gruppen ist;
jedes R⁵ unabhängig ausgewählt ist aus Wasserstoff und Methyl;
jedes R⁶ unabhängig ausgewählt ist aus Wasserstoff, Methyl, CH₂OH, CH(CH₃)OH, CH₂NH₂ oder CH(CH₃)NH₂;
jedes R⁷ unabhängig Halo, -CF₃, -CN, -OR⁸, -N(R⁸)₂, -C(O)CH₃, -C(O)OCH₃, -SO₂(C₁-C₃-Alkyl), -C(O)N(R⁸)₂, -O(CH₂)₂-OR⁸, SO₂N(R⁸)₂, Heteroaryl, -C₁-C₃-Haloalkyl, C₁-C₃-Alkyl, optional substituiert mit -OR⁸ oder -N(R⁸)₂ ist; und
jedes R⁸ unabhängig H oder C₁-C₃-Alkyl ist.

3. Verbindung nach Anspruch 1 oder 2, wobei jedes R² und R³ unabhängig Aryl, optional substituiert mit einer bis drei R⁷-Gruppen, ist.

4. Verbindung nach Anspruch 3, wobei R² Phenyl, optional substituiert mit einer bis zwei R⁷-Gruppen, ist, und jedes R⁷ unabhängig F, Cl oder Methyl ist.

5. Verbindung nach Anspruch 3, wobei R³ Phenyl, optional substituiert mit einer bis zwei R⁷-Gruppen, ist, wobei jedes R⁷ unabhängig F, CN, -SO₂NH₂, -SO₂NH(CH₃), Acrylamido oder Oxadiazolyl ist.

6. Verbindung nach Anspruch 3, wobei R⁴ 4-6-gliedriges gesättigtes Heterocyclyl, -CH₂-Heterocyclyl, 1H-Indol-2-yl, Indolin-2-yl, 1,2,3,4-Tetrahydroquinolin-2-yl, Imidazo[1,2-*a*]pyridin-5-yl, 5,6,7,8-Tetrahydroimidazo[1,2-*a*]pyridin-5-yl, -(CR⁵R⁶)₁₋₄N(R⁵)C(O)O(C₁-C₆-Alkyl) oder -(CR⁵R⁶)₁₋₄N(R⁵)SO₂(C₁-C₆-Alkyl) ist, wobei jedes gesättigte Heterocyclyl, Heteroaryl oder Heterocyclyl unabhängig optional substituiert mit einer bis drei R⁷-Gruppen ist; oder
R⁴: 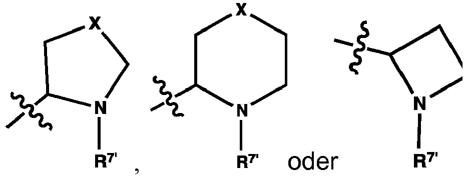 ist;
wobei X CH(R⁷"), O, NH oder NC(O)CH₃ ist; R⁷' H, -C(O)CH₃, -C(O)OCH₃,-SO₂(C₁-C₃-Alkyl), -C(O)N(R⁸)₂, Pyrimidinyl, Pyridyl ist; und R⁷" H, -O(CH₂)₂-OCH₃, -O(CH₂)₂-OCH, OH, OCH₃, NH₂, oder F ist; oder
R⁴-(CR⁵R⁶)N(R⁵)C(O)O(C₁-C₄-Alkyl) ist, wobei jedes R⁵ unabhängig H oder Methyl ist und R⁶ Methyl oder CH₂OH ist.

7. Verbindung nach Anspruch 5, wobei R⁴ 1,2,3,4-Tetrahydroquinolin-2-yl oder 5,6,7,8-Tetrahydroimidazo[1,2-*a*]pyridin-5-yl ist.

8. Verbindung ausgewählt aus irgendeiner der folgenden Verbindungen:

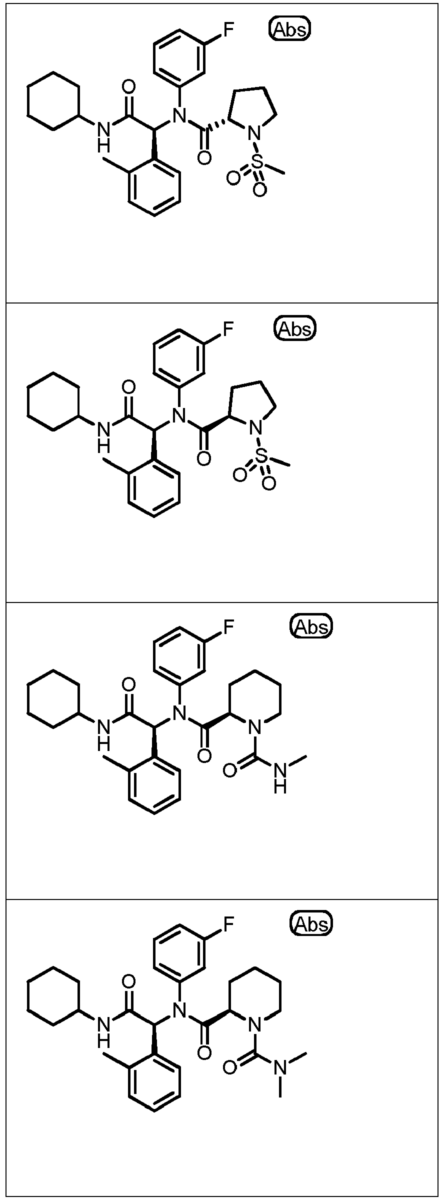

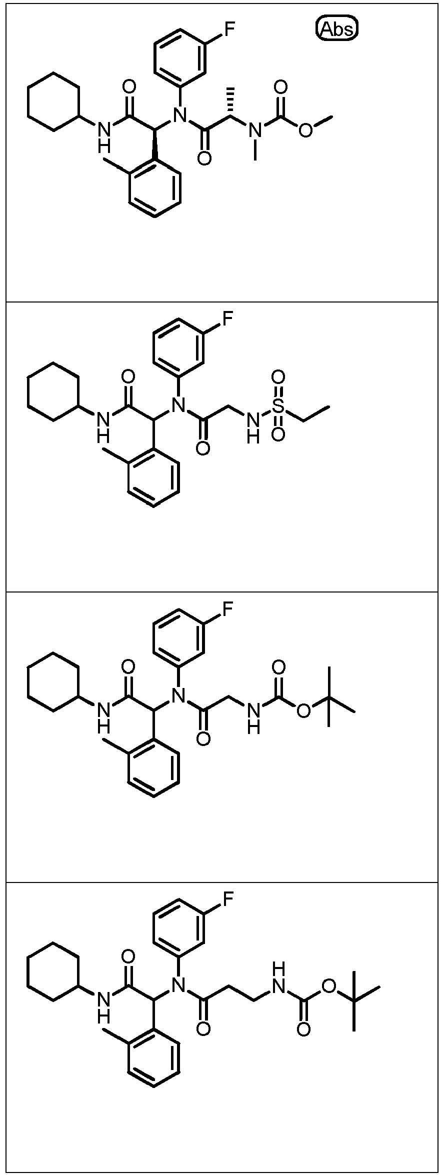

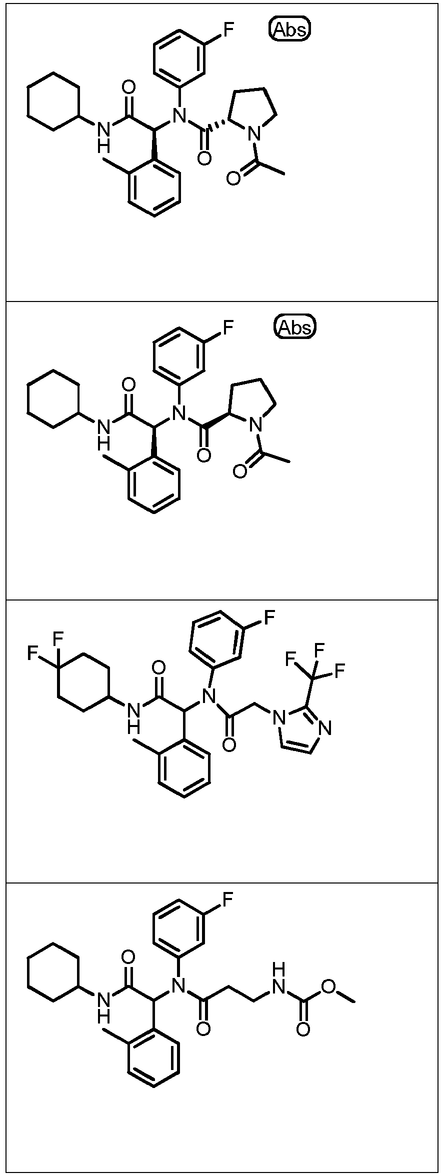

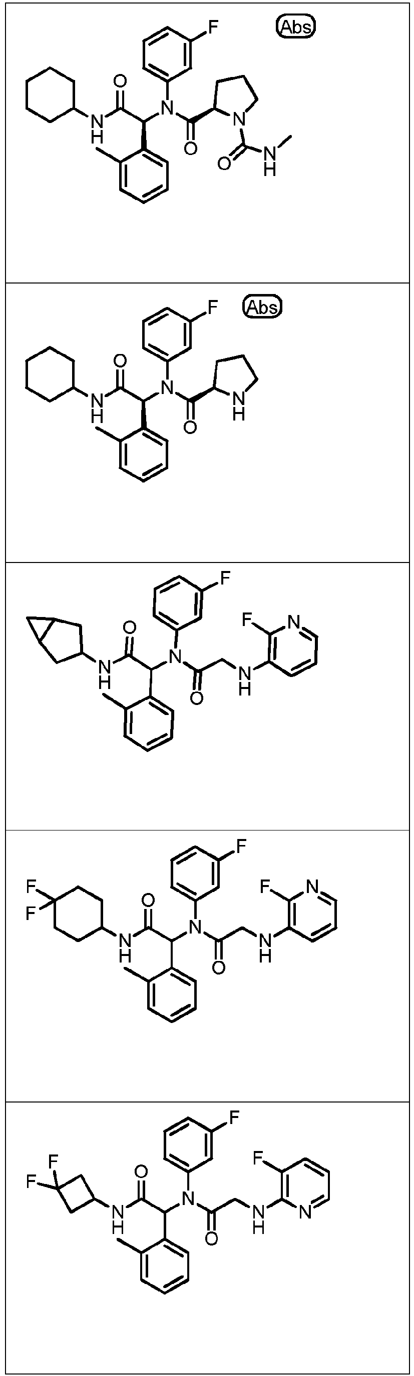

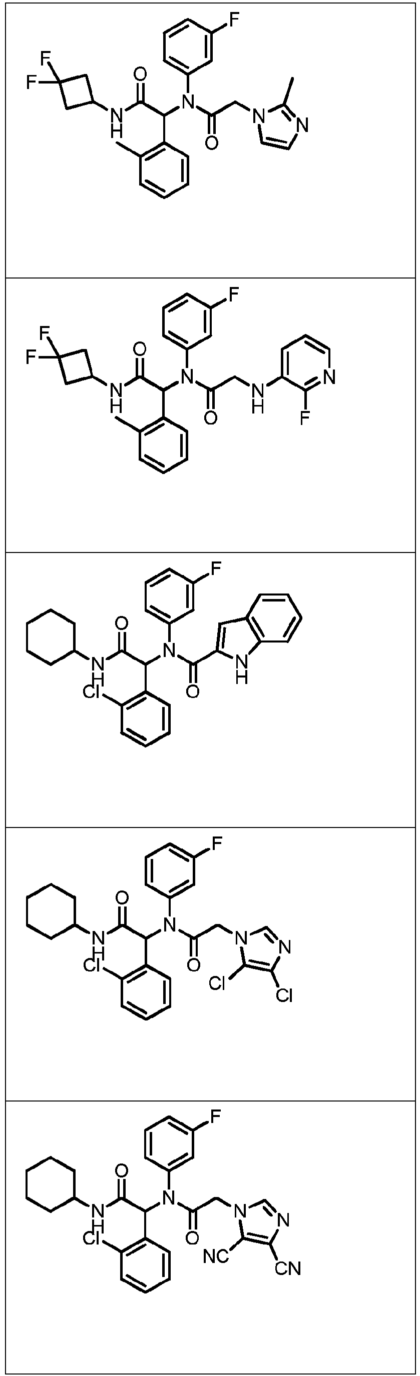

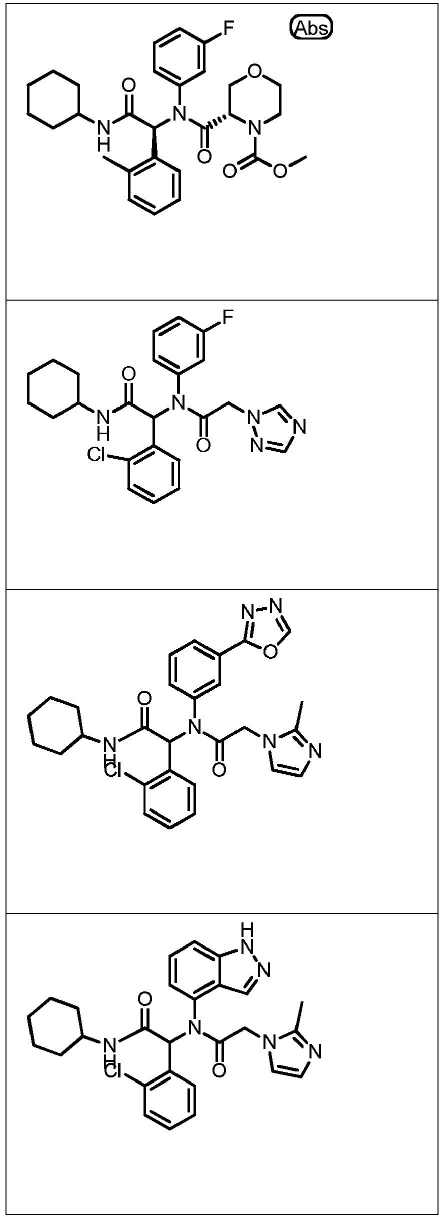

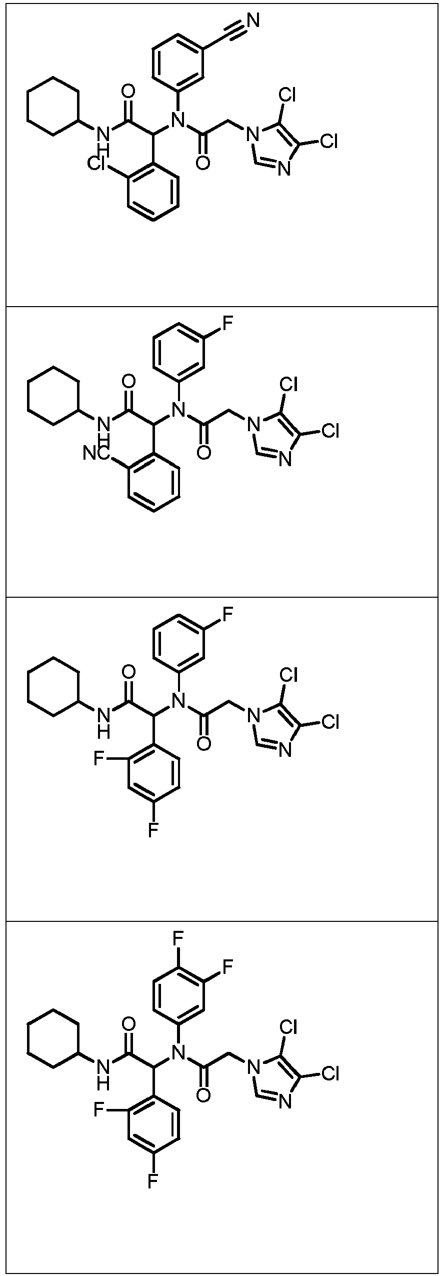

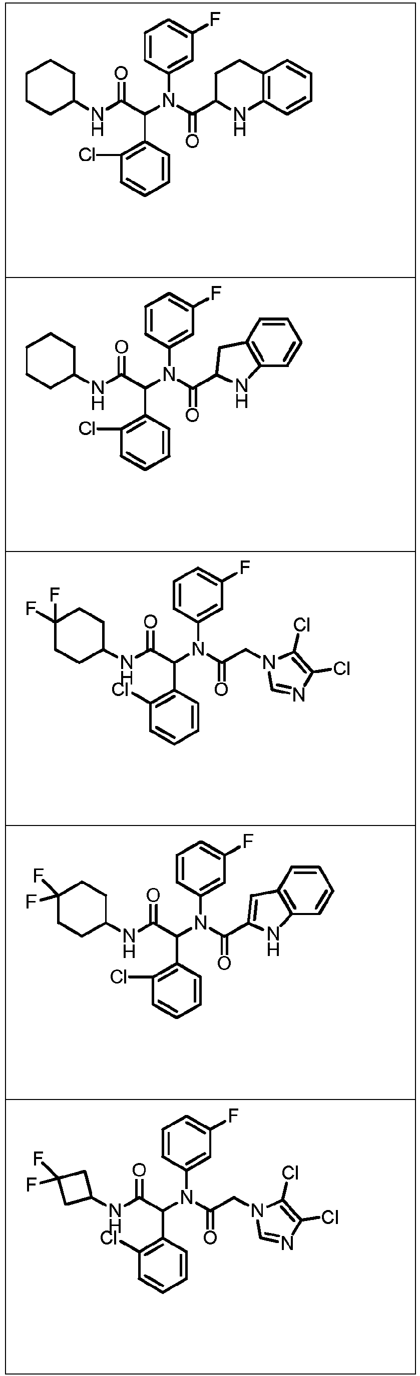

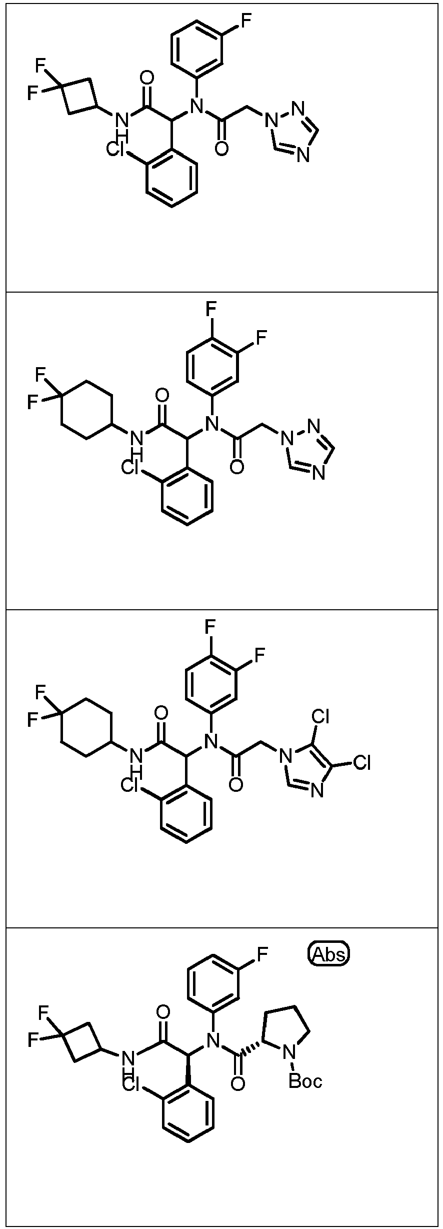

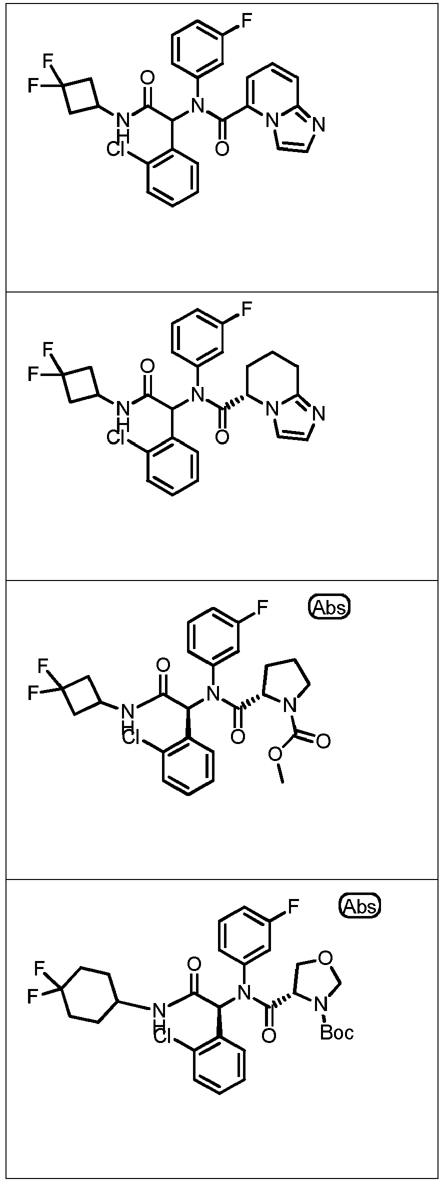

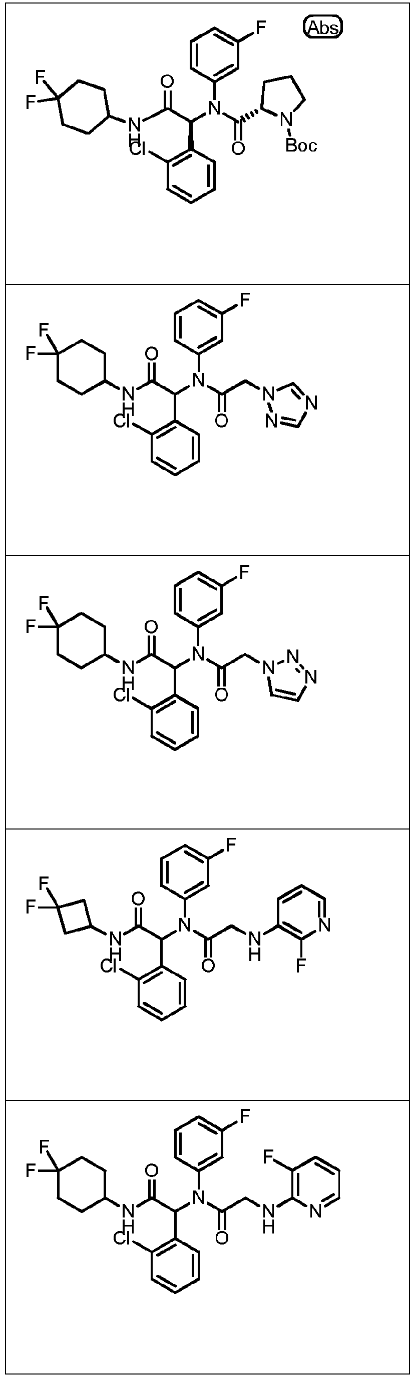

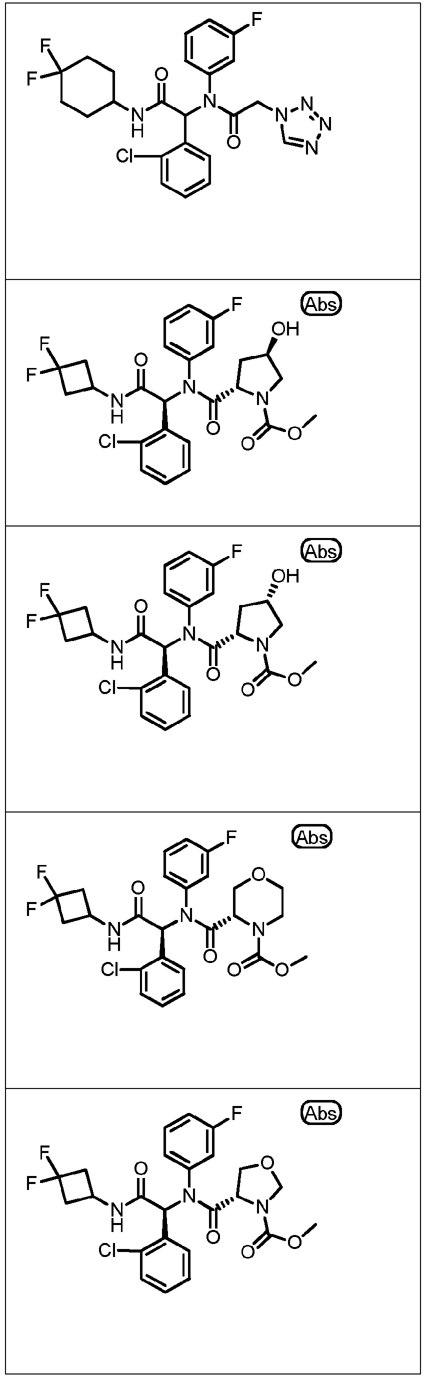

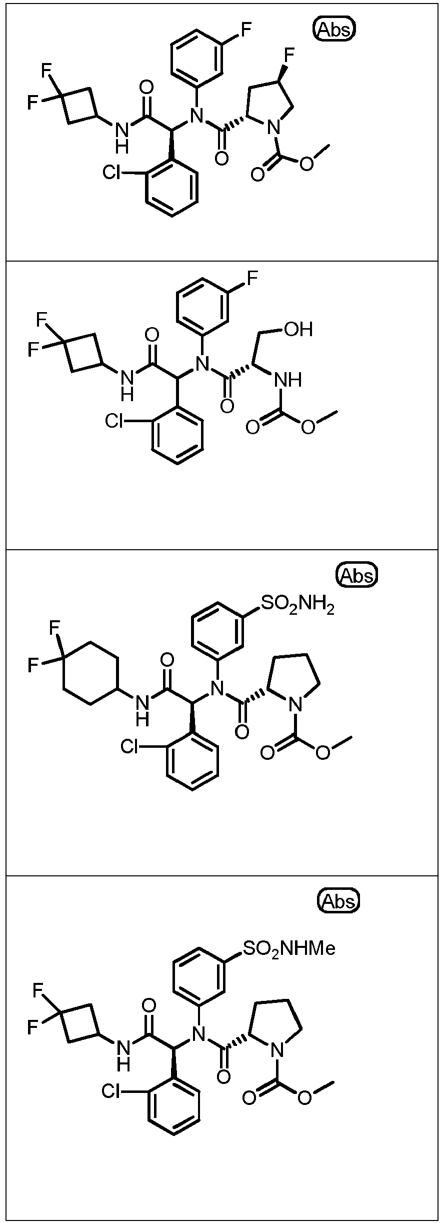

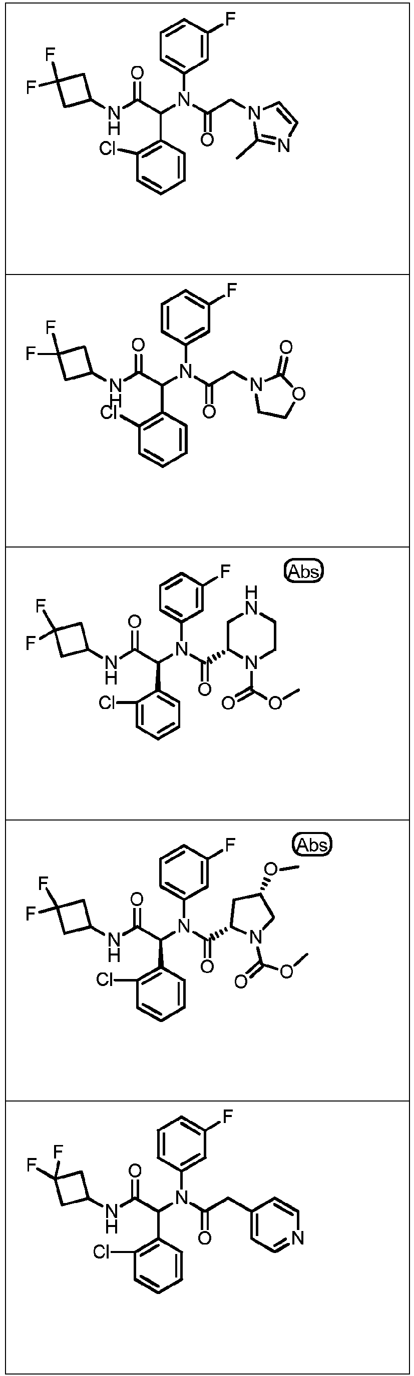

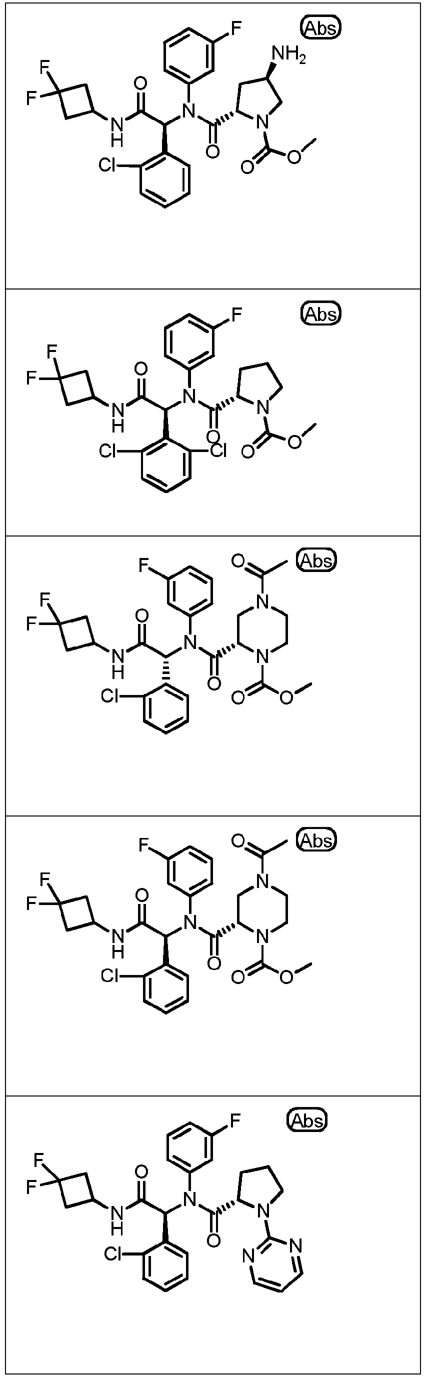

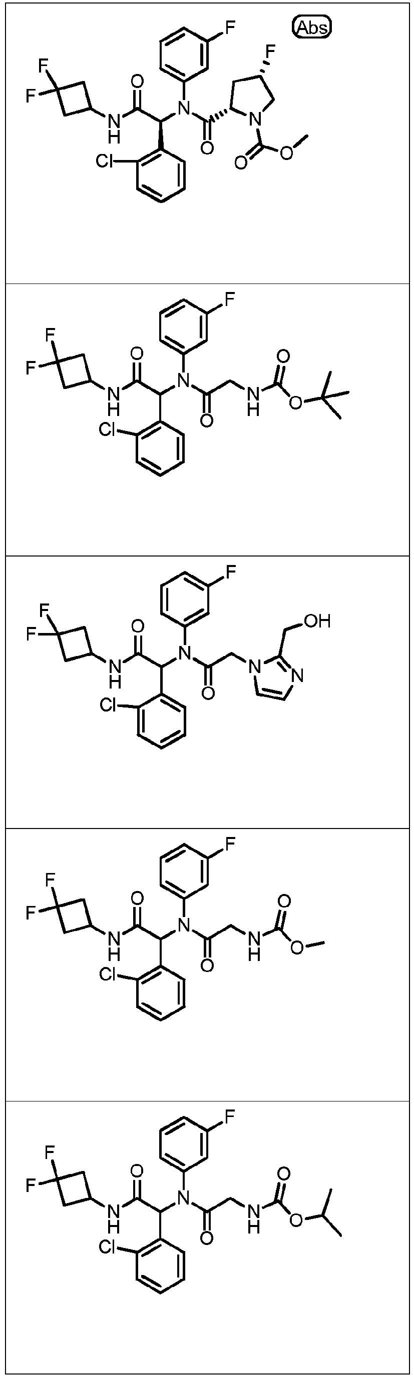

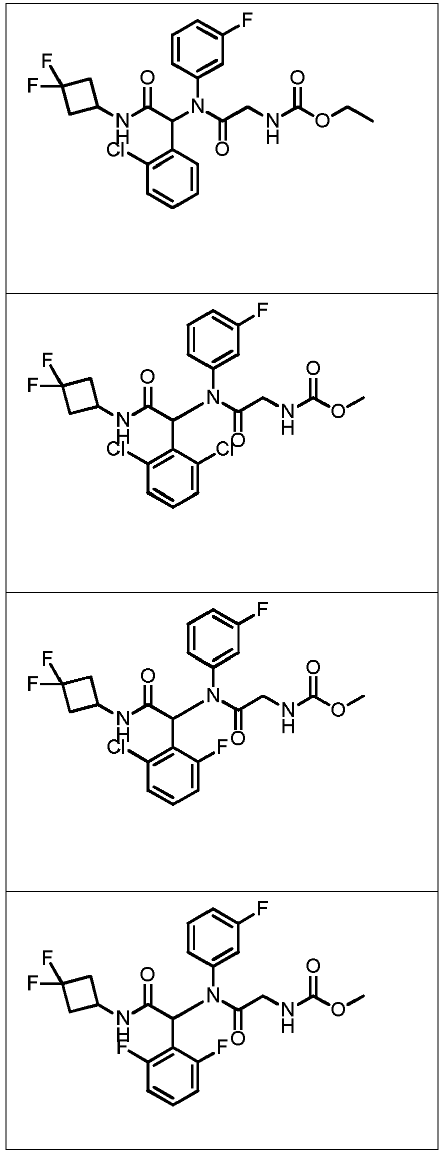

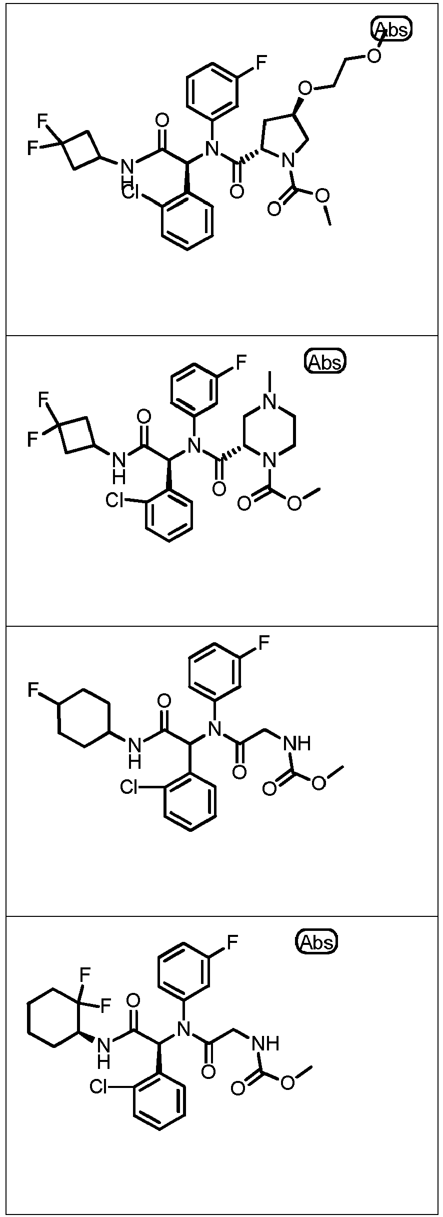

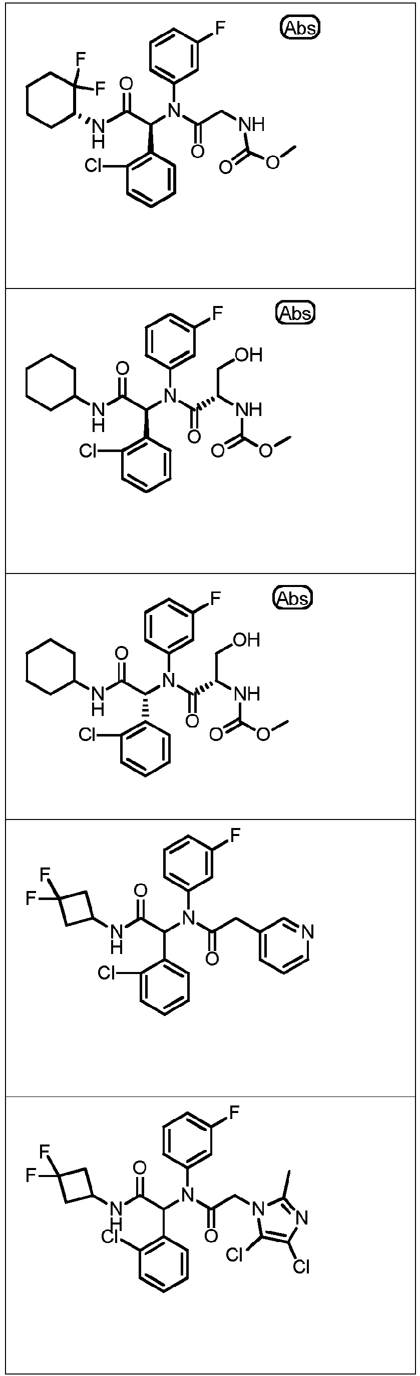

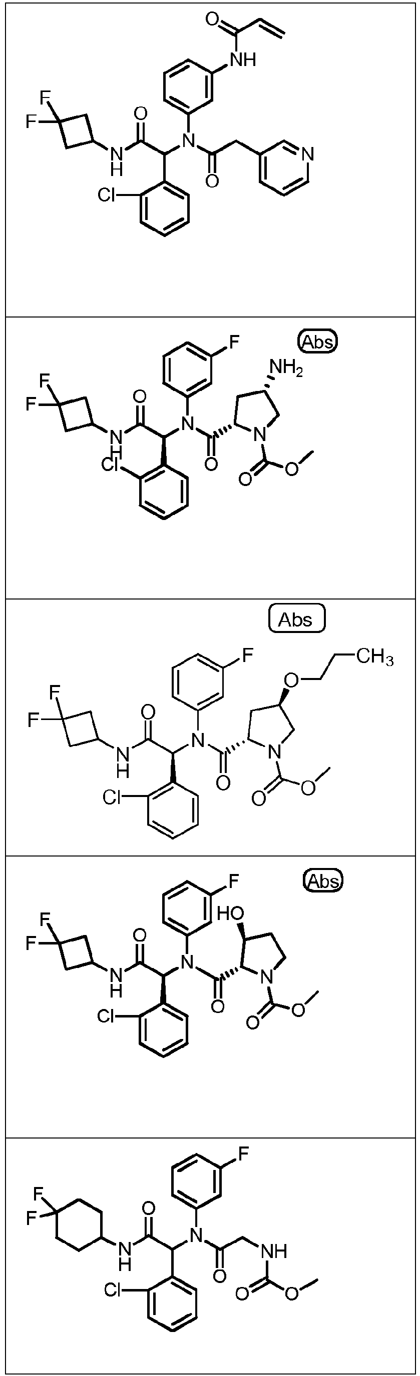

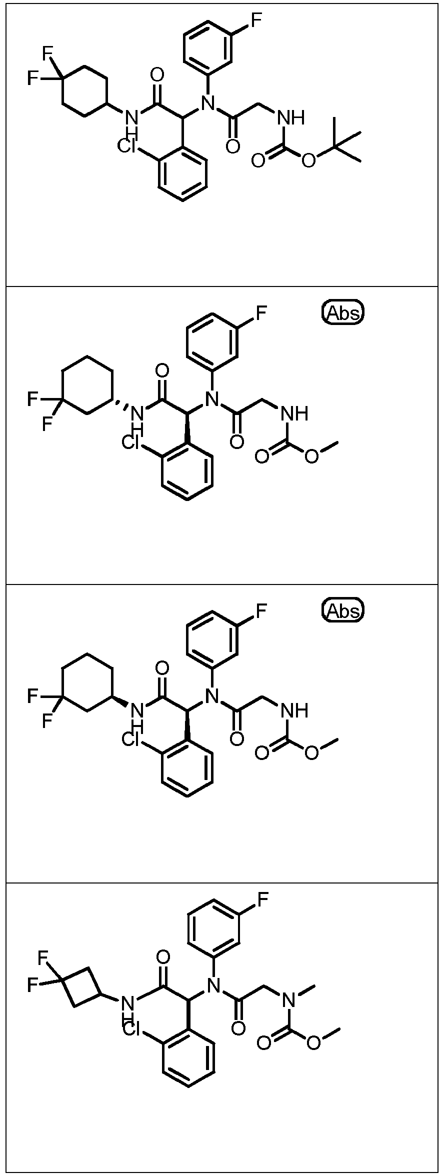

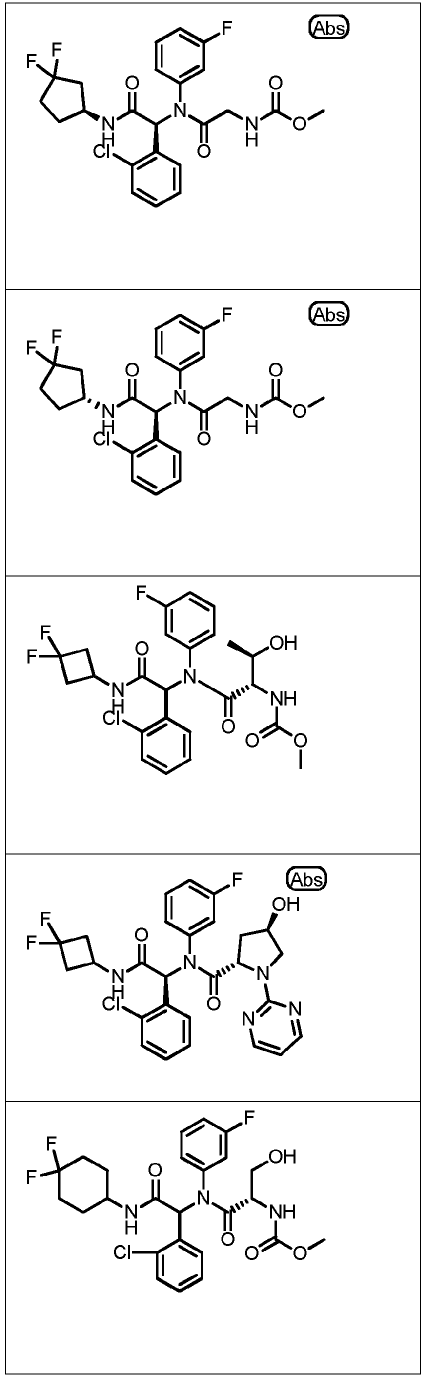

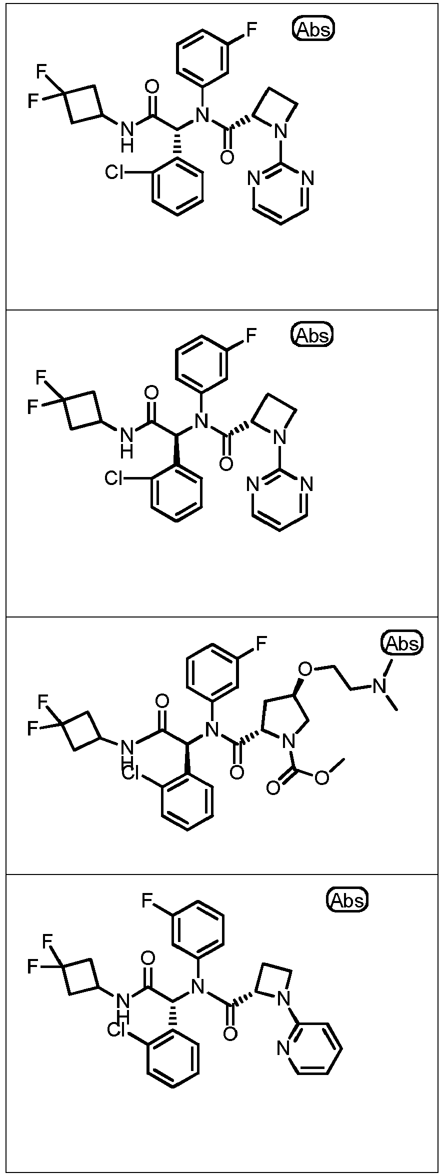

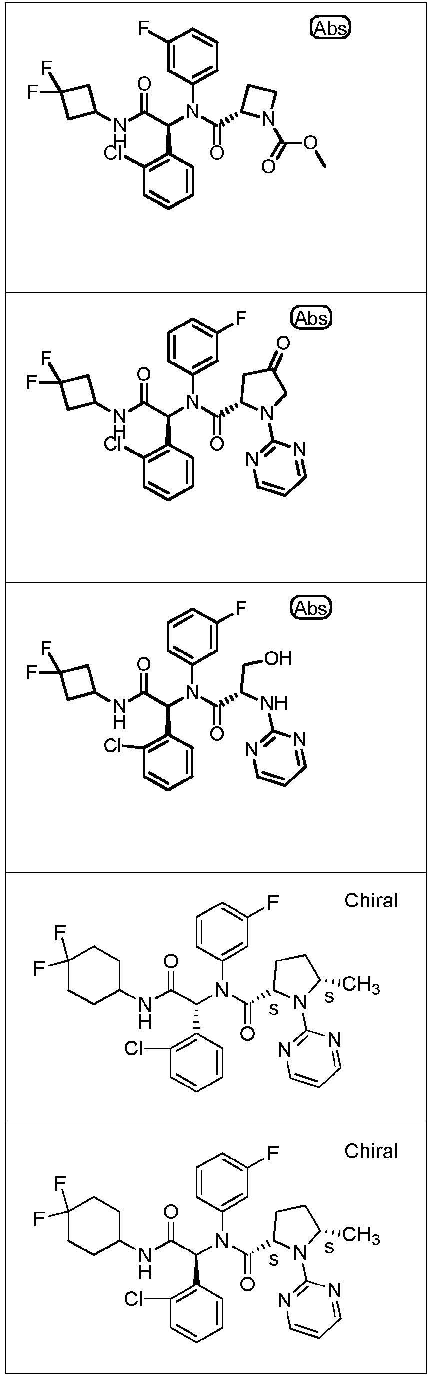

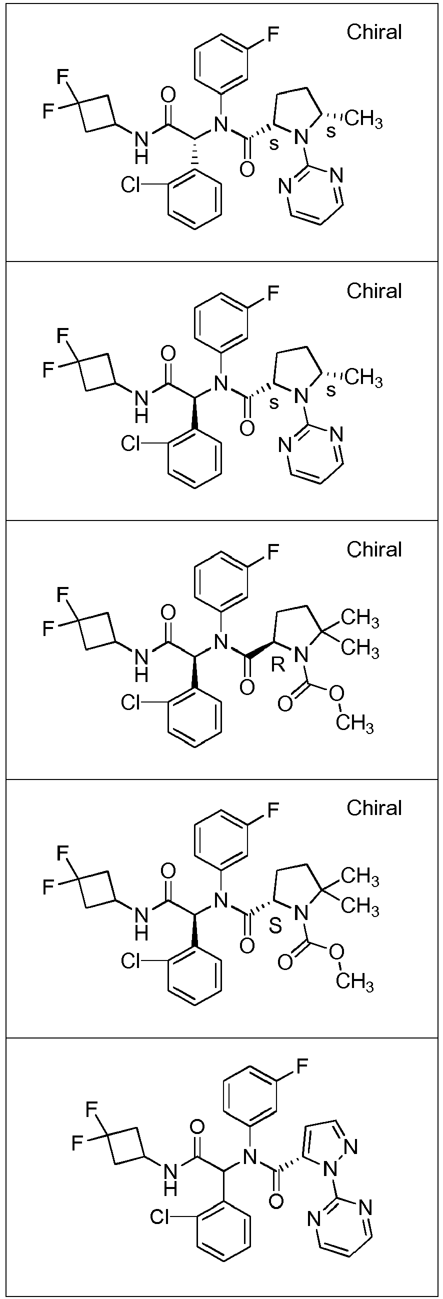

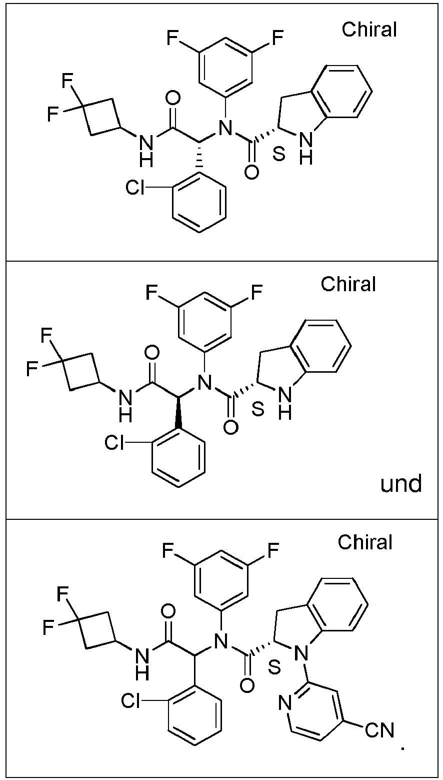

9. Verbindung der Formel I oder ein pharmazeutisch verträgliches Salz oder Hydrat davon, wobei: 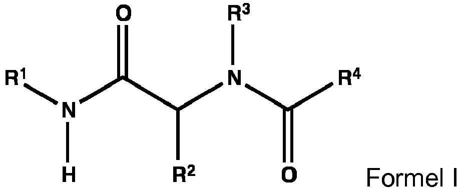 R¹: 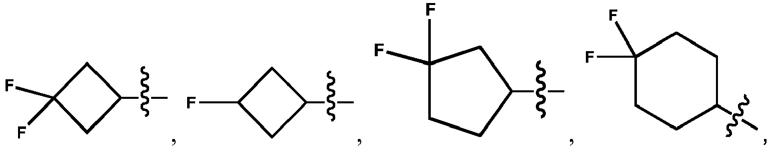 oder 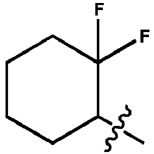 ist;
jedes R² und R³ unabhängig ausgewählt ist aus optional substituiertem Aryl oder optional substituiertem Heteroaryl;
R⁴ gesättigtes Heterocyclyl, -CH(R⁵)N(R⁵)-Aryl, -CH(R⁵)N(R⁵)-Heterocyclyl, -CH(R⁵)N(R⁵)-Carbocyclyl, -CH₂-Heterocyclyl, 1H-Indol-2-yl, Indolin-2-yl, 1,2,3,4-Tetrahydroquinolin-2-yl, Imidazo[1,2-*a*]pyridin-5-yl, 5,6,7,8-Tetrahydroimidazo[1,2-*a*]pyridin-5-yl, -(CR⁵R⁶)₁₋₄N(R⁵)C(O)O(C₁-C₆-Alkyl) oder -(CR⁵R⁶)₁₋₄N(R⁵)SO₂(C₁-C₆-Alkyl) ist, wobei jedes gesättigte Heterocyclyl, Heteroaryl, Aryl, Heterocyclyl oder Carbocyclyl unabhängig optional substituiert ist; oder
R⁴ -CH₂-Heteroaryl ist, wobei Heteroaryl Imidazolyl, substituiert mit einer oder zwei R⁷-Gruppen, ist, ausgewählt aus der Gruppe bestehend aus-CN, -CH₂OH, Cl und F; oder Heteroaryl Triazolyl, Tetrazolyl oder Pyridinyl, optional substituiert mit einer oder zwei R⁷-Gruppen, ist; oder
R⁴ -CH(R⁵)N(R⁵)-Heteroaryl ist, wobei Heteroaryl Pyrimidinyl, optional substituiert mit einer oder zwei R⁷-Gruppen, ist, ausgewählt aus der Gruppe bestehend aus Cl und F;
jedes R⁵ unabhängig ausgewählt ist aus Wasserstoff und Methyl; und jedes R⁶ unabhängig ausgewählt ist aus Wasserstoff, Methyl, CH₂OH, CH(CH₃)OH, CH₂NH₂ oder CH(CH₃)NH₂.

10. Verbindung ausgewählt aus der Gruppe bestehend aus:
| | |
|---|---|
| **33** | 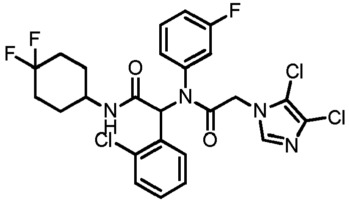 |
| **35** | 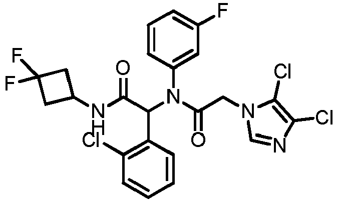 |
| **36** | 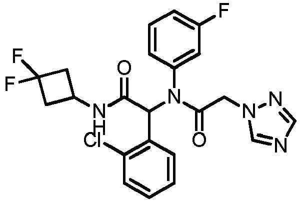 |
| **37** | 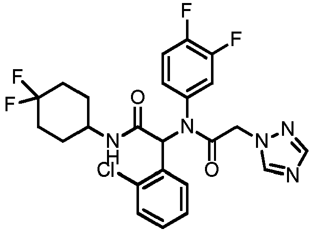 |
| **38** | 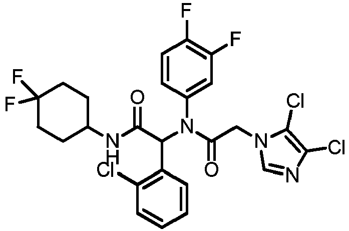 |
| **45** | 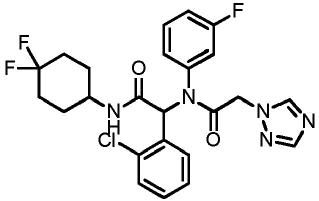 |
| **46** | 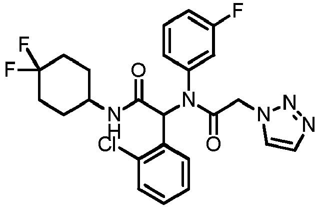 |
| **49** | 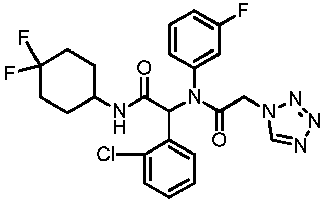 |
| **59** | 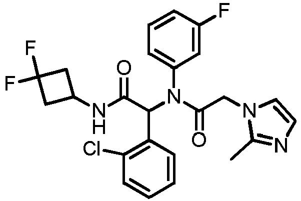 |
| **64** | 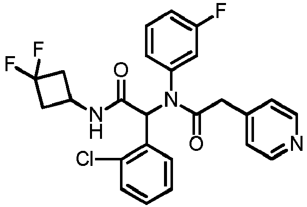 |
| **72** | 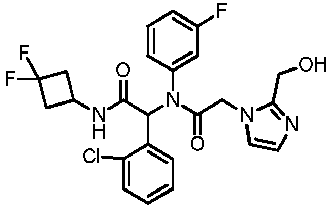 |
| **86** | 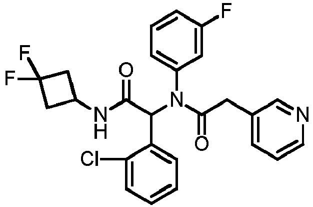 |
| **87** | 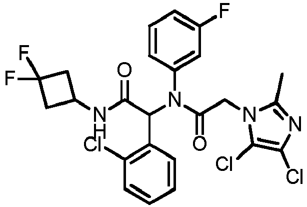 |
| **88** | 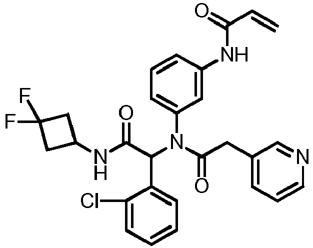 |
| **108** | 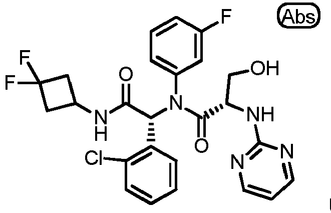 |
| | und |
| **109** | 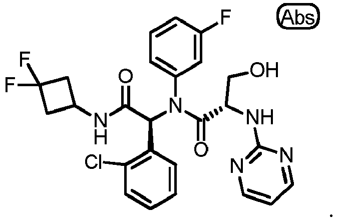 |

11. Pharmazeutische Zusammensetzung umfassend eine Verbindung nach irgendeinem der Ansprüche 1 bis 10; und einem pharmazeutisch verträglichen Träger.

12. Pharmazeutische Zusammensetzung nach Anspruch 11, ferner umfassend einen zweiten therapeutischen Wirkstoff, der nützlich bei der Behandlung von Krebs ist.

13. Verbindung oder pharmazeutische Zusammensetzung nach irgendeinem der Ansprüche 1 bis 11 zur Verwendung in einem Verfahren zur Behandlung eines Krebses, charakterisiert durch das Vorhandensein einer IDH1-Mutation, wobei die IDH1-Mutation in einer neuen Fähigkeit des Enzyms resultiert, die NADPH-abhängige Reduktion von α-Ketoglutarat zu *R*(-)-2-Hydroxyglutarat in einem Patienten zu katalysieren.

14. Verbindung oder pharmazeutische Zusammensetzung zur Verwendung nach Anspruch 13, wobei die IDH1-Mutation eine IDH1 R132H- oder R132C-Mutation ist.

15. Verbindung oder pharmazeutische Zusammensetzung zur Verwendung nach Anspruch 13, wobei der Krebs ausgewählt ist aus Gliom (Glioblastom), akuter myelogener Leukämie, Melanom, nicht-kleinzelligem Lungenkrebs (NSCLC), Cholangiokarzinomen, Chondrosarkom, myelodysplastischen Syndromen (MDS), myeloproliferativem Neoplasma (MPN), Darmkrebs in einem Patienten.

16. Verbindung oder pharmazeutische Zusammensetzung zur Verwendung nach irgendeinem der Ansprüche 13 bis 15, ferner umfassend Verabreichung an den Patienten, der dessen bedarf, eines zweiten therapeutischen Wirkstoffs, der nützlich bei der Behandlung von Krebs ist.

## Revendications

1. Composé de formule I ou un sel ou hydrate pharmaceutiquement acceptable de celui-ci, 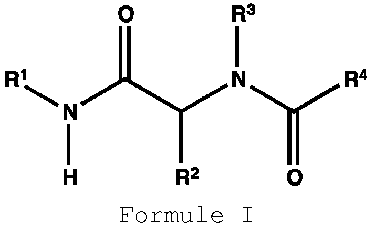 formule dans laquelle R¹ est : 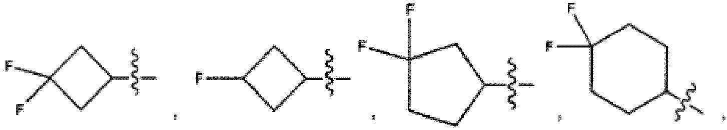 ou 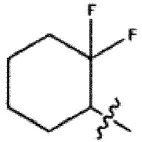
chaque R² et R³ est indépendamment choisi parmi les radicaux aryle éventuellement substitués et hétéroaryle éventuellement substitués ;
R⁴ est un radical entièrement saturé hétérocyclyle, -CH(R⁵)N(R⁵)-aryle, -CH(R⁵)N(R⁵)-hétérocyclyle, -CH(R⁵)N(R⁵)-carbocyclyle, -CH₂-hétérocyclyle, 1H-indol-2-yle, indolin-2-yle, 1,2,3,4-tétrahydroquinolin-2-yle, imidazo[1,2-a]pyridin-5-yle, 5,6,7,8-tétrahydroimidazo[1,2-a]pyridin-5-yle, -(CR⁵R⁶)₁₋₄N(R⁵)C(O)O-alkyle en C₁ à C₆, ou -(CR⁵R⁶)₁₋₄N(R⁵)SO₂-alkyle en C₁ à C₆, où chaque radical entièrement saturé hétérocyclyle, hétéroaryle, aryle, hétérocyclyle ou carbocyclyle est indépendamment éventuellement substitué ;
chaque R⁵ est indépendamment choisi parmi l'hydrogène et le radical méthyle ; et
chaque R⁶ est indépendamment choisi parmi l'hydrogène et les radicaux méthyle, CH₂OH, CH(CH₃)OH, CH₂NH₂ et CH(CH₃)NH₂ ;
où :
"hétérocyclyle" se réfère à une structure cyclique monocyclique, bicyclique ou tricyclique qui n'est pas entièrement aromatique et contient un à quatre hétéroatomes indépendamment choisis parmi N, O et S dans un ou plusieurs des cycles ; et
"carbocyclyle" se réfère à un système cyclique hydrocarboné monocyclique, bicyclique ou tricyclique qui n'est pas entièrement aromatique, dont n'importe quel atome de cycle susceptible d'une substitution peut être substitué par un ou plusieurs substituants.

2. Composé selon la revendication 1, dans lequel :
chaque R² et R³ est indépendamment choisi parmi les radicaux aryle et hétéroaryle, où ledit aryle ou hétéroaryle est indépendamment éventuellement substitué par un à trois groupes R⁷ ou acrylamido ;
R⁴ est un radical saturé hétérocyclyle, -CH(R⁵)N(R⁵)-aryle, -CH(R⁵)N(R⁵)-hétérocyclyle, -CH(R⁵)N(R⁵)-carbocyclyle, -CH₂-hétérocyclyle, 1H-indol-2-yle, indolin-2-yle, 1,2,3,4-tétrahydroquinolin-2-yle, imidazo[1,2-a]pyridin-5-yle, 5,6,7,8-tétrahydroimidazo[1,2-*a*]pyridin-5-yle, -(CR⁵R⁶)₁₋₄N(R⁵)-C(O)O-alkyle en C₁ à C₆, ou -(CR⁵R⁶)₁₋₄N(R⁵)SO₂-alkyle en C₁ à C₆, où chaque radical saturé hétérocyclyle, hétéroaryle, aryle, hétérocyclyle ou carbocyclyle est indépendamment éventuellement substitué par un à trois groupes R⁷ ;
chaque R⁵ est indépendamment choisi parmi l'hydrogène et le radical méthyle ;
chaque R⁶ est indépendamment choisi parmi l'hydrogène et les radicaux méthyle, CH₂OH, CH(CH₃)OH, CH₂NH₂ et CH(CH₃)NH₂ ;
chaque R⁷ est indépendamment un radical halogéno, -CF₃, -CN, -OR⁸, -N(R⁸)₂, -C(O)CH₃, -C(O)OCH₃, -SO₂-alkyle en C₁ à C₃, -C(O)N(R⁸)₂, -O(CH₂)₂-OR⁸, SO₂N(R⁸)₂, hétéroaryle, halogénoalkyle en C₁ à C₃, alkyle en C₁ à C₃ éventuellement substitué par -OR⁸ ou -N(R⁸)₂ ; et
chaque R⁸ est indépendamment H ou un radical alkyle en C₁ à C₃.

3. Composé selon la revendication 1 ou 2, dans lequel chaque R² et R³ est indépendamment un radical aryle éventuellement substitué par un à trois groupes R⁷.

4. Composé selon la revendication 3, dans lequel R² est un radical phényle éventuellement substitué par un ou deux groupes R⁷ et chaque R⁷ est indépendamment F, Cl ou le radical méthyle.

5. Composé selon la revendication 3, dans lequel R³ est un radical phényle éventuellement substitué par un ou deux groupes R⁷ et chaque R⁷ est indépendamment F ou un radical CN, -SO₂NH₂, -SO₂NH(CH₃), acrylamido ou oxadiazolyle.

6. Composé selon la revendication 3, dans lequel R⁴ est un radical saturé à 4 à 6 chaînons hétérocyclyle, -CH₂-hétérocyclyle, 1H-indol-2-yle, indolin-2-yle, 1,2,3,4-tétrahydroquinolin-2-yle, imidazo[1,2-*a*]pyridin-5-yle, 5,6,7,8-tétrahydroimidazo[1,2-*a*]pyridin-5-yle, -(CR⁵R⁶)₁₋₄N(R⁵)C(O)O-alkyle en C₁ à C₆, ou -(CR⁵R⁶)₁₋₄N(R⁵)SO₂-alkyle en C₁ à C₆, ou chaque dit radical saturé hétérocyclyle, hétéroaryle ou hétérocyclyle est indépendamment éventuellement substitué par un à trois groupes R⁷ ; ou
R⁴ est : 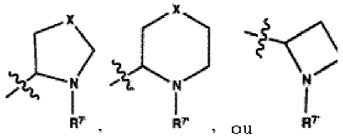
où X est CH(R^{7"}), O, NH ou NC(O)CH₃ ; R^{7'} est H, -C(O)CH₃, -C(O)OCH₃, -SO₂-alkyle en C₁ à C₃, -C(O)N-(R⁸)₂, pyrimidinyle, pyridyle ; et R^{7"} est H, -O(CH₂)₂-OCH₃, -O(CH₂)₂-OCH, OH, OCH₃, NH₂ ou F ; ou
R⁴ est un radical -(CR⁵R⁶)N(R⁵)C(O)O-alkyle en C₁ à C₄ où chaque R⁵ est indépendamment H ou le radical méthyle et R⁶ est le radical méthyle ou CH₂OH.

7. Composé selon la revendication 5, dans lequel R⁴ est le radical 1,2,3,4-tétrahydroquinolin-2-yle ou 5,6,7,8-tétrahydroimidazo[1,2-*a*]pyridin-5-yle.

8. Composé choisi parmi n'importe lesquels des composés suivants :

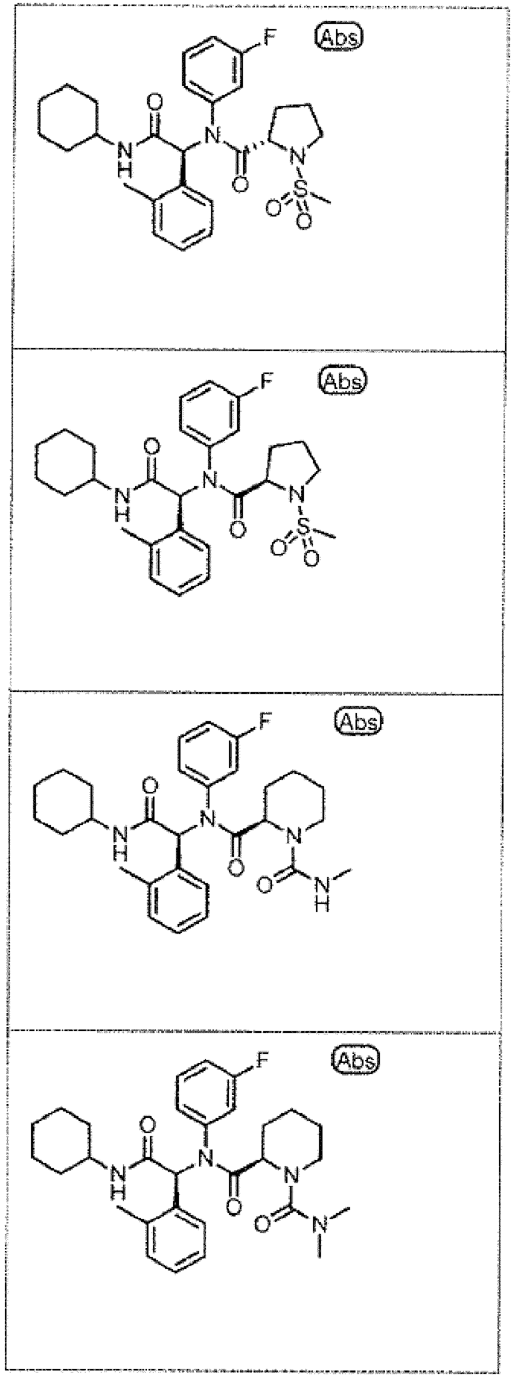

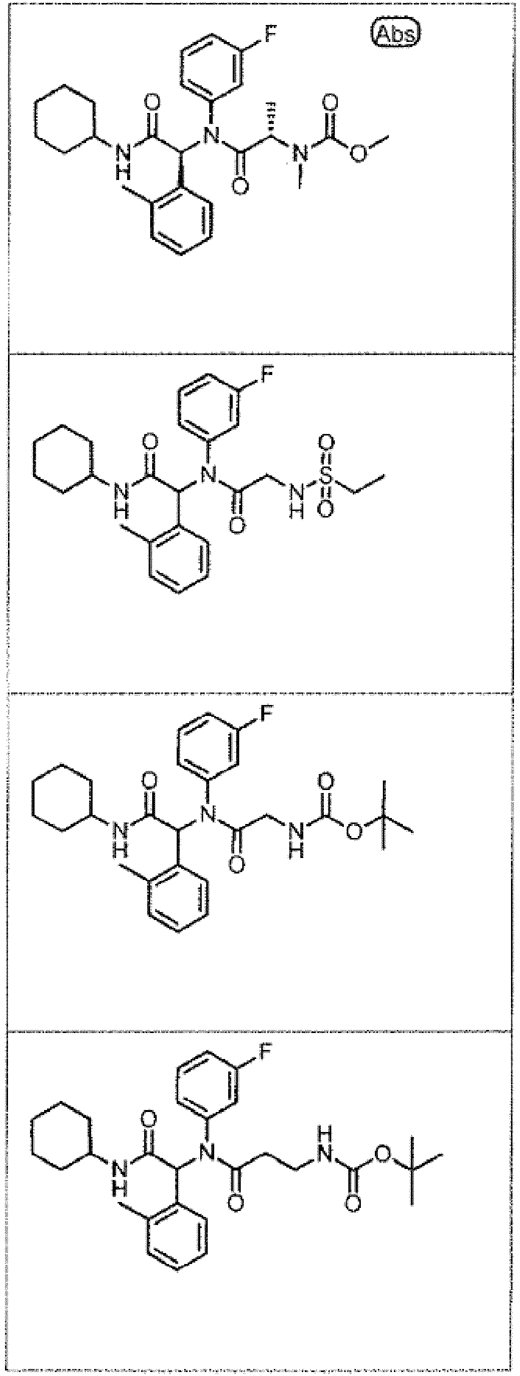

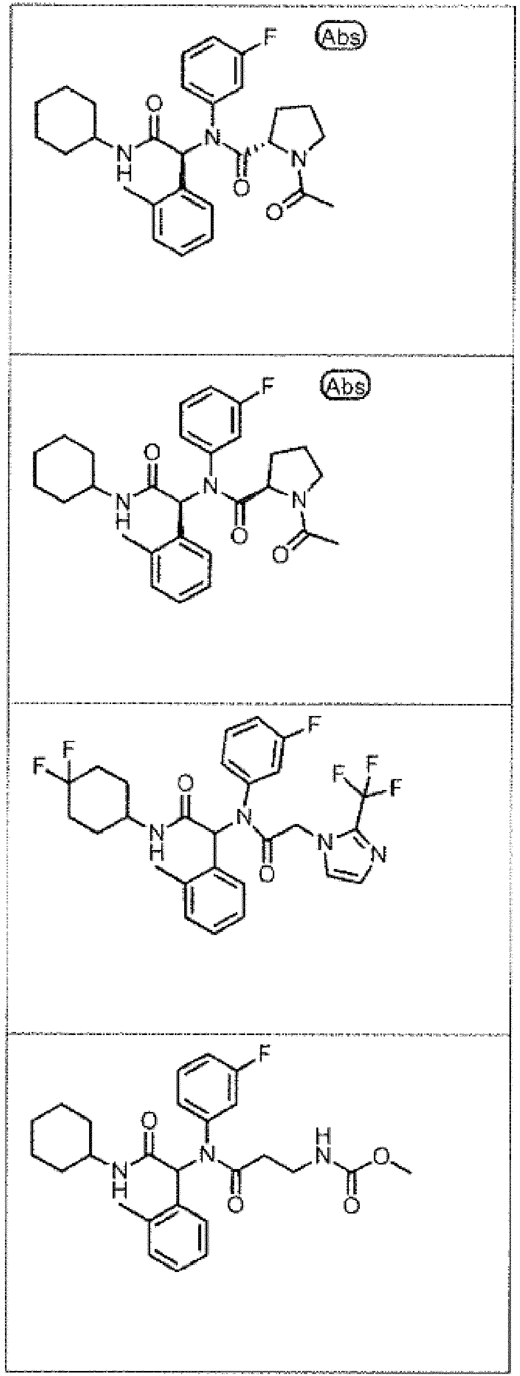

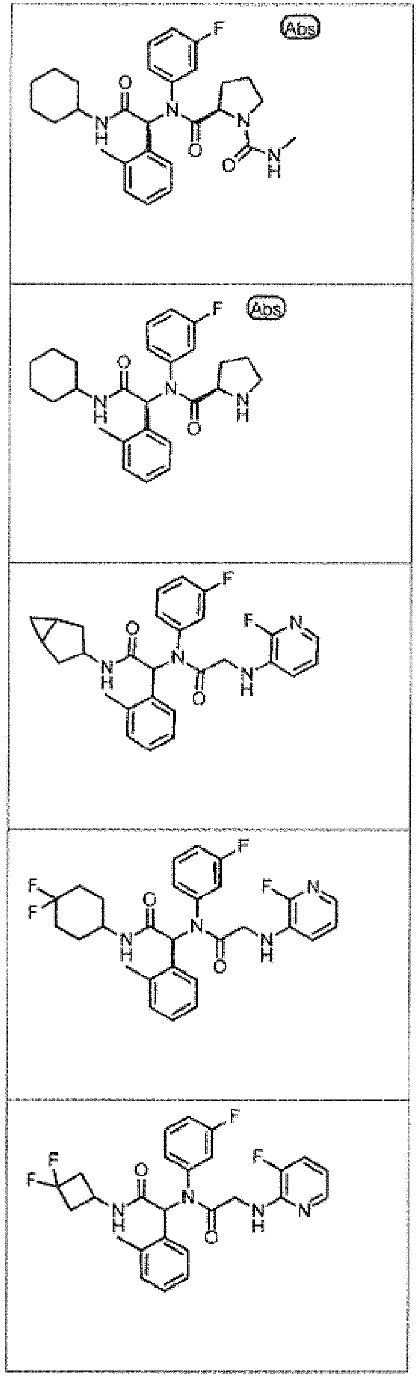

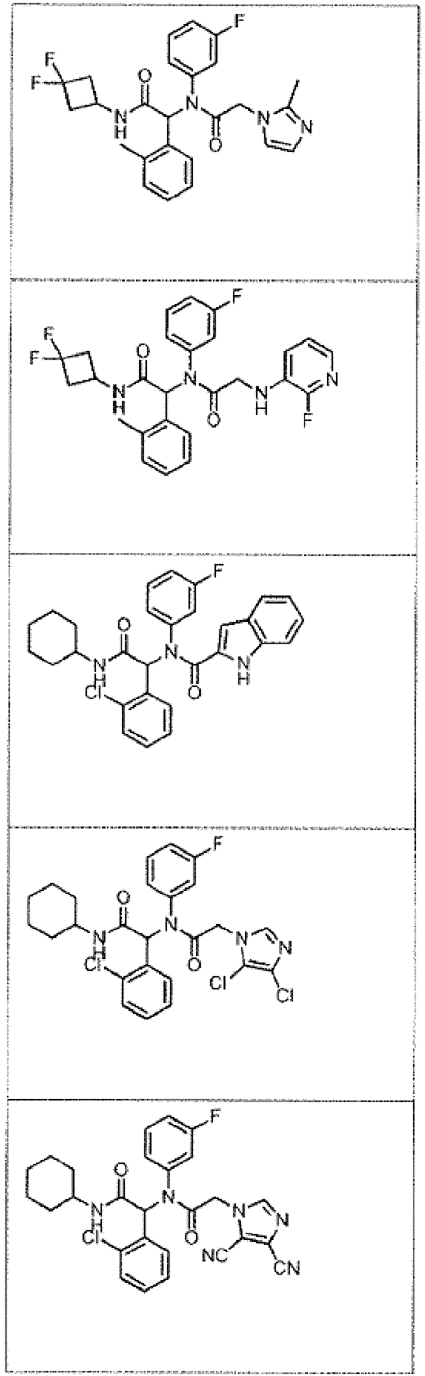

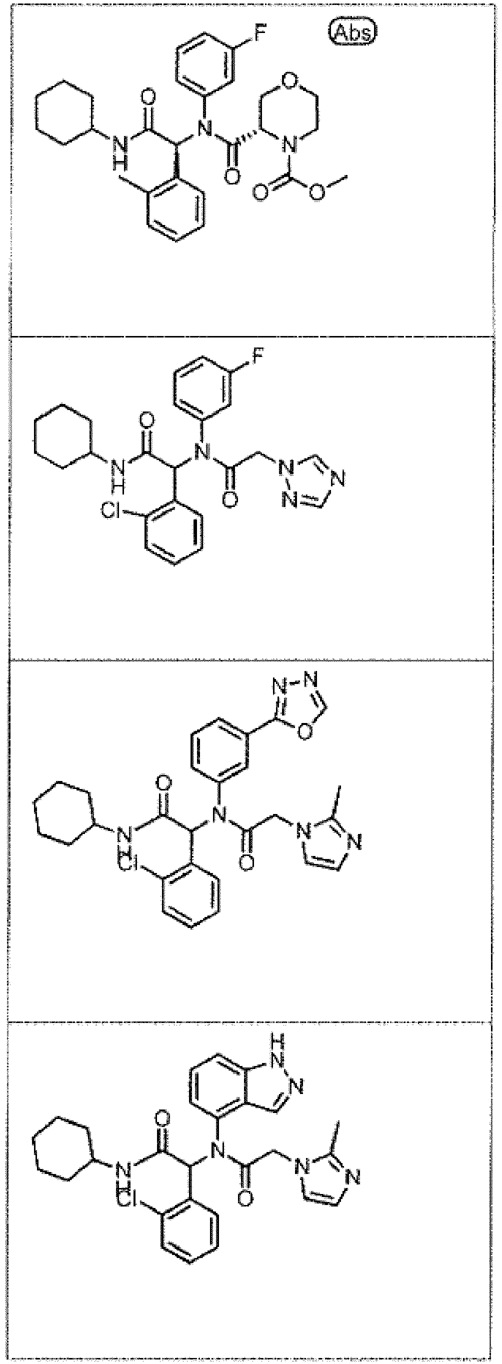

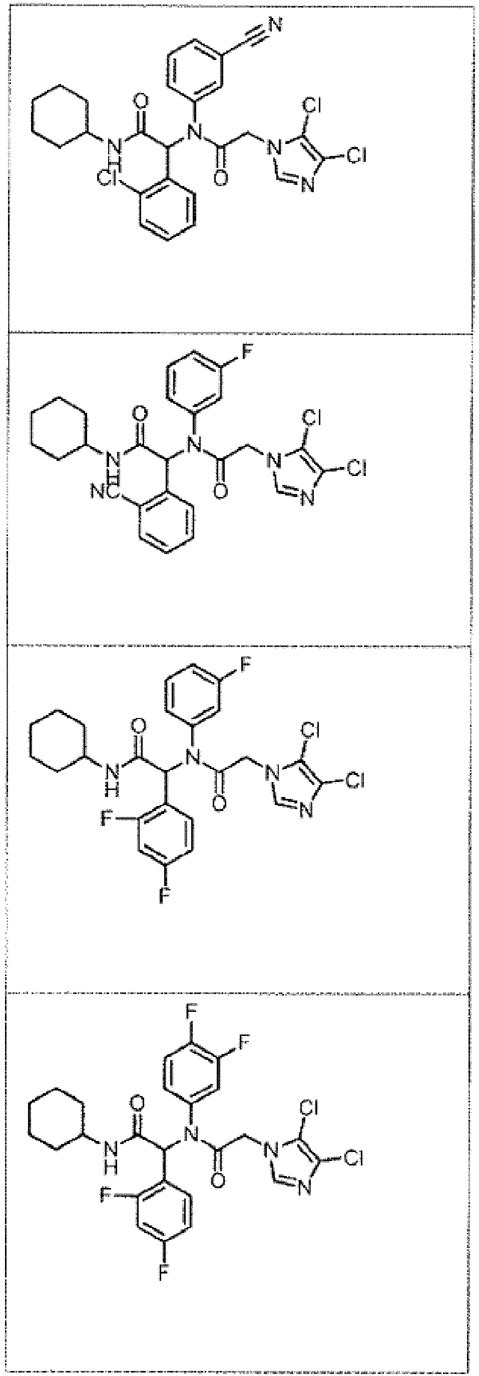

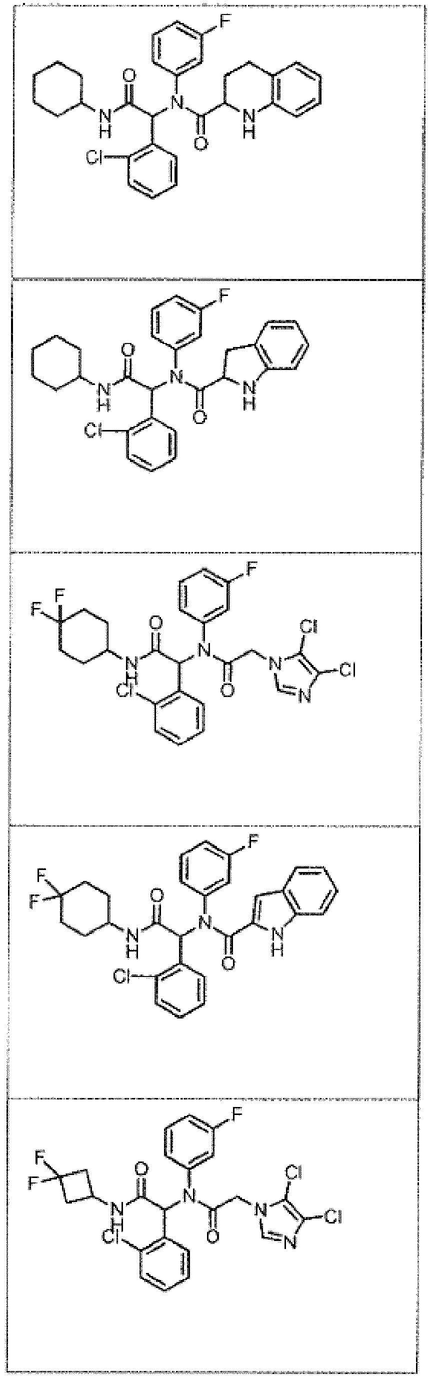

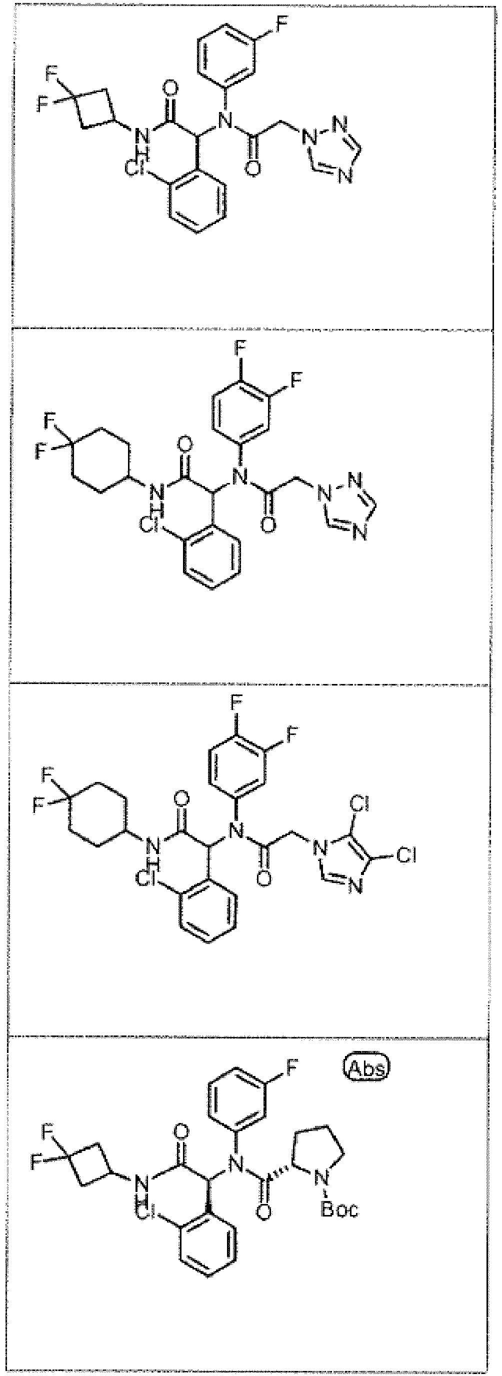

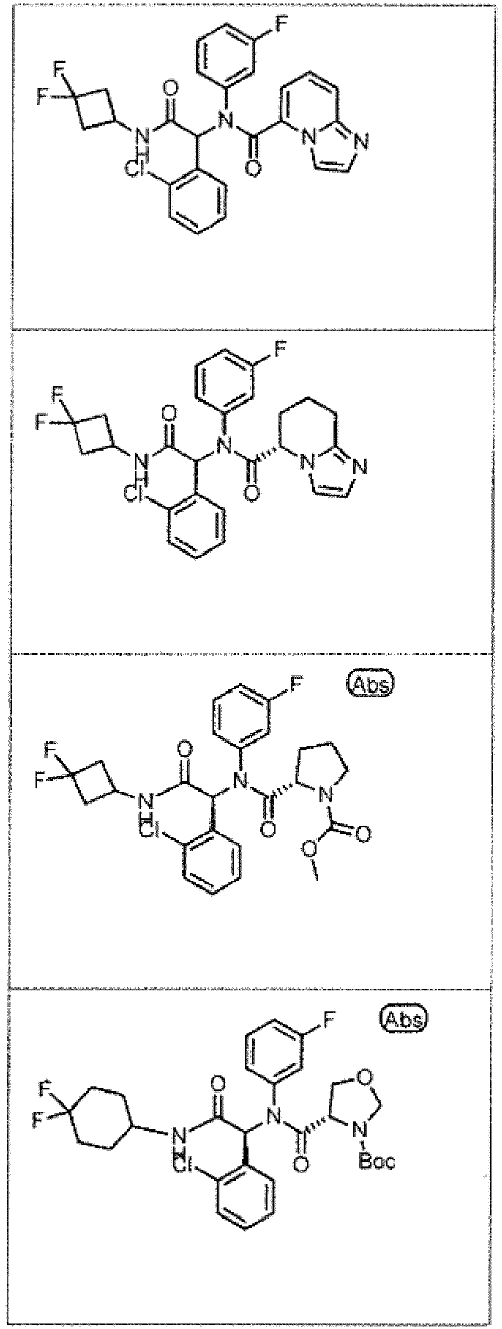

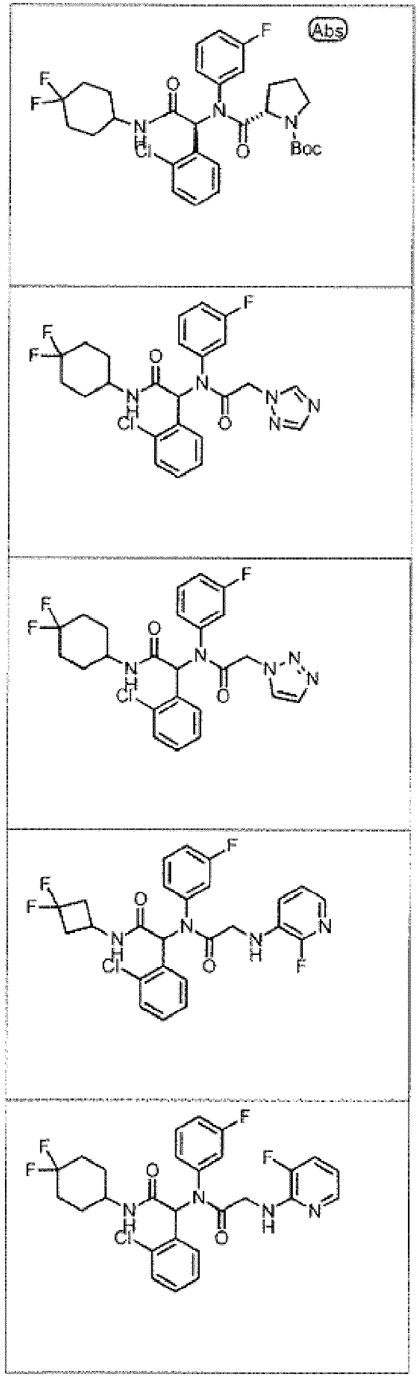

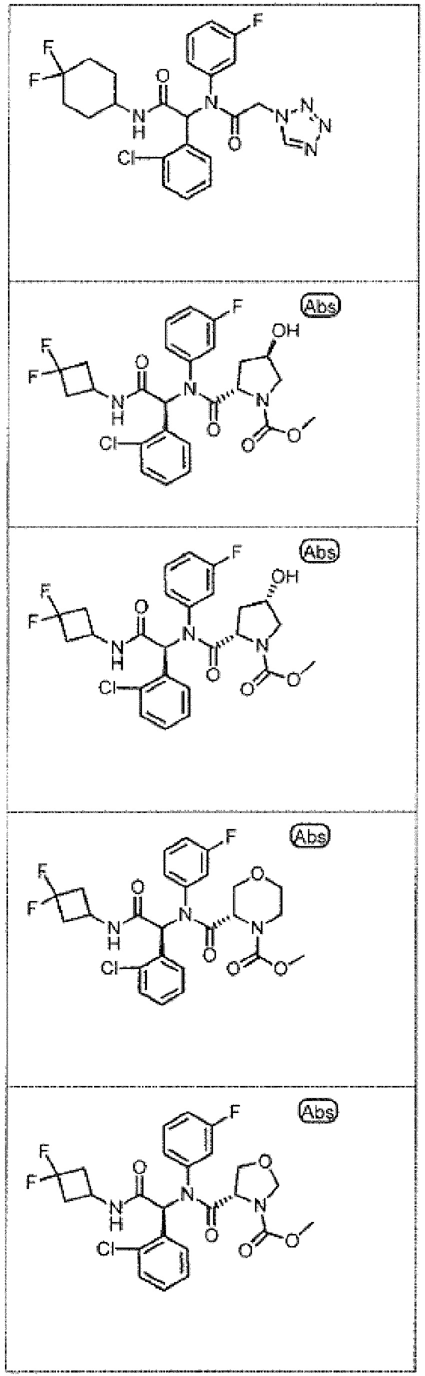

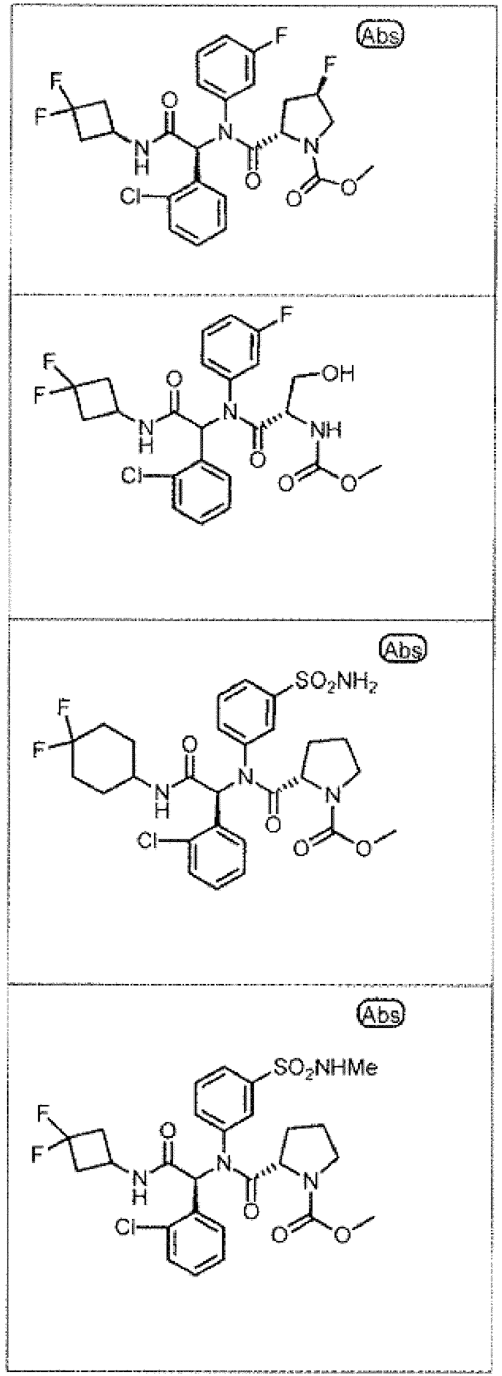

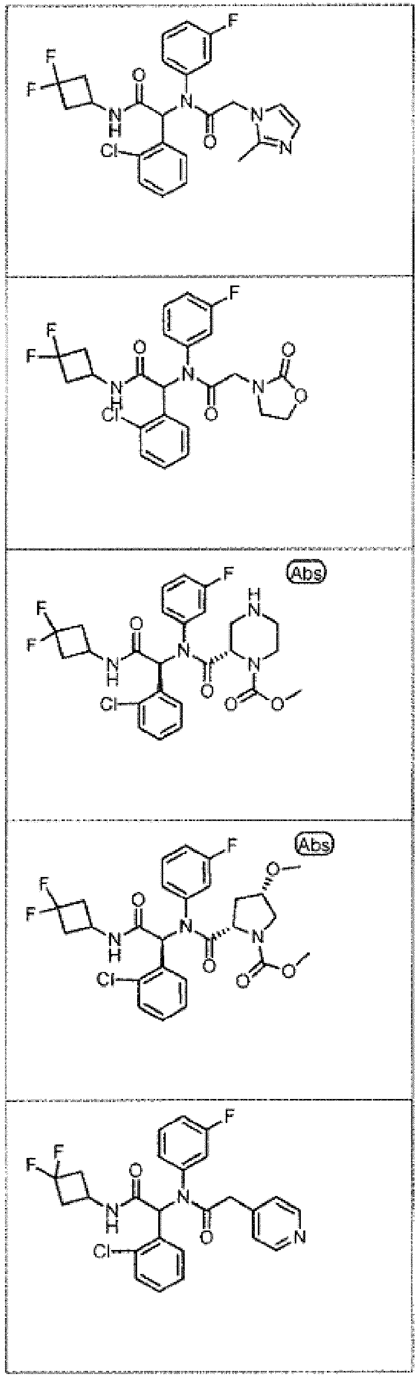

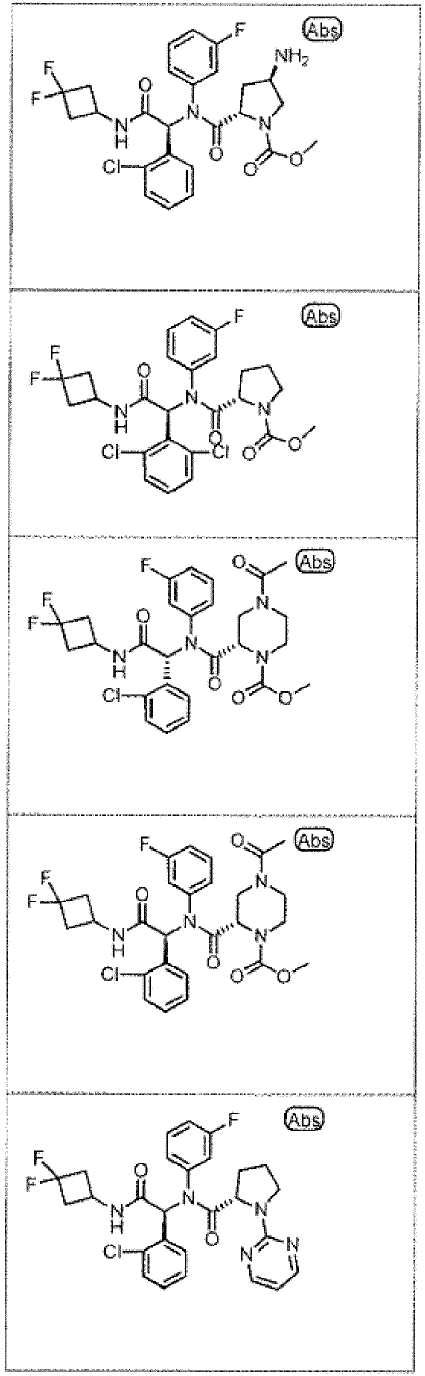

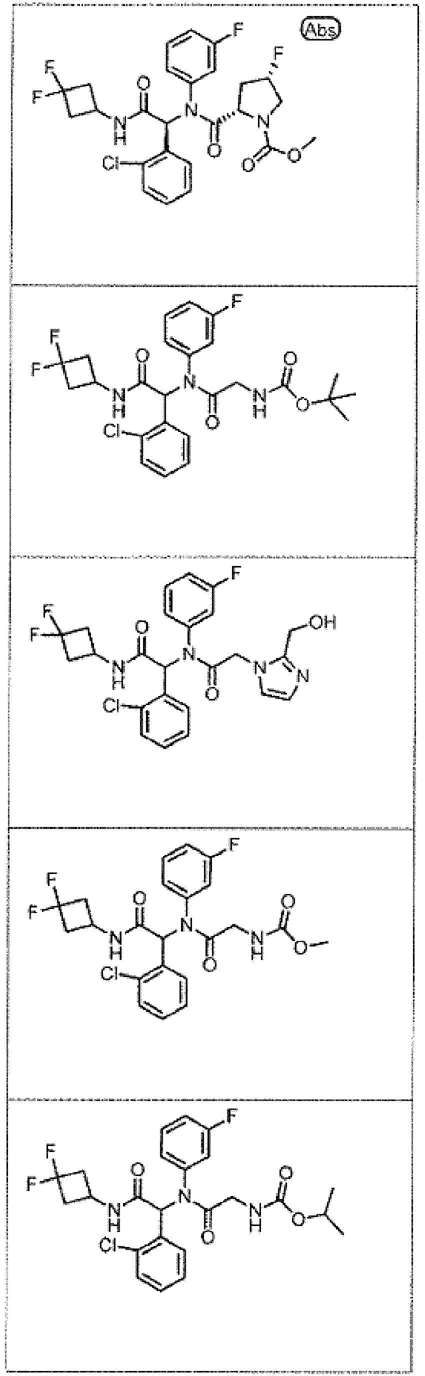

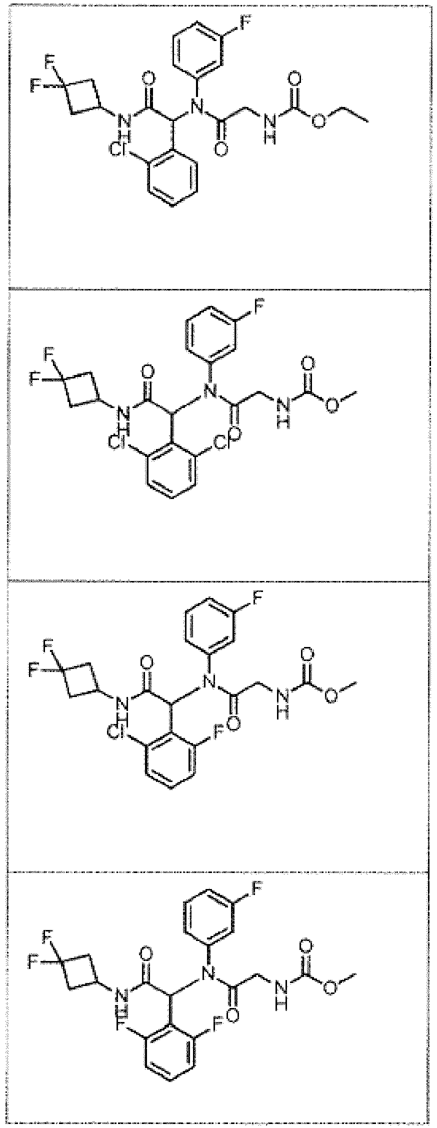

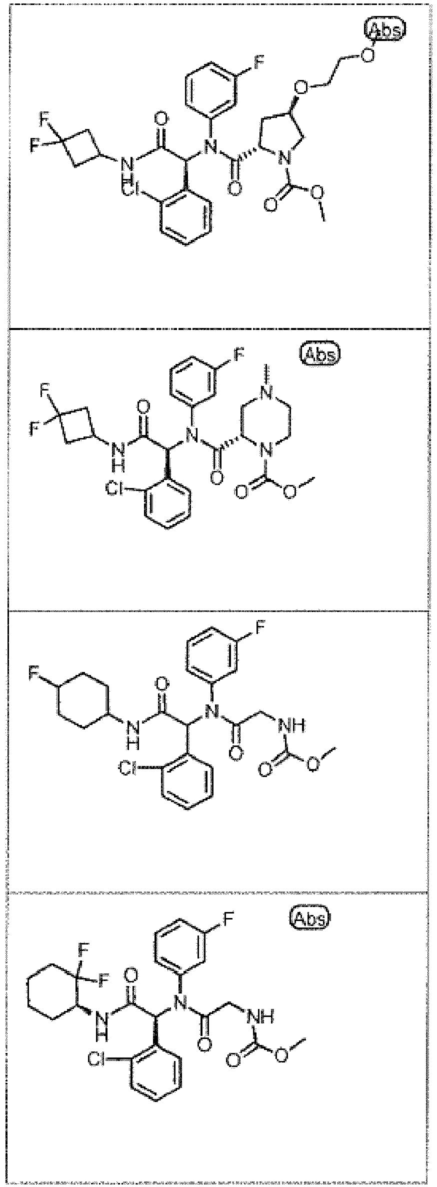

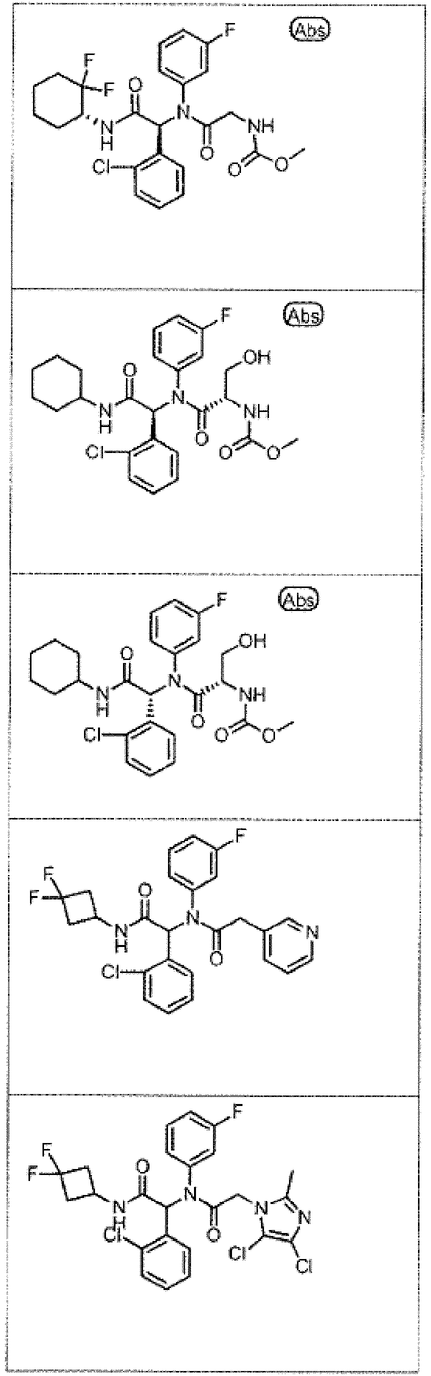

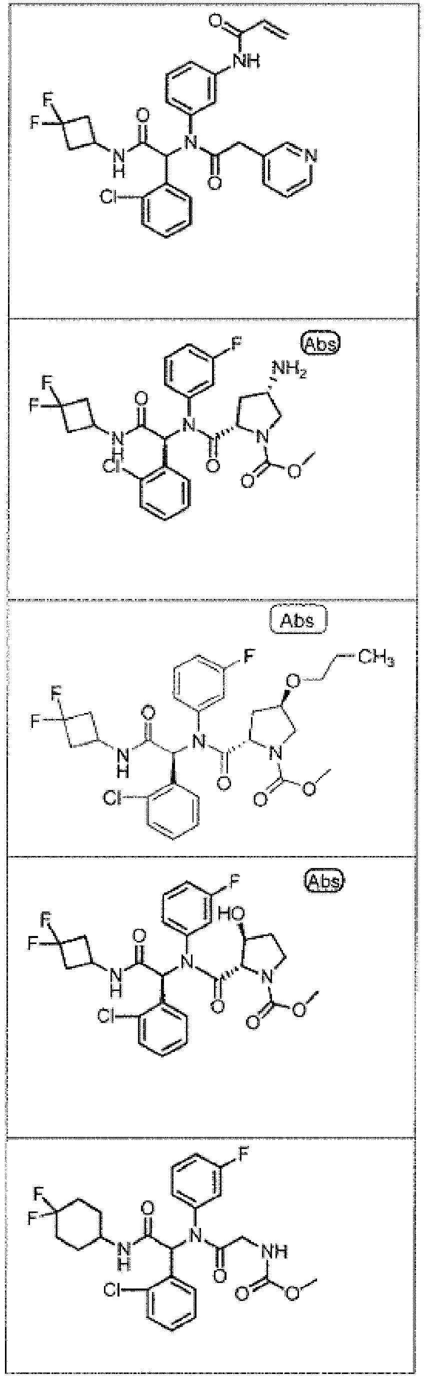

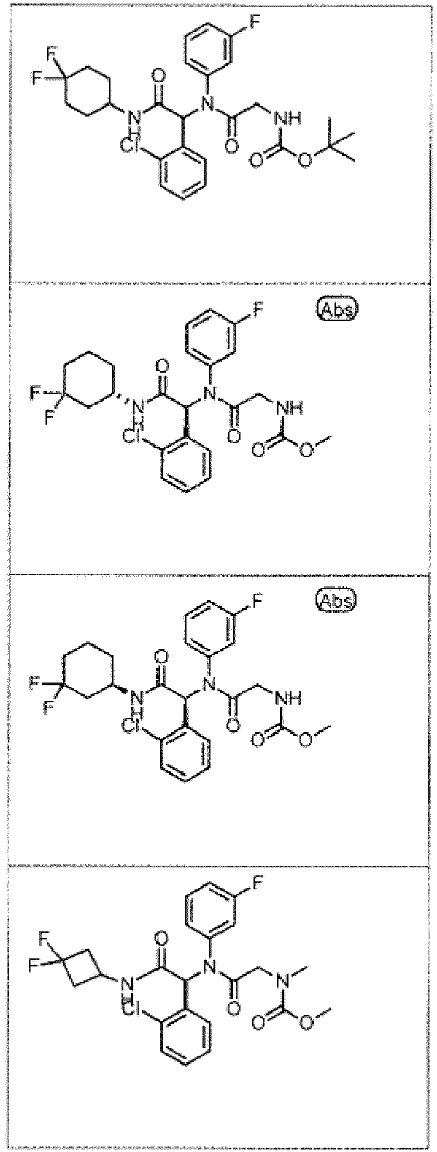

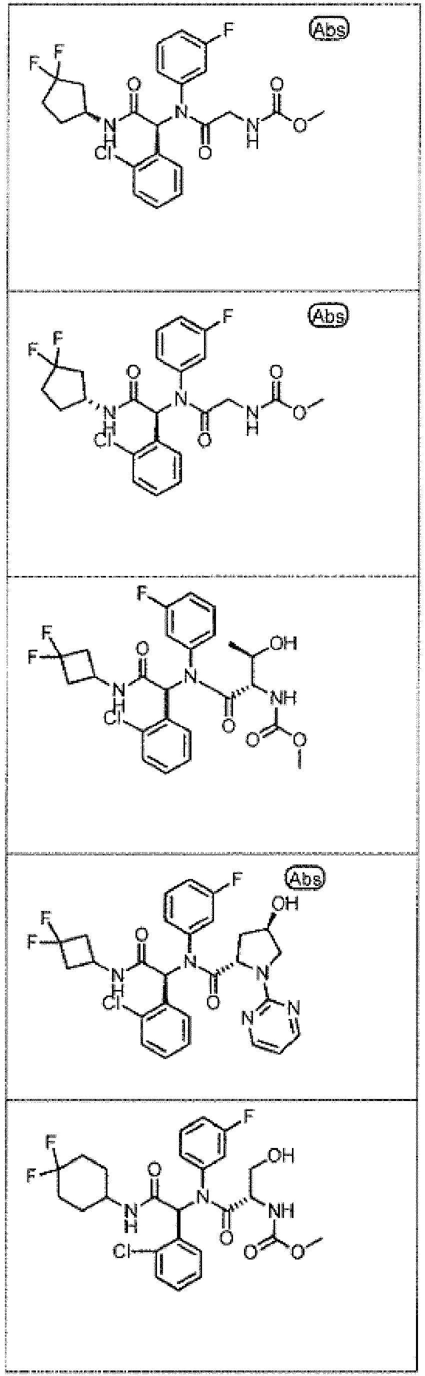

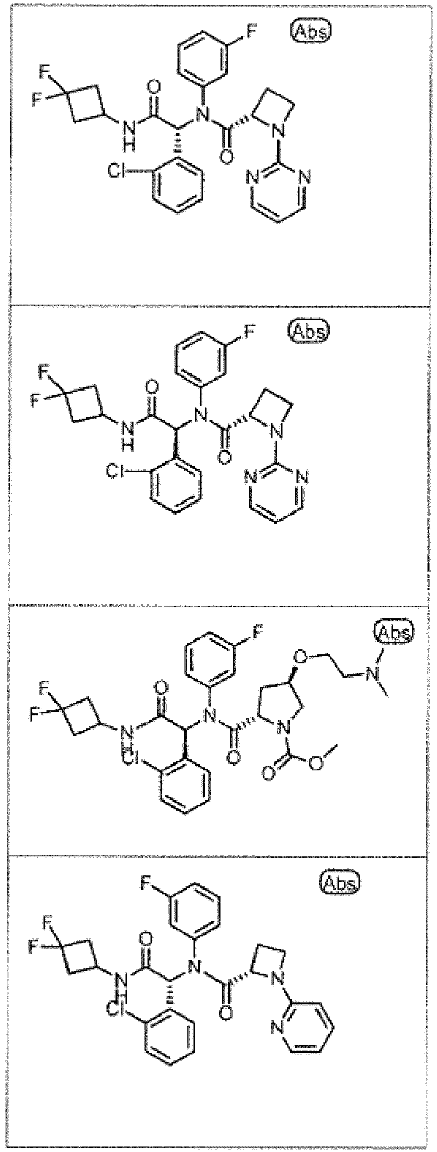

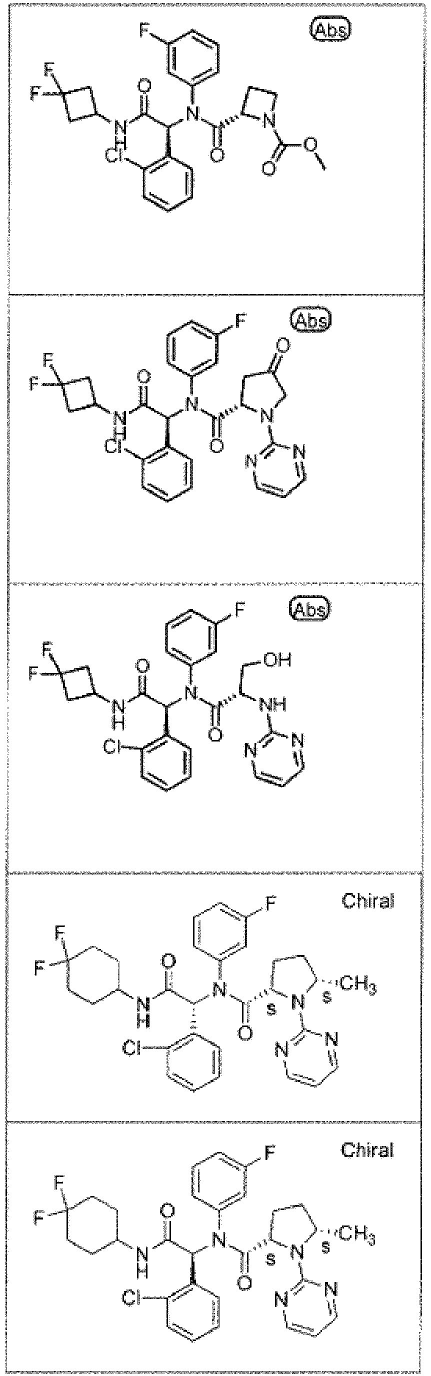

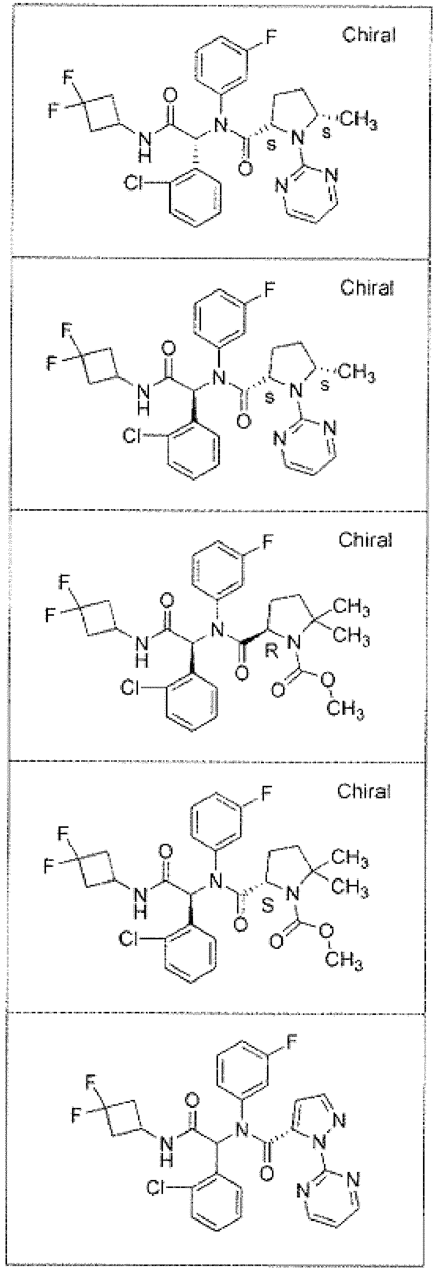

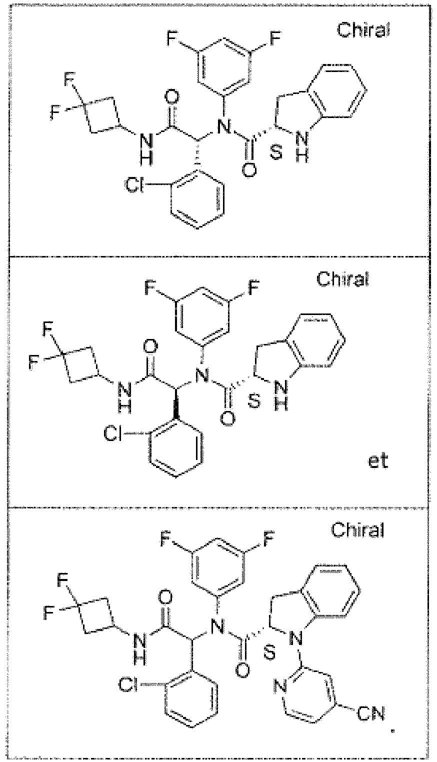

9. Composé de formule I ou un sel ou hydrate pharmaceutiquement acceptable de celui-ci, 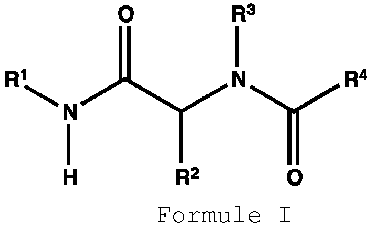 formule dans laquelle
R¹ est : 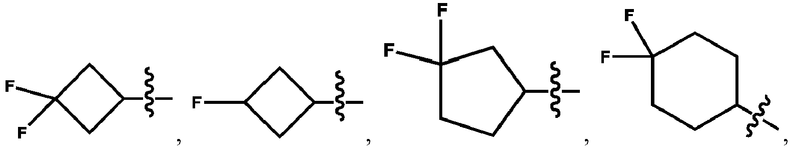 ou 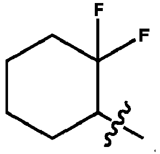
chaque R² et R³ est indépendamment choisi parmi les radicaux aryle éventuellement substitués et hétéroaryle éventuellement substitués ;
R⁴ est un radical saturé hétérocyclyle, -CH(R⁵)N(R⁵)-aryle, -CH(R⁵)N(R⁵)-hétérocyclyle, -CH(R⁵)N(R⁵)-carbocyclyle, -CH₂-hétérocyclyle, 1H-indol-2-yle, indolin-2-yle, 1,2,3,4-tétrahydroquinolin-2-yle, imidazo[1,2-a]pyridin-5-yle, 5,6,7,8-tétrahydroimidazo[1,2-*a*]pyridin-5-yle, -(CR⁵R⁶)₁₋₄N(R⁵)C(O)O-alkyle en C₁ à C₆, ou -(CR⁵R⁶)₁₋₄N(R⁵)SO₂-alkyle en C₁ à C₆, où chaque radical entièrement saturé hétérocyclyle, hétéroaryle, aryle, hétérocyclyle ou carbocyclyle est indépendamment éventuellement substitué ; ou
R⁴ est un radical -CH₂-hétéroaryle où l'hétéroaryle est un imidazolyle substitué par un ou deux groupes R⁷ choisis dans l'ensemble constitué par -CN, -CH₂OH, Cl, et F ; ou l'hétéroaryle est un triazolyle, tétrazolyle ou pyridinyle éventuellement substitué par un ou deux groupes R⁷ ; ou
R⁴ est un radical -CH(R⁵)N(R⁵)-hétéroaryle où l'hétéroaryle est un pyrimidinyle éventuellement substitué par un ou deux groupes R⁷ choisis dans l'ensemble constitué par Cl et F ;
chaque R⁵ est indépendamment choisi parmi l'hydrogène et le radical méthyle ; et
chaque R⁶ est indépendamment choisi parmi l'hydrogène et les radicaux méthyle, CH₂OH, CH(CH₃)OH, CH₂NH₂ et CH(CH₃)NH₂.

10. Composé choisi dans l'ensemble constitué par les suivants :
| | |
|---|---|
| **33** | 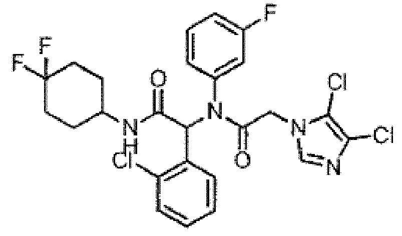 |
| **35** | 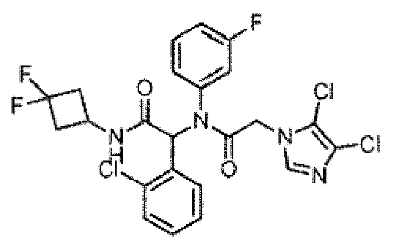 |
| **36** | 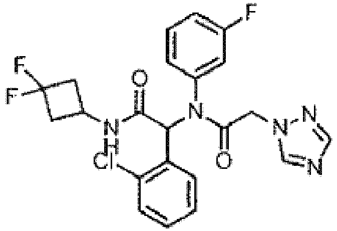 |
| **37** | 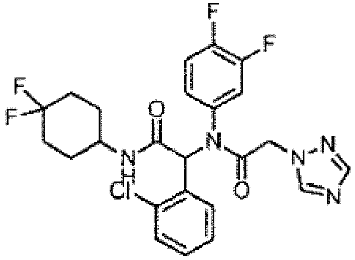 |
| **38** | 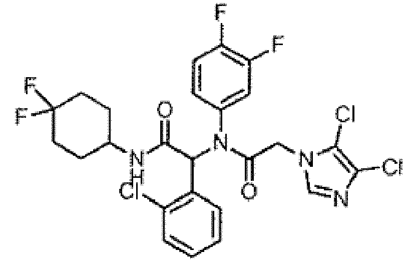 |
| **45** | 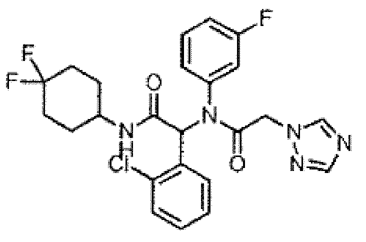 |
| **46** | 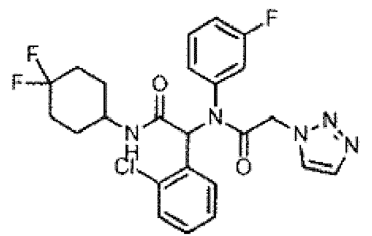 |
| **49** | 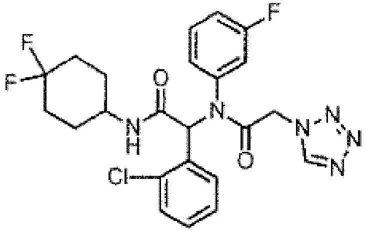 |
| **59** | 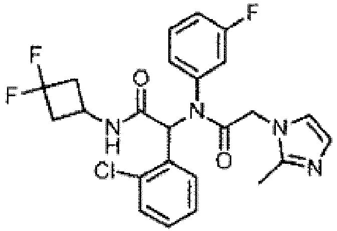 |
| **64** | 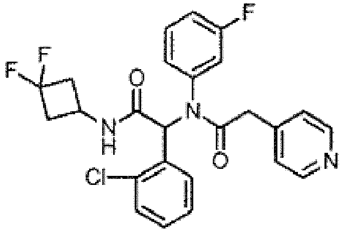 |
| **72** | 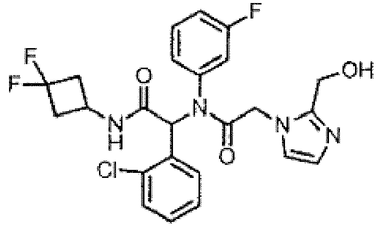 |
| **86** | 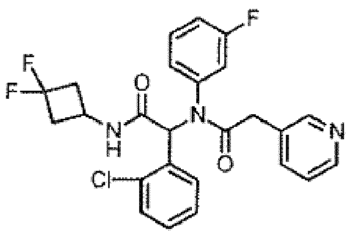 |
| **87** | 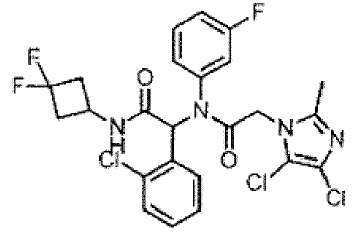 |
| **88** | 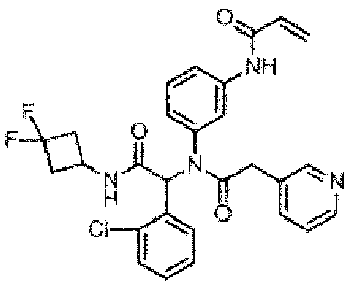 |
| **108** | 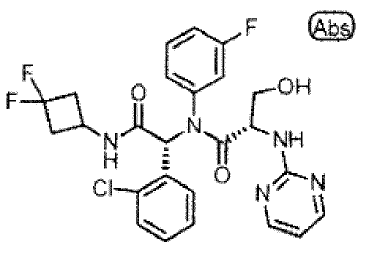 |
| | et |
| **109** | 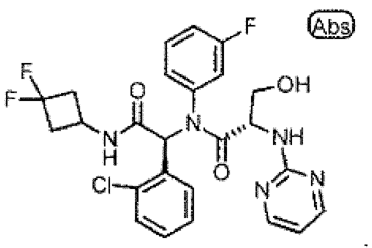 |

11. Composition pharmaceutique comprenant un composé selon l'une quelconque des revendications 1 à 10 ; et un véhicule pharmaceutiquement acceptable.

12. Composition pharmaceutique selon la revendication 11, comprenant en outre un deuxième agent thérapeutique utile dans le traitement d'un cancer.

13. Composé ou composition pharmaceutique selon l'une quelconque des revendications 1 à 11, pour une utilisation dans une méthode de traitement d'un cancer **caractérisé par** la présence d'une mutation d'IDH1, dans lequel la mutation d'IDH1 a pour résultat une nouvelle capacité de l'enzyme à catalyser la réduction dépendante du NADPH de α-cétoglutarate en *R*-(-)-2-hydroxyglutarate chez un patient.

14. Composé ou composition pharmaceutique pour une utilisation selon la revendication 13, dans lequel la mutation d'IDH1 est une mutation d'IDH1 R132H ou R132C.

15. Composé ou composition pharmaceutique pour une utilisation selon la revendication 13, dans lequel le cancer est choisi parmi un gliome (glioblastome), une leucémie myéloïde aiguë, un mélanome, un cancer du poumon non à petites cellules (NSCLC), un cholangiocarcinome, un chondrosarcome, un syndrome myélodysplasique (MDS), un néoplasme myéloprolifératif (MPN), un cancer du côlon chez un patient.

16. Composé ou composition pharmaceutique pour une utilisation selon l'une quelconque des revendications 13 à 15, comprenant en outre l'administration, au patient en ayant besoin, d'un deuxième agent thérapeutique utile dans le traitement d'un cancer.
